# EUROPEAN PATENT APPLICATION

(11) **EP 4 831 180 A1**
(43) Date of publication of application: **28.10.2026**
(21) Application number: 24906556.6
(22) Date of filing: 20.12.2024
(51) Int. Cl.: C07D 491/22, A61K 31/4375, A61P 35/00

(54) **NOVEL KINASE INHIBITOR**

(30) Priority: 22.12.2023 CN 202311785897; 01.02.2024 CN 202410145188; 05.03.2024 CN 202410251564; 21.03.2024 CN 202410329787; 22.04.2024 CN 202410488461; 15.05.2024 CN 202410605099; 04.07.2024 CN 202410895024; 23.08.2024 CN 202411174428; 29.11.2024 CN 202411745850
(71) Applicant: Shouyao Holdings (Beijing) Co., Ltd., Beijing 100176 (CN)
(72) Inventor: LIU, Tao, Beijing 100195 (CN); WANG, Jinghan, Beijing 100195 (CN); XUE, Mengting, Beijing 100195 (CN); GAO, Yuhao, Beijing 100195 (CN); ZHANG, Saifei, Beijing 100195 (CN); DUAN, Xiaowei, Beijing 100195 (CN); CHEN, Sanxu, Beijing 100195 (CN); LI, Haizhou, Beijing 100195 (CN); XIE, Bowen, Beijing 100195 (CN); MIN, Wangyang, Beijing 100195 (CN); BAI, Yun, Beijing 100195 (CN); ZHANG, Kai, Beijing 100195 (CN); CHEN, Xi, Beijing 100195 (CN); LIU, Xijie, Beijing 100195 (CN); HUANG, Yuanyu, Beijing 100195 (CN)
(74) Representative: f & e patent
(86) International application number: PCT/CN2024/141075
(87) International publication number: WO 2025/131085

(57) **Abstract**

The present invention provides a novel kinase inhibitor, and a preparation method and use thereof. Specifically, the present invention provides a compound of formula (II), or a pharmaceutically acceptable salt, solvate, polymorph or isomer thereof, pharmaceutical compositions comprising these compounds, and the use of such compounds and compositions in treating related diseases.

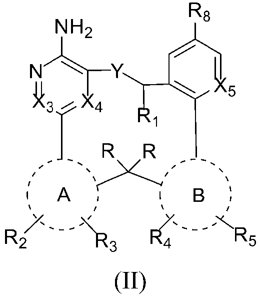

## Description

### Cross-Reference

This application claims priority to Chinese Patent Application No. 202311785897.9, filed on December 22, 2023, titled "Novel Kinase Inhibitor"; Chinese Patent Application No. 202410145188.2, filed on February 1, 2024, titled "Novel Kinase Inhibitor"; Chinese Patent Application No. 202410251564.6, filed on March 5, 2024, titled "Novel Kinase Inhibitor"; Chinese Patent Application No. 202410329787.X, filed on March 21, 2024, titled "Novel Kinase Inhibitor"; Chinese Patent Application No. 202410488461.1, filed on April 22, 2024, titled "Novel Kinase Inhibitor"; Chinese Patent Application No. 202410605099.1, filed on May 15, 2024, titled "Novel Kinase Inhibitor"; Chinese Patent Application No. 202410895024.1, filed on July 4, 2024, titled "Novel Kinase Inhibitor"; Chinese Patent Application No. 202411174428.8, filed on August 23, 2024, titled "Novel Kinase Inhibitor"; and Chinese Patent Application No. 202411745850.4, filed on November 29, 2024, titled "Novel Kinase Inhibitor". The entire disclosures of all of the foregoing applications are incorporated herein by reference.

### Technical Field

The present invention relates to novel pyrrolopyrimidine derivatives having protein kinase inhibitory activity, methods for preparing the same, pharmaceutical compositions containing the same, and the use of such compounds and pharmaceutical compositions thereof in treating protein kinase-related diseases.

### Background

Chromosomal rearrangements of the anaplastic lymphoma kinase (ALK) gene generate oncogenic fusion proteins, which occur in 3% to 5% of patients with non-small cell lung cancer (NSCLC). These fusion proteins exhibit abnormal dimerization or oligomerization, leading to constitutive ALK activation.

The fusion of ALK with the echinoderm microtubule-associated protein-like 4 (EML4) gene, forming EML4-ALK, has been found to be highly oncogenic in preclinical NSCLC models. Targeting the kinase domain of EML4-ALK with small-molecule ALK inhibitors provides clinical benefits to patients with ALK-positive NSCLC.

To date, three generations of ALK inhibitors have been approved, including first-generation (crizotinib), second-generation (alectinib, brigatinib, ceritinib), and third-generation (lorlatinib) therapies. However, resistance has emerged to all of these therapies. The development of resistance is due in part to the appearance of ALK mutations that interfere with drug binding. Crizotinib was approved in 2011 for ALK-positive NSCLC patients, with an objective response rate of 74% in the first-line setting. However, crizotinib treatment can lead to resistance mutations in the ALK active site, such as G1269A and C1156Y, as well as the gatekeeper residue mutation L1196M, which contribute to the development of drug resistance. Second-generation ALK inhibitors (alectinib, brigatinib, ceritinib) have been approved for ALK-positive NSCLC patients, but they are also susceptible to resistance due to ALK mutations, such as the solvent-front mutation (e.g., G1202R), found in 33% to 37% of relapsed patients, and the I1171 mutation in the hydrophobic regulatory spine (24% to 26%). The third-generation ALK inhibitor lorlatinib is approved for ALK-positive NSCLC patients who have previously received crizotinib and at least one other ALK inhibitor, or for patients who have received first-line treatment with alectinib or ceritinib. However, in plasma samples from patients with disease progression after lorlatinib treatment, ALK mutations were detected in 76% of cases: L1196M (38%), G1202R (28%), D1203N (24%), F1174C/L (14%), and I1171X (14%). These mutations are a mixture of single and compound mutations, with compound mutations found in 35% to 48% of treated patients. Among the compound mutations, the G1202R/L1196M mutation accounts for the highest proportion, while currently approved ALK inhibitors show poor inhibitory activity against the G1202R/L1196M mutant ALK kinase. The re-emergence of single mutations such as L1196M and G1202R may be due to the moderate potency of lorlatinib against these mutations (L1196M IC₅₀ 18-30 nmol/L, G1202R IC₅₀ 37-63 nmol/L). Therefore, there is a need for a CNS-penetrant, potent wild-type (WT) ALK inhibitor that is not susceptible to resistance from mutations generated by treatment with first-generation, second-generation, and third-generation ALK inhibitors.

The present patent invention provides a series of novel ALK inhibitors, which exhibit strong inhibitory activity against the G1202R/L1196M compound mutant ALK kinase.

### Summary of the Invention

In one aspect, the present invention provides a compound of Formula (II), or a pharmaceutically acceptable salt, solvate, polymorph, or isomer thereof 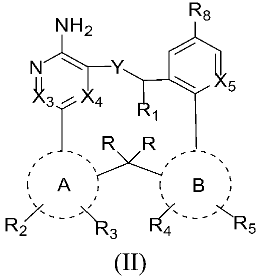 wherein,
Each R is independently H, halogen, -CN, or C₁₋₄ alkyl, or two R are taken together to form a 3-4 membered carbocycle,
Y is -O- or -NR₁₀-,
R₁₀ is H or C₁₋₆ alkyl,
R₁ is H, C₁₋₆ alkyl, halo-C₁₋₆ alkyl, C₂₋₆ alkenyl, or 3-6 membered cycloalkyl, the alkyl, alkenyl and cycloalkyl are optionally substituted with halogen, -OH or -NH₂, or
R₁ and R₁₀ are optionally joined together to form a 4-8 membered heterocycle, the heterocycle is optionally substituted with halogen, -CN, -OH or -NH₂,
X₅ is N or CR₉,
R₈ and R₉ are each independently H, F, Cl, or ethynyl,
X₄ is N and CH,
X₃ is N and CH,
Ring A and Ring B are 5-6 membered aromatic rings,
R₂ and R₃ are each independently H, halogen, -CN, C₁₋₄ alkyl, -O-C₁₋₄ alkyl, halo-C₁₋₄ alkyl, 3-4 membered cycloalkylmethyl, 3-6 membered cycloalkyl, 3-6 membered heterocyclyl, 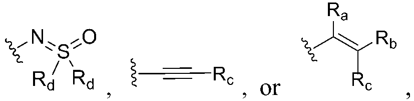 the alkyl, methyl, cycloalkyl and heterocyclyl are optionally substituted with halogen, -CN, -OH, -NH₂, C₂₋₆ alkenyl, or C₂₋₆ alkynyl,
R₄ and R₅ are each independently H, halogen, -CN, C₁₋₄ alkyl, -O-C₁₋₄ alkyl, halo-C₁₋₄ alkyl, 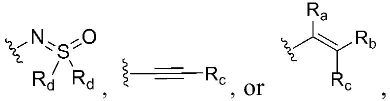 the alkyl is optionally substituted with halogen, -CN, - OH, -NH₂, C₂₋₆ alkenyl, or C₂₋₆ alkynyl,
X₃, R₂, R₃, R₄ and R₅ satisfy the following condition:
   when X₃ is CH,
   at least one of R₂, R₃, R₄ and R₅ is 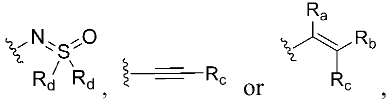
   Rₐ is H, halogen, C₁₋₆ alkyl, or halo-C₁₋₆ alkyl,
   R_{b} is H, halogen, C₁₋₆ alkyl, or halo-C₁₋₆ alkyl,
   R_{c} is H, halogen, C₁₋₆ alkyl, 3-8 membered cycloalkyl, 3-8 membered heterocyclyl, 6-10 membered aryl, 5-12 membered heteroaryl, -(CO)-NH₂, -(CO)-NH-C₁₋₆ alkyl, -(CO)-O-C₁₋₆ alkyl, -(CO)-C₁₋₆ alkyl, the alkyl, cycloalkyl, heterocyclyl, aryl and heteroaryl are optionally substituted with halogen, -OH, -NH₂, -CN, -O-C₁₋₆ alkyl, -NH-C₁₋₆ alkyl, or -N(C₁₋₆ alkyl)(C₁₋₆ alkyl), or
   R_{b} and R_{c} are optionally joined together to form a 4-8 membered carbocycle or 4-8 membered heterocycle, the carbocycle and heterocycle are optionally substituted with halogen, - OH, -NH₂, -CN, C₁₋₆ alkyl, C₂₋₆ alkenyl, C₂₋₆ alkynyl, -O-C₁₋₆ alkyl, -NH-C₁₋₆ alkyl, or -N(C₁₋₆ alkyl)(C₁₋₆ alkyl), the alkyl, alkenyl and alkynyl are optionally substituted with halogen, -OH, - NH₂, or -CN,

Each R_{d} is independently C₁₋₆ alkyl.

In some embodiments, Y is -O-.

In some embodiments, R₁ is H, C₁₋₆ alkyl, halo-C₁₋₆ alkyl, C₂₋₆ alkenyl, or 3-6 membered cycloalkyl, the alkyl, alkenyl and cycloalkyl are optionally substituted with halogen, -OH or - NH₂.

In some embodiments, R₈ and R₉ are each independently H or F.

In some embodiments, R_{d} is methyl.

In some embodiments, R₂ and R₃ are each independently H, halogen, -CN, C₁₋₄ alkyl, -O-C₁₋₄ alkyl, halo-C₁₋₄ alkyl, 3-4 membered cycloalkylmethyl, 3-6 membered cycloalkyl, 3-6 membered heterocyclyl, 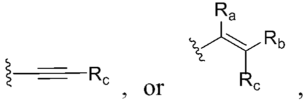 the alkyl, methyl, cycloalkyl and heterocyclyl are optionally substituted with halogen, -CN, -OH, -NH₂, C₂₋₆ alkenyl, or C₂₋₆ alkynyl,
R₄ and R₅ are each independently H, halogen, -CN, C₁₋₄ alkyl, -O-C₁₋₄ alkyl, halo-C₁₋₄ alkyl, 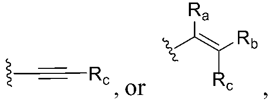 the alkyl is optionally substituted with halogen, -CN, -OH, -NH₂, C₂₋₆ alkenyl, or C₂₋₆ alkynyl,
X₃, R₂, R₃, R₄ and R₅ satisfy the following condition:
   when X₃ is CH,
   at least one of R₂, R₃, R₄ and R₅ is 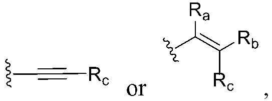 Rₐ, R_{b} and R_{c} are as defined above.

In some embodiments, R is H.

In some embodiments, R₈ is F,

In some embodiments, R₁ is methyl or ethyl.

In some embodiments, the present invention provides the following compound, or a pharmaceutically acceptable salt, solvate, polymorph, or isomer thereof

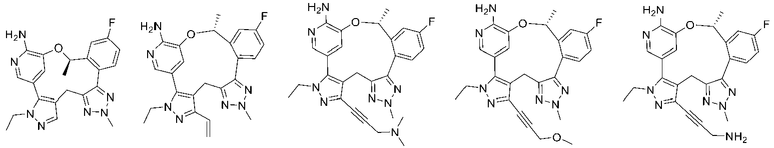

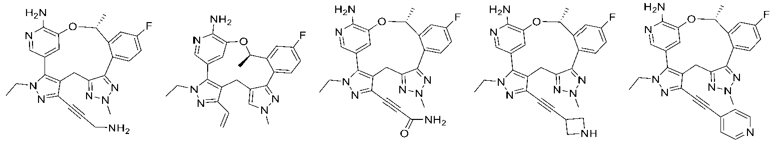

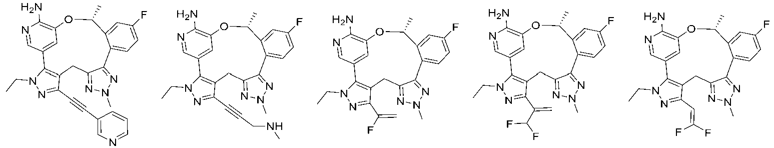

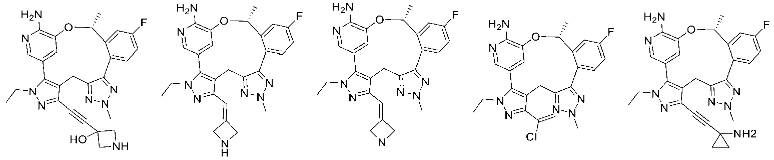

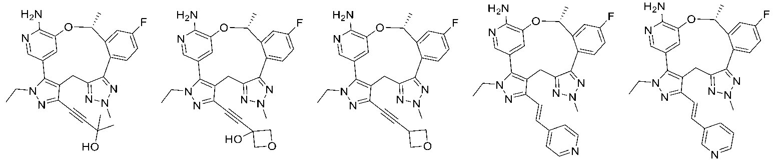

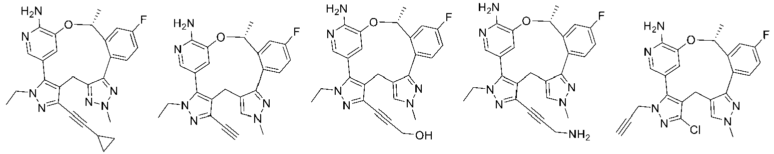

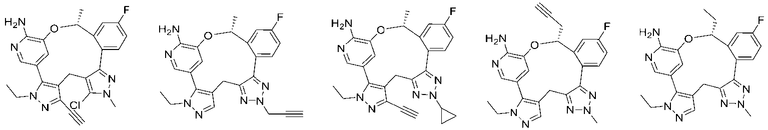

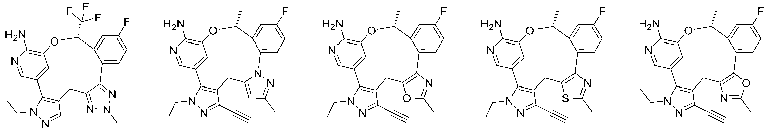

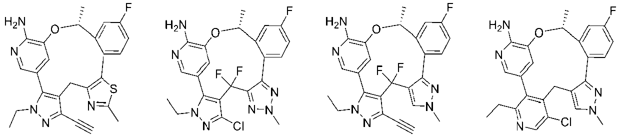

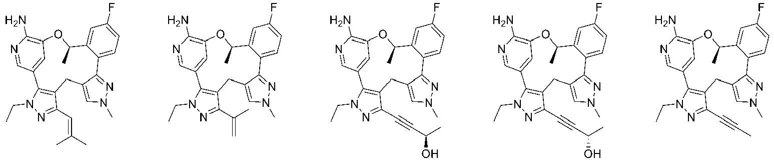

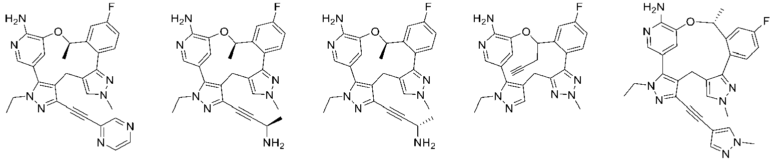

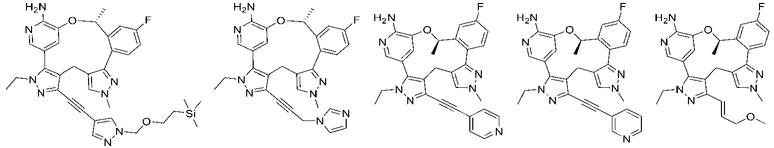

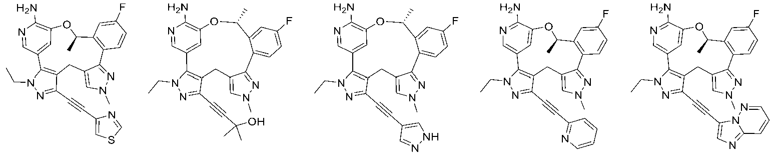

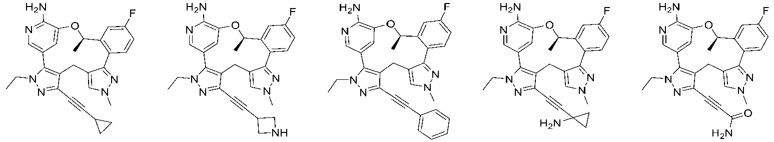

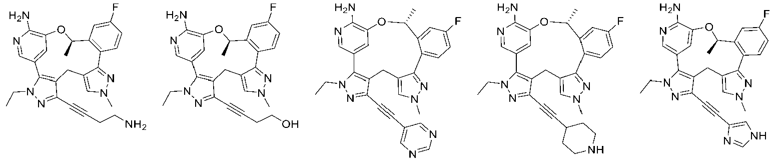

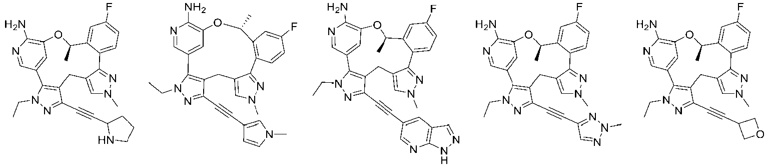

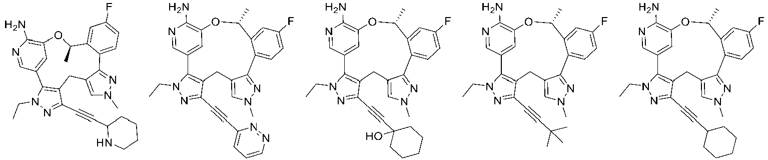

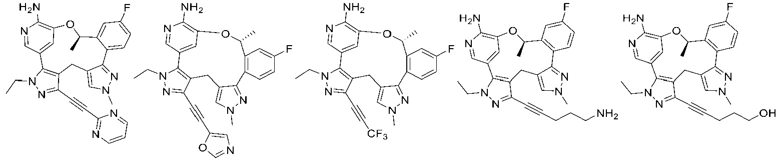

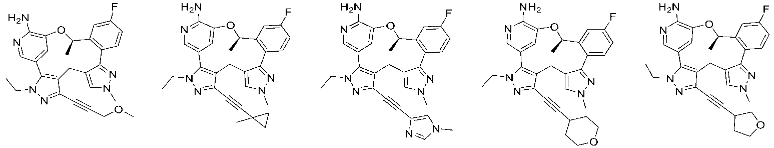

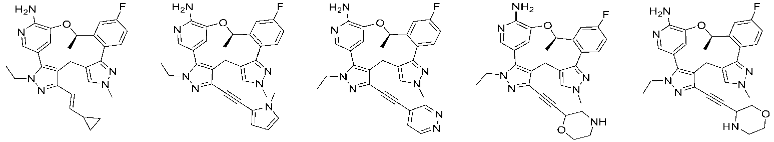

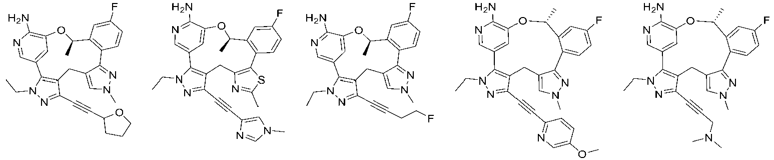

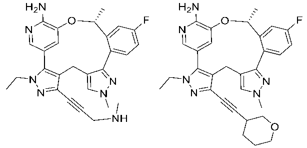

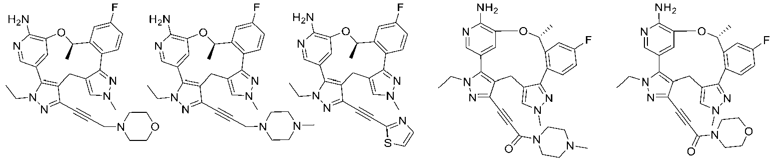

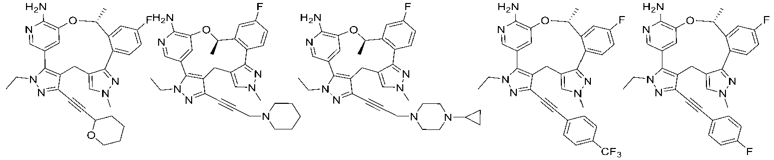

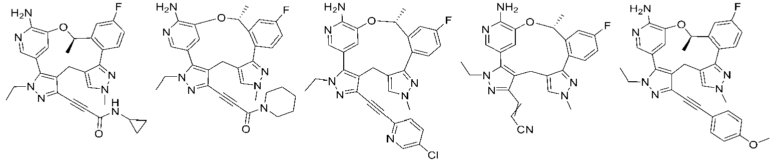

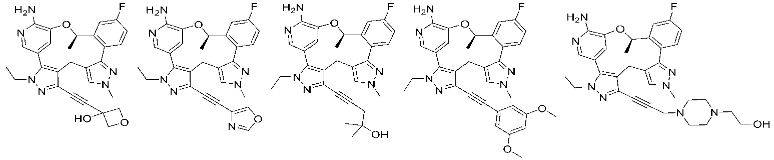

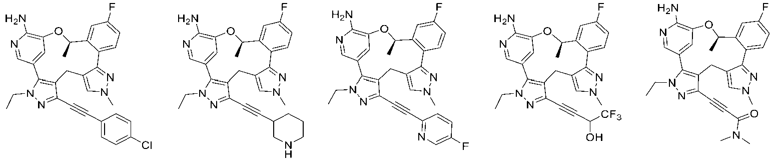

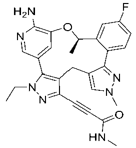

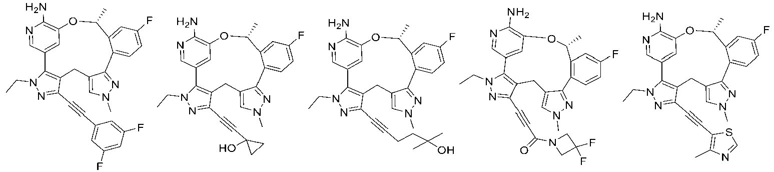

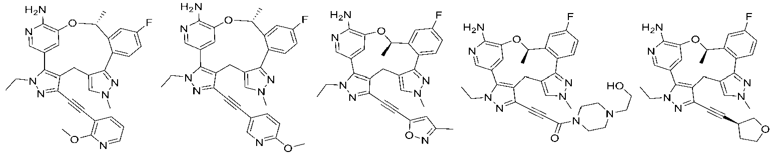

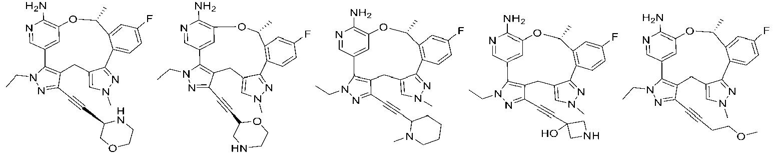

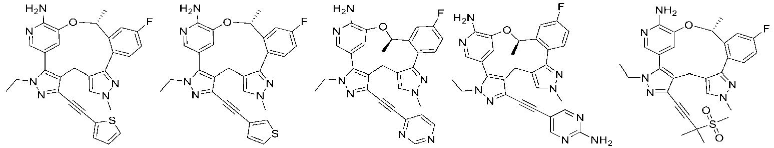

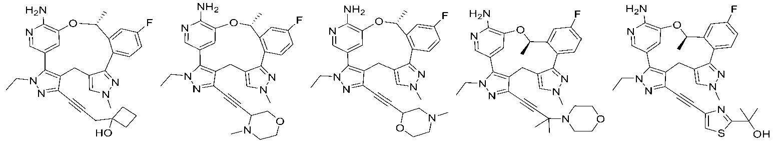

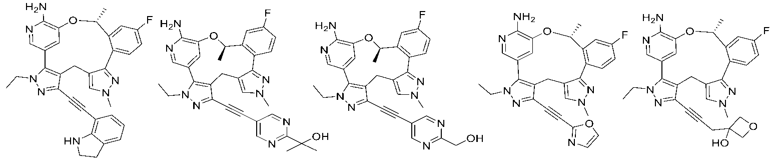

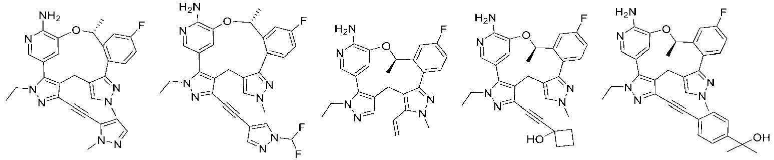

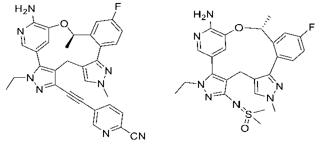

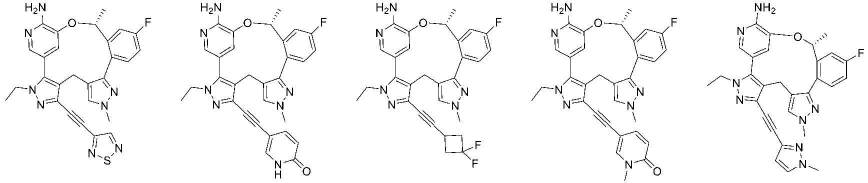

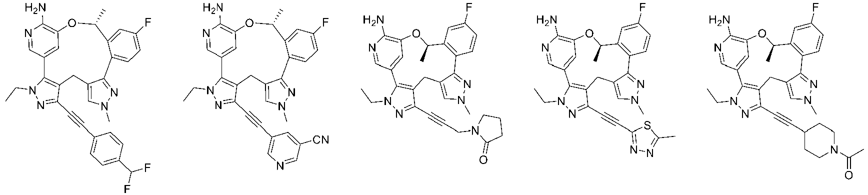

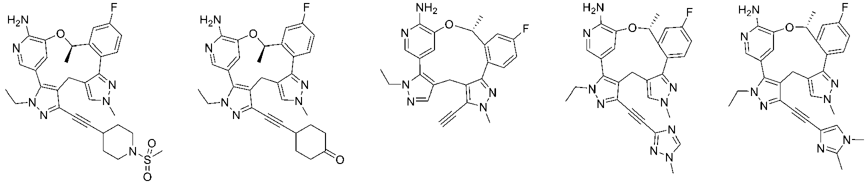

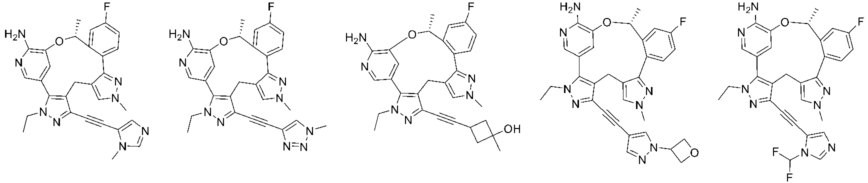

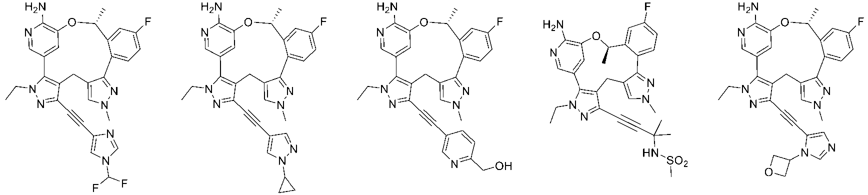

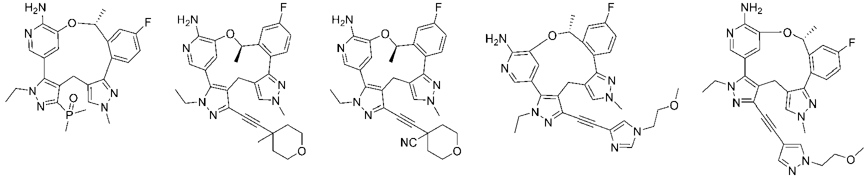

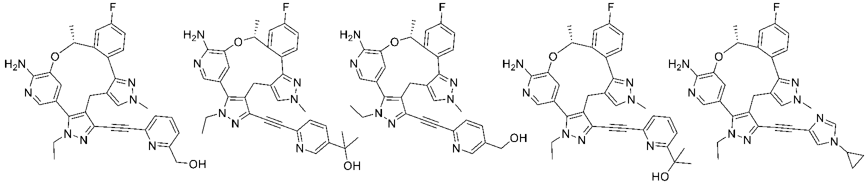

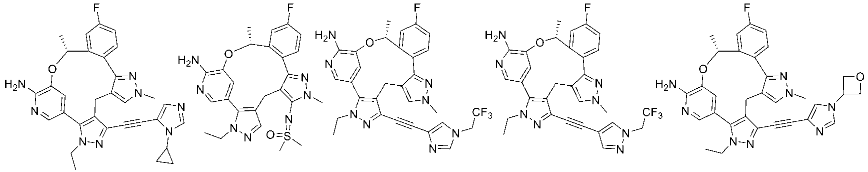

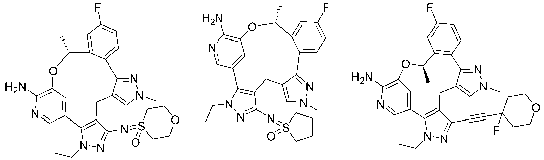

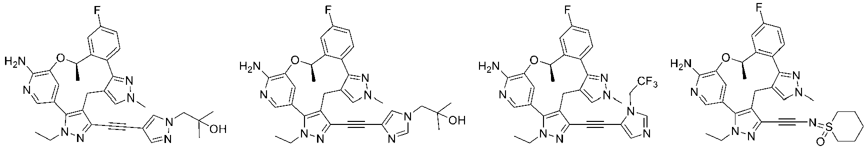

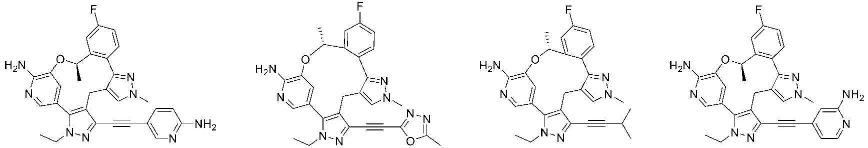

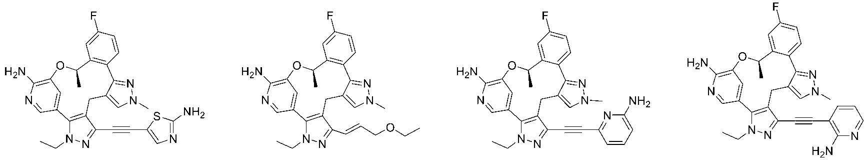

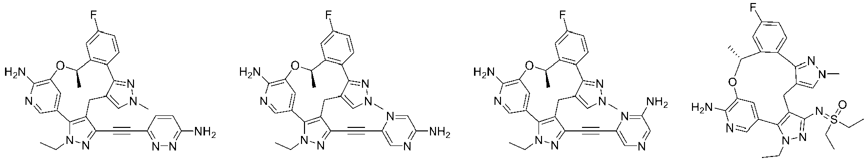

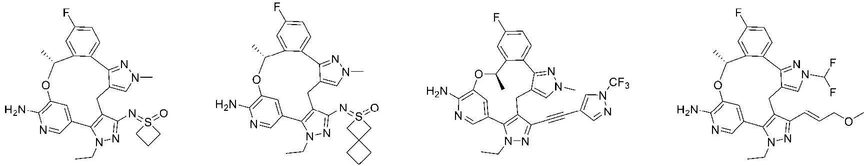

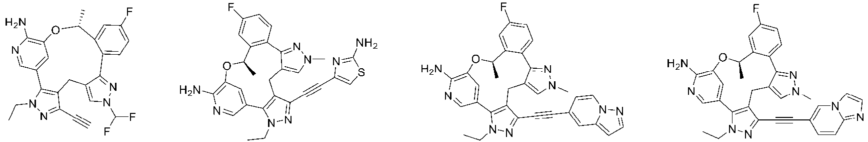

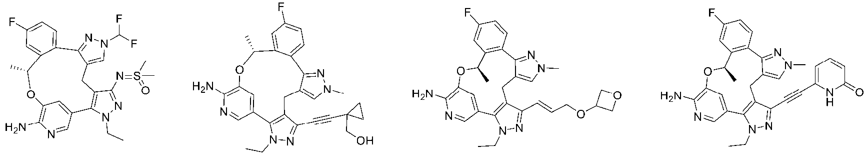

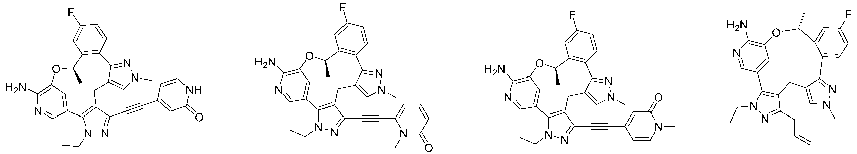

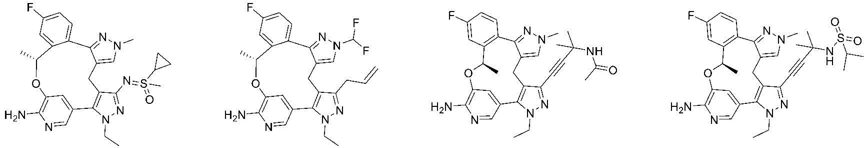

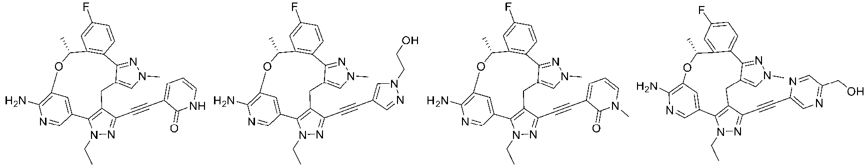

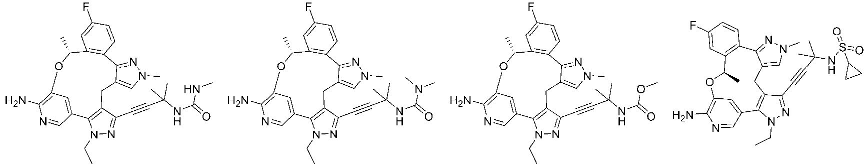

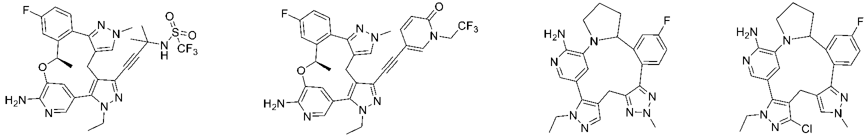

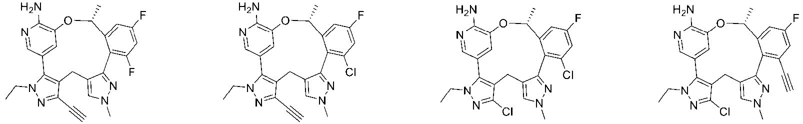

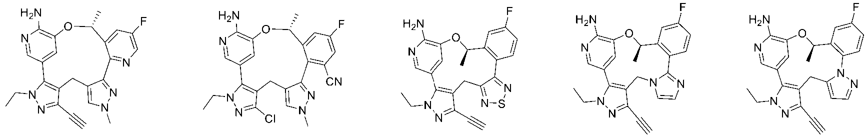

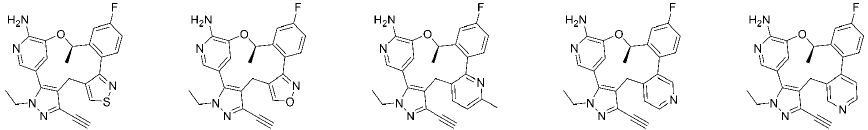

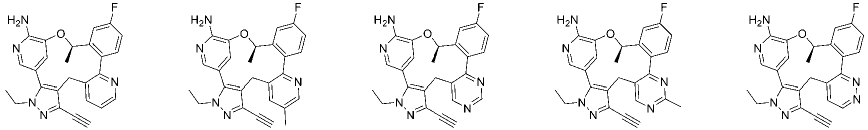

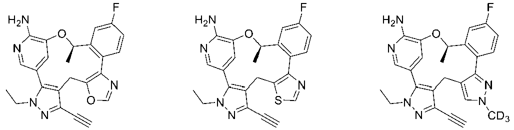

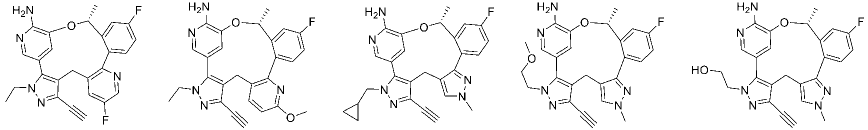

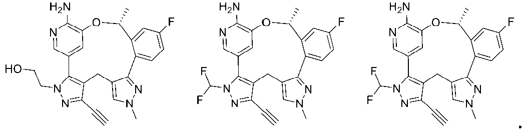

In another aspect, the present invention provides a pharmaceutical composition comprising a compound of the present invention or a pharmaceutically acceptable salt, solvate, polymorph, or isomer thereof, and optionally a pharmaceutically acceptable carrier.

In another aspect, the present invention provides a method for treating an ALK-mediated disease, comprising administering to a subject an effective amount of a compound of the present invention or a pharmaceutically acceptable salt, solvate, polymorph, or isomer thereof, or the composition described above. In some embodiments, the ALK-mediated disease has a G1202R ALK site mutation and/or an L1196M ALK site mutation; preferably, the ALK-mediated disease has a compound mutation comprising both the G1202R ALK site mutation and the L1196M ALK site mutation. In some embodiments, the ALK-mediated disease is ALK-positive non-small cell lung cancer, anaplastic large cell lymphoma, inflammatory myofibroblastic tumor, nasopharyngeal carcinoma, breast cancer, colorectal cancer, diffuse large B-cell lymphoma, systemic histiocytosis, or neuroblastoma.

In some embodiments of the present invention, the subject is a mammal, including a human.

In another aspect, the present invention provides use of a compound of the present invention or a pharmaceutically acceptable salt, solvate, polymorph, or isomer thereof, or the composition described above, in the manufacture of a medicament for treating an ALK-mediated disease. In some embodiments, the ALK-mediated disease has a G1202R ALK site mutation and/or an L1196M ALK site mutation; preferably, the ALK-mediated disease has a compound mutation comprising both the G1202R ALK site mutation and the L1196M ALK site mutation. In some embodiments, the ALK-mediated disease is ALK-positive non-small cell lung cancer, anaplastic large cell lymphoma, inflammatory myofibroblastic tumor, nasopharyngeal carcinoma, breast cancer, colorectal cancer, diffuse large B-cell lymphoma, systemic histiocytosis, or neuroblastoma.

### Detailed Description

Unless otherwise defined, all technical and scientific terms used herein have the same meaning as commonly understood by one of ordinary skill in the art to which the claimed subject matter belongs. Unless otherwise indicated, all patents, patent applications, and published materials cited herein are incorporated by reference in their entirety.

It is to be understood that both the foregoing brief summary and the following detailed description are exemplary and explanatory, and are not restrictive of any subject matter of the present invention.

### Certain Chemical Terminology

The terms "optional" or "optionally" mean that the subsequently described event or circumstance may or may not occur, and the description includes instances where said event or circumstance occurs and instances where it does not. For example, "optionally substituted alkyl" means "unsubstituted alkyl" or "substituted alkyl." Furthermore, an optionally substituted group may be unsubstituted (e.g., -CH₂CH₃), fully substituted (e.g., -CF₂CF₃), monosubstituted (e.g., - CH₂CH₂F), or substituted at any level between monosubstituted and fully substituted (e.g., - CH₂CHF₂, -CF₂CH₃, -CFHCHF₂, etc.). Those skilled in the art will appreciate that for any group containing one or more substituents, no substitution or substitution pattern that is sterically impossible and/or cannot be synthesized will be introduced.

Definitions of standard chemical terms may be found in references including Carey and Sundberg, Advanced Organic Chemistry, Fourth Edition, Volumes A (2000) and B (2001), Plenum Press, New York. Unless otherwise indicated, conventional methods within the skill of the art are employed, such as mass spectrometry, NMR, HPLC, IR, and UV/Vis spectroscopy, and pharmacological methods. Unless specific definitions are provided, the terms relating to analytical chemistry, synthetic organic chemistry, and medicinal and pharmaceutical chemistry, as well as the experimental procedures and techniques described herein, are those known in the art. Standard techniques may be used for chemical synthesis, chemical analysis, pharmaceutical preparation, formulation and delivery, and treatment of patients. For example, reactions and purifications may be carried out using manufacturer's instructions for kits, or in a manner known in the art or as described in the present invention. Typically, the above techniques and methods may be carried out according to conventional methods well known in the art, as described in various general and more specific references cited and discussed in this specification. In this specification, groups and substituents thereof may be selected by those skilled in the art to provide stable moieties and compounds.

Some chemical groups named herein may be denoted by shorthand notations indicating the total number of carbon atoms. For example, "C₁-₆ alkyl" describes an alkyl group, as defined below, having a total of 1 to 6 carbon atoms. The total number of carbon atoms indicated by the shorthand notation does not include carbon atoms from possible substituents.

The term "fused" or "fused ring", as used herein alone or in combination, refers to a ring structure in which two or more rings share one or more bonds.

The term "spiro" or "spiro ring," as used herein alone or in combination, refers to a ring structure in which two or more rings share one or more atoms.

The term "alkyl" as used herein alone or as part of another group (e.g., haloalkyl) refers to an optionally substituted straight-chain or optionally substituted branched-chain monovalent saturated hydrocarbon having 1 to 12 carbon atoms, preferably 1 to 8 carbon atoms, more preferably 1 to 6 carbon atoms, attached to the rest of the molecule by a single bond, for example, methyl, ethyl, isopropyl, n-butyl, isobutyl, n-pentyl, n-heptyl, 2-methylhexyl, n-octyl, and the like.

The term "alkenyl" as used herein alone or in combination refers to an optionally substituted straight-chain or optionally substituted branched-chain monovalent hydrocarbon group having one or more C=C double bonds and having from 2 to about 10 carbon atoms, more preferably from 2 to about 6 carbon atoms. The double bonds in these groups may be in the cis or trans configuration, and are understood to include both isomers. Examples include, but are not limited to, vinyl (CH=CH₂), 1-propenyl (CH₂CH=CH₂), isopropenyl (C(CH₃)=CH₂), butenyl, 1,3-butadienyl, and the like. When a numerical range is given for the alkenyl group as defined herein, for example, "C₂-C₆ alkenyl" or "C₂-₆ alkenyl", it refers to an alkenyl group that may be composed of 2 carbon atoms, 3 carbon atoms, 4 carbon atoms, 5 carbon atoms, or 6 carbon atoms. The alkenyl groups herein also encompass cases where no numerical range is specified.

The term "alkynyl" as used herein alone or in combination refers to an optionally substituted straight-chain or branched-chain monovalent hydrocarbon group having one or more C≡C triple bonds and having from 2 to about 10 carbon atoms, more preferably from 2 to about 6 carbon atoms. Examples include, but are not limited to, ethynyl, 2-propynyl, 2-butynyl, 1,3-butadiynyl, and the like. When a numerical range is given for the alkynyl group as defined herein, for example, "C₂-C₆ alkynyl" or "C₂-₆ alkynyl", it refers to an alkynyl group that may be composed of 2 carbon atoms, 3 carbon atoms, 4 carbon atoms, 5 carbon atoms, or 6 carbon atoms. The alkynyl groups herein also encompass cases where no numerical range is specified.

The term "aryl" refers to an all-carbon monocyclic or fused ring having a fully conjugated π-electron system, containing 6 to 14 carbon atoms, preferably 6 to 12 carbon atoms, and most preferably 6 carbon atoms. The aryl group may be unsubstituted or substituted with one or more substituents, examples of which include, but are not limited to, alkyl, alkyloxy, aryl, aralkyl, amino, halogen, hydroxy, sulfonyl, sulfinyl, phosphoryl, and heterocycloaliphatic groups. Non-limiting examples of unsubstituted aryl include, but are not limited to, phenyl, naphthyl, and anthracenyl.

The term "heteroaryl" refers to a monocyclic or fused ring of 5 to 12 ring atoms, having 5, 6, 7, 8, 9,10, 11, or 12 ring atoms, which contains 1, 2, 3, or 4 ring atoms selected from N, O, and S, with the remaining ring atoms being C, and having a fully conjugated π-electron system. The heteroaryl group may be unsubstituted or substituted, wherein the substituents include, but are not limited to, alkyl, alkyloxy, aryl, aralkyl, amino, halogen, hydroxy, cyano, nitro, carbonyl, and heterocycloaliphatic groups. Non-limiting examples of unsubstituted heteroaryl include, but are not limited to, pyrrolyl, furanyl, thienyl, imidazolyl, oxazolyl, pyrazolyl, pyridinyl, pyrimidinyl, pyrazinyl, quinolinyl, isoquinolinyl, tetrazolyl, and triazinyl.

The term "cycloalkyl" as used herein alone or as part of another group refers to a stable, monovalent, non-aromatic, monocyclic or polycyclic hydrocarbon group containing only carbon and hydrogen atoms, which may include fused, spiro, or bridged ring systems, containing 3 to 15 ring-forming carbon atoms, preferably 3 to 10 ring-forming carbon atoms, more preferably 3 to 8 ring-forming carbon atoms, which may be saturated or unsaturated, and is attached to the rest of the molecule by a single bond. Non-limiting examples of "cycloalkyl" include cyclopropyl, cyclohexyl, cyclohexanonyl, cyclooctyl, 1H-indenyl, decahydronaphthyl, and the like.

The term "heterocyclyl" or "heterocycle" as used herein alone or as part of another group refers to a stable, 3- to 18-membered monovalent non-aromatic ring containing 2 to 12 carbon atoms and 1 to 6 heteroatoms selected from nitrogen, oxygen, and sulfur. Unless otherwise specified, the heterocyclyl group may be a monocyclic, bicyclic, tricyclic, or tetracyclic ring system, which may include fused, spiro, or bridged ring systems. The nitrogen, carbon, or sulfur atoms of the heterocyclyl group may be optionally oxidized, the nitrogen atoms may be optionally quaternized, and the heterocyclyl group may be partially or fully saturated. The heterocyclyl group may be attached to the rest of the molecule by a single bond through a ring carbon atom or a ring heteroatom. A heterocyclyl group containing fused rings may include one or more aromatic or heteroaromatic rings, provided that the point of attachment to the rest of the molecule is on a non-aromatic ring atom. The heterocyclyl group is preferably a stable 4- to 11-membered monovalent non-aromatic monocyclic or bicyclic ring containing 1 to 3 heteroatoms selected from nitrogen, oxygen, and sulfur, and more preferably a stable 4- to 8-membered monovalent non-aromatic monocyclic ring containing 1 to 3 heteroatoms selected from nitrogen, oxygen, and sulfur. Non-limiting examples of heterocyclyl groups include azepanyl, azetidinyl, decahydroisoquinolinyl, dihydrofuranyl, dihydroindolyl, dioxolanyl, morpholinyl, 2-oxopiperidinyl, tetrahydroisoquinolinyl, trithianyl, and the like.

The term "carbocycle" as used herein alone or in combination refers to a structure formed by covalently closed rings of carbon atoms, which may be saturated or partially unsaturated. The carbocycle may be formed from 3,4, 5, 6, 7, 8, 9, or more than 9 atoms. The term "carbocycle" herein may be monocyclic or polycyclic, and polycyclic carbocycles include spiro, fused, and bridged rings. The carbocycle may be optionally substituted. The "carbocycle" herein preferably contains from about 5 to about 20, or from 5 to 10, or from 5 to 8, or from 5 to 6 skeletal ring atoms.

The term "halogen," "halo," or "halide" as used herein alone or in combination refer to fluorine, chlorine, bromine, and iodine.

Unless otherwise specified, the olefinic double bonds contained in the compounds of the present invention include both E and Z isomers.

It is to be understood that the compounds of the present invention may contain asymmetric centers. These asymmetric centers may independently be in the R or S configuration. Some compounds of the present invention may also exhibit cis-trans isomerism, as will be apparent to those skilled in the art. It is to be understood that the compounds of the present invention include their individual geometric isomers and stereoisomers, as well as mixtures thereof, including racemic mixtures. These isomers may be separated from their mixtures by carrying out or modifying known methods, such as chromatographic techniques and recrystallization techniques, or may be prepared individually from suitable isomers of their intermediates.

The term "pharmaceutically acceptable salt" as used herein includes both acid addition salts and base addition salts.

"Pharmaceutically acceptable acid addition salts" refer to those salts that retain the biological effectiveness and properties of the free base of the compound, are not biologically or otherwise undesirable, and are formed with inorganic acids such as, but not limited to, hydrochloric acid, hydrobromic acid, sulfuric acid, nitric acid, phosphoric acid, and the like, or with organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, lauryl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecenoic acid, and the like.

"Pharmaceutically acceptable base addition salts" refer to those salts that retain the biological effectiveness and properties of the free acid of the compound, are not biologically or otherwise undesirable. These salts are prepared by reacting the free acid with an inorganic base or an organic base. Salts formed by reaction with inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are ammonium salts, sodium salts, potassium salts, calcium salts, and manganese salts.

Organic bases for salt formation include, but are not limited to, primary amines, secondary amines, tertiary amines, substituted amines (including naturally occurring substituted amines), cyclic amines, and basic ion exchange resins, such as ammonia, isopropylamine, trimethylamine, diethylamine, triethylamine, tripropylamine, diethanolamine, ethanolamine, dimethylethanolamine, 2-dimethylaminoethanol, 2-diethylaminoethanol, dicyclohexylamine, lysine, arginine, histidine, caffeine, procaine, hydrabamine, choline, betaine, N-benzylphenethylamine, benzathine, ethylenediamine, glucosamine, meglumine, theobromine, triethanolamine, tromethamine, purines, piperazine, piperidine, N-ethylpiperidine, polyamine resins, and the like. Particularly preferred organic bases are isopropylamine, diethylamine, ethanolamine, trimethylamine, dicyclohexylamine, choline, and caffeine.

The compound of the present invention or a salt thereof may be administered alone as an active substance, preferably in the form of a pharmaceutical composition thereof.

The term "pharmaceutical composition" as used herein refers to a preparation comprising a mixture of a compound of the present invention and a vehicle that is generally accepted in the art for delivering biologically active compounds to mammals (e.g., humans). Such vehicles include all pharmaceutically acceptable carriers.

The term "acceptable" as used herein with respect to a formulation, composition, or ingredient means having no persistent detrimental effect on the overall health of the subject being treated.

The term "pharmaceutically acceptable" as used herein refers to a substance (e.g., a carrier or diluent) that does not affect the biological activity or properties of the compound of the present invention and is relatively non-toxic, i.e., the substance may be administered to an individual without causing an adverse biological reaction or interacting in an adverse manner with any component contained in the composition.

"Pharmaceutically acceptable carriers" include, but are not limited to, adjuvants, carriers, excipients, auxiliary agents, deodorants, diluents, preservatives, dyes/colorants, flavor enhancers, surfactants and wetting agents, dispersing agents, suspending agents, stabilizing agents, isotonic agents, solvents, or emulsifiers that have been approved by the relevant governmental administrative department for use in humans and domestic animals.

The pharmaceutical compositions of the present invention may be formulated into solid, semi-solid, liquid, or gaseous formulations, such as tablets, pills, capsules, powders, granules, ointments, emulsions, suspensions, solutions, suppositories, injections, inhalants, gels, microspheres, aerosols, and the like.

Typical routes of administration of the compound of the present invention or a pharmaceutically acceptable salt thereof or a pharmaceutical composition thereof include, but are not limited to, oral, rectal, transmucosal, enteral, or topical, transdermal, inhalation, parenteral, sublingual, intravaginal, intranasal, intraocular, intraperitoneal, intramuscular, subcutaneous, intravenous administration. The preferred route of administration is oral administration.

The pharmaceutical compositions of the present invention may be manufactured by methods well known in the art, such as conventional mixing, dissolving, granulating, dragee-making, levigating, emulsifying, lyophilizing, and the like.

In preferred embodiments, the pharmaceutical composition is in an oral form. For oral administration, the pharmaceutical composition may be formulated by mixing the active compound with pharmaceutically acceptable carriers well known in the art. Such carriers enable the compounds of the present invention to be formulated as tablets, pills, lozenges, dragees, capsules, liquids, gels, syrups, suspensions, and the like, for oral administration to a patient.

Solid oral pharmaceutical compositions may be prepared by conventional mixing, filling, or tableting methods. For example, they may be obtained by mixing the active compound with solid excipients, optionally grinding the resulting mixture, adding other suitable auxiliary agents if necessary, and then processing the mixture into granules to obtain the core of tablets or dragees. Suitable excipients include, but are not limited to: binders, diluents, disintegrants, lubricants, glidants, sweeteners, or flavoring agents, etc., such as microcrystalline cellulose, glucose solution, acacia mucilage, gelatin solution, sucrose and starch paste; talc, starch, magnesium stearate, calcium stearate or stearic acid; lactose, sucrose, starch, mannitol, sorbitol, or dicalcium phosphate; silicon dioxide; croscarmellose sodium, pregelatinized starch, sodium starch glycolate, alginic acid, corn starch, potato starch, methylcellulose, agar, carboxymethylcellulose, cross-linked polyvinylpyrrolidone, and the like. The cores of the dragees may be optionally coated, particularly with enteric coatings, according to methods well known in ordinary pharmaceutical practice.

The pharmaceutical compositions may also be suitable for parenteral administration, such as sterile solutions, suspensions, or lyophilized products in appropriate unit dosage forms. Suitable excipients, such as fillers, buffers, or surfactants, may be used.

### Specific Embodiments

The compounds of the present invention, as well as their preparation methods and uses, are exemplarily described below in conjunction with the examples.

The following non-limiting examples are illustrative only and are not intended to limit the present invention in any way.

Unless otherwise indicated, temperatures are in degrees Celsius. Reagents were purchased from commercial suppliers such as Sinopharm Chemical Reagent Beijing Co., Ltd., Alfa Aesar, or Beijing J&K Scientific Technology Co., Ltd., and were used directly without further purification unless otherwise indicated.

Unless otherwise indicated, the following reactions were carried out at room temperature, in anhydrous solvents, under a positive pressure of nitrogen or argon, or using a drying tube; reaction vessels were fitted with rubber septa for the addition of substrates and reagents via syringe; glassware was dried and/or heat-dried.

Unless otherwise indicated, column chromatography purification was performed using 200-300 mesh silica gel from Qingdao Haiyang Chemical Factory; preparative thin layer chromatography was performed using thin layer chromatography silica gel pre-coated plates (HSGF254) produced by Yantai Institute of Chemical Industry; MS was determined on a Thermo LCQ Fleet (ESI) liquid chromatograph-mass spectrometer; optical rotations were measured on an SGW-3 automatic polarimeter from Shanghai Shenguang Instrument Co., Ltd.

NMR data (¹H NMR) were recorded on a Varian instrument operating at 400 MHz. Solvents used for NMR data include CDCl₃, CD₃OD, D₂O, DMSO-d6, etc., with tetramethylsilane (0.00 ppm) as the reference or with residual solvent as the reference (CDCl₃: 7.26 ppm; CD₃OD: 3.31 ppm; D₂O: 4.79 ppm; d6-DMSO: 2.50 ppm). When peak multiplicities are indicated, the following abbreviations represent different peak shapes: s (singlet), d (doublet), t (triplet), q (quartet), m (multiplet), br (broad), dd (doublet of doublets), dt (doublet of triplets). Where coupling constants are given, they are reported in Hertz (Hz).

### Abbreviation:

| | |
|---|---|
| cataCXium A | butyldi(1-adamantyl)phosphine |
| NIS | N-iodosuccinimide |
| Pd(dppf)Cl₂ | (1,1'-bis(diphenylphosphino)ferrocene)dichloropalladium(II) |
| Pd(PPh₃)₂Cl₂ | bis(triphenylphosphine)palladium(II) dichloride |

### Synthesis Method

### General Synthesis Method A

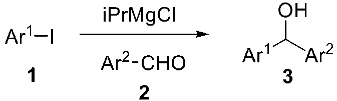

Under nitrogen protection, a solution of isopropylmagnesium chloride in tetrahydrofuran (2 M, 1 eq) was added dropwise to iodide **1** (1.05 eq) at -35 °C. Subsequently, the reaction mixture was warmed to 0 °C and stirred for an additional 1 hour. At 0 °C, a solution of compound **2** (1 eq) in tetrahydrofuran was added dropwise to the above reaction mixture, and the mixture was allowed to warm to room temperature and stirred overnight. Saturated aqueous ammonium chloride solution was added to the reaction mixture. The resulting mixture was extracted with ethyl acetate. The extract was washed with brine, dried over anhydrous sodium sulfate, and concentrated under reduced pressure. The resulting residue was purified by flash silica gel column chromatography to obtain compound **3.**

### General Synthesis Method B

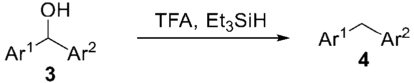

Under nitrogen protection, triethylsilane (3.0 eq) was added to a solution of compound **3** (1.0 eq) in dichloromethane. The temperature of the reaction mixture was then lowered to 0 °C, and trifluoroacetic acid (4.0 eq) was slowly added dropwise. After the addition was complete, the reaction mixture was warmed to room temperature and stirred overnight. The reaction mixture was concentrated under reduced pressure, and the resulting residue was purified by flash silica gel column chromatography to obtain compound **4.**

### General Synthesis Method C

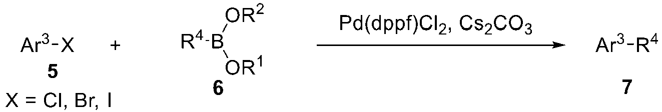

Under nitrogen protection, a solution of aromatic halide **5** (1.0 eq), boronic acid or boronic ester **6** (1.1 eq), Pd(dppf)Cl₂ (0.1 eq), and cesium carbonate (2.0 eq) in dioxane was heated to 80 °C and stirred overnight. The reaction mixture was concentrated under reduced pressure, and the resulting residue was purified by flash silica gel column chromatography to obtain compound **7.**

### General Synthesis Method D

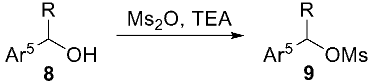

At 0 °C, methanesulfonic anhydride (1.5 eq) was added to a solution of compound **8** (1.0 eq) and triethylamine (2.0 eq) in dichloromethane. The reaction mixture was stirred at 0 °C for an additional 2 hours. Saturated aqueous sodium bicarbonate solution was added to the reaction mixture to quench the reaction. The resulting solution was extracted with dichloromethane. The extract was dried over anhydrous sodium sulfate, filtered, and concentrated under reduced pressure. The resulting residue **9** was used directly in the next step without further purification.

### General Synthesis Method E

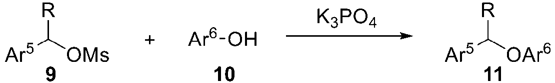

Under nitrogen protection, a solution of compound **9** (1.0 eq), compound **10** (1.2 eq), and anhydrous potassium phosphate (2.0 eq) in *N,N*-dimethylacetamide was heated to 65 °C and stirred overnight. The reaction mixture was poured into water, and the resulting aqueous solution was extracted with ethyl acetate. The extract was dried over anhydrous sodium sulfate, filtered, and concentrated under reduced pressure. The resulting residue was purified by flash silica gel column chromatography to obtain compound **11.**

### General Synthesis Method F

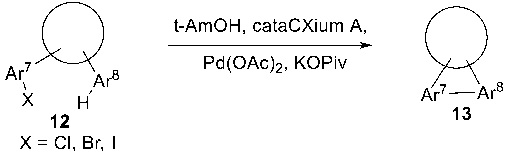

Under nitrogen protection, a solution of compound **12** (1.0 eq), cataCXium A (0.2 eq), potassium pivalate (2.0 eq), and palladium acetate (0.1 eq) in *tert-*amyl alcohol was heated to 100 °C and stirred overnight. The reaction mixture was concentrated under reduced pressure, and the resulting residue was purified by flash silica gel column chromatography to obtain compound **13.**

### General Synthesis Method G

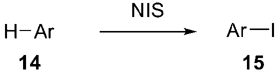

To a solution of compound **14** (1.0 eq) in trifluoroacetic acid was added NIS (2.5 eq), and the reaction mixture was heated to 30 °C and stirred for 48 hours. The reaction mixture was concentrated under reduced pressure, and saturated aqueous sodium bicarbonate solution was added to the resulting residue. The resulting mixture was extracted with ethyl acetate. The extract was washed with saturated sodium thiosulfate solution, dried over anhydrous sodium sulfate, and concentrated under reduced pressure. The residue was purified by flash silica gel column chromatography to obtain compound **15.**

### General Synthesis Method H

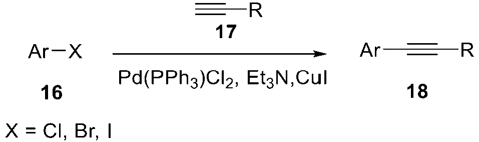

Under nitrogen protection, to a solution of compound **16** (1.0 eq), Pd(PPh₃)₂Cl₂ (0.1 eq), triethylamine (3.0 eq), and copper(I) iodide (0.2 eq) in DMF was added compound **17** (2.0 eq). The reaction mixture was heated to 80 °C and stirred overnight. The reaction mixture was concentrated under reduced pressure, and the resulting residue was purified by flash silica gel column chromatography to obtain compound **18.**

### General Synthesis Method I

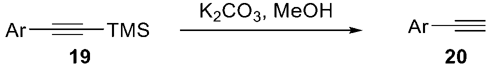

At room temperature, anhydrous potassium carbonate (2.0 eq) was added to a solution of compound **19** (1.0 eq) in methanol. The reaction mixture was stirred at room temperature until the reaction was complete. The reaction mixture was filtered, and the resulting filtrate was concentrated under reduced pressure. The resulting residue was purified by flash silica gel column chromatography to obtain compound **20.**

### General Synthesis Method J

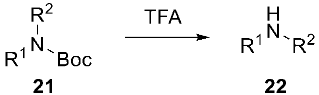

At room temperature, trifluoroacetic acid (dichloromethane:trifluoroacetic acid = 3:1) was added to a solution of compound **21** in dichloromethane, and the mixture was stirred at room temperature until the reaction was complete. The reaction mixture was concentrated under reduced pressure. Saturated aqueous sodium bicarbonate solution was added to the resulting residue, and the mixture was extracted with ethyl acetate. The organic extract was dried over anhydrous sodium sulfate, filtered, and concentrated under reduced pressure. The resulting residue was purified by flash silica gel column chromatography to obtain compound **22**.

### Example 1: (19R)-3-ethyl-5-ethynyl-16-fluoro-10,19-dimethyl-3,4,9,10,11,23-hexaaza-2 0-oxapentacyclo[19.3.1.0^{2,6}.0^{8,12}.0^{13,18}]pentacosa-1(25),2(6),4,8(9),11,13(14),15,17,21(22),23-d ecaen-22-amine

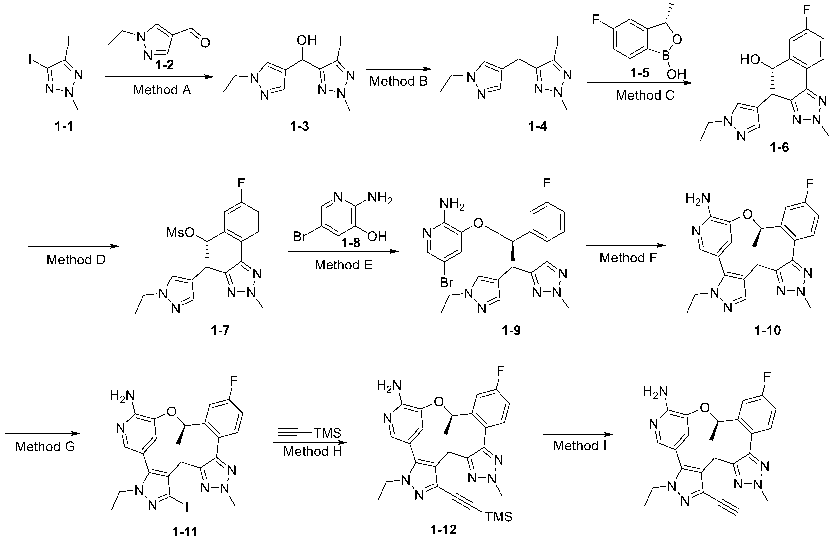

### Step 1

At -30 °C, to a solution of compound **1-1** (21 g) in tetrahydrofuran (400 mL) was slowly added a solution of isopropylmagnesium chloride in furan (29 mL, 2M), and the reaction solution was stirred at -30 °C for 30 minutes. At -30 °C, to the reaction solution was slowly added a solution of compound **1-2** (7.42 g) in tetrahydrofuran (50 mL), and the reaction solution was reacted at room temperature for 2 hours. To the reaction solution was added saturated aqueous ammonium chloride solution (400 mL) to quench the reaction. The mixture was extracted with ethyl acetate (200 mL x 3), the extract was washed with brine (300 mL), dried over anhydrous sodium sulfate, and concentrated under reduced pressure, and the residue was purified through silica gel chromatography (petroleum ether:ethyl acetate = 3:1) to obtain compound **1-3** (15 g).

### Step 2

At 0 °C, to a solution of compound **1-3** (15 g) in dichloromethane (150 mL) were added triethylsilane (15.7 g) and trifluoroacetic acid (20 g), and the reaction solution was stirred at room temperature overnight. The reaction solution was poured into saturated aqueous sodium bicarbonate solution (300 mL), the mixture was extracted with ethyl acetate (150 mL x 3), the extract was dried over anhydrous sodium sulfate and concentrated under reduced pressure, and the residue was purified through silica gel chromatography (petroleum ether:ethyl acetate = 5:1) to obtain compound **1-4** (11 g).

### Step 3

Under nitrogen protection, a solution of compound **1-4** (8.5 g), compound **1-5** (5.3 g), anhydrous sodium carbonate (9 g) and Pd(dppf)Cl₂ (7.8 g) in tetrahydrofuran /water (80 mL/ 8 mL) was heated to 65 °C and reacted overnight. The reaction solution was concentrated under reduced pressure, and the residue was purified through silica gel chromatography (petroleum ether:ethyl acetate = 2:1 to 1:1) to obtain compound **1-6** (9.6 g).

### Step 4

At 0 °C, to a solution of compound **1-6** (7.5 g) and triethylamine (9.2 g) in dichloromethane (150 mL) was added methanesulfonic anhydride (7.9 g), and the reaction solution was stirred at 0 °C for 1 hour. To the reaction solution was added water (200 mL) to quench the reaction, the mixture was extracted with dichloromethane (100 mL x 3), and the extract was dried over anhydrous sodium sulfate and concentrated under reduced pressure to obtain compound **1-7.**

### Step 5

A solution of compound **1-7** (8.4 g), **1-8** (3.9 g) and cesium carbonate (9.2 g) in *N-*methylpyrrolidone (20 mL) was heated to 45 °C and reacted for 3 hours. The reaction solution was poured into water (200 mL), the mixture was extracted with ethyl acetate (100 mL x 3), the extract was dried over anhydrous sodium sulfate and concentrated under reduced pressure, and the residue was purified through silica gel chromatography (dichloromethane:ethyl acetate = 2:1 to 1:1) to obtain compound **1-9** (7.8 g).

### Step 6

Under nitrogen protection, a solution of compound **1-9** (7.0 g), cataCXium A (1.2 g), potassium pivalate (5.8 g) and palladium acetate (0.37 g) in *tert-*amyl alcohol (140 mL) was heated to 100 °C and reacted overnight. The reaction solution was concentrated under reduced pressure, and the residue was purified through silica gel chromatography (dichloromethane:ethyl acetate = 2:1 to 1:1) to obtain compound **1-10** (3.5 g).

### Step 7

To a solution of compound **1-10** (1.0 g) in trifluoroacetic acid (20 mL) was added NIS (1.34 g), and the reaction solution was heated to 30 °C and reacted for 48 hours. The reaction solution was concentrated under reduced pressure, and to the residue was added saturated aqueous sodium bicarbonate solution (100 mL). The mixture was extracted with ethyl acetate (80 mL x 3), the extract was washed with saturated aqueous sodium thiosulfate solution (80 mL), dried over anhydrous sodium sulfate, and concentrated under reduced pressure, and the residue was purified through silica gel chromatography (dichloromethane:methanol = 30:1) to obtain compound **1-11** (720 mg).

### Step 8

Under nitrogen protection, to a solution of compound **1-11** (100 mg), Pd(PPh₃)₂Cl₂ (13 mg), triethylamine (80 µL) and copper(I) iodide (7 mg) in DMF (2 mL) was added trimethylsilylacetylene (0.1 mL), and the reaction solution was heated to 80 °C and reacted overnight. The reaction solution was concentrated under reduced pressure, and the residue was purified through silica gel chromatography (dichloromethane:methanol = 40:1) to obtain compound **1-12** (85 mg).

### Step 9

At room temperature, to a solution of compound **1-12** (85 mg) in methanol (2 mL) was added anhydrous potassium carbonate (45 mg), and the reaction solution was stirred at room temperature until the reaction was complete. The reaction solution was filtered, the filtrate was concentrated under reduced pressure, and the residue was purified through silica gel chromatography (dichloromethane:methanol = 40:1) to obtain the target compound. LC-MS (ESI) [M+H]⁺: 444.2. ¹H NMR (400 MHz, CDCl₃), 7.52 (brs, 1H), 7.31 (dd, J = 9.6 Hz, 2.8 Hz, 1H), 7.09 (dd, J = 8.4 Hz, 5.6 Hz, 1H), 7.03 (td, J = 8.4 Hz, 2.8 Hz, 1H), 6.22 (d, J = 1.6 Hz, 1H), 5.24-5.29 (m, 1H), 5.04 (s, 2H), 4.22 (s, 3H), 4.04-4.13 (m, 2H), 3.98 (d, J = 15.6 Hz, 1H), 3.31 (s, 1H), 3.19 (d, J = 15.6 Hz, 1H), 1.79 (d, J = 6.0 Hz, 3H), 1.37 (t, J = 7.2 Hz, 3H).

### Example 2: (19R)-3-ethyl-16-fluoro-10,19-dimethyl-5-vinyl-3,4,9,10,11,23-hexaaza-20-o xapentacyclo[19.3.1.0^{8,12}.0^{13,18}.0^{2,6}]pentacosa-1(25),2(6),4,8(9),11,13(18),14,16,21(22),23-deca en-22-amine

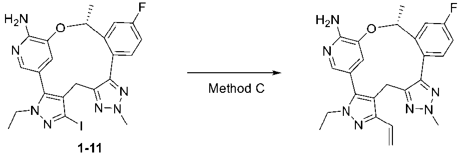

Under nitrogen protection, a solution of **1-11** (100 mg), **2-1** (35 mg), Pd(dppf)Cl₂ (14 mg) and anhydrous sodium carbonate (40 mg) in dioxane/water (2 ml/0.5 mL) was heated to 80 °C and reacted overnight. The reaction solution was concentrated under reduced pressure, and the residue was purified through silica gel chromatography (dichloromethane:methanol = 40:1) to obtain the target compound (42 mg). LC-MS (ESI) [M+H]⁺: 446.4. ¹H NMR (400 MHz, CDCl₃), 7.52 (s, 1H), 7.32 (dd, J = 10.0 Hz, 2.4 Hz, 1H), 7.08 (dd, J = 8.8 Hz, 6.0 Hz, 1H), 7.02 (td, J = 8.0 Hz, 2.4 Hz, 1H), 6.95 (dd, J = 18.0 Hz, 11.2 Hz, 1H), 6.27 (s, 1H), 6.19 (dd, J = 18.0 Hz, 1.6 Hz, 1H), 5.43 (dd, J = 11.2 Hz, 1.2 Hz, 1H), 5.23-5.30 (m, 1H), 4.85 (s, 2H), 4.21 (s, 3H), 4.03-4.18 (m, 2H), 3.84 (d, J = 15.6 Hz, 1H), 3.19 (d, J = 15.6 Hz, 1H), 1.79 (d, J = 6.4 Hz, 3H), 1.37 (t, J = 7.2 Hz, 3H).

### Example 3: (19R)-5-(3-(dimethylamino)prop-1-yn-1-yl)-3-ethyl-16-fluoro-10,19-dimeth yl-3,4,9,10,11,23-hexaaza-20-oxapentacyclo[19.3.1.0^{8,12}.0^{13,18}.0^{2,6}]pentacosa-1(25),2(6),4,8(9), 11,13(18),14,16,21(22),23-decaen-22-amine

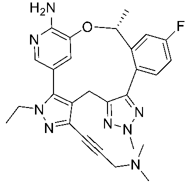

The synthetic method of Example 3 is the same as that of Example 1. LC-MS (ESI) [M+H]⁺: 501.3. ¹H NMR (400 MHz, CDCl₃), 7.52 (d, J = 1.2 Hz, 1H), 7.32 (dd, J = 10.0 Hz, 2.4 Hz, 1H), 7.11 (dd, J = 8.4 Hz, 6.0 Hz, 1H), 7.03 (td, J = 8.4 Hz, 2.4 Hz, 1H), 6.23 (d, J = 1.2 Hz, 1H), 5.24-5.29 (m, 1H), 4.49 (s, 2H), 4.20 (s, 3H), 4.06-4.15 (m, 2H), 3.97 (d, J = 15.6 Hz, 1H), 3.65 (s, 2H), 3.18 (d, J = 15.6 Hz, 1H), 2.48 (s, 6H), 1.79 (d, J = 6.0 Hz, 3H), 1.39 (t, J = 7.2 Hz, 3H).

### Example 4: (19R)-3-ethyl-16-fluoro-5-(3-methoxyprop-1-yn-1-yl)-10,19-dimethyl-3,4,9, 10,11,23-hexaaza-20-oxapentacyclo[19.3.1.0^{8,12}.0^{13,18}.0^{2,6}]pentacosa-1(25),2(6),4,8(9),11,13(1 8),14,16,21(22),23-decaen-22-amine

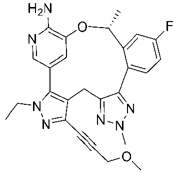

The synthetic method of Example 4 is the same as that of Example 1. LC-MS (ESI) [M+H]⁺: 488.3. ¹H NMR (400 MHz, CDCl₃), 7.45-7.56 (brs, 1H), 7.32 (dd, J = 10.0 Hz, 2.4 Hz, 1H), 7.11 (dd, J = 8.4 Hz, 6.0 Hz, 1H), 7.04 (td, J = 8.4 Hz, 2.4 Hz, 1H), 6.24 (s, 1H), 5.22-5.31 (m, 1H), 4.91 (s, 2H), 4.39-4.47 (m, 2H), 4.21 (s, 3H), 4.07-4.15 (m, 2H), 3.98 (d, J = 15.6 Hz, 1H), 3.52 (s, 3H), 3.18 (d, J = 15.6 Hz, 1H), 1.80 (d, J = 6.4 Hz, 3H), 1.39 (t, J = 7.2 Hz, 3H).

### Example 5: (19R)-5-(3-aminoprop-1-yn-1-yl)-3-ethyl-16-fluoro-10,19-dimethyl-3,4,9,10, 11,23-hexaaza-20-oxapentacyclo[19.3.1.0^{8,12}.0^{13,18}0^{2,6}]pentacosa-1(25),2(6),4,8(9),11,13(18),1 4,16,21(22),23-decaen-22-amine

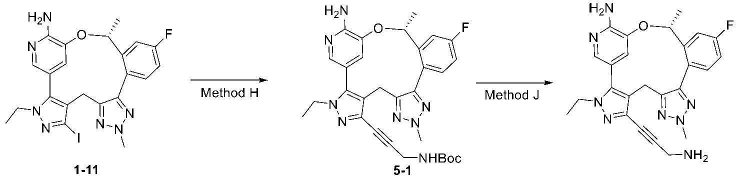

### Step 1

Under nitrogen protection, to a solution of compound **1-11** (150 mg), Pd(PPh₃)₂Cl₂ (25 mg), triethylamine (115 µL) and copper(I) iodide (11 mg) in DMF (2 mL) was added compound **17** (85 mg), and the reaction solution was heated to 80 °C and reacted overnight. The reaction solution was concentrated under reduced pressure, and the residue was purified through silica gel chromatography (dichloromethane:methanol = 40:1) to obtain compound **5-1** (80 mg).

### Step 2

At room temperature, to a solution of compound **5-1** (80 mg) in dichloromethane (3 mL) was added trifluoroacetic acid (1 mL), and the reaction solution was stirred at room temperature until the reaction was complete. The reaction solution was concentrated under reduced pressure, to the residue was added saturated aqueous sodium bicarbonate solution (30 mL), and the mixture was extracted with ethyl acetate (20 mL x 3). The extract was dried over anhydrous sodium sulfate and concentrated under reduced pressure, and the residue was purified through silica gel chromatography (dichloromethane:methanol = 15:1) to obtain the target compound (52 mg). LC-MS (ESI) [M+H]⁺: 473.2. ¹H NMR (400 MHz, CDCl₃), 7.50 (d, J = 1.2 Hz, 1H), 7.32 (dd, J = 10.0 Hz, 2.4 Hz, 1H), 7.10 (dd, J = 8.4 Hz, 6.0 Hz, 1H), 7.03 (td, J = 8.4 Hz, 2.4 Hz, 1H), 6.21 (d, J = 1.2 Hz, 1H), 5.24-5.29 (m, 1H), 4.84 (s, 2H), 4.23 (s, 3H), 4.06-4.12 (m, 2H), 3.95 (d, J = 15.6 Hz, 1H), 3.74 (s, 2H), 3.17 (d, J = 15.6 Hz, 1H), 1.90-2.14 (brs, 2H), 1.79 (d, J = 6.0 Hz, 3H), 1.38 (t, J = 7.2 Hz, 3H).

### Example 6: 3-((19R)-22-amino-3-ethyl-16-fluoro-10,19-dimethyl-3,4,9,10,11,23-hexaaza -20-oxapentacyclo[19.3.1.0^{8,12}.0^{13,18}.0^{2,6}]pentacosa-1(25),2(6),4,8(9),11,13(18),14,16,21(22),23 -decaen-5-yl)prop-2-yn-1-ol

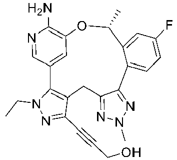

The synthetic method of Example 6 is the same as that of Example 1. LC-MS (ESI) [M+H]⁺: 474.1. ¹H NMR (400 MHz, CDCl₃), 7.52 (d, J = 1.2 Hz, 1H), 7.31 (dd, J = 9.6 Hz, 2.4 Hz, 1H), 7.09 (dd, J = 8.4 Hz, 5.6 Hz, 1H), 7.02 (dd, J = 8.4 Hz, 2.4 Hz, 1H), 6.21 (d, J = 1.2 Hz, 1H), 5.20-5.28 (m, 1H), 4.99 (s, 2H), 4.57 (s, 2H), 4.21 (s, 3H), 4.04-4.14 (m, 2H), 3.95 (d, J = 15.6 Hz, 1H), 3.16 (d, J = 15.6 Hz, 1H), 2.56-2.96 (brs, 1H), 1.78 (d, J = 6.4 Hz, 3H), 1.36 (t, J = 7.2 Hz, 3H).

### Example 7: (19R)-3-ethyl-16-fluoro-10,19-dimethyl-5-vinyl-20-oxa-3,4,10,11,23-pentaaz apentacyclo[19.3.1.0^{8,12}0.^{13,18}.0^{2,6}]pentacosa-1(25),2(6),4,8(9),11,13(18),14,16,21(22),23-decae n-22-amine

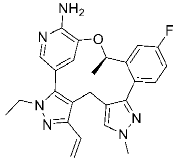

The synthetic method of Example 7 is the same as that of Example 2. LC-MS (ESI) [M+H]⁺: 445.3. ¹H NMR (400 MHz, CD₃OD), 7.54 (dd, J = 10.0 Hz, 2.8 Hz, 1H), 7.43 (s, 1H), 7.41 (d, J = 1.6 Hz, 1H), 7.21 (dd, J = 8.4 Hz, 6.0 Hz, 1H), 7.07 (td, J = 8.4 Hz, 2.8 Hz, 1H), 6.89 (dd, J = 17.6 Hz, 11.6 Hz, 1H), 6.49 (d, J = 1.6 Hz, 1H), 6.00 (dd, J = 17.6 Hz, 1.6 Hz, 1H), 5.38-5.44 (m, 2H), 4.02-4.19 (m, 2H), 3.90 (s, 3H), 3.75 (d, J = 16.0 Hz, 1H), 2.90 (d, J = 16.0 Hz, 1H), 1.80 (d, J = 6.4 Hz, 3H), 1.31 (t, J = 7.2 Hz, 3H).

### Example 8: 3-((3R)-24-amino-19-ethyl-6-fluoro-3,12-dimethyl-11,12,13,18,19,23-hexaaz a-2-oxapentacyclo[19.3.1.0^{4,9}.0^{10,14}.0^{16,20}]pentacosa-1(25),4(5),6,8,10(11),13,16(20),17,21(22), 23-decaen-17-yl)prop-2-ynamide

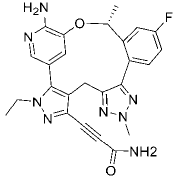

The synthetic method of Example 8 is the same as that of Example 1. LC-MS (ESI) [M+H]⁺: 487.2. ¹H NMR (400 MHz, CDCl₃), 7.51 (d, J = 1.6 Hz, 1H), 7.31 (dd, J = 9.6 Hz, 2.4 Hz, 1H), 7.08-7.12 (m, 1H), 7.04 (td, J = 8.0 Hz, 2.8 Hz, 1H), 6.18-6.25 (m, 2H), 6.09-6.17 (brs, 1H), 5.19-5.30 (m, 3H), 4.21 (s, 3H), 4.13 (q, J = 7.2 Hz, 2H), 4.01 (d, J = 15.6 Hz, 1H), 3.19 (d, J = 15.6 Hz, 1H), 1.78 (d, J = 6.0 Hz, 3H), 1.40 (t, J = 7.2 Hz, 3H).

### Example 9: (3R)-17-(azetidin-3-ylethynyl)-19-ethyl-6-fluoro-3,12-dimethyl-11,12,13,18, 19,23-hexaaza-2-oxapentacyclo[19.3.1.0^{4,9}.0^{10,14}.0^{16,20}]pentacosa-1(25),4(5),6,8,10(11),13,16(2 0),17,21(22),23-decaen-24-amine

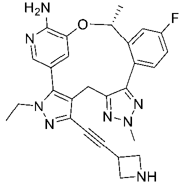

The synthetic method of Example 9 is the same as that of Example 5. LC-MS (ESI) [M+H]+: 499.4. ¹H NMR (400 MHz, CDCl₃), 7.50 (d, J = 1.6 Hz, 1H), 7.31 (dd, J = 9.6 Hz, 2.4 Hz, 1H), 7.12 (dd, J = 8.4 Hz, 5.6 Hz, 1H), 7.02 (dd, J = 8.0 Hz, 2.8 Hz, 1H), 6.21 (d, J = 1.6 Hz, 1H), 5.24-5.29 (m, 1H), 4.60-5.18 (m, 4H), 4.22 (s, 3H), 3.90-4.18 (m, 7H), 3.17 (d, J = 15.2 Hz, 1H), 1.78 (d, J = 6.0 Hz, 3H), 1.37 (t, J = 7.2 Hz, 3H).

### Example 10: (3R)-19-ethyl-6-fluoro-3, 12-dimethyl-17-(pyridin-4-ylethynyl)-11, 12, 13, 18, 19,23-hexaaza-2-oxapentacyclo[19.3.1.0^{4,9}.0^{10,14}.0^{16,20}]pentacosa-1(25),4(5),6,8,10(11),13,16(2 0),17,21(22),23-decaen-24-amine

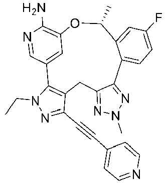

The synthetic method of Example 10 is the same as that of Example 1. LC-MS (ESI) [M+H]+: 521.3. ¹H NMR (400 MHz, CDCl₃), 8.61 (d, J = 6.0 Hz, 2H), 7.53 (d, J = 1.6 Hz, 1H), 7.47 (d, J = 6.0 Hz, 2H), 7.33 (dd, J = 9.6 Hz, 2.4 Hz, 1H), 7.14 (dd, J = 8.4 Hz, 5.6 Hz, 1H), 7.05 (td, J = 8.0 Hz, 2.8 Hz, 1H), 6.25 (d, J = 1.6 Hz, 1H), 5.25-5.32 (m, 1H), 4.90 (s, 2H), 4.22 (s, 3H), 4.12-4.19 (m, 2H), 4.02 (d, J = 15.6 Hz, 1H), 3.25 (d, J = 15.6 Hz, 1H), 1.81 (d, J = 6.4 Hz, 3H), 1.43 (t, J = 7.2 Hz, 3H).

### Example 11: (3R)-19-ethyl-6-fluoro-3, 12-dimethyl-17-(pyridin-3-ylethynyl)-11, 12, 13, 18, 19,23-hexaaza-2-oxapentacyclo[19.3.1.0^{4,9}.0^{10,14}.0^{16,20}]pentacosa-1(25),4(5),6,8,10(11),13,16(2 0),17,21(22),23-decaen-24-amine

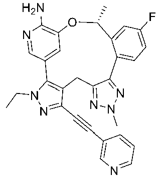

The synthetic method of Example 11 is the same as that of Example 1. LC-MS (ESI) [M+H]+: 521.3. ¹H NMR (400 MHz, CDCl₃), 8.85 (s, 1H), 8.56 (d, J = 4.4 Hz, 1H), 7.90 (dt, J = 8.0 Hz, 2.0 Hz, 1H), 7.54 (d, J = 1.2 Hz, 1H), 7.29-7.34 (m, 2H), 7.14 (dd, J = 8.4 Hz, 5.6 Hz, 1H), 7.05 (td, J = 8.0 Hz, 2.8 Hz, 1H), 6.27 (d, J = 1.2 Hz, 1H), 5.25-5.32 (m, 1H), 4.94 (s, 2H), 4.22 (s, 3H), 4.12-4.18 (m, 2H), 4.04 (d, J = 15.6 Hz, 1H), 3.25 (d, J = 15.6 Hz, 1H), 1.80 (d, J = 6.8 Hz, 3H), 1.42 (t, J = 7.2 Hz, 3H).

### Example 12: (3R)-19-ethyl-6-fluoro-3,12-dimethyl-17-(3-(methylamino)prop-1-yn-1-yl)-11,12,13,18,19,23-hexaaza-2-oxapentacyclo[19.3.1.0^{4,9}.0^{10,14}.0^{16,20}]pentacosa-1(25),4(5),6,8,10 (11),13,16(20),17,21(22),23-decaen-24-amine

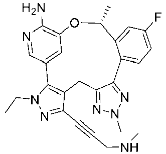

The synthetic method of Example 12 is the same as that of Example 5. LC-MS (ESI) [M+H]⁺: 487.3. ¹H NMR (400 MHz, CD₃OD), 7.60 (dd, J = 10.4 Hz, 2.8 Hz, 1H), 7.43 (d, J = 1.6 Hz, 1H), 7.17 (dd, J = 8.4 Hz, 6.0 Hz, 1H), 7.11 (td, J = 8.4 Hz, 2.8 Hz, 1H), 6.21 (d, J = 1.6 Hz, 1H), 5.34-5.41 (m, 1H), 4.21 (s, 2H), 4.19 (s, 3H), 4.03-4.18 (m, 2H), 3.96 (d, J = 15.6 Hz, 1H), 3.21 (d, J = 15.6 Hz, 1H), 2.88 (s, 3H), 1.80 (d, J = 6.0 Hz, 3H), 1.32 (t, J = 7.2 Hz, 3H).

### Example 13: (3R)-19-ethyl-6-fluoro-17-(1-fluorovinyl)-3,12-dimethyl-11,12,13,18,19,23 -hexaaza-2-oxapentacyclo[19.3.1.0^{4,9}.0^{10,14}.0^{16,20}]pentacosa-1(25),4(5),6,8,10(11),13,16(20),17, 21(22),23-decaen-24-amine

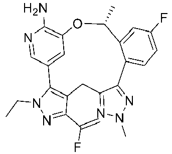

The synthetic method of Example 13 is the same as that of Example 1. LC-MS (ESI) [M+H]⁺: 464.2. ¹H NMR (400 MHz, CDCl₃), 7.52 (d, J = 1.2 Hz, 1H), 7.31 (dd, J = 9.6 Hz, 2.4Hz, 1H), 7.10 (dd, J = 8.4 Hz, 5.6 Hz, 1H), 7.04 (td, J = 8.0 Hz, 2.8 Hz, 1H), 6.29 (d, J = 1.2 Hz, 1H), 5.52 (dd, J = 50.8 Hz, 3.2 Hz, 1H), 5.14-5.43 (m, 3H), 5.02 (dd, J = 18.0 Hz, 3.2 Hz, 1H), 4.22 (s, 3H), 4.06-4.17 (m, 2H), 3.98 (d, J = 16.0 Hz, 1H), 3.18 (dd, J = 16.0 Hz, 1.6 Hz, 1H), 1.81 (d, J = 6.8 Hz, 3H), 1.37 (t, J = 7.2 Hz, 3H).

### Example 14: (3R)-17-(3,3-difluoroprop-1-en-2-yl)-19-ethyl-6-fluoro-3,12-dimethyl-11,1 2,13,18,19,23-hexaaza-2-oxapentacyclo[19.3.1.0^{4,9}.0^{10,14}.0^{16,20}]pentacosa-1(25),4(5),6,8,10(11), 13,16(20),17,21(22),23-decaen-24-amine

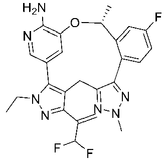

The synthetic method of Example 14 is the same as that of Example 2. LC-MS (ESI) [M+H]+: 496.2.

### Example 15: (3R)-17-(2,2-difluorovinyl)-19-ethyl-6-fluoro-3, 12-dimethyl-11, 12, 13, 18, 1 9,23-hexaaza-2-oxapentacyclo[19.3.1.0^{4,9}.0^{10,14}.0^{16,20}]pentacosa-1(25),4(5),6,8,10(11),13,16(2 0),17,21(22),23-decaen-24-amine

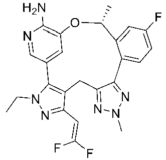

The synthetic method of Example 15 is the same as that of Example 2. LC-MS (ESI) [M+H]+: 482.2.

### Example 16: 3-(((3R)-24-amino-19-ethyl-6-fluoro-3,12-dimethyl-11,12,13,18,19,23-hexa aza-2-oxapentacyclo[19.3.1.0^{4,9}.0^{10,14}.0^{16,20}]pentacosa-1(25),4(5),6,8,10(11),13,16(20),17,21(2 2),23-decaen-17-yl)ethynyl)azetidin-3-ol

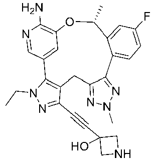

The synthetic method of Example 16 is the same as that of Example 5. LC-MS (ESI) [M+H]+: 515.2. ¹H NMR (400 MHz, CDCl₃+CD₃OD), 7.39 (d, J = 1.6 Hz, 1H), 7.29 (dd, J = 9.6 Hz, 2.4 Hz, 1H), 7.09 (dd, J = 8.8 Hz, 5.6 Hz, 1H), 7.01 (td, J = 8.0 Hz, 2.4 Hz, 1H), 6.15 (d, J = 1.6 Hz, 1H), 5.16-5.23 (m, 1H), 4.27-4.35 (m, 4H), 4.17 (s, 3H), 4.03-4.09 (m, 2H), 3.92 (d, J = 15.6 Hz, 1H), 3.15 (d, J = 15.6 Hz, 1H), 1.75 (d, J = 6.0 Hz, 3H), 1.33 (t, J = 7.2 Hz, 3H).

### Example 17: (3R)-17-(azetidin-3-ylidenemethyl)-19-ethyl-6-fluoro-3,1 2-dimethyl-11, 12, 13,18,19,23-hexaaza-2-oxapentacyclo[19.3.1.0^{4.9}.0^{10,14}.0^{16,20}]pentacosa-1(25),4(5),6,8,10(11),1 3,16(20),17,21(22),23-decaen-24-amine

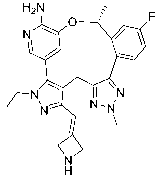

The synthetic method of Example 17 is the same as that of Example 2. LC-MS (ESI) [M+H]+: 487.2. ¹H NMR (400 MHz, CDCl₃), 7.51 (d, J = 1.6 Hz, 1H), 7.32 (dd, J = 10.0 Hz, 2.8 Hz, 1H), 7.07 (dd, J = 8.4 Hz, 6.0 Hz, 1H), 7.02 (td, J = 8.4 Hz, 2.8 Hz, 1H), 6.41-6.45 (m, 1H), 6.24 (d, J = 1.6 Hz, 1H), 5.22-5.28 (m, 1H), 4.73-4.84 (m, 3H), 4.56-4.67 (m, 2H), 4.44-4.51 (m, 1H), 4.20 (s, 3H), 4.05 (q, J = 7.2 Hz, 2H), 3.74 (d, J = 16.0 Hz, 1H), 3.15 (d, J = 16.0 Hz, 1H), 1.78 (d, J = 6.0 Hz, 3H), 1.35 (t, J = 7.2 Hz, 3H).

### Example 18: (3R)-19-ethyl-6-fluoro-3,12-dimethyl-17-((1-methyl azetidin-3-ylidene)me thyl)-11,12,13,18,19,23-hexaaza-2-oxapentacyclo[19.3.1.0^{4,9}.0^{10,14}.0^{16,20}]pentacosa-1(25),4(5), 6,8,10(11),13,16(20),17,21(22),23-decaen-24-amine

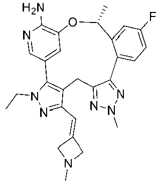

The synthetic method of Example 18 is the same as that of Example 2. LC-MS (ESI) [M+H]+: 501.2. ¹H NMR (400 MHz, CDCl₃), 7.50 (d, J = 1.6 Hz, 1H), 7.31 (dd, J = 9.6 Hz, 2.4 Hz, 1H), 7.06 (dd, J = 8.4 Hz, 5.6 Hz, 1H), 7.01 (td, J = 8.0 Hz, 2.8 Hz, 1H), 6.42-6.45 (m, 1H), 6.24 (d, J = 1.6 Hz, 1H), 5.22-5.27 (m, 1H), 4.79 (s, 2H), 4.31-4.44 (m, 2H), 4.19 (s, 3H), 4.18 (s, 2H), 4.04 (q, J = 7.2 Hz, 2H), 3.72 (d, J = 15.6 Hz, 1H), 3.14 (d, J = 15.6 Hz, 1H), 2.57 (s, 3H), 1.78 (d, J = 6.0 Hz, 3H), 1.35 (t, J = 7.2 Hz, 3H).

### Example 19: (3R)-17-(1-chlorovinyl)-19-ethyl-6-fluoro-3,12-dimethyl-11,12,13,18,19,23 -hexaaza-2-oxapentacyclo[19.3.1.0^{4,9}.0^{10,14}.0^{16,20}]pentacosa-1(25),4(5),6,8,10(11),13,16(20),17, 21(22),23-decaen-24-amine

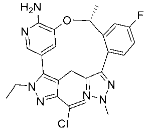

The synthetic method of Example 19 is the same as that of Example 2. LC-MS (ESI) [M+H]+: 480.3.

### Example 20: (3R)-17-((aminocyclopropyl)ethynyl)-19-ethyl-6-fluoro-3,12-dimethyl-11,1 2,13,18,19,23-hexaaza-2-oxapentacyclo[19.3.1.0^{4,9}.0^{10,14}.0^{16,20}]pentacosa-1(25),4(5),6,8,10(11), 13,16(20),17,21(22),23-decaen-24-amine

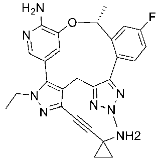

The synthetic method of Example 20 is the same as that of Example 5. LC-MS (ESI) [M+H]+: 499.3. ¹H NMR (400 MHz, CDCl₃), 7.47 (d, J = 1.6 Hz, 1H), 7.31 (dd, J = 9.6 Hz, 2.4 Hz, 1H), 7.11 (dd, J = 8.4 Hz, 5.6 Hz, 1H), 7.02 (td, J = 8.0 Hz, 2.8 Hz, 1H), 6.21 (d, J = 1.6 Hz, 1H), 5.22-5.29 (m, 1H), 5.05 (s, 2H), 4.22 (s, 3H), 4.03-4.13 (m, 2H), 3.91 (d, J = 15.6 Hz, 1H), 3.15 (d, J = 15.6 Hz, 1H), 2.46-2.99 (brs, 2H), 1.78 (d, J = 6.0 Hz, 3H), 1.36 (t, J = 7.2 Hz, 3H), 1.12-1.20 (m, 4H).

### Example 21: 4-((3R)-24-amino-19-ethyl-6-fluoro-3,12-dimethyl-11,12,13,18,19,23-hexa aza-2-oxapentacyclo[19.3.1.0^{4,9}.0^{10,14}.0^{16,20}]pentacosa-1(25),4(5),6,8,10(11),13,16(20),17,21(2 2),23-decaen-17-yl)-2-methylbut-3-yn-2-ol

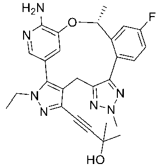

The synthetic method of Example 21 is the same as that of Example 1. LC-MS (ESI) [M+H]+: 502.3.

### Example 22: 3-(((3R)-24-amino-19-ethyl-6-fluoro-3,12-dimethyl-11,12,13,18,19,23-hexa aza-2-oxapentacyclo[19.3.1.0^{4,9}.0^{10,14}.0^{16,20}]pentacosa-1(25),4(5),6,8,10(11),13,16(20),17,21(2 2),23-decaen-17-yl)ethynyl)oxetan-3-ol

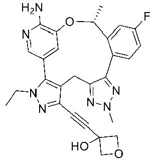

The synthetic method of Example 22 is the same as that of Example 1. LC-MS (ESI) [M+H]+: 516.2. ¹H NMR (400 MHz, CDCl₃), 7.53 (d, J = 1.6 Hz, 1H), 7.32 (dd, J = 9.6 Hz, 2.4 Hz, 1H), 7.09 (dd, J = 8.4 Hz, 5.6 Hz, 1H), 7.03 (td, J = 8.4 Hz, 2.8 Hz, 1H), 6.21 (d, J = 1.6 Hz, 1H), 5.22-5.29 (m, 1H), 5.01-5.04 (m, 2H), 4.87 (s, 2H), 4.81-4.84 (m, 2H), 4.21 (s, 3H), 4.05-4.15 (m, 2H), 3.89 (d, J = 15.6 Hz, 1H), 3.14 (d, J = 15.6 Hz, 1H), 1.78 (d, J = 6.0 Hz, 3H), 1.37 (t, J = 7.2 Hz, 3H).

### Example 23: (3R)-19-ethyl-6-fluoro-3,12-dimethyl-17-(oxetan-3-ylethynyl)-11,12,13,18, 19,23-hexaaza-2-oxapentacyclo[19.3.1.0^{4,9}.0^{10,14}.0^{16,20}]pentacosa-1(25),4(5),6,8,10(11),13,16(2 0),17,21(22),23-decaen-24-amine

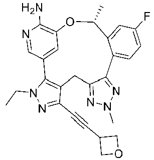

The synthetic method of Example 23 is the same as that of Example 1. LC-MS (ESI) [M+H]+: 500.3. ¹H NMR (400 MHz, CDCl₃), 7.51 (d, J = 1.2 Hz, 1H), 7.32 (dd, J = 10.0 Hz, 2.8 Hz, 1H), 7.12 (dd, J = 8.4 Hz, 5.6 Hz, 1H), 7.03 (td, J = 8.0 Hz, 2.8 Hz, 1H), 6.23 (d, J = 1.2 Hz, 1H), 5.24-5.30 (m, 1H), 4.88-4.94 (m, 4H), 4.86 (s, 2H), 4.23 (s, 3H), 4.16-4.20 (m, 1H), 4.07-4.14 (m, 2H), 3.94 (d, J = 15.6 Hz, 1H), 3.18 (d, J = 15.6 Hz, 1H), 1.79 (d, J = 6.4 Hz, 3H), 1.39 (t, J = 7.2 Hz, 3H).

### Example 24: (3R)-19-ethyl-6-fluoro-3,12-dimethyl-17-((1E)-2-(pyridin-4-yl)vinyl)-11,12, 13,18,19,23-hexaaza-2-oxapentacyclo[19.3.1.0^{4,9}.0^{10,14}.0^{16,20}]pentacosa-1(25),4(5),6,8,10(11),1 3,16(20),17,21(22),23-decaen-24-amine

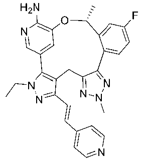

The synthetic method of Example 24 is the same as that of Example 2. LC-MS (ESI) [M+H]+: 523.2. ¹H NMR (400 MHz, CDCl₃), 8.32-8.82 (brs, 2H), 7.42-7.60 (m, 5H), 7.34 (dd, J = 9.6 Hz, 2.8 Hz, 1H), 7.11 (dd, J = 8.4 Hz, 5.6 Hz, 1H), 7.04 (td, J = 8.0 Hz, 2.8 Hz, 1H), 6.30 (s, 1H), 5.25-5.32 (m, 1H), 5.00 (s, 2H), 4.22 (s, 3H), 4.10-4.21 (m, 2H), 3.91 (d, J = 16.0 Hz, 1H), 3.26 (d, J = 16.0 Hz, 1H), 1.81 (d, J = 6.4 Hz, 3H), 1.42 (t, J = 7.2 Hz, 3H).

### Example 25: (3R)-19-ethyl-6-fluoro-3,12-dimethyl-17-((1E)-2-(pyridin-3-yl)vinyl)-11,12, 13,18,19,23-hexaaza-2-oxapentacyclo[19.3.1.0^{4,9}.0^{10,14}.0^{16,20}]pentacosa-1(25),4(5),6,8,10(11),1 3,16(20),17,21(22),23-decaen-24-amine

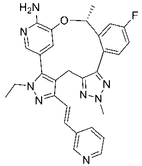

The synthetic method of Example 25 is the same as that of Example 2. LC-MS (ESI) [M+H]+: 523.2. ¹H NMR (400 MHz, CDCl₃), 8.80 (s, 1H), 8.49 (d, J = 4.4 Hz, 1H), 7.92 (d, J = 8.0 Hz, 1H), 7.53-7.58 (m, 2H), 7.40 (d, J = 16.4 Hz, 1H), 7.30-7.35 (m, 2H), 7.12 (dd, J = 8.4 Hz, 5.6 Hz, 1H), 7.04 (td, J = 8.0 Hz, 2.8 Hz, 1H), 6.32 (d, J = 1.6 Hz, 1H), 5.25-5.32 (m, 1H), 5.15 (s, 2H), 4.22 (s, 3H), 4.08-4.19 (m, 2H), 3.92 (d, J = 16.0 Hz, 1H), 3.25 (d, J = 16.0 Hz, 1H), 1.80 (d, J = 6.4 Hz, 3H), 1.41 (t, J = 7.2 Hz, 3H).

### Example 26: (3R)-17-(cyclopropylethynyl)-19-ethyl-6-fluoro-3,12-dimethyl-11,12,13,18, 19,23-hexaaza-2-oxapentacyclo[19.3.1.0^{4,9}.0^{10,14}.0^{16,20}]pentacosa-1(25),4(5),6,8,10(11),13,16(2 0),17,21(22),23-decaen-24-amine

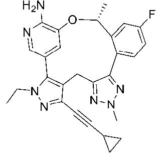

The synthetic method of Example 26 is the same as that of Example 1. LC-MS (ESI) [M+H]+: 484.3.

### Example 27: (3R)-19-ethyl-17-ethynyl-6-fluoro-3,12-dimethyl-2-oxa-11,12,18,19,23-pen taazapentacyclo[19.3.1.0^{4,9}.0^{10,14}.0^{16,20}]pentacosa-1(25),4(5),6,8,10(11),13,16(20),17,21(22),23-decaen-24-amine

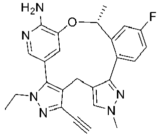

The synthetic method of Example 27 is the same as that of Example 1. LC-MS (ESI) [M+H]+: 443.2. ¹H NMR (400 MHz, CD₃OD), 7.55 (s, 1H), 7.53 (dd, J = 10.4 Hz, 2.8 Hz, 1H), 7.43 (s, 1H), 7.19 (dd, J = 8.4 Hz, 5.6 Hz, 1H), 7.08 (td, J = 8.4 Hz, 2.8 Hz, 1H), 6.47 (s, 1H), 5.38-5.45 (m, 1H), 4.03-4.18 (m, 2H), 3.92 (s, 3H), 3.86 (s, 1H), 3.78 (d, J = 15.6 Hz, 1H), 2.90 (d, J = 15.6 Hz, 1H), 1.81 (d, J = 6.4 Hz, 3H), 1.32 (t, J = 7.2 Hz, 3H).

### Example 28: 3-((3R)-24-amino-19-ethyl-6-fluoro-3,12-dimethyl-2-oxa-11,12,18,19,23-pe ntaazapentacyclo[19.3.1.0^{4,9}.0^{10,14}.0^{16.20}]pentacosa-1(25),4(5),6,8,10(11),13,16(20),17,21(22),2 3-decaen-17-yl)prop-2-yn-1-ol

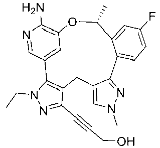

The synthetic method of Example 28 is the same as that of Example 1. LC-MS (ESI) [M+H]+: 473.4. ¹H NMR (400 MHz, CDCl₃), 7.52 (d, J = 1.6 Hz, 1H), 7.40 (s, 1H), 7.28 (dd, J = 10.0 Hz, 2.8 Hz, 1H), 7.10 (dd, J = 8.4 Hz, 5.6 Hz, 1H), 6.98 (td, J = 8.0 Hz, 2.8 Hz, 1H), 6.50 (d, J = 1.6 Hz, 1H), 5.36-5.42 (m, 1H), 4.85 (s, 2H), 4.56 (s, 2H), 4.07-4.13 (m, 2H), 3.90 (s, 3H), 3.73 (d, J = 15.2 Hz, 1H), 2.87 (d, J = 15.2 Hz, 1H), 1.78 (d, J = 6.0 Hz, 3H), 1.38 (t, J = 7.2 Hz, 3H).

### Example 29: (3R)-17-(3-aminoprop-1-yn-1-yl)-19-ethyl-6-fluoro-3,12-dimethyl-2-oxa-1 1,12,18,19,23-pentaazapentacyclo[19.3.1.0^{4,9}.0^{10,14}.0^{16,20}]pentacosa-1(25),4(5),6,8,10(11),13,16 (20),17,21(22),23-decaen-24-amine

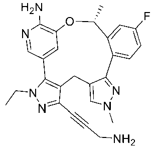

The synthetic method of Example 29 is the same as that of Example 5. LC-MS (ESI) [M+H]+: 472.3. ¹H NMR (400 MHz, CDCl₃), 7.50 (d, J = 1.6 Hz, 1H), 7.43 (s, 1H), 7.28 (dd, J = 10.0 Hz, 2.4 Hz, 1H), 7.10 (dd, J = 8.4 Hz, 5.6 Hz, 1H), 6.97 (td, J = 8.0 Hz, 2.4 Hz, 1H), 6.49 (d, J = 1.6 Hz, 1H), 5.35-5.42 (m, 1H), 4.92 (s, 2H), 4.06-4.14 (m, 2H), 3.91 (s, 3H), 3.85 (s, 2H), 3.72 (d, J = 15.6 Hz, 1H), 3.26-3.63 (brs, 2H), 2.87 (d, J = 15.6 Hz, 1H), 1.76 (d, J = 6.4 Hz, 3H), 1.37 (t, J = 7.2 Hz, 3H).

### Example 30: (3R)-6-fluoro-3,12-dimethyl-18-(prop-2-yn-1-yl)-2-oxa-11,12,18,19,23-pen taazapentacyclo[19.3.1.0^{4,9}.0^{10,14}.0^{16,20}]pentacosa-1(25),4(5),6,8,10(11),13,16(17),19,21(22),23-decaen-24-amine

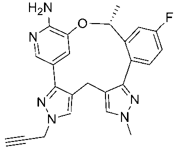

The synthetic method of Example 30 is the same as that of Example 1. LC-MS (ESI) [M+H]+: 429.3. ¹H NMR (400 MHz, CDCl₃), 7.69 (s, 1H), 7.65 (d, J = 1.2 Hz, 1H), 7.28-7.31 (m, 2H), 7.20-7.23 (m, 1H), 7.08-7.13 (m, 1H), 6.94 (d, J = 1.2 Hz, 1H), 5.28-5.35 (m, 1H), 4.91 (d, J = 2.4 Hz, 2H), 3.92 (s, 3H), 3.76 (d, J = 16.0 Hz, 1H), 3.09 (d, J = 16.0 Hz, 1H), 2.57 (t, J = 2.4 Hz, 1H), 1.85 (d, J = 6.4 Hz, 3H).

### Example 31: (3R)-13-chloro-19-ethyl-17-ethynyl-6-fluoro-3,12-dimethyl-2-oxa-11,12,18, 19,23-pentaazapentacyclo[19.3.1.0^{4,9}.0^{10,14}.0^{16,20}]pentacosa-1(25),4(5),6,8,10(11),13,16(20),17, 21(22),23-decaen-24-amine

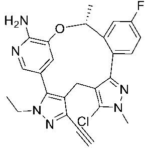

The synthetic method of Example 31 is the same as that of Example 1. LC-MS (ESI) [M+H]+: 477.3.

### Example 32: (3R)-19-ethyl-6-fluoro-3-methyl-12-(prop-2-yn-1-yl)-11, 12, 13, 18, 19,23-he xaaza-2-oxapentacyclo[19.3.1.0^{4,9}.0^{10,14}.0^{16,20}]pentacosa-1(25),4(5),6,8,10(11),13,16(20),17,21 (22),23-decaen-24-amine

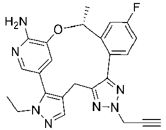

The synthetic method of Example 32 is the same as that of Example 1. LC-MS (ESI) [M+H]+: 444.3.

### Example 33: (3R)-12-cyclopropyl-19-ethyl-17-ethynyl-6-fluoro-3-methyl-11,12,13,18,19, 23-hexaaza-2-oxapentacyclo[19.3.1.0^{4,9}.0^{10,14}.0^{16,20}]pentacosa-1(25),4(5),6,8,10(11),13,16(20), 17,21(22),23-decaen-24-amine

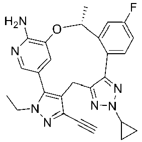

The synthetic method of Example 33 is the same as that of Example 1. LC-MS (ESI) [M+H]+: 444.3.

### Example 34: (3R)-19-ethyl-6-fluoro-12-methyl-3-(prop-2-yn-1-yl)-11, 12, 13, 18, 19,23-he xaaza-2-oxapentacyclo[19.3.1.0^{4,9}.0^{10,14}.0^{16,20}]pentacosa-1(25),4(5),6,8,10(11),13,16(20),17,21 (22),23-decaen-24-amine

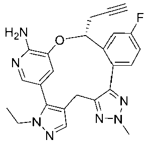

The synthetic method of Example 34 is the same as that of Example 1. LC-MS (ESI) [M+H]+: 444.2.

### Example 35: (3R)-3,19-diethyl-6-fluoro-12-methyl-11,12,13,18,19,23-hexaaza-2-oxapent acyclo[19.3.1.0^{4,9}.0^{10,14}.0^{16,20}]pentacosa-1(25),4(5),6,8,10(11),13,16(20),17,21(22),23-decaen-2 4-amine

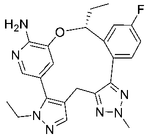

The synthetic method of Example 35 is the same as that of Example 1. LC-MS (ESI) [M+H]+: 434.3. ¹H NMR (400 MHz, DMSO), 7.70 (dd, J = 10.4 Hz, 2.4 Hz, 1H), 7.55 (s, 1H), 7.41 (d, J = 1.6 Hz, 1H), 7.14-7.21 (m, 2H), 6.14 (s, 2H), 6.04 (d, J = 1.6 Hz, 1H), 4.97 (td, J = 7.2 Hz, 1.6 Hz, 1H), 4.13 (s, 3H), 3.92-4.02 (m, 2H), 3.75 (d, J = 15.6 Hz, 1H), 2.94 (d, J = 15.6 Hz, 1H), 2.13-2.24 (m, 1H), 1.94-2.04 (m, 1H), 1.24 (t, J = 7.2 Hz, 3H), 0.94 (t, J = 7.2 Hz, 3H).

### Example 36: (3S)-19-ethyl-6-fluoro-12-methyl-3-(trifluoromethyl)-11, 12, 13, 18, 19,23-he xaaza-2-oxapentacyclo[19.3.1.0^{4,9}.0^{10,14}.0^{16,20}]pentacosa-1(25),4(5),6,8,10(11),13,16(20),17,21 (22),23-decaen-24-amine

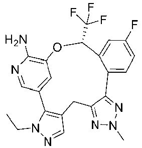

The synthetic method of Example 36 is the same as that of Example 1. LC-MS (ESI) [M+H]+: 474.2. ¹H NMR (400 MHz, CDCl₃), 7.71 (s, 1H), 7.58 (s, 1H), 7.49-7.54 (m, 1H), 7.18-7.22 (m, 2H), 6.23 (s, 1H), 5.47-5.52 (m, 1H), 5.14 (s, 2H), 4.24 (s, 3H), 4.02-4.16 (m, 2H), 3.83 (d, J = 15.6 Hz, 1H), 3.16 (d, J = 15.6 Hz, 1H), 1.40 (t, J = 7.2 Hz, 3H).

### Example 37: (3R)-19-ethyl-17-ethynyl-6-fluoro-3,12-dimethyl-2-oxa-10,11,18,19,23-pen taazapentacyclo[19.3.1.0^{4,9}.0^{10,14}.0^{16,20}]pentacosa-1(25),4(5),6,8,11,13,16(20),17,21(22),23-dec aen-24-amine

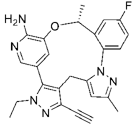

The synthetic method of Example 37 is the same as that of Example 1. LC-MS (ESI) [M+H]+: 443.2.

### Example 38: (3R)-19-ethyl-17-ethynyl-6-fluoro-3,12-dimethyl-2,13-dioxa-11,18,19,23-te traazapentacyclo[19.3.1.0^{4,9}.0^{10,14}.0^{16,20}]pentacosa-1(25),4(5),6,8,10(14),11,16(20),17,21(22),23 -decaen-24-amine

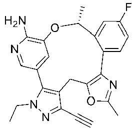

The synthetic method of Example 38 is the same as that of Example 1. LC-MS (ESI) [M+H]+: 444.2. ¹H NMR (400 MHz, CDCl₃), 7.51 (s, 1H), 7.30 (dd, J = 9.6 Hz, 2.8 Hz, 1H), 7.07 (dd, J = 8.4 Hz, 5.6 Hz, 1H), 6.98 (td, J = 8.4 Hz, 2.8 Hz, 1H), 6.57 (d, J = 1.2 Hz, 1H), 5.39-5.45 (m, 1H), 4.86 (s, 2H), 4.07-4.17 (m, 2H), 3.97 (d, J = 15.6 Hz, 1H), 3.30 (d, J = 15.6 Hz, 1H), 3.29 (s, 1H), 2.49 (s, 3H), 1.80 (d, J = 6.4 Hz, 3H), 1.41 (t, J = 7.2 Hz, 3H).

### Example 39: (3R)-19-ethyl-17-ethynyl-6-fluoro-3,12-dimethyl-2-oxa-11,18,19,23-tetraaz a-13-thiapentacyclo[19.3.1.0^{4,9}.0^{10,14}.0^{16,20}]pentacosa-1(25),4(5),6,8,10(14),11,16(20),17,21(22), 23-decaen-24-amine

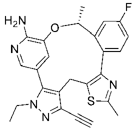

The synthetic method of Example 39 is the same as that of Example 1. LC-MS (ESI) [M+H]+: 460.3. ¹H NMR (400 MHz, CD₃OD), 7.55 (dd, J = 10.0 Hz, 2.8 Hz, 1H), 7.45 (d, J = 1.6 Hz, 1H), 7.22 (dd, J = 8.4 Hz, 6.0 Hz, 1H), 7.08 (td, J = 8.4 Hz, 2.8 Hz, 1H), 6.50 (d, J = 1.6 Hz, 1H), 5.23-5.30 (m, 1H), 4.06-4.20 (m, 2H), 4.03 (d, J = 16.0 Hz, 1H), 3.89 (s, 1H), 3.37 (d, J = 16.0 Hz, 1H), 2.69 (s, 3H), 1.82 (d, J = 6.4 Hz, 3H), 1.33 (t, J = 7.2 Hz, 3H).

### Example 40: (3R)-19-ethyl-17-ethynyl-6-fluoro-3,12-dimethyl-2,11-dioxa-13,18,19,23-te traazapentacyclo[19.3.1.0^{4,9}.0^{10,14}.0^{16,20}]pentacosa-1(25),4(5),6,8,10(14),12,16(20),17,21(22),23 -decaen-24-amine

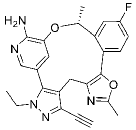

The synthetic method of Example 40 is the same as that of Example 1. LC-MS (ESI) [M+H]+: 444.3. ¹H NMR (400 MHz, CDCl₃), 7.52 (d, J = 1.6 Hz, 1H), 7.32 (dd, J = 10.0 Hz, 2.8 Hz, 1H), 7.15 (dd, J = 8.4 Hz, 5.6 Hz, 1H), 7.04 (td, J = 8.4 Hz, 2.8 Hz, 1H), 6.71 (d, J = 1.6 Hz, 1H), 4.98-5.30 (m, 3H), 4.02-4.15 (m, 2H), 3.85 (d, J = 15.2 Hz, 1H), 3.32 (s, 1H), 3.09 (d, J = 15.2 Hz, 1H), 2.51 (s, 3H), 1.79 (d, J = 6.4 Hz, 3H), 1.37 (t, J = 7.2 Hz, 3H).

### Example 41: (3R)-19-ethyl-17-ethynyl-6-fluoro-3,12-dimethyl-2-oxa-13,18,19,23-tetraaz a-11-thiapentacyclo[19.3.1.0^{4,9}.0^{10,14}.0^{16,20}]pentacosa-1(25),4(5),6,8,10(14),12,16(20),17,21(22), 23-decaen-24-amine

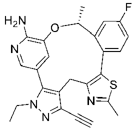

The synthetic method of Example 41 is the same as that of Example 1. LC-MS (ESI) [M+H]⁺: 460.3. ¹H NMR (400 MHz, CDCl₃), 7.54 (s, 1H), 7.27 (dd, J = 9.6 Hz, 2.8 Hz, 1H), 7.15 (dd, J = 8.4 Hz, 5.6 Hz, 1H), 7.02 (td, J = 8.4 Hz, 2.8 Hz, 1H), 6.75 (s, 1H), 5.44-5.50 (m, 1H), 4.87 (s, 2H), 4.15 (q, J = 7.2 Hz, 2H), 3.92 (d, J = 15.2 Hz, 1H), 3.29 (s, 1H), 3.23 (d, J = 15.2 Hz, 1H), 2.72 (s, 3H), 1.74 (d, J = 6.4 Hz, 3H), 1.43 (t, J = 7.2 Hz, 3H).

### Example 42: (3R)-17-chloro-19-ethyl-6,15,15-trifluoro-3,12-dimethyl-11,12,13,18,19,23-hexaaza-2-oxapentacyclo[19.3.1.0^{4,9}.0^{10,14}.0^{16,20}]pentacosa-1(25),4(5),6,8,10(11),13,16(20),17,2 1(22),23-decaen-24-amine

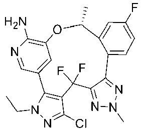

The synthetic method of Example 42 is the same as that of Example 1. LC-MS (ESI) [M+H]+: 490.2.

### Example 43: (3R)-19-ethyl-17-ethynyl-6,15,15-trifluoro-3,12-dimethyl-2-oxa-11,12,18,1 9,23-pentaazapentacyclo[19.3.1.0^{4,9}.0^{10,14}.0^{16,20}]pentacosa-1(25),4(5),6,8,10(11),13,16(20),17,2 1(22),23-decaen-24-amine

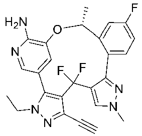

The synthetic method of Example 43 is the same as that of Example 1. LC-MS (ESI) [M+H]+: 479.2.

### Example 44: (3R)-17-chloro-20-ethyl-6-fluoro-3,12-dimethyl-2-oxa-11,12,19,24-tetraaza pentacyclo[20.3.1.0^{4,9}.0^{10,14}.0^{16,21}]hexacosa-1(26),4(5),6,8,10(11),13,16(17),18,20,22(23),24-un decaen-25-amine

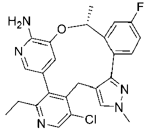

The synthetic method of Example 44 is the same as that of Example 1. LC-MS (ESI) [M+H]⁺: 479.2.

### Example 45: (19R)-3-ethyl-16-fluoro-10,19-dimethyl-5-(2-methylprop-1-en-1-yl)-20-oxa -3,4,10,11,23-pentaazapentacyclo[19.3.10^{8,12}.0^{13,18}.0^{2,6}]pentacosa-1(25),2(6),4,8(9),11,13(18),1 4,16,21(22),23-decaen-22-amine

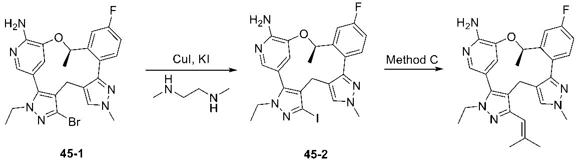

The synthetic method of compound **45-1** is the same as that of compound **1-10.** Under nitrogen protection, to a solution of compound **45-1** (1.7 g) in 1,4-dioxane (35 mL) were added copper(I) iodide (583 mg), potassium iodide (5.64 g) and N,N'-dimethylethylenediamine (0.56 mL). The reaction solution was heated under microwave to 135 °C and reacted at this temperature for 2 hours. The reaction solution was filtered, and the resulting filtrate was concentrated under reduced pressure and purified through flash silica gel chromatography (dichloromethane:ethyl acetate = 3:1 to 1:1) to obtain compound **45-2** (1.3 g). Compound **45-2** was used as the starting material to synthesize Example 45 through General Synthesis Method C. LC-MS (ESI) [M+H]⁺: 473.3. ¹H NMR (400 MHz, CDCl₃), 7.47 (d, J = 1.2 Hz, 1H), 7.28 (dd, J = 10.0 Hz, 2.8 Hz, 1H), 7.22 (s, 1H), 7.13 (dd, J = 8.4 Hz, 5.6 Hz, 1H), 7.00 (td, J = 8.4 Hz, 2.8 Hz, 1H), 6.56 (d, J = 1.2 Hz, 1H), 6.26 (s, 1H), 5.36-5.43 (m, 1H), 5.16 (s, 2H), 4.10 (q, J = 7.2 Hz, 2H), 3.90 (s, 3H), 3.57 (d, J = 16.0 Hz, 1H), 2.86 (d, J = 16.0 Hz, 1H), 2.15 (s, 3H), 1.99 (s, 3H), 1.78 (d, J = 6.0 Hz, 3H), 1.40 (t, J = 7.2 Hz, 3H).

### Example 46: (19R)-3-ethyl-16-fluoro-10,19-dimethyl-5-(prop-1-en-2-yl)-20-oxa-3,4,10, 11,23-pentaazapentacyclo[19.3.1.0^{8,12}.0^{13,18}.0^{2,6}]pentacosa-1(25),2(6),4,8(9),11,13(18),14,16,21 (22),23-decaen-22-amine

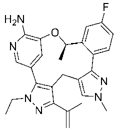

The synthetic method of Example 46 is the same as that of Example 45. LC-MS (ESI) [M+H]⁺: 459.4. ¹H NMR (400 MHz, CDCl₃), 7.38 (s, 1H), 7.32 (s, 1H), 7.28 (d, J = 10.0 Hz, 2.4 Hz, 1H), 7.17 (dd, J = 8.4 Hz, 5.6 Hz, 1H), 7.03 (td, J = 8.0 Hz, 2.8 Hz, 1H), 6.62 (s, 1H), 5.70-5.89 (brs, 2H), 5.59 (s, 1H), 5.40-5.47 (m, 1H), 5.29 (s, 1H), 4.04-4.16 (m, 2H), 3.91 (s, 3H), 3.80 (d, J = 16.0 Hz, 1H), 2.94 (d, J = 16.0 Hz, 1H), 2.27 (s, 3H), 1.81 (d, J = 6.4 Hz, 3H), 1.38 (t, J = 7.2 Hz, 3H).

### Example 47: (2R)-4-((19R)-22-amino-3-ethyl-16-fluoro-10,19-dimethyl-20-oxa-3,4,10,1 1,23-pentaazapentacyclo[19.3.1.0^{8,12}.0^{13,18}.0^{2,6}]pentacosa-1(25),2(6),4,8(9),11,13(18),14,16,21 (22),23-decaen-5-yl)but-3-yn-2-ol

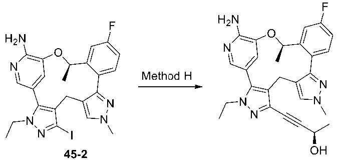

Under nitrogen protection, to a solution of compound **45-2** (100 mg), Pd(PPh₃)₂Cl₂ (17 mg), triethylamine (51 µL) and copper(I) iodide (7 mg) in DMF (2 mL) was added compound **17** (30 mg), and the reaction solution was heated to 80 °C and reacted overnight. The reaction solution was concentrated under reduced pressure, and the residue was purified through silica gel chromatography (dichloromethane : methanol =40:1) to obtain the target compound (53 mg). LC-MS (ESI) [M+H]⁺: 487.4. ¹H NMR (400 MHz, CD₃OD), 7.59 (s, 1H), 7.53 (dd, J = 10.0 Hz, 2.4 Hz, 1H), 7.43 (s, 1H), 7.19 (d, J = 8.4 Hz, 5.6 Hz, 1H), 7.08 (td, J = 8.0 Hz, 2.8 Hz, 1H), 6.48 (d, J = 1.2 Hz, 1H), 5.38-5.45 (m, 1H), 4.78 (q, J = 6.8 Hz, 1H), 4.02-4.17 (m, 2H), 3.92 (s, 3H), 3.77 (d, J = 15.6 Hz, 1H), 2.88 (d, J = 15.6 Hz, 1H), 1.81 (d, J = 6.4 Hz, 3H), 1.55 (d, J = 6.4 Hz, 3H), 1.31 (t, J = 7.2 Hz, 3H).

### Example 48: (2S)-4-((19R)-22-amino-3-ethyl-16-fluoro-10,19-dimethyl-20-oxa-3,4,10,1 1,23-pentaazapentacyclo[19.3.1.0^{8,12}.0^{13,18}.0^{2,6}]pentacosa-1(25),2(6),4,8(9),11,13(18),14,16,21 (22),23-decaen-5-yl)but-3-yn-2-ol

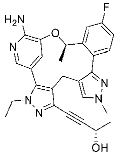

The synthetic method of Example 48 is the same as that of Example 47. LC-MS (ESI) [M+H]⁺: 487.3. ¹H NMR (400 MHz, CD₃OD), 7.59 (s, 1H), 7.53 (dd, J = 10.0 Hz, 2.4 Hz, 1H), 7.35-7.50 (brs, 1H), 7.18 (dd, J = 8.4 Hz, 5.6 Hz, 1H), 7.07 (td, J = 8.0 Hz, 2.8 Hz, 1H), 6.48 (s, 1H), 5.39-5.44 (m, 1H), 4.78 (q, J = 6.8 Hz, 1H), 4.02-4.17 (m, 2H), 3.92 (s, 3H), 3.77 (d, J = 15.6 Hz, 1H), 2.88 (d, J = 15.6 Hz, 1H), 1.81 (d, J = 6.8 Hz, 3H), 1.56 (d, J = 6.4 Hz, 3H), 1.31 (t, J = 7.2 Hz, 3H).

### Example 49: (19R)-3-ethyl-16-fluoro-10,19-dimethyl-5-(prop-1-yn-1-yl)-20-oxa-3,4,10, 11,23-pentaazapentacyclo[19.3.1.0^{8,12}.0^{13,18}.0^{2,6}]pentacosa-1(25),2(6),4,8(9),11,13(18),14,16,21 (22),23-decaen-22-amine

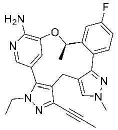

The synthetic method of Example 49 is the same as that of Example 47. LC-MS (ESI) [M+H]⁺: 487.3. ¹H NMR (400 MHz, CD₃OD), 7.51-7.54 (m, 2H), 7.41 (d, J = 1.6 Hz, 1H), 7.18 (dd, J = 8.4 Hz, 5.6 Hz, 1H), 7.07 (td, J = 8.4 Hz, 2.8 Hz, 1H), 6.46 (d, J = 1.6 Hz, 1H), 5.37-5.44 (m, 1H), 4.00-4.15 (m, 2H), 3.92 (s, 3H), 3.74 (d, J = 16.0 Hz, 1H), 2.85 (d, J = 16.0 Hz, 1H), 2.14 (s, 3H), 1.81 (d, J = 6.4 Hz, 3H), 1.30 (t, J = 7.2 Hz, 3H).

### Example 50: (19R)-3-ethyl-16-fluoro-10,19-dimethyl-5-(pyrazin-2-ylethynyl)-20-oxa-3, 4,10,11,23-pentaazapentacyclo[19.3.1.0^{8,12}.0^{13,18}.0^{2,6}]pentacosa-1(25),2(6),4,8(9),11,13(18),14, 16,21(22),23-decaen-22-amine

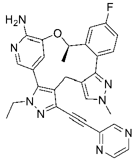

The synthetic method of Example 50 is the same as that of Example 47. LC-MS (ESI) [M+H]⁺: 521.3. ¹H NMR (400 MHz, CD₃OD), 8.87 (d, J = 1.2 Hz, 1H), 8.64-8.65 (m, 1H), 8.59 (d, J = 2.8 Hz, 1H), 7.63 (s, 1H), 7.54 (dd, J = 10.0 Hz, 2.8 Hz, 1H), 7.41-7.52 (brs, 1H), 7.22 (dd, J = 8.4 Hz, 6.0 Hz, 1H), 7.08 (td, J = 8.4 Hz, 2.8 Hz, 1H), 6.50 (s, 1H), 5.39-5.45 (m, 1H), 4.09-4.24 (m, 2H), 3.91 (s, 3H), 3.90 (d, J = 15.6 Hz, 1H), 2.98 (d, J = 15.6 Hz, 1H), 1.80 (d, J = 6.0 Hz, 3H), 1.36 (t, J = 7.2 Hz, 3H).

### Example 51: (19R)-5-((3S)-3-aminobut-1-yn-1-yl)-3-ethyl-16-fluoro-10, 19-dimethyl-20-oxa-3,4,10,11,23-pentaazapentacyclo[19.3.1.0^{8,12}.0^{13,18}.0^{2,6}]pentacosa-1(25),2(6),4,8(9),11,13(1 8),14,16,21(22),23-decaen-22-amine

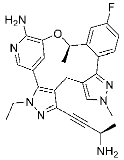

The synthetic method of Example 51 is the same as that of Example 47. LC-MS (ESI) [M+H]⁺: 486.3. ¹H NMR (400 MHz, CDCl₃), 7.50 (d, J = 2.0 Hz, 1H), 7.40 (s, 1H), 7.28 (dd, J = 10.0 Hz, 2.8 Hz, 1H), 7.13 (dd, J = 8.4 Hz, 5.6 Hz, 1H), 6.99 (td, J = 8.0 Hz, 2.8 Hz, 1H), 6.49 (d, J = 2.0 Hz, 1H), 5.40 (qd, J = 6.0 Hz, 1.6 Hz, 1H), 4.81 (s, 2H), 4.07-4.13 (m, 2H), 4.00 (q, J = 6.4 Hz, 1H), 3.91 (s, 3H), 3.74 (d, J = 15.6 Hz, 1H), 2.89 (d, J = 15.6 Hz, 1H), 1.78 (d, J = 6.4 Hz, 3H), 1.50 (d, J = 6.8 Hz, 3H), 1.39 (t, J = 7.2 Hz, 3H).

### Example 52: (19R)-5-((3S)-3-aminobut-1-yn-1-yl)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,13(1 8),14,16,21(22),23-decaen-22-amine

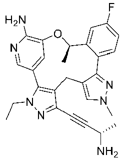

The synthetic method of Example 52 is the same as that of Example 47. LC-MS (ESI) [M+H]⁺: 486.3. ¹H NMR (400 MHz, CDCl₃), 7.50 (s, 2H), 7.28 (dd, J = 10.0 Hz, 2.8 Hz, 1H), 7.08-7.12 (m, 1H), 6.95 (td, J = 8.0 Hz, 2.4 Hz, 1H), 6.49 (d, J = 1.2 Hz, 1H), 5.37-5.42 (m, 1H), 4.90 (s, 2H), 4.25 (q, J = 6.8 Hz, 1H), 4.06-4.16 (m, 2H), 3.92 (s, 3H), 3.73 (d, J = 15.6 Hz, 1H), 2.88 (d, J = 15.6 Hz, 1H), 1.78 (d, J = 6.4 Hz, 3H), 1.65 (d, J = 6.8 Hz, 3H), 1.37 (t, J = 7.2 Hz, 3H).

### Example 53: 3-ethyl-16-fluoro-10-methyl-19-(prop-2-yn-1-yl)-3,4,9,10,11,23-hexaaza-2 0-oxapentacyclo[19.3.1.0^{8,12}.0^{13,18}.0^{2,6}]pentacosa-1(25),2(6),4,8(9),11,13(18),14,16,21(22),23-d ecaen-22-amine

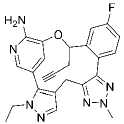

The synthetic method of Example 53 is the same as that of compound **45-1.** LC-MS (ESI) [M+H]⁺: 444.2. ¹H NMR (400 MHz, CDCl₃), 7.71 (s, 1H), 7.54 (d, J = 1.6 Hz, 1H), 7.38 (dd, J = 9.6 Hz, 2.4 Hz, 1H), 7.11 (dd, J = 8.4 Hz, 5.6 Hz, 1H), 7.06 (td, J = 8.0 Hz, 2.8 Hz, 1H), 6.27 (d, J = 1.6 Hz, 1H), 5.24-5.28 (m, 1H), 5.04 (s, 2H), 4.22 (s, 3H), 4.02-4.15 (m, 2H), 3.79 (d, J = 15.6 Hz, 1H), 3.19 (d, J = 15.6 Hz, 1H), 3.02-3.09 (m, 1H), 2.92-2.98 (m, 1H), 2.09 (t, J = 2.4 Hz, 1H), 1.38 (t, J = 7.2 Hz, 3H).

### Example 54: (19R)-3-ethyl-16-fluoro-10,19-dimethyl-5-((1-methylpyrazol-4-yl)ethynyl)-20-oxa-3,4,10,11,23-pentaazapentacyclo[19.3.1.0^{8,12}.0^{13,18}.0^{2,6}]pentacosa-1(25),2(6),4,8(9),11,1 3(18),14,16,21(22),23-decaen-22-amine

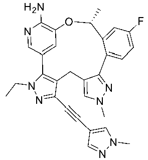

The synthetic method of Example 54 is the same as that of Example 47. LC-MS (ESI) [M+H]⁺: 523.3. ¹H NMR (400 MHz, CDCl₃), 7.68 (s, 1H), 7.60 (s, 1H), 7.50 (d, J = 1.6 Hz, 1H), 7.44 (s, 1H), 7.29 (dd, J = 10.0 Hz, 2.4 Hz, 1H), 7.14 (dd, J = 8.4 Hz, 5.6 Hz, 1H), 7.00 (td, J = 8.0 Hz, 2.8 Hz, 1H), 6.54 (d, J = 1.6 Hz, 1H), 5.38-5.45 (m, 1H), 5.07 (s, 2H), 4.09-4.15 (m, 2H), 3.92 (s, 3H), 3.90 (s, 3H), 3.82 (d, J = 15.6 Hz, 1H), 2.93 (d, J = 15.6 Hz, 1H), 1.79 (d, J = 6.4 Hz, 3H), 1.41 (t, J = 6.8 Hz, 3H).

### Example 55: (19R)-5-((1-(5,5-dimethyl-2-oxa-5-silahexan-1-yl)pyrazol-4-yl)ethynyl)-3-e thyl-16-fluoro-10,19-dimethyl-20-oxa-3,4,10,11,23-pentaazapentacyclo(19.3.1.0^{8,12}.0^{13,18}.0^{2,6}]p entacosa-1(25),2(6),4,8(9),11,13(18),14,16,21(22),23-decaen-22-amine

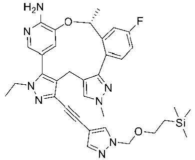

The synthetic method of Example 55 is the same as that of Example 47. LC-MS (ESI) [M+H]⁺: 639.4. ¹H NMR (400 MHz, CDCl₃), 7.81 (s, 1H), 7.73 (s, 1H), 7.45 (s, 1H), 7.22-7.30 (m, 2H), 7.17 (dd, J = 8.4 Hz, 5.6 Hz, 1H), 7.04 (td, J = 8.4 Hz, 2.4 Hz, 1H), 6.62 (s, 1H), 5.62-6.00 (brs, 2H), 5.40-5.48 (m, 3H), 4.08-4.16 (m, 2H), 3.91 (s, 3H), 3.86 (d, J = 15.6 Hz, 1H), 3.57-3.61 (m, 2H), 2.94 (d, J = 15.6 Hz, 1H), 1.82 (d, J = 6.0 Hz, 3H), 1.42 (t, J = 7.2 Hz, 3H), 0.90-0.94 (m, 2H), -0.01 (s, 9H).

### Example 56: (19R)-3-ethyl-16-fluoro-5-(3-(imidazol-1-yl)prop-1-yn-1-yl)-10,19-dimethy 1-20-oxa-3,4,10,11,23-pentaazapentacyclo[19.3.1.0^{8,12}.0^{13,18}.0^{2,6}]pentacosa-1(25),2(6),4,8(9),11, 13(18),14,16,21(22),23-decaen-22-amine

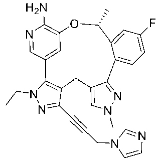

The synthetic method of Example 56 is the same as that of Example 47. LC-MS (ESI) [M+H]⁺: 523.4. ¹H NMR (400 MHz, CDCl₃), 7.92 (s, 1H), 7.48 (d, J = 1.6 Hz, 1H), 7.23-7.30 (m, 2H), 7.10-7.19 (m, 3H), 7.00 (td, J = 8.4 Hz, 2.8 Hz, 1H), 6.50 (d, J = 1.6 Hz, 1H), 5.36-5.41 (m, 1H), 4.83-5.24 (m, 4H), 4.11 (q, J = 7.2 Hz, 2H), 3.89 (s, 3H), 3.71 (d, J = 15.6 Hz, 1H), 2.91 (d, J = 15.6 Hz, 1H), 1.78 (d, J = 6.0 Hz, 3H), 1.40 (t, J = 7.2 Hz, 3H).

### Example 57: (19R)-3-ethyl-16-fluoro-10,19-dimethyl-5-(pyridin-4-ylethynyl)-20-oxa-3,4, 10,11,23-pentaazapentacyclo[19.3.1.0^{2,6}.0^{8,12}.0^{13,18}]pentacosa-1(25),2(6),4,8(9),11,13(14),15,17, 21(22),23-decaen-22-amine

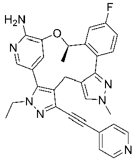

The synthetic method of Example 57 is the same as that of Example 47. LC-MS (ESI) [M+H]⁺: 520.4. ¹H NMR (400 MHz, CDCl₃), 8.51-8.54 (m, 2H), 7.54 (s, 1H), 7.45 (d, J = 4.8 Hz, 2H), 7.41 (s, 1H), 7.30 (dd, J = 9.6 Hz, 2.8 Hz, 1H), 7.16 (dd, J = 8.4 Hz, 5.6 Hz, 1H), 7.01 (td, J = 8.4 Hz, 2.8 Hz, 1H), 6.52 (s, 1H), 5.38-5.45 (m, 1H), 4.89 (s, 2H), 4.17 (q, J = 7.2 Hz, 2H), 3.91 (s, 3H), 3.84 (d, J = 15.6 Hz, 1H), 2.99 (d, J = 15.6 Hz, 1H), 1.79 (d, J = 6.4 Hz, 3H), 1.43 (t, J = 7.2 Hz, 3H).

### Example 58: (19R)-3-ethyl-16-fluoro-10,19-dimethyl-5-(pyridin-3-ylethynyl)-20-oxa-3,4, 10,11,23-pentaazapentacyclo[19.3.1.0^{2,6}.0^{8,12}.0^{13,18}]pentacosa-1(25),2(6),4,8(9),11,13(14),15,17, 21(22),23-decaen-22-amine

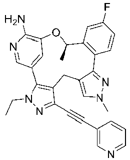

The synthetic method of Example 58 is the same as that of Example 47. LC-MS (ESI) [M+H]⁺: 520.4. ¹H NMR (400 MHz, CDCl₃), 8.83 (d, J = 1.2 Hz, 1H), 8.57 (dd, J = 4.8 Hz, 1.2 Hz, 1H), 7.87 (dt, J = 8.0 Hz, 2.0 Hz, 1H), 7.54 (s, 1H), 7.44 (s, 1H), 7.28-7.32 (m, 2H), 7.16 (dd, J = 8.4 Hz, 5.6 Hz, 1H), 7.00 (td, J = 8.4 Hz, 2.4 Hz, 1H), 6.53 (d, J = 1.2 Hz, 1H), 5.38-5.45 (m, 1H), 4.88 (s, 2H), 4.13-4.19 (m, 2H), 3.90 (s, 3H), 3.85 (d, J = 15.6 Hz, 1H), 2.98 (d, J = 15.6 Hz, 1H), 1.79 (d, J = 6.4 Hz, 3H), 1.43 (t, J = 7.2 Hz, 3H).

### Example 59: (19R)-3-ethyl-16-fluoro-5-((1E)-3-methoxyprop-1-en-1-yl)-10,19-dimethyl-20-oxa-3,4,10,11,23-pentaazapentacyclo[19.3.1.0^{2,6}.0^{8,12}.0^{13,18}]pentacosa-1(24),2(6),4,8(9),11,1 3(18),14,16,21(25),22-decaen-22-amine

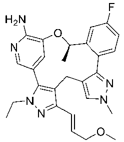

The synthetic method of Example 59 is the same as that of Example 45. LC-MS (ESI) [M+H]⁺: 489.3. ¹H NMR (400 MHz, CDCl₃), 7.42-7.56 (brs, 1H), 7.26-7.29 (m, 2H), 7.14 (dd, J = 8.4 Hz, 5.6 Hz, 1H), 7.02 (td, J = 8.4 Hz, 2.4 Hz, 1H), 6.76 (dt, J = 16.4 Hz, 1.6 Hz, 1H), 6.55-6.62 (m, 2H), 5.18-5.56 (m, 3H), 4.16 (dd, J = 5.6 Hz, 1.2 Hz, 2H), 4.05-4.13 (m, 2H), 3.90 (s, 3H), 3.70 (d, J = 16.0 Hz, 1H), 3.44 (s, 3H), 2.93 (d, J = 16.0 Hz, 1H), 1.79 (d, J = 6.0 Hz, 3H), 1.39 (t, J = 7.2 Hz, 3H).

### Example 60: (19R)-3-ethyl-16-fluoro-10,19-dimethyl-5-(1,3-thiazol-4-ylethynyl)-20-oxa-3,4,10,11,23-pentaazapentacyclo[19.3.10^{8,12}.0^{13,18}.0^{2,6}]pentacosa-1(24),2(6),4,8(9),11,13(14),1 5,17,21(25),22-decaen-22-amine

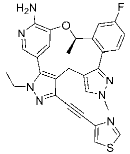

The synthetic method of Example 60 is the same as that of Example 47. LC-MS (ESI) [M+H]⁺: 526.4. ¹H NMR (400 MHz, CDCl₃), 8.83 (d, J = 2.0 Hz, 1H), 7.66 (d, J = 2.0 Hz, 1H), 7.53 (d, J = 1.6 Hz, 1H), 7.49 (s, 1H), 7.28 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.55 (d, J = 1.6 Hz, 1H), 5.38-5.45 (m, 1H), 4.95 (s, 2H), 4.11-4.17 (m, 2H), 3.90 (d, J = 15.6 Hz, 1H), 3.89 (s, 3H), 2.95 (d, J = 15.6 Hz, 1H), 1.79 (d, J = 6.4 Hz, 3H), 1.41 (t, J = 7.2 Hz, 3H).

### Example 61: 4-((19R)-22-amino-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,13(18),14,16,21(22),2 3-decaen-5-yl)-2-methylbut-3-yn-2-ol

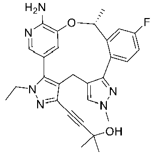

The synthetic method of Example 61 is the same as that of Example 47. LC-MS (ESI) [M+H]⁺: 501.4. ¹H NMR (400 MHz, CDCl₃), 7.48 (d, J = 1.6 Hz, 1H), 7.41 (s, 1H), 7.28 (dd, J = 10.0 Hz, 2.8 Hz, 1H), 7.14 (dd, J = 8.4 Hz, 5.6 Hz, 1H), 7.00 (td, J = 8.4 Hz, 2.8 Hz, 1H), 6.53 (d, J = 1.6 Hz, 1H), 5.38-5.45 (m, 1H), 5.20 (s, 2H), 4.04-4.14 (m, 2H), 3.90 (s, 3H), 3.73 (d, J = 15.6 Hz, 1H), 2.89 (d, J = 15.6 Hz, 1H), 1.79 (d, J = 6.4 Hz, 3H), 1.68 (s, 6H), 1.38 (t, J = 7.2 Hz, 3H).

### Example 62: (19R)-3-ethyl-16-fluoro-5-(1H-pyrazol-4-ylethynyl)-10,19-dimethyl-20-oxa -3,4,10,11,23-pentaazapentacyclo[19.3.1.0^{8,12}.0^{13,18}.0^{2,6}]pentacosa-1(25),2(6),4,8(9),11,13(18),1 4,16,21(22),23-decaen-22-amine

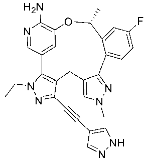

The synthetic method of Example 62 is the same as that of Example 47. LC-MS (ESI) [M+H]⁺: 509.4. ¹H NMR (400 MHz, CDCl₃), 10.10-11.20 (brs, 1H), 7.73-7.97 (brs, 2H), 7.49 (d, J = 1.2 Hz, 1H), 7.46 (s, 1H), 7.29 (dd, J = 10.0 Hz, 2.8 Hz, 1H), 7.16 (dd, J = 8.4 Hz, 5.6 Hz, 1H), 7.02 (td, J = 8.4 Hz, 2.8 Hz, 1H), 6.58 (d, J = 1.2 Hz, 1H), 5.32-5.48 (m, 3H), 4.10-4.16 (m, 2H), 3.91 (s, 3H), 3.85 (d, J = 15.6 Hz, 1H), 2.95 (d, J = 15.6 Hz, 1H), 1.80 (d, J = 6.4 Hz, 3H), 1.42 (t, J = 7.2 Hz, 3H).

### Example 63: (19R)-3-ethyl-16-fluoro-10,19-dimethyl-5-(pyridin-2-ylethynyl)-20-oxa-3,4, 10,11,23-pentaazapentacyclo[19.3.1.0^{8,12}.0^{13,18}.0^{2,6}]pentacosa-1(25),2(6),4,8(9),11,13(18),14,16, 21(22),23-decaen-22-amine

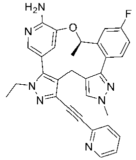

The synthetic method of Example 63 is the same as that of Example 47. LC-MS (ESI) [M+H]⁺: 520.4. ¹H NMR (400 MHz, CDCl₃), 8.65 (d, J = 4.8 Hz, 1H), 7.71 (td, J = 8.0 Hz, 2.0 Hz, 1H), 7.62 (d, J = 7.6 Hz, 1H), 7.54 (d, J = 1.6 Hz, 1H), 7.49 (s, 1H), 7.26-7.30 (m, 2H), 7.14 (dd, J = 8.4 Hz, 5.6 Hz, 1H), 7.00 (td, J = 8.4 Hz, 2.8 Hz, 1H), 6.56 (d, J = 1.6 Hz, 1H), 5.39-5.46 (m, 1H), 4.81 (s, 2H), 4.14-4.20 (m, 2H), 3.94 (d, J = 16.0 Hz, 1H), 3.89 (s, 3H), 2.97 (d, J = 16.0 Hz, 1H), 1.79 (d, J = 6.4 Hz, 3H), 1.44 (t, J = 7.2 Hz, 3H).

### Example 64: (19R)-3-ethyl-16-fluoro-5-(imidazo[2,3-f][1,2]pyridazin-3-ylethynyl)-10,19 -dimethyl-20-oxa-3,4,10,11,23-pentaazapentacyclo[19.3.1.0^{8,12}.0^{13,18}.0^{2,6}]pentacosa-1(25),2(6), 4,8(9),11,13(18),14,16,21(22),23-decaen-22-amine

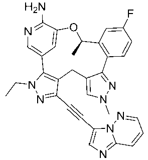

The synthetic method of Example 64 is the same as that of Example 47. LC-MS (ESI) [M+H]⁺: 560.4. ¹H NMR (400 MHz, CDCl₃), 8.46 (dd, J = 4.4 Hz, 1.6 Hz, 1H), 8.09 (s, 1H), 8.01 (dd, J = 8.8 Hz, 1.6 Hz, 1H), 7.64 (s, 1H), 7.56 (d, J = 1.6 Hz, 1H), 7.30 (dd, J = 9.6 Hz, 2.8 Hz, 1H), 7.12-7.16 (m, 2H), 7.00 (td, J = 8.4 Hz, 2.8 Hz, 1H), 6.57 (d, J = 1.6 Hz, 1H), 5.41-5.48 (m, 1H), 4.82 (s, 2H), 4.15-4.20 (m, 2H), 3.93 (d, J = 15.6 Hz, 1H), 3.91 (s, 3H), 3.00 (d, J = 15.6 Hz, 1H), 1.80 (d, J = 6.4 Hz, 3H), 1.43 (t, J = 7.2 Hz, 3H).

### Example 65: (19R)-5-(cyclopropylethynyl)-3-ethyl-16-fluoro-10,19-dimethyl-20-oxa-3,4, 10,11,23-pentaazapentacyclo[19.3.1.0^{2,6}.0^{8,12}.0^{13,18}]pentacosa-1(25),2(6),4,8(9),11,13(18),14,16, 21(22),23-decaen-22-amine

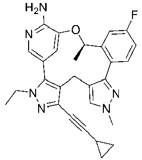

The synthetic method of Example 65 is the same as that of Example 47. LC-MS (ESI) [M+H]⁺: 483.4. ¹H NMR (400 MHz, CDCl₃), 7.50 (d, J = 1.6 Hz, 1H), 7.41 (s, 1H), 7.25-7.28 (m, 1H), 7.14 (dd, J = 8.4 Hz, 5.6 Hz, 1H), 6.99 (td, J = 8.4 Hz, 2.8 Hz, 1H), 6.52 (d, J = 1.6 Hz, 1H), 5.36-5.42 (m, 1H), 5.02-5.29 (brs, 2H), 4.01-4.10 (m, 2H), 3.91 (s, 3H), 3.74 (d, J = 15.6 Hz, 1H), 3.86 (d, J = 15.6 Hz, 1H), 1.78 (d, J = 6.0 Hz, 3H), 1.49-1.55 (m, 1H), 1.34 (t, J = 7.2 Hz, 3H), 0.45-0.93 (m, 4H).

### Example 66: (19R)-5-(azetidin-3-ylethynyl)-3-ethyl-16-fluoro-10,19-dimethyl-20-oxa-3, 4,10,11,23-pentaazapentacyclo[19.3.1.0^{2,6}.0^{8,12}.0^{13,18}]pentacosa-1(24),2(6),4,8(9),11,13(18),14, 16,21(25),22-decaen-22-amine

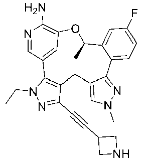

The synthetic method of Example 66 is the same as that of Example 47. LC-MS (ESI) [M+H]⁺: 498.4. ¹H NMR (400 MHz, CD₃OD), 7.57 (s, 1H), 7.52 (dd, J = 9.6 Hz, 2.4 Hz, 1H), 7.30-7.51 (brs, 1H), 7.17 (dd, J = 8.4 Hz, 5.6 Hz, 1H), 7.06 (t, J = 8.4 Hz, 2.4 Hz, 1H), 6.46 (s, 1H), 5.37-5.43 (m, 1H), 4.00-4.46 (m, 7H), 3.92 (s, 3H), 3.76 (d, J = 15.6 Hz, 1H), 2.87 (d, J = 15.6 Hz, 1H), 1.79 (d, J = 6.0 Hz, 3H), 1.30 (t, J = 7.2 Hz, 3H).

### Example 67: (19R)-3-ethyl-16-fluoro-10,19-dimethyl-5-(phenylethynyl)-20-oxa-3,4,10,1 1,23-pentaazapentacyclo[19.3.1.0^{2,6}.0^{8,12}.0^{13,18}]pentacosa-1(24),2(6),4,8(9),11,13(18),14,16,21 (25),22-decaen-22-amine

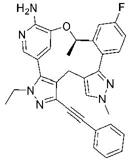

The synthetic method of Example 67 is the same as that of Example 47. LC-MS (ESI) [M+H]⁺: 519.4. ¹H NMR (400 MHz, CD₃OD), 7.28-7.75 (m, 8H), 7.22 (dd, J = 8.4 Hz, 5.6 Hz, 1H), 7.07 (td, J = 8.4 Hz, 2.8 Hz, 1H), 6.50 (s, 1H), 5.39-5.45 (m, 1H), 4.06-4.21 (m, 2H), 3.91 (s, 3H), 3.87 (d, J = 15.6 Hz, 1H), 2.95 (d, J = 15.6 Hz, 1H), 1.81 (d, J = 6.4 Hz, 3H), 1.35 (t, J = 7.2 Hz, 3H).

### Example 68: (19R)-5-((aminocyclopropyl)ethynyl)-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(24),2(6),4,8(9),11,13(1 8),14,16,21(25),22-decaen-22-amine

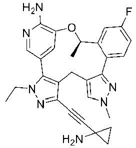

The synthetic method of Example 68 is the same as that of Example 47. LC-MS (ESI) [M+H]⁺: 498.4. ¹H NMR (400 MHz, CDCl₃), 7.46 (d, J = 1.2 Hz, 1H), 7.40 (s, 1H), 7.29 (dd, J = 10.0 Hz, 2.8 Hz, 1H), 7.14 (dd, J = 8.4 Hz, 5.6 Hz, 1H), 7.00 (td, J = 8.0 Hz, 2.8 Hz, 1H), 6.53 (d, J = 1.2 Hz, 1H), 5.38-5.45 (m, 1H), 5.16-5.36 (brs, 2H), 4.06-4.12 (m, 2H), 3.92 (s, 3H), 3.72 (d, J = 15.6 Hz, 1H), 2.88 (d, J = 15.6 Hz, 1H), 1.80 (d, J = 6.4 Hz, 3H), 1.38 (t, J = 7.2 Hz, 3H), 1.22-1.27 (m, 2H), 1.11-1.16 (m, 2H).

### Example 69: (19R)-5-((aminocyclopropyl)ethynyl)-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(24),2(6),4,8(9),11,13(1 8),14,16,21(25),22-decaen-22-amine

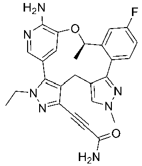

The synthetic method of Example 69 is the same as that of Example 47. LC-MS (ESI) [M+H]⁺: 486.3. ¹H NMR (400 MHz, CD₃OD), 7.57 (s, 1H), 7.53 (dd, J = 10.0 Hz, 2.8 Hz, 1H), 7.44 (s, 1H), 7.20 (dd, J = 8.4 Hz, 5.6 Hz, 1H), 7.08 (td, J = 8.4 Hz, 2.8 Hz, 1H), 6.48 (d, J = 1.6 Hz, 1H), 5.39-5.45 (m, 1H), 4.06-4.21 (m, 2H), 3.92 (s, 3H), 3.85 (d, J = 15.6 Hz, 1H), 2.93 (d, J = 15.6 Hz, 1H), 1.81 (d, J = 6.4 Hz, 3H), 1.33 (t, J = 7.2 Hz, 3H).

### Example 70: (19R)-5-(4-aminobut-1-yn-1-yl)-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(24),2(6),4,8(9),11,13(14),1 5,17,21(25),22-decaen-22-amine

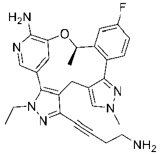

The synthetic method of Example 70 is the same as that of Example 47. LC-MS (ESI) [M+H]⁺: 486.4. ¹H NMR (400 MHz, CDCl₃), 7.50 (d, J = 1.6 Hz, 1H), 7.42 (s, 1H), 7.28 (dd, J = 10.0 Hz, 2.8 Hz, 1H), 7.13 (dd, J = 8.8 Hz, 6.0 Hz, 1H), 6.99 (td, J = 8.0 Hz, 2.8 Hz, 1H), 6.50 (d, J = 1.6 Hz, 1H), 5.37-5.43 (m, 1H), 4.82 (s, 2H), 4.07-4.13 (m, 2H), 3.91 (s, 3H), 3.74 (d, J = 15.6 Hz, 1H), 3.03 (t, J = 6.4 Hz, 2H), 2.89 (d, J = 15.6 Hz, 1H), 2.71 (t, J = 6.4 Hz, 2H), 2.16-2.42 (brs, 2H), 1.78 (d, J = 6.0 Hz, 3H), 1.38 (t, J = 7.2 Hz, 3H).

### Example 71: 4-((19R)-22-amino-3-ethyl-16-fluoro-10,19-dimethyl-20-oxa-3,4,10,11, 23 -pentaazapentacyclo[19.3.1.0^{2,6}.0^{8,12}.0^{13,18}]pentacosa-1(24),2(6),4,8(9),11,13(14),15,17,21(25), 22-decaen-5-yl)but-3-yn-1-ol

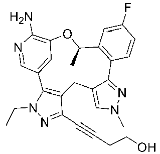

The synthetic method of Example 71 is the same as that of Example 47. LC-MS (ESI) [M+H]⁺: 487.4. ¹H NMR (400 MHz, CDCl₃), 7.49 (d, J = 1.2 Hz, 1H), 7.42 (s, 1H), 7.28 (dd, J = 10.0 Hz, 2.4 Hz, 1H), 7.12 (dd, J = 8.4 Hz, 5.6 Hz, 1H), 6.98 (td, J = 8.0 Hz, 2.8 Hz, 1H), 6.50 (d, J = 1.2 Hz, 1H), 5.36-5.43 (m, 1H), 4.88 (s, 2H), 4.06-4.12 (m, 2H), 3.90 (s, 3H), 3.88 (t, J = 6.4 Hz, 2H), 3.73 (d, J = 15.6 Hz, 1H), 2.87 (d, J = 15.6 Hz, 1H), 2.78 (t, J = 6.4 Hz, 2H), 1.78 (d, J = 6.0 Hz, 3H), 1.38 (t, J = 7.2 Hz, 3H).

### Example 72: (19R)-3-ethyl-16-fluoro-10,19-dimethyl-5-(pyrimidin-5-ylethynyl)-20-oxa-3,4,10,11,23-pentaazapentacyclo[19.3.1.0^{8,12}.0^{13,18}.0^{2,6}]pentacosa-1(25),2(6),4,8(9),11,13(18),1 4,16,21(22),23-decaen-22-amine

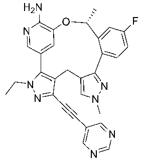

The synthetic method of Example 72 is the same as that of Example 47. LC-MS (ESI) [M+H]⁺: 521.4. ¹H NMR (400 MHz, CDCl₃), 9.17 (s, 1H), 8.92 (s, 2H), 7.53 (d, J = 1.6 Hz, 1H), 7.40 (s, 1H), 7.29 (dd, J = 10.0 Hz, 2.8 Hz, 1H), 7.17 (dd, J = 8.4 Hz, 5.6 Hz, 1H), 7.02 (td, J = 8.0 Hz, 2.8 Hz, 1H), 6.56 (d, J = 1.6 Hz, 1H), 5.39-5.46 (m, 1H), 5.25 (s, 2H), 4.13-4.19 (m, 2H), 3.92 (s, 3H), 3.84 (d, J = 15.6 Hz, 1H), 3.00 (d, J = 15.6 Hz, 1H), 1.80 (d, J = 6.4 Hz, 3H), 1.43 (t, J = 7.2 Hz, 3H).

### Example 73: (19R)-3-ethyl-16-fluoro-5-(piperidin-4-ylethynyl)-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,13(18),1 4,16,21(22),23-decaen-22-amine

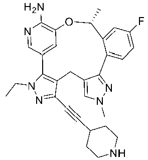

The synthetic method of Example 73 is the same as that of Example 47. LC-MS (ESI) [M+H]⁺: 526.4. ¹H NMR (400 MHz, CDCl₃), 7.50 (d, J = 1.6 Hz, 1H), 7.40 (s, 1H), 7.28 (dd, J = 10.0 Hz, 2.8 Hz, 1H), 7.14 (dd, J = 8.4 Hz, 5.6 Hz, 1H), 6.99 (td, J = 8.0 Hz, 2.8 Hz, 1H), 6.49 (d, J = 1.6 Hz, 1H), 5.37-5.43 (m, 1H), 4.82 (s, 2H), 4.07-4.13 (m, 2H), 3.91 (s, 3H), 3.73 (d, J = 15.6 Hz, 1H), 2.92-3.10 (m, 1H), 3.15-3.20 (m, 2H), 2.89 (d, J = 15.6 Hz, 1H), 2.76-2.84 (m, 2H), 1.98-2.06 (m, 2H), 1.74-1.84 (m, 5H), 1.38 (t, J = 7.2 Hz, 3H).

### Example 74: (19R)-3-ethyl-16-fluoro-5-(1H-imidazol-4-ylethynyl)-10,19-dimethyl-20-ox a-3,4,10,11,23-pentaazapentacyclo[19.3.1.0^{8,12}.0^{13,18}.0^{2,6}]pentacosa-1(25),2(6),4,8(9),11,13(18), 14,16,21(22),23-decaen-22-amine

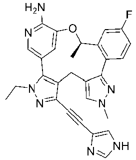

The synthetic method of Example 74 is the same as that of Example 47. LC-MS (ESI) [M+H]⁺: 509.4. ¹H NMR (400 MHz, CDCl₃), 11.92-12.16 (brs, 1H), 7.68 (s, 1H), 7.51 (d, J = 1.6 Hz, 1H), 7.47 (s, 1H), 7.40 (s, 1H), 7.28 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.56 (d, J = 1.6 Hz, 1H), 5.39-5.45 (m, 1H), 4.95 (s, 2H), 4.11-4.17 (m, 2H), 3.90 (s, 3H), 3.86 (d, J = 15.6 Hz, 1H), 1.94 (d, J = 15.6 Hz, 1H), 1.79 (d, J = 6.0 Hz, 3H), 1.42 (t, J = 7.2 Hz, 3H).

### Example 75: (19R)-3-ethyl-16-fluoro-10,19-dimethyl-5-(tetrahydro-1H-pyrrol-2-ylethyn yl)-20-oxa-3,4,10,11,23-pentaazapentacyclo[19.3.1.0.^{8,12}.0^{13,18}.0^{2,6}]pentacosa-1(25),2(6),4,8(9),1 1,13(18),14,16,21(22),23-decaen-22-amine

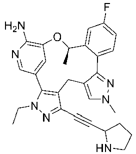

The synthetic method of Example 75 is the same as that of Example 47. LC-MS (ESI) [M+H]⁺: 512.4. ¹H NMR (400 MHz, CD₃OD), 7.57 (s, 1H), 7.54 (dd, J = 10.0 Hz, 2.8 Hz, 1H), 7.43 (s, 1H), 7.17 (dd, J = 8.4 Hz, 5.6 Hz, 1H), 7.08 (td, J = 8.4 Hz, 2.8 Hz, 1H), 6.46 (d, J = 1.6 Hz, 1H), 5.36-5.44 (m, 1H), 4.43-4.50 (m, 1H), 4.05-4.17 (m, 2H), 3.93 (s, 3H), 3.77 (d, J = 16.0 Hz, 1H), 3.31-3.37 (m, 1H), 3.14-3.24 (m, 1H), 2.90 (d, J = 16.0 Hz, 1H), 2.34-2.44 (m, 1H), 1.96-2.22 (m, 3H), 1.81 (d, J = 6.4 Hz, 3H), 1.31 (t, J = 7.2 Hz, 3H).

### Example 76: (19R)-3-ethyl-16-fluoro-10,19-dimethyl-5-((1-methylpyrrol-3-yl)ethynyl)-2 0-oxa-3,4,10,11,23-pentaazapentacyclo[19.3.1.0^{8,12}.0^{13,18}.0^{2,6}]pentacosa-1(24),2(6),4,8(9),11,13 (14),15,17,21(25),22-decaen-22-amine

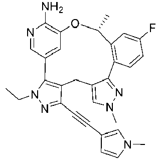

The synthetic method of Example 76 is the same as that of Example 47. LC-MS (ESI) [M+H]⁺: 522.4. ¹H NMR (400 MHz, CDCl₃), 7.74 (s, 1H), 7.49 (s, 1H), 7.24-7.28 (m, 1H), 7.15 (dd, J = 8.4 Hz, 5.6 Hz, 1H), 7.01 (td, J = 8.4 Hz, 2.8 Hz, 1H), 6.93 (s, 1H), 6.54 (s, 2H), 6.35 (s, 1H), 5.32-5.57 (m, 3H), 4.06-4.14 (m, 2H), 3.90 (s, 3H), 3.84 (d, J = 15.6 Hz, 1H), 3.66 (s, 3H), 2.91 (d, J = 15.6 Hz, 1H), 1.79 (d, J = 6.0 Hz, 3H), 1.36 (t, J = 7.2 Hz, 3H).

### Example 77: (19R)-3-ethyl-16-fluoro-5-(1H-pyrazolo[3,4-b]pyridin-5-ylethynyl)-10,19-d imethyl-20-oxa-3,4,10,11,23-pentaazapentacyclo[19.3.1.0^{2,6}.0^{8,12}.0^{13,18}]pentacosa-1(24),2(6),4, 8(9),11,13(18),14,16,21(25),22-decaen-22-amine

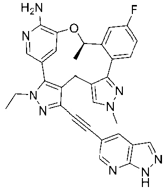

The synthetic method of Example 77 is the same as that of Example 47. LC-MS (ESI) [M+H]⁺: 560.4. ¹H NMR (400 MHz, CD₃OD), 8.73 (d, J = 2.0 Hz, 1H), 8.48 (d, J = 2.0 Hz, 1H), 8.17 (s, 1H), 7.66 (s, 1H), 7.55 (dd, J = 10.0 Hz, 5.6 Hz, 1H), 7.48 (s, 1H), 7.25 (dd, J = 8.4 Hz, 2.8 Hz, 1H), 7.10 (td, J = 8.0 Hz, 2.8 Hz, 1H), 6.58 (d, J = 1.6 Hz, 1H), 5.44-5.50 (m, 1H), 4.10-4.22 (m, 2H), 3.90-3.94 (m, 4H), 2.98 (d, J = 15.6 Hz, 1H), 1.83 (d, J = 6.4 Hz, 3H), 1.37 (t, J = 7.2 Hz, 3H).

### Example 78: (19R)-3-ethyl-16-fluoro-10,19-dimethyl-5-((2-methyl-1,2,3-triazol-,4-yl)eth ynyl)-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine

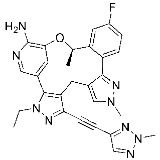

The synthetic method of Example 78 is the same as that of Example 47. LC-MS (ESI) [M+H]⁺: 524.4. ¹H NMR (400 MHz, CD₃OD), 7.90 (s, 1H), 7.59 (s, 1H), 7.54 (dd, J = 10.0 Hz, 2.8 Hz, 1H), 7.40-7.52 (brs, 1H), 7.21 (dd, J = 8.4 Hz, 5.6 Hz, 1H), 7.08 (td, J = 8.4 Hz, 2.8 Hz, 1H), 6.49 (s, 1H), 5.39-5.45 (m, 1H), 4.21 (s, 3H), 4.07-4.20 (m, 2H), 3.91 (s, 3H), 3.83 (d, J = 15.6 Hz, 1H), 2.94 (d, J = 15.6 Hz, 1H), 1.81 (d, J = 6.0 Hz, 3H), 1.34 (t, J = 7.2 Hz, 3H).

### Example 79: (19R)-3-ethyl-16-fluoro-10,19-dimethyl-5-(oxetan-3-ylethynyl)-20-oxa-3,4, 10,11,23-pentaazapentacyclo[19.3.1.0^{8,12}.0^{13,18}.0^{2,6}]pentacosa-1(25),2(6),4,8(9),11,13(18),14,16, 21(22),23-decaen-22-amine

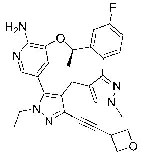

The synthetic method of Example 79 is the same as that of Example 47. LC-MS (ESI) [M+H]⁺: 499.4. ¹H NMR (400 MHz, CD₃OD), 7.57 (s, 1H), 7.54 (dd, J = 10.0 Hz, 2.8 Hz, 1H), 7.43 (s, 1H), 7.20 (dd, J = 8.4 Hz, 5.6 Hz, 1H), 7.08 (td, J = 8.4 Hz, 2.8 Hz, 1H), 6.47 (s, 1H), 5.38-5.44 (m, 1H), 4.96 (dd, J = 8.8 Hz, 5.6 Hz, 2H), 4.80-4.83 (m, 2H), 4.19-4.27 (m, 1H), 4.03-4.18 (m, 2H), 3.92 (s, 3H), 3.76 (d, J = 15.6 Hz, 1H), 2.89 (d, J = 15.6 Hz, 1H), 1.81 (d, J = 6.4 Hz, 3H), 1.32 (t, J = 7.2 Hz, 3H).

### Example 80: (19R)-3-ethyl-16-fluoro-5-(piperidin-2-ylethynyl)-10,19-dimethyl-20-oxa-3, 4,10,11,23-pentaazapentacyclo[19.3.1.0^{8,12}.0^{13,18}.0^{2,6}]pentacosa-1(25),2(6),4,8(9),11,13(18),14, 16,21(22),23-decaen-22-amine

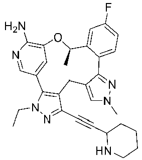

The synthetic method of Example 80 is the same as that of Example 47. LC-MS (ESI) [M+H]⁺: 526.4. ¹H NMR (400 MHz, CD₃OD), 7.61 (s, 0.5H), 7.60 (s, 0.5H), 7.54 (dd, J = 1.0 Hz, 2.8 Hz, 1H), 7.43 (d, J = 1.6 Hz, 1H), 7.18 (dd, J = 8.8 Hz, 6.0 Hz, 1H), 7.08 (td, J = 8.4 Hz, 2.8 Hz, 1H), 6.47 (d, J = 1.6 Hz, 1H), 5.38-5.45 (m, 1H), 4.02-4.17 (m, 2H), 3.88-3.97 (m, 4H), 3.79 (d, J = 15.6 Hz, 1H), 3.11-3.17 (m, 1H), 2.89 (d, J = 15.6 Hz, 1H), 2.72-2.78 (m, 1H), 1.85-2.03 (m, 2H), 1.72-1.84 (m, 4H), 1.54-1.64 (m, 3H), 1.31 (t, J = 7.6 Hz, 3H).

### Example 81: (19R)-5-(1,2-pyridazin-3-ylethynyl)-3-ethyl-16-fluoro-10,19-dimethyl-20-o xa-3,4,10,11,23-pentaazapentacyclo[19.3.1.0^{8,12}.0^{13,18}.0^{2,6}]pentacosa-1(25),2(6),4,8(9),11,13(1 8),14,16,21(22),23-decaen-22-amine

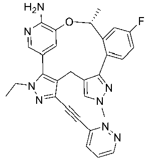

The synthetic method of Example 81 is the same as that of Example 47. LC-MS (ESI) [M+H]⁺: 521.4. ¹H NMR (400 MHz, CDCl₃), 9.17 (dd, J = 4.8 Hz, 1.6 Hz, 1H), 7.74 (dd, J = 8.0 Hz, 1.6 Hz, 1H), 7.47-7.52 (m, 3H), 7.29 (dd, J = 10.0 Hz, 2.8 Hz, 1H), 7.16 (dd. J = 8.4 Hz, 5.6 Hz, 1H), 7.03 (td, J = 8.4 Hz, 2.8 Hz, 1H), 6.63 (d, J = 1.2 Hz, 1H), 5.40-5.52 (m, 3H), 4.14-4.20 (m, 2H), 3.98 (d, J = 15.6 Hz, 1H), 3.90 (s, 3H), 2.99 (d, J = 15.6 Hz, 1H), 1.81 (d, J = 6.4 Hz, 3H), 1.45 (t, J = 7.2 Hz, 3H).

### Example 82: 1-(((19R)-22-amino-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,13(18),14,16,21(22),2 3-decaen-5-yl)ethynyl)cyclohexan-1-ol

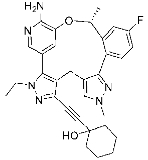

The synthetic method of Example 82 is the same as that of Example 47. LC-MS (ESI) [M+H]⁺: 541.4. ¹H NMR (400 MHz, CDCl₃), 7.43 (s, 1H), 7.41 (s, 1H), 7.28 (dd, J = 10.0 Hz, 2.8 Hz, 1H), 7.17 (dd, J = 8.4 Hz, 5.6 Hz, 1H), 7.04 (td, J = 8.4 Hz, 2.8 Hz, 1H), 6.60 (d, J = 1.6 Hz, 1H), 5.76-6.02 (brs, 2H), 5.41-5.48 (m, 1H), 4.04-4.14 (m, 2H), 3.91 (s, 3H), 3.78 (d, J = 15.6 Hz, 1H), 2.91 (d, J = 15.6 Hz, 1H), 2.03-2.12 (m, 2H), 1.54-1.85 (m, 10H), 1.39 (t, J = 7.2 Hz, 3H), 1.28-1.36 (m, 1H).

### Example 83: (19R)-5-(3,3-dimethylbut-1-yn-1-yl)-3-ethyl-16-fluoro-10,19-dimethyl-20-o xa-3,4,10,11,23-pentaazapentacyclo[19.3.1.08,12.013,18.02,6]pentacosa-1(25),2(6),4,8(9),11,1 3(18),14,16,21(22),23-decaen-22-amine

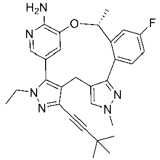

The synthetic method of Example 83 is the same as that of Example 47. LC-MS (ESI) [M+H]⁺: 499.4. ¹H NMR (400 MHz, CDCl₃), 7.46 (s, 1H), 7.43 (s, 1H), 7.28 (d, J = 10.0 Hz, 2.8 Hz, 1H), 7.17 (dd, J = 8.4 Hz, 5.6 Hz, 1H), 7.01 (td, J = 8.4 Hz, 2.8 Hz, 1H), 6.55 (d, J = 1.6 Hz, 1H), 5.40-5.46 (m, 1H), 5.24-5.39 (brs, 2H), 4.03-4.14 (m, 2H), 3.91 (s, 3H), 3.75 (d, J = 15.6 Hz, 1H), 2.88 (d, J = 15.6 Hz, 1H), 1.80 (d, J = 6.4 Hz, 3H), 1.36-1.39 (m, 12H).

### Example 84: (19R)-5-(cyclohexylethynyl)-3-ethyl-16-fluoro-10,19-dimethyl-20-oxa-3,4, 10,11,23-pentaazapentacyclo[19.3.1.0^{8,12}.0^{13,18}.0^{2,6}]pentacosa-1(25),2(6),4,8(9),11,13(18),14,16, 21(22),23-decaen-22-amine

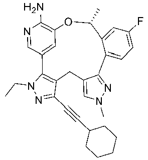

The synthetic method of Example 84 is the same as that of Example 47. LC-MS (ESI) [M+H]⁺: 525.4. ¹H NMR (400 MHz, CDCl₃), 7.51 (s, 1H), 7.43 (s, 1H), 7.24-7.28 (m, 1H), 7.14 (dd, J = 8.4 Hz, 5.6 Hz, 1H), 7.00 (td, J = 8.4 Hz, 2.8 Hz, 1H), 6.53 (d, J = 1.2 Hz, 1H), 5.30-5.44 (m, 3H), 3.99-4.09 (m, 2H), 3.90 (s, 3H), 3.75 (d, J = 15.6 Hz, 1H), 2.86 (d, J = 15.6 Hz, 1H), 2.63-2.72 (m, 1H), 1.89-1.98 (m, 2H), 1.71-1.83 (m, 5H), 1.52-1.65 (m, 3H), 1.29-1.42 (m, 6H).

### Example 85: (19R)-3-ethyl-16-fluoro-10,19-dimethyl-5-(pyrimidin-2-ylethynyl)-20-oxa-3,4,10,11,23-pentaazapentacyclo[19.3.1.0^{2,6}.0^{8,12}.0^{13,18}]pentacosa-1(24),2(6),4,8(9),11,13(18),1 4,16,21(25),22-decaen-22-amine

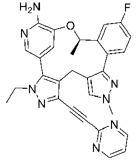

The synthetic method of Example 85 is the same as that of Example 47. LC-MS (ESI) [M+H]⁺: 521.4. ¹H NMR (400 MHz, CD₃OD), 8.84 (d, J = 5.2 Hz, 2H), 7.65 (s, 1H), 7.55 (dd, J = 10.0 Hz, 2.8 Hz, 1H), 7.45-7.52 (m, 2H), 7.23 (dd, J = 8.4 Hz, 5.6 Hz, 1H), 7.09 (td, J = 8.4 Hz, 2.8 Hz, 1H), 6.52 (s, 1H), 5.41-5.48 (m, 1H), 4.08-4.23 (m, 2H), 3.95 (d, J = 7.6 Hz, 1H), 3.91 (s, 3H), 2.98 (d, J = 15.6 Hz, 1H), 1.82 (d, J = 6.4 Hz, 3H), 1.36 (t, J = 7.2 Hz, 3H).

### Example 86: (19R)-3-ethyl-16-fluoro-10,19-dimethyl-5-(1,3-oxazol-5-ylethynyl)-20-oxa-3,4,10,11,23-pentaazapentacyclo[19.3.1.0^{8,12}.0^{2,6}.0^{13,18}]pentacosa-1(25),2(6),4,8(9),11,13(14),1 5,17,21(22),23-decaen-22-amine

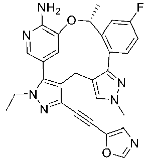

The synthetic method of Example 86 is the same as that of Example 47. LC-MS (ESI) [M+H]⁺: 510.4. ¹H NMR (400 MHz, CDCl₃), 7.91 (s, 1H), 7.53 (d, J = 1.6 Hz, 1H), 7.41 (s, 1H), 7.39 (s, 1H), 7.29 (dd, J = 10.0 Hz, 2.8 Hz, 1H), 7.14 (dd, J = 8.4 Hz, 5.6 Hz, 1H), 7.01 (td, J = 8.4 Hz, 2.8 Hz, 1H), 6.54 (d, J = 1.6 Hz, 1H), 5.39-5.45 (m, 1H), 4.92 (s, 2H), 4.16 (q, J = 7.2 Hz, 2H), 3.92 (s, 3H), 3.82 (d, J = 15.6 Hz, 1H), 2.98 (d, J = 15.6 Hz, 1H), 1.80 (d, J = 6.4 Hz, 3H), 1.43 (t, J = 7.2 Hz, 3H).

### Example 87: (19R)-3-ethyl-16-fluoro-10,19-dimethyl-5-(3,3,3-trifluoroprop-1-yn-1-yl)-2 0-oxa-3,4,10,11,23-pentaazapentacyclo[19.3.1.0^{1,12}.0^{2,6}.0^{13,18}]pentacosa-1(25),2(6),4,8(9),11,13 (14),15,17,21(22),23-decaen-22-amine

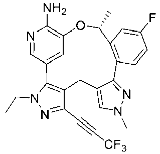

The synthetic method of Example 87 is the same as that of Example 47. LC-MS (ESI) [M+H]⁺: 511.3. ¹H NMR (400 MHz, CDCl₃), 7.47-7.87 (brs, 1H), 7.32 (s, 1H), 7.22-7.26 (m, 1H), 7.17 (dd, J = 8.4 Hz, 5.6 Hz, 1H), 7.05 (td, J = 8.4 Hz, 2.8 Hz, 1H), 6.60 (s, 1H), 6.14-6.48 (brs, 2H), 5.32-5.44 (m, 1H), 4.02-4.24 (m, 2H), 3.93 (s, 3H), 3.78 (d, J = 15.6 Hz, 1H), 2.97 (d, J = 15.6 Hz, 1H), 1.81 (d, J = 6.4 Hz, 3H), 1.40 (t, J = 7.2 Hz, 3H).

### Example 88: (19R)-5-(5-aminopent-1-yn-1-yl)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(24),2(6),4,8(9),11,13(14),1 5,17,21(25),22-decaen-22-amine

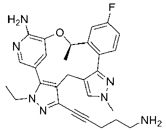

The synthetic method of Example 88 is the same as that of Example 47. LC-MS (ESI) [M+H]⁺: 500.4. ¹H NMR (400 MHz, CDCl₃), 7.44 (d, J = 1.6 Hz, 1H), 7.37 (s, 1H), 7.28 (dd, J = 10.0 Hz, 2.8 Hz, 1H), 7.13 (dd, J = 8.4 Hz, 5.6 Hz, 1H), 6.99 (td, J = 8.4 Hz, 2.4 Hz, 1H), 6.51 (d, J = 1.6 Hz, 1H), 5.22-5.44 (m, 3H), 4.03-4.09 (m, 2H), 3.90 (s, 3H), 3.71 (d, J = 15.6 Hz, 1H), 3.21 (t, J = 7.2 Hz, 2H), 2.87 (d, J = 15.6 Hz, 1H), 2.68 (t, J = 7.2 Hz, 2H), 1.99-2.09 (m, 2H), 1.79 (d, J = 6.0 Hz, 3H), 1.34 (t, J = 7.2 Hz, 3H).

### Example 89: 5-((19R)-22-amino-3-ethyl-16-fluoro-10,19-dimethyl-20-oxa-3,4,10, 11,23 -pentaazapentacyclo[19.3.1.0^{8,12}.0^{13,18}.0^{2,6}]pentacosa-1(24),2(6),4,8(9),11,13(14),15,17,21(25), 22-decaen-5-yl)pent-4-yn-1-ol

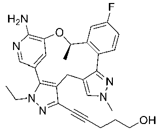

The synthetic method of Example 89 is the same as that of Example 47. LC-MS (ESI) [M+H]⁺: 501.4. ¹H NMR (400 MHz, CDCl₃), 7.49 (d, J = 1.6 Hz, 1H), 7.41 (s, 1H), 7.28 (dd, J = 10.0 Hz, 2.8 Hz, 1H), 7.12 (dd, J = 8.4 Hz, 5.6 Hz, 1H), 6.98 (td, J = 8.4 Hz, 2.8 Hz, 1H), 6.50 (d, J = 1.6 Hz, 1H), 5.36-5.42 (m, 1H), 4.89 (s, 2H), 4.06-4.12 (m, 2H), 3.90 (s, 3H), 3.83 (t, J = 6.0 Hz, 2H), 3.73 (d, J = 15.6 Hz, 1H), 2.87 (d, J = 15.6 Hz, 1H), 2.62 (t, J = 7.2 Hz, 2H), 1.96-2.22 (brs, 1H), 1.88-1.94 (m, 2H), 1.78 (d, J = 6.4 Hz, 3H), 1.38 (t, J = 7.2 Hz, 3H).

### Example 90: (19R)-3-ethyl-16-fluoro-5-(3-methoxyprop-1-yn-1-yl)-10,19-dimethyl-20-o xa-3,4,10,11,23-pentaazapentacyclo[19.3.1.0^{8,12}.0^{13,18}.0^{2,6}]pentacosa-1(24),2(6),4,8(9),11,13(1 4),15,17,21(25),22-decaen-22-amine

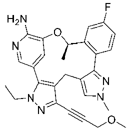

The synthetic method of Example 90 is the same as that of Example 47. LC-MS (ESI) [M+H]⁺: 487.4. ¹H NMR (400 MHz, CDCl₃), 7.49 (d, J = 1.6 Hz, 1H), 7.41 (s, 1H), 7.28 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.53 (d, J = 1.6 Hz, 1H), 5.37-5.44 (m, 1H), 4.99 (s, 2H), 4.42 (s, 2H), 4.09-4.14 (m, 2H), 3.91 (s, 3H), 3.78 (d, J = 15.6 Hz, 1H), 3.50 (s, 3H), 2.91 (d, J = 15.6 Hz, 1H), 1.79 (d, J = 6.4 Hz, 3H), 1.40 (t, J = 7.2 Hz, 3H).

### Example 91: (19R)-3-ethyl-16-fluoro-10,19-dimethyl-5-((methylcyclopropyl)ethynyl)-20 -oxa-3,4,10,11,23-pentaazapentacyclo[19.3.1.0^{8,12}.0^{13,18}.0^{2,6}]pentacosa-1(25),2(6),4,8(9),11,13 (18),14,16,21(22),23-decaen-22-amine

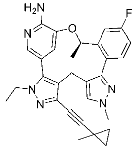

The synthetic method of Example 91 is the same as that of Example 47. LC-MS (ESI) [M+H]⁺: 497.4. ¹H NMR (400 MHz, CDCl₃), 7.50 (s, 1H), 7.41 (s, 1H), 7.26-7.29 (m, 1H), 7.15 (dd, J = 8.8 Hz, 5.6 Hz, 1H), 6.98-7.02 (m, 1H), 6.52 (s, 1H), 5.37-5.43 (m, 1H), 4.78-5.02 (brs, 2H), 4.02-4.12 (m, 2H), 3.92 (s, 3H), 3.74 (d, J = 15.6 Hz, 1H), 2.86 (d, J = 15.6 Hz, 1H), 1.79 (d, J = 6.4 Hz, 3H), 1.41 (s, 3H), 1.36 (t, J = 7.2 Hz, 3H), 1.06-1.09 (m, 2H), 0.71-0.73 (m, 2H).

### Example 92: (19R)-3-ethyl-16-fluoro-10,19-dimethyl-5-((1-methyl imidazol-4-yl)ethyny 1)-20-oxa-3,4,10,11,23-pentaazapentacyclo[19.3.1.0^{8,12}.0^{13,18}.0^{2,6}]pentacosa-1(25),2(6),4,8(9),1 1,13(18),14,16,21(22),23-decaen-22-amine

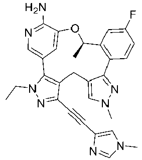

The synthetic method of Example 92 is the same as that of Example 47. LC-MS (ESI) [M+H]⁺: 423.4. ¹H NMR (400 MHz, Acetone), 8.03-8.52 (m, 2H), 7.62 (s, 1H), 7.53 (s, 1H), 7.44 (d, J = 9.6 Hz, 1H), 7.16-7.25 (m, 2H), 6.45 (d, J = 1.2 Hz, 1H), 5.40-5.46 (m, 1H), 4.10-4.20 (m, 1H), 3.86-4.04 (m, 7H), 3.12-3.36 (m, 1H), 2.58-2.74 (m, 1H), 1.73 (d, J = 6.4 Hz, 3H), 1.33 (t, J = 7.2 Hz, 3H).

### Example 93: (19R)-3-ethyl-16-fluoro-10,19-dimethyl-5-(3,4,5,6-tetrahydro-2H-pyran-4-y lethynyl)-20-oxa-3,4,10,11,23-pentaazapentacyclo[19.3.1.0^{2,6}.0^{8,12}.0^{13,18}]pentacosa-1(24),2(6),4, 8(9),11,13(18),14,16,21(25),22-decaen-22-amine

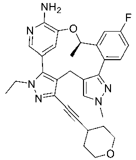

The synthetic method of Example 93 is the same as that of Example 47. LC-MS (ESI) [M+H]⁺: 527.4. ¹H NMR (400 MHz, CDCl₃), 7.41-7.70 (brs, 1H), 7.40 (s, 1H), 7.25-7.28 (m, 1H), 7.13-7.16 (m, 1H), 6.97-7.04 (m, 1H), 6.51 (s, 1H), 5.36-5.42 (m, 1H), 5.11-5.30 (brs, 2H), 4.03-4.14 (m, 2H), 3.94-4.02 (m, 2H), 3.91 (s, 3H), 3.74 (d, J = 15.6 Hz, 1H), 3.54-3.59 (m, 2H), 2.90-2.99 (m, 1H), 2.88 (d, J = 15.6 Hz, 1H), 1.93-2.02 (m, 2H), 1.74-1.89 (m, 5H), 1.34 (t, J = 7.2 Hz, 3H).

### Example 94: (19R)-3-ethyl-16-fluoro-10,19-dimethyl-5-( tetrahydrofuran-3-ylethynyl)-2 0-oxa-3,4,10,11,23-pentaazapentacyclo[19.3.1.0^{8,12}.0^{13,18}.0^{2,6}]pentacosa-1(24),2(6),4,8(9),11,13 (14),15,17,21(25),22-decaen-22-amine

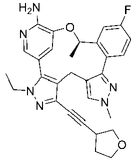

The synthetic method of Example 94 is the same as that of Example 47. LC-MS (ESI) [M+H]⁺: 513.4. ¹H NMR (400 MHz, CDCl₃), 7.50 (d, J = 1.6 Hz, 1H), 7.39 (s, 1H), 7.28 (dd, J = 10.0 Hz, 2.8 Hz, 1H), 7.14 (dd, J = 8.4 Hz, 6.0 Hz, 1H), 6.99 (td, J = 8.4 Hz, 2.8 Hz, 1H), 6.50 (d, J = 1.6 Hz, 1H), 5.37-5.44 (m, 1H), 4.87 (s, 2H), 4.07-4.15 (m, 3H), 3.86-4.03 (m, 5H), 3.79-3.83 (m, 1H), 3.73 (d, J = 15.6 Hz, 1H), 3.26-3.33 (m, 1H), 2.89 (d, J = 15.6 Hz, 1H), 2.29-2.38 (m, 1H), 2.12-2.21 (m, 1H), 1.78 (d, J = 6.0 Hz, 3H), 1.39 (t, J = 7.2 Hz, 3H).

### Example 95: (19R)-5-((1E)-2-cyclopropylvinyl)-3-ethyl-16-fluoro-10,19-dimethyl-20-ox a-3,4,10,11,23-pentaazapentacyclo[19.3.1.0^{8,12}.0^{13,18}.0^{2,6}]pentacosa-1(24),2(6),4,8(9),11,13(14), 15,17,21(25),22-decaen-22-amine

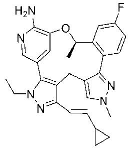

The synthetic method of Example 95 is the same as that of Example 45. LC-MS (ESI) [M+H]⁺: 485.4. ¹H NMR (400 MHz, CDCl₃), 7.50 (d, J = 1.2 Hz, 1H), 7.27-7.30 (m, 2H), 7.14 (dd, J = 8.4 Hz, 6.0 Hz, 1H), 6.99 (dd, J = 8.4 Hz, 2.8 Hz, 1H), 6.59 (d, J = 16.0 Hz, 1H), 6.50 (d, J = 1.6 Hz, 1H), 6.07 (dd, J = 16.0 Hz, 8.8 Hz, 1H), 5.36-5.42 (m, 1H), 4.80 (s, 2H), 4.04-4.15 (m, 2H), 3.91 (s, 3H), 3.62 (d, J = 16.0 Hz, 1H), 2.90 (d, J = 16.0 Hz, 1H), 1.78 (d, J = 6.4 Hz, 3H), 1.57-1.66 (m, 1H), 1.37 (d, J = 7.2 Hz, 3H), 0.82-0.87 (m, 2H), 0.57-0.60 (m, 2H).

### Example 96: (19R)-3-ethyl-16-fluoro-10,19-dimethyl-5-((1-methylpyrrol-2-yl)ethynyl)-2 0-oxa-3,4,10,11,23-pentaazapentacyclo[19.3.1.0^{8,12}.0^{13,18}.0^{2,6}]pentacosa-1(24),2(6),4,8(9),11,13 (14),15,17,21(25),22-decaen-22-amine

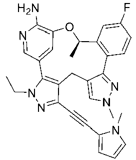

The synthetic method of Example 96 is the same as that of Example 47. LC-MS (ESI) [M+H]⁺: 522.4. ¹H NMR (400 MHz, CDCl₃), 7.78 (s, 1H), 7.46 (s, 1H), 7.24-7.28 (m, 1H), 7.15 (dd, J = 8.4 Hz, 6.0 Hz, 1H), 7.01 (td, J = 8.4 Hz, 2.4 Hz, 1H), 6.69-6.70 (m, 1H), 6.53-6.56 (m, 2H), 6.12-6.14 (m, 1H), 5.58 (s, 2H), 5.35-5.41 (m, 1H), 4.09 (q, J = 7.2 Hz, 2H), 3.90 (s, 3H), 3.82 (d, J = 15.6 Hz, 1H), 3.77 (s, 3H), 2.95 (d, J = 15.6 Hz, 1H), 1.78 (d, J = 6.0 Hz, 3H), 1.32 (t, J = 7.2 Hz, 3H).

### Example 97: (19R)-5-(1,2-pyridazin-4-ylethynyl)-3-ethyl-16-fluoro-10,19-dimethyl-20-o xa-3,4,10,11,23-pentaazapentacyclo[19.3.1.0^{8,12}.0^{13,18}.0^{2,6}]pentacosa-1(25),2(6),4,8(9),11,13(1 8),14,16,21(22),23-decaen-22-amine

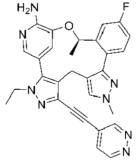

The synthetic method of Example 97 is the same as that of Example 47. LC-MS (ESI) [M+H]⁺: 521.4. ¹H NMR (400 MHz, CDCl₃), 9.28 (s, 1H), 9.20 (d, J = 5.2 Hz, 1H), 7.59 (dd, J = 5.2 Hz, 2.0 Hz, 1H), 7.54 (s, 1H), 7.37 (s, 1H), 7.29 (dd, J = 9.6 Hz, 2.8 Hz, 1H), 7.16 (dd, J = 8.4 Hz, 5.6 Hz, 1H), 7.11 (td, J = 8.4 Hz, 2.8 Hz, 1H), 6.52 (s, 1H), 5.37-5.44 (m, 1H), 5.05 (s, 2H), 4.17 (q, J = 7.2 Hz, 2H), 3.91 (s, 3H), 3.83 (d, J = 15.6 Hz, 1H), 3.01 (d, J = 15.6 Hz, 1H), 1.79 (d, J = 6.4 Hz, 3H), 1.43 (t, J = 7.2 Hz, 3H).

### Example 98: (19R)-3-ethyl-16-fluoro-10,19-dimethyl-5-(1,4-oxaazacyclohexan-2-ylethy nyl)-20-oxa-3,4,10,11,23-pentaazapentacyclo[19.3.1.0^{2,6}.0^{8,12}.0^{13,18}]pentacosa-1(25),2(6),4,8(9), 11,13(14),15,17,21(22),23-decaen-22-amine

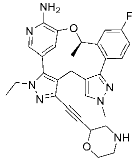

The synthetic method of Example 98 is the same as that of Example 47. LC-MS (ESI) [M+H]⁺: 528.4. ¹H NMR (400 MHz, CDCl₃), 7.51 (s, 1H), 7.43 (s, 0.5H), 7.41 (s, 0.5H), 7.28 (dd, J = 10.0 Hz, 2.8 Hz, 1H), 7.11-7.15 (m, 1H), 7.00 (td, J = 8.4 Hz, 2.8 Hz, 1H), 6.51 (s, 1H), 5.38-5.44 (m, 1H), 4.82 (s, 2H), 4.64-4.71 (m, 1H), 4.01-4.15 (m, 3H), 3.91 (s, 3H), 3.66-3.80 (m, 2H), 3.26-3.30 (m, 1H), 3.06-3.13 (m, 1H), 2.90-3.00 (m, 3H), 1.79 (d, J = 6.0 Hz, 3H), 1.39 (t, J = 7.2 Hz, 3H).

### Example 99: (19R)-3-ethyl-16-fluoro-10,19-dimethyl-5-(1,4-oxaazacyclohexan-3-ylethy nyl)-20-oxa-3,4,10,11,23-pentaazapentacyclo[19.3.1.0^{2,6}.0^{8,12}0^{13,18}]pentacosa-1(25),2(6),4,8(9), 11,13(14),15,17,21(22),23-decaen-22-amine

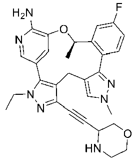

The synthetic method of Example 99 is the same as that of Example 47. LC-MS (ESI) [M+H]⁺: 528.4. ¹H NMR (400 MHz, CDCl₃), 7.50 (s, 1H), 7.46 (s, 1H), 7.28 (dd, J = 10.0 Hz, 2.8 Hz, 1H), 7.13 (dd, J = 8.4 Hz, 5.6 Hz, 1H), 6.99 (td, J = 8.4 Hz, 2.8 Hz, 1H), 6.50 (s, 1H), 5.36-5.43 (m, 1H), 4.85 (s, 2H), 4.07-4.14 (m, 2H), 3.99-4.06 (m, 2H), 3.91 (s, 3H), 3.65-3.84 (m, 4H), 3.12-3.20 (m, 1H), 2.88-2.99 (m, 2H), 1.78 (d, J = 6.0 Hz, 3H), 1.37-1.41 (m, 3H).

### Example 100: (19R)-3-ethyl-16-fluoro-10,19-dimethyl-5-(tetrahydrofuran-2-ylethynyl)-2 0-oxa-3,4,10,11,23-pentaazapentacyclo[19.3.1.0^{8,12}.0^{13,18}.0^{2,6}]pentacosa-1(24),2(6),4,8(9),11,13 (14),15,17,21(25),22-decaen-22-amine

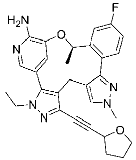

The synthetic method of Example 100 is the same as that of Example 47. LC-MS (ESI) [M+H]⁺: 513.4. ¹H NMR (400 MHz, CDCl₃), 7.50 (s, 1H), 7.43 (s, 0.5H), 7.42 (s, 0.5H), 7.28 (dd, J = 10.0 Hz, 2.8 Hz, 1H), 7.11-7.15 (m, 1H), 6.99 (td, J = 8.4 Hz, 2.8 Hz, 1H), 6.51 (s, 1H), 5.38-5.43 (m, 1H), 4.90-4.94 (m, 1H), 4.85 (s, 2H), 3.99-4.15 (m, 3H), 3.86-3.92 (m, 4H), 3.76 (d, J = 15.6 Hz, 1H), 2.89 (d, J = 15.6 Hz, 1H), 2.08-2.32 (m, 2.5H), 1.90-2.04 (m, 1.5H), 1.78 (d, J = 6.0 Hz, 3H), 1.38 (t, J = 7.2 Hz, 3H).

### Example 101: (19R)-3-ethyl-16-fluoro-10,19-dimethyl-5-((1-methyl imidazol-4-yl)ethyn yl)-20-oxa-3,4,9,23-tetraaza-11-thiapentacyclo[19.3.1.0^{8,12}.0^{13,18}.0^{2,6}]pentacosa-1(25),2(6),4,8(1 2),9,13(18),14,16,21(22),23-decaen-22-amine

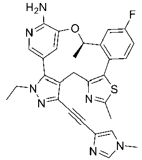

The synthetic method of Example 101 is the same as that of Example 47. LC-MS (ESI) [M+H]⁺: 540.4. ¹H NMR (400 MHz, CD₃OD+CDCl₃), 7.91 (s, 1H), 7.58-7.79 (brs, 1H), 7.23-7.26 (m, 2H), 7.10-7.15 (m, 1H), 7.00 (td, J = 8.0 Hz, 2.4 Hz, 1H), 6.71 (s, 1H), 5.35-5.43 (m, 1H), 4.00-4.16 (m, 2H), 3.82-3.92 (m, 1H), 3.75 (s, 3H), 2.96-3.10 (m, 1H), 2.64 (s, 3H), 1.70 (d, J = 6.4 Hz, 3H), 1.35 (t, J = 7.2 Hz, 3H).

### Example 102: (19R)-3-ethyl-16-fluoro-5-(4-fluorobut-1-yn-1-yl)-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,13(18),1 4,16,21(22),23-decaen-22-amine

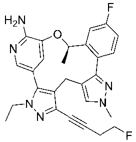

The synthetic method of Example 102 is the same as that of Example 47. LC-MS (ESI) [M+H]⁺: 489.4. ¹H NMR (400 MHz, CDCl₃), 7.48 (s, 1H), 7.43 (s, 1H), 7.28 (dd, J = 10.0 Hz, 2.8 Hz, 1H), 7.15 (dd, J = 8.4 Hz, 5.6 Hz, 1H), 7.01 (td, J = 8.4 Hz, 2.8 Hz, 1H), 6.57 (s, 1H), 5.06-5.57 (m, 3H), 4.65 (dt, J = 46.4 Hz, 6.4 Hz, 2H), 4.06-4.12 (m, 2H), 3.91 (s, 3H), 3.77 (d, J = 15.6 Hz, 1H), 2.94 (dt, J = 20 Hz, 6.4 Hz, 2H), 2.89 (d, J = 15.6 Hz, 1H), 1.80 (d, J = 6.4 Hz, 3H), 1.39 (t, J = 7.2 Hz, 3H).

### Example 103: bis((19R)-3-ethyl-16-fluoro-5-((5-methoxypyridin-2-yl)ethynyl)-10,19-di methyl-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine)

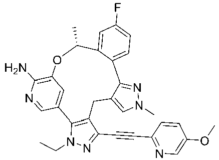

The synthetic method of Example 103 is the same as that of Example 47. LC-MS (ESI) [M+H]⁺: 550.4. ¹H NMR (400 MHz, CDCl₃), 8.33 (d, J = 3.2 Hz, 1H), 7.56 (d, J = 5.2 Hz, 1H), 7.53 (s, 1H), 7.50 (s, 1H), 7.28 (dd, J = 10.0 Hz, 2.8 Hz, 1H), 7.19 (dd, J = 8.8 Hz, 2.8 Hz, 1H), 7.13 (dd, J = 8.4 Hz, 5.6 Hz, 1H), 6.99 (td, J = 8.4 Hz, 2.8 Hz, 1H), 6.55 (s, 1H), 5.39-5.45 (m, 1H), 4.88 (s, 2H), 4.12-4.18 (m, 2H), 3.92 (d, J = 15.6 Hz, 1H), 3.88 (s, 6H), 2.95 (d, J = 15.6 Hz, 1H), 1.78 (d, J = 6.0 Hz, 3H), 1.42 (t, J = 7.2 Hz, 3H).

### Example 104: (19R)-5-(3-(dimethylamino)prop-1-yn-1-yl)-3-ethyl-16-fluoro-10,19-dime thyl-20-oxa-3,4,10,11,23-pentaazapentacyclo[19.3.1.0^{8,12}.0^{13,18}.0^{2,6}]pentacosa-1(25),2(6),4,8(9), 11,13(18),14,16,21(22),23-decaen-22-amine

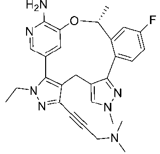

The synthetic method of Example 104 is the same as that of Example 47. LC-MS (ESI) [M+H]⁺: 500.4. ¹H NMR (400 MHz, CDCl₃), 7.50 (d, J = 2.0 Hz, 1H), 7.42 (s, 1H), 7.29 (dd, J = 10.0 Hz, 2.8 Hz, 1H), 7.13 (dd, J = 8.4 Hz, 5.6 Hz, 1H), 6.99 (td, J = 8.4 Hz, 2.8 Hz, 1H), 6.50 (d, J = 2.0 Hz, 1H), 5.38-5.43 (m, 1H), 4.81 (s, 2H), 4.08-4.14 (m, 2H), 3.90 (s, 3H), 3.77 (d, J = 15.6 Hz, 1H), 3.61 (s, 2H), 2.91 (d, J = 15.6 Hz, 1H), 2.41 (s, 6H), 1.78 (d, J = 6.0 Hz, 3H), 1.39 (t, J = 7.2 Hz, 3H).

### Example 105: (19R)-3-ethyl-16-fluoro-10,19-dimethyl-5-(3-(methylamino)prop-1-yn-1-y l)-20-oxa-3,4,10,11,23-pentaazapentacyclo[19.3.1.0^{8,12}.0^{13,18}.0^{2,6}]pentacosa-1(25),2(6),4,8(9),1 1,13(18),14,16,21(22),23-decaen-22-amine

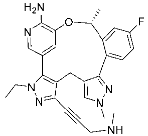

The synthetic method of Example 105 is the same as that of Example 47. LC-MS (ESI) [M+H]⁺: 486.4. ¹H NMR (400 MHz, CDCl₃), 7.45-7.56 (m, 2H), 7.28 (d, J = 10.0 Hz, 2.8 Hz, 1H), 7.11 (dd, J = 8.4 Hz, 2.8 Hz, 1H), 6.98 (td, J = 8.4 Hz, 2.8 Hz, 1H), 6.49 (s, 1H), 5.36-5.42 (m, 1H), 4.90 (s, 2H), 4.07-4.13 (m, 2H), 3.83-4.01 (m, 5H), 3.73 (d, J = 15.6 Hz, 1H), 2.89 (d, J = 15.6 Hz, 1H), 2.74 (s, 3H), 1.77 (d, J = 6.4 Hz, 3H), 1.38 (t, J = 7.2 Hz, 3H).

### Example 106: (19R)-3-ethyl-16-fluoro-10,19-dimethyl-5-(3,4,5,6-tetrahydro-2H-pyran-3-ylethynyl)-20-oxa-3,4,10,11,23-pentaazapentacyclo[19.3.1.0^{8,12}.0^{13,18}.0^{2,6}]pentacosa-1(25),2(6), 4,8(9),11,13(18),14,16,21(22),23-decaen-22-amine

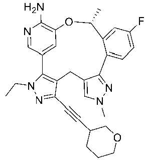

The synthetic method of Example 106 is the same as that of Example 47. LC-MS (ESI) [M+H]⁺: 527.4. ¹H NMR (400 MHz, CDCl₃), 7.40-7.56 (m, 2H), 7.28 (dd, J = 10.0 Hz, 2.8 Hz, 1H), 7.15 (dd, J = 8.4 Hz, 5.6 Hz, 1H), 7.01 (td, J = 8.4 Hz, 2.8 Hz, 1H), 6.53 (s, 1H), 5.38-5.45 (m, 1H), 5.01-5.24 (brs, 2H), 4.03-4.14 (m, 3H), 3.84-3.94 (m, 4H), 3.74 (d, J = 15.2 Hz, 1H), 3.46-3.56 (m, 2H), 2.84-2.91 (m, 2H), 2.15-2.23 (m, 1H), 1.80 (d, J = 6.4 Hz, 3H), 1.59-1.77 (m, 3H), 1.39 (t, J = 7.2 Hz, 3H).

### Example 107: (19R)-3-ethyl-16-fluoro-10,19-dimethyl-5-(3-(1,4-oxaazacyclohexan-4-yl) prop-1-yn-1-yl)-20-oxa-3,4,10,11,23-pentaazapentacyclo[19.3.1.0^{8,12}.0^{13,18}.0^{2,6}]pentacosa-1(24), 2(6),4,8(9),11,13(14),15,17,21(25),22-decaen-22-amine

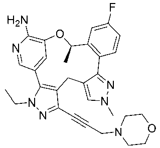

The synthetic method of Example 107 is the same as that of Example 47. LC-MS (ESI) [M+H]⁺: 542.4. ¹H NMR (400 MHz, CDCl₃), 7.51 (d, J = 1.6 Hz, 1H), 7.43 (s, 1H), 7.29 (dd, J = 10.0 Hz, 2.8 Hz, 1H), 7.13 (dd, J = 8.4 Hz, 2.8 Hz, 1H), 6.99 (td, J = 8.4 Hz, 2.8 Hz, 1H), 6.50 (d, J = 1.6 Hz, 1H), 5.37-5.43 (m, 1H), 4.83 (s, 2H), 4.07-4.16 (m, 2H), 3.91 (s, 3H), 3.73-3.78 (m, 5H), 3.65 (s, 2H), 2.91 (d, J = 15.6 Hz, 1H), 2.69-2.72 (m, 4H), 1.79 (d, J = 6.0 Hz, 3H), 1.39 (t, J = 7.2 Hz, 3H).

### Example 108: (19R)-3-ethyl-16-fluoro-10,19-dimethyl-5-(3-(4-methylpiperazin-1-yl)pro p-1-yn-1-yl)-20-oxa-3,4,10,11,23-pentaazapentacyclo[19.3.1.0^{8,12}.0^{13,18}.0^{2,6}]pentacosa-1(24),2 (6),4,8(9),11,13(14),15,17,21(25),22-decaen-22-amine

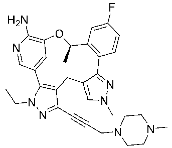

The synthetic method of Example 108 is the same as that of Example 47. LC-MS (ESI) [M+H]⁺: 555.4. ¹H NMR (400 MHz, CDCl₃), 7.49 (d, J = 2.0 Hz, 1H), 7.45 (s, 1H), 7.28 (dd, J = 10.0 Hz, 2.8 Hz, 1H), 7.13 (dd, J = 8.4 Hz, 5.6 Hz, 1H), 6.98 (td, J = 8.4 Hz, 2.8 Hz, 1H), 6.49 (d, J = 2.0 Hz, 1H), 5.36-5.43 (m, 1H), 4.83 (s, 2H), 4.07-4.13 (m, 2H), 3.93 (s, 3H), 3.75 (d, J = 15.6 Hz, 1H), 3.66 (s, 2H), 2.57-3.02 (m, 9H), 2.48 (s, 3H), 1.78 (d, J = 6.4 Hz, 3H), 1.38 (t, J = 7.2 Hz, 3H).

### Example 109: (19R)-3-ethyl-16-fluoro-10,19-dimethyl-5-(1,3-thiazol-2-ylethynyl)-20-ox a-3,4,10,11,23-pentaazapentacyclo[19.3.1.0^{8,12}.0^{13,18}.0^{2,6}]pentacosa-1(24),2(6),4,8(9),11,13(14), 15,17,21(25),22-decaen-22-amine

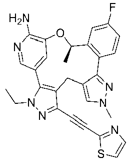

The synthetic method of Example 109 is the same as that of Example 47. LC-MS (ESI) [M+H]⁺: 526.4. ¹H NMR (400 MHz, CDCl₃), 7.90 (d, J = 2.8 Hz, 1H), 7.46 (s, 1H), 7.44 (d, J = 2.8 Hz, 1H), 7.24-7.40 (m, 2H), 7.17 (dd, J = 8.4 Hz, 6.0 Hz, 1H), 7.06 (td, J = 8.4 Hz, 2.8 Hz, 1H), 6.69 (s, 1H), 6.21-6.52 (brs, 2H), 5.44-5.50 (m, 1H), 4.09-4.17 (m, 2H), 3.95 (d, J = 15.6 Hz, 1H), 3.90 (s, 3H), 2.97 (d, J = 15.6 Hz, 1H), 1.84 (d, J = 6.4 Hz, 3H), 1.45 (t, J = 7.2 Hz, 3H).

### Example 110: 3-((19R)-22-amino-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(25),2(6),4,8(9),11,13(14),15,17,21(22),2 3-decaen-5-yl)-1-(4-methylpiperazin-1-yl)prop-2-yn-1-one

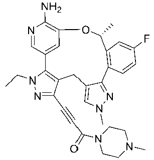

The synthetic method of Example 110 is the same as that of Example 47. LC-MS (ESI) [M+H]⁺: 569.4. ¹H NMR (400 MHz, CDCl₃), 7.51 (s, 1H), 7.40 (s, 1H), 7.28 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.53 (s, 1H), 5.38-5.44 (m, 1H), 4.88 (s, 2H), 4.13-4.19 (m, 2H), 3.97-4.08 (m, 2H), 3.91 (s, 3H), 3.68-3.90 (m, 3H), 2.96 (d, J = 15.6 Hz, 1H), 2.28-2.80 (m, 7H), 1.79 (d, J = 6.4 Hz, 3H), 1.41 (t, J = 7.2 Hz, 3H).

### Example 111: 3-((19R)-22-amino-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(25),2(6),4,8(9),11,13(14),15,17,21(22),2 3-decaen-5-yl)-1-(1,4-oxaazacyclohexan-4-yl)prop-2-yn-1-one

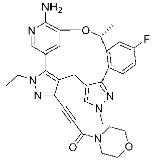

The synthetic method of Example 111 is the same as that of Example 47. LC-MS (ESI) [M+H]⁺: 556.4. ¹H NMR (400 MHz, CDCl₃), 7.48 (d, J = 1.6 Hz, 1H), 7.41 (s, 1H), 7.28 (dd, J = 10.0 Hz, 2.8 Hz, 1H), 7.13 (dd, J = 8.4 Hz, 5.6 Hz, 1H), 7.02 (td, J = 8.4 Hz, 2.8 Hz, 1H), 6.56 (d, J = 1.6 Hz, 1H), 5.40-5.46 (m, 1H), 5.13-5.27 (brs, 2H), 4.12-4.18 (m, 2H), 3.88-3.93 (m, 5H), 3.84 (d, J = 15.6 Hz, 1H), 3.75-3.77 (m, 2H), 3.72 (s, 4H), 2.96 (d, J = 15.6 Hz, 1H), 1.80 (d, J = 6.4 Hz, 3H), 1.41 (t, J = 6.4 Hz, 3H).

### Example 112: (19R)-3-ethyl-16-fluoro-10,19-dimethyl-5-(3,4,5,6-tetrahydro-2H-pyran-2-ylethynyl)-20-oxa-3,4,10,11,23-pentaazapentacyclo[19.3.1.0^{8,12}.0^{13,18}.0^{2,6}]pentacosa-1(25),2(6), 4,8(9),11,13(18),14,16,21(22),23-decaen-22-amine

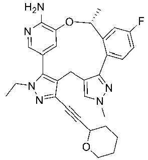

The synthetic method of Example 112 is the same as that of Example 47. LC-MS (ESI) [M+H]⁺: 527.4. ¹H NMR (400 MHz, CDCl₃), 7.42-7.54 (m, 2H), 7.28 (d, J = 10.0 Hz, 2.8 Hz, 1H), 7.12-7.16 (m, 1H), 7.00 (td, J = 8.4 Hz, 2.8 Hz, 1H), 6.54 (s, 1H), 5.39-5.45 (m, 1H), 4.96-5.20 (brs, 2H), 4.63-4.67 (m, 1H), 4.04-4.16 (m, 3H), 3.91 (s, 3H), 3.77-3.82 (m, 1H), 3.60-3.66 (m, 1H), 2.90 (d, J = 15.6 Hz, 1H), 1.91-2.03 (m, 2H), 1.79-1.90 (m, 4H), 1.52-1.75 (m, 3H), 1.39 (t, J = 7.2 Hz, 3H).

### Example 113: (19R)-3-ethyl-16-fluoro-5-(3-(piperidin-1-yl)prop-1-yn-1-yl)-10,19-dimeth yl-20-oxa-3,4,10,11,23-pentaazapentacyclo[19.3.1.0^{8,12}.0^{13,18}.0^{2,6}]pentacosa-1(24),2(6),4,8(9),1 1,13(14),15,17,21(25),22-decaen-22-amine

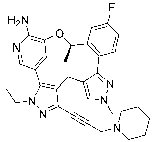

The synthetic method of Example 113 is the same as that of Example 47. LC-MS (ESI) [M+H]⁺: 540.4. ¹H NMR (400 MHz, CDCl₃), 7.48-7.55 (brs, 1H), 7.46 (s, 1H), 7.29 (dd, J = 10.0 Hz, 2.8 Hz, 1H), 7.13 (dd, J = 8.8 Hz, 5.6 Hz, 1H), 7.00 (td, J = 8.4 Hz, 2.8 Hz, 1H), 6.51 (s, 1H), 5.37-5.43 (m, 1H), 4.74-4.94 (brs, 2H), 4.07-4.16 (m, 2H), 3.92 (s, 3H), 3.65-3.78 (m, 3H), 2.90 (d, J = 15.6 Hz, 1H), 2.60-2.84 (m, 4H), 1.79 (d, J = 6.0 Hz, 3H), 1.66-1.76 (m, 4H), 1.43-1.52 (m, 2H), 1.39 (t, J = 7.2 Hz, 3H).

### Example 114: (19R)-5-(3-(4-cyclopropylpiperazin-1-yl)prop-1-yn-1-yl)-3-ethyl-16-fluor o-10,19-dimethyl-20-oxa-3,4,10,11,23-pentaazapentacyclo[19.3.1.0^{8,12}.0^{2,6}.0^{13,18}]pentacosa-1(2 4),2(6),4,8(9),11,13(14),15,17,21(25),22-decaen-22-amine

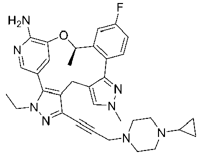

The synthetic method of Example 114 is the same as that of Example 47. LC-MS (ESI) [M+H]⁺: 581.4. ¹H NMR (400 MHz, CDCl₃), 7.50 (d, J = 1.6 Hz, 1H), 7.42 (s, 1H), 7.28 (dd, J = 10.0 Hz, 2.8 Hz, 1H), 7.12 (dd, J = 8.4 Hz, 5.6 Hz, 1H), 6.99 (td, J = 8.4 Hz, 2.8 Hz, 1H), 6.49 (d, J = 1.6 Hz, 1H), 5.36-5.42 (m, 1H), 4.89 (s, 2H), 4.05-4.14 (m, 2H), 3.90 (s, 3H), 3.74 (d, J = 15.6 Hz, 1H), 3.64 (s, 2H), 2.88 (d, J = 15.6 Hz, 1H), 2.60-2.84 (m, 8H), 1.77 (d, J = 6.0 Hz, 3H), 1.60-1.70 (m, 1H), 1.37 (t, J = 7.2 Hz, 3H), 0.37-0.51 (m, 4H).

### Example 115: (19R)-3-ethyl-16-fluoro-10,19-dimethyl-5-((4-(trifluoromethyl)phenyl)eth ynyl)-20-oxa-3,4,10,11,23-pentaazapentacyclo[19.3. 1.0^{8,12}.0^{13,18}.0^{2,6}]pentacosa-1(25),2(6),4,8 (9),11,13(18),14,16,21(22),23-decaen-22-amine

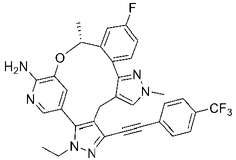

The synthetic method of Example 115 is the same as that of Example 47. LC-MS (ESI) [M+H]⁺: 587.4. ¹H NMR (400 MHz, CDCl₃), 7.70 (d, J = 8.4 Hz, 2H), 7.63 (d, J = 8.4 Hz, 2H), 7.45-7.60 (brs, 1H), 7.44 (s, 1H), 7.30 (dd, J = 9.6 Hz, 2.8 Hz, 1H), 7.17 (dd, J = 8.4 Hz, 5.6 Hz, 1H), 7.02 (td, J = 8.4 Hz, 2.8 Hz, 1H), 6.55 (s, 1H), 5.39-5.46 (m, 1H), 4.85-5.04 (brs, 2H), 4.12-4.21 (m, 2H), 3.91 (s, 3H), 3.86 (d, J = 15.6 Hz, 1H), 2.99 (d, J = 15.6 Hz, 1H), 1.80 (d, J = 6.4 Hz, 3H), 1.44 (t, J = 7.2 Hz, 3H).

### Example 116: (19R)-3-ethyl-16-fluoro-5-((4-fluorophenyl)ethynyl)-10,19-dimethyl-20-o xa-3,4,10,11,23-pentaazapentacyclo[19.3.1.0^{8,12}.0^{13,18}.0^{2,6}]pentacosa-1(25),2(6),4,8(9),11,13(1 8),14,16,21(22),23-decaen-22-amine

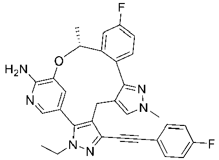

The synthetic method of Example 116 is the same as that of Example 47. LC-MS (ESI) [M+H]⁺: 537.4. ¹H NMR (400 MHz, CDCl₃), 7.50-7.63 (m, 3H), 7.45 (s, 1H), 7.28-7.30 (m, 1H), 7.14-7.17 (m, 1H), 6.96-7.10 (m, 3H), 6.54 (s, 1H), 5.36-5.45 (m, 1H), 5.02 (s, 2H), 4.05-4.20 (m, 2H), 3.90 (s, 3H), 3.84 (d, J = 15.6 Hz, 1H), 2.95 (d, J = 15.6 Hz, 1H), 1.79 (d, J = 5.6 Hz, 3H), 1.39 (t, J = 7.2 Hz, 3H).

### Example 117: 3-((19R)-22-amino-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(25),2(6),4,8(9),11,13(14),15,17,21(22),2 3-decaen-5-yl)-N-cyclopropylprop-2-ynamide

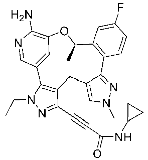

The synthetic method of Example 117 is the same as that of Example 47. LC-MS (ESI) [M+H]⁺: 526.4. ¹H NMR (400 MHz, CDCl₃), 7.42 (d, J = 2.4 Hz, 1H), 7.38 (s, 1H), 7.25-7.29 (m, 1H), 7.14 (dd, J = 8.4 Hz, 5.6 Hz, 1H), 7.04 (td, J = 8.4 Hz, 2.4 Hz, 1H), 6.60 (s, 1H), 6.12 (s, 1H), 5.48-5.84 (brs, 2H), 5.40-5.46 (m, 1H), 4.12 (q, J = 7.2 Hz, 2H), 3.91 (s, 3H), 3.84 (d, J = 15.6 Hz, 1H), 2.93 (d, J = 15.6 Hz, 1H), 2.78-2.85 (m, 1H), 1.81 (d, J = 6.4 Hz, 3H), 1.41 (t, J = 7.2 Hz, 3H), 0.84-0.89 (m, 2H), 0.59-0.63 (m, 2H).

### Example 118: 3-((19R)-22-amino-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(25),2(6),4,8(9),11,13(14),15,17,21(22),2 3-decaen-5-yl)-1-(piperidin-1-yl)prop-2-yn-1-one

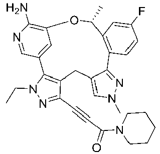

The synthetic method of Example 118 is the same as that of Example 47. LC-MS (ESI) [M+H]⁺: 554.4. ¹H NMR (400 MHz, CDCl₃), 7.48 (s, 1H), 7.44 (s, 1H), 7.28 (dd, J = 10.0 Hz, 2.8 Hz, 1H), 7.13 (dd, J = 8.4 Hz, 5.6 Hz, 1H), 7.01 (td, J = 8.4 Hz, 2.4 Hz, 1H), 6.56 (s, 1H), 5.40-5.45 (m, 1H), 5.18 (s, 2H), 4.08-4.21 (m, 2H), 3.90 (s, 3H), 3.83-3.86 (m, 3H), 3.63-3.66 (m, 2H), 2.94 (d, J = 15.6 Hz, 1H), 1.80 (d, J = 6.4 Hz, 3H), 1.56-1.74 (m, 6H), 1.40 (t, J = 7.2 Hz, 3H).

### Example 119: (19R)-5-((5-chloropyridin-2-yl)ethynyl)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,1 3(18),14,16,21(22),23-decaen-22-amine

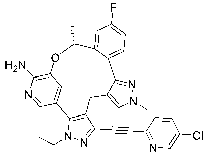

The synthetic method of Example 119 is the same as that of Example 47. LC-MS (ESI) [M+H]⁺: 554.3. ¹H NMR (400 MHz, CDCl₃), 8.59 (d, J = 2.4 Hz, 1H), 7.69 (dd, J = 8.4 Hz, 2.4 Hz, 1H), 7.51-7.59 (m, 2H), 7.46 (s, 1H), 7.28 (dd, J = 10.0 Hz, 2.4 Hz, 1H), 7.14 (dd, J = 8.4 Hz, 5.6 Hz, 1H), 7.01 (td, J = 8.4 Hz, 2.4 Hz, 1H), 6.57 (s, 1H), 5.37-5.45 (m, 1H), 4.82-5.22 (brs, 2H), 4.09-4.20 (m, 2H), 3.86-3.94 (m, 4H), 2.97 (d, J = 15.6 Hz, 1H), 1.79 (d, J = 6.4 Hz, 3H), 1.42 (t, J = 7.2 Hz, 3H).

### Example 120: 3-((19R)-22-amino-3-ethyl-16-fluoro-10,19-dimethyl-20-oxa-3,4,10,11,23 -pentaazapentacyclo[19.3.1.0^{2,6}.0^{8,12}.0^{13,18}]pentacosa-1(24),2(6),4,8(9),11,13(14),15,17,21(25), 22-decaen-5-yl)prop-2-enenitrile

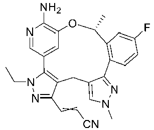

The synthetic method of Example 120 is the same as that of Example 45. LC-MS (ESI) [M+H]⁺: 469.4. ¹H NMR (400 MHz, CDCl₃), 7.46-7.58 (m, 2H), 7.24-7.30 (m, 1H), 7.12-7.20 (m, 2H), 7.04-7.09 (m, 1H), 6.61 (s, 1H), 6.19-6.46 (m, 3H), 5.36-5.46 (m, 1H), 4.06-4.22 (m, 2H), 3.93 (s, 3H), 3.66 (d, J = 15.6 Hz, 1H), 2.99 (d, J = 15.6 Hz, 1H), 1.82 (d, J = 6.0 Hz, 3H), 1.41 (t, J = 7.2 Hz, 3H).

### Example 121: (19R)-3-ethyl-16-fluoro-5-((4-methoxyphenyl)ethynyl)-10,19-dimethyl-20 -oxa-3,4,10,11,23-pentaazapentacyclo[19.3.1.0^{8,12}.0^{13,18}.0^{2,6}]pentacosa-1(24),2(6),4,8(9),11,13 (14),15,17,21(25),22-decaen-22-amine

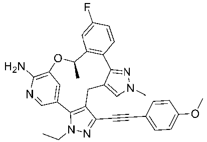

The synthetic method of Example 121 is the same as that of Example 47. LC-MS (ESI) [M+H]⁺: 549.4. ¹H NMR (400 MHz, CD₃OD+CDCl₃), 7.38-7.48 (m, 3H), 7.29-7.34 (m, 1H), 7.22 (s, 1H), 7.11-7.14 (m, 1H), 6.99-7.03 (m, 1H), 6.83 (d, J = 8.4 Hz, 2H), 8.62 (s, 1H), 5.29-5.39 (m, 1H), 3.96-4.12 (m, 2H), 3.81-3.85 (m, 4H), 3.76 (s, 3H), 2.85 (d, J = 16.0 Hz, 1H), 1.80 (d, J = 5.6 Hz, 3H), 1.29-1.39 (m, 3H).

### Example 122: 3-(((19R)-22-amino-3-ethyl-16-fluoro-10,19-dimethyl-20-oxa-3,4, 10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)oxetan-3-ol

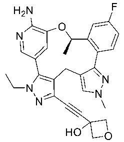

The synthetic method of Example 122 is the same as that of Example 47. LC-MS (ESI) [M+H]⁺: 515.4. ¹H NMR (400 MHz, CDCl₃), 7.36 (s, 1H), 7.22-7.31 (m, 2H), 7.10 (dd, J = 8.4 Hz, 5.6 Hz, 1H), 6.99 (td, J = 8.4 Hz, 2.4 Hz, 1H), 6.49 (s, 1H), 5.34-5.44 (m, 1H), 5.08-5.32 (brs, 2H), 5.00 (d, J = 6.8 Hz, 1H), 4.94 (d, J = 6.8 Hz, 1H), 4.83-4.85 (m, 2H), 3.98-4.15 (m, 2H), 3.90 (s, 3H), 3.63 (d, J = 15.6 Hz, 1H), 2.79 (d, J = 15.6 Hz, 1H), 1.79 (d, J = 5.6 Hz, 3H), 1.32 (t, J = 6.4 Hz, 3H).

### Example 123: (19R)-3-ethyl-16-fluoro-10,19-dimethyl-5-(1,3-oxazol-4-ylethynyl)-20-ox a-3,4,10,11,23-pentaazapentacyclo[19.3.1.0^{8,12}.0^{13,18}.0^{2,6}]pentacosa-1(24),2(6),4,8(9),11,13(14), 15,17,21(25),22-decaen-22-amine

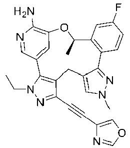

The synthetic method of Example 123 is the same as that of Example 47. LC-MS (ESI) [M+H]⁺: 510.4. ¹H NMR (400 MHz, CDCl₃), 7.97 (s, 1H), 7.94 (s, 1H), 7.47 (s, 1H), 7.24-7.31 (m, 2H), 7.18 (dd, J = 8.4 Hz, 5.6 Hz, 1H), 7.09 (td, J = 8.4 Hz, 2.4 Hz, 1H), 6.76 (s, 1H), 5.46-5.53 (m, 1H), 4.02-4.17 (m, 2H), 3.94 (d, J = 15.6 Hz, 1H), 3.91 (s, 3H), 2.95 (d, J = 15.6 Hz, 1H), 1.86 (d, J = 5.6 Hz, 3H), 1.43 (t, J = 7.2 Hz, 3H).

### Example 124: 5-((19R)-22-amino-3-ethyl-16-fluoro-10,19-dimethyl-20-oxa-3,4,10,11,23 -pentaazapentacyclo[19.3.1.0^{8,12}.0^{13,18}.0^{2,6}]pentacosa-1(25),2(6),4,8(9),11,13(18),14,16,21(22), 23-decaen-5-yl)-2-methylpent-4-yn-2-ol

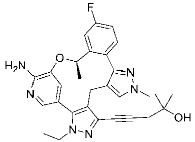

The synthetic method of Example 124 is the same as that of Example 47. LC-MS (ESI) [M+H]⁺: 515.4. ¹H NMR (400 MHz, CDCl₃), 7.50 (d, J = 1.6 Hz, 1H), 7.42 (s, 1H), 7.28 (dd, J = 10.0 Hz, 2.8 Hz, 1H), 7.13 (dd, J = 8.4 Hz, 5.6 Hz, 1H), 6.99 (td, J = 8.4 Hz, 2.8 Hz, 1H), 6.50 (d, J = 1.6 Hz, 1H), 5.37-5.43 (m, 1H), 4.91 (s, 2H), 4.05-4.15 (m, 2H), 3.90 (s, 3H), 3.74 (d, J = 15.6 Hz, 1H), 2.90 (d, J = 15.6 Hz, 1H), 2.71 (s, 2H), 2.06-2.20 (brs, 1H), 1.78 (d, J = 6.0 Hz, 3H), 1.41 (s, 6H), 1.38 (t, J = 7.2 Hz, 3H).

### Example 125: (19R)-5-((3,5-dimethoxyphenyl)ethynyl)-3-ethyl-16-fluoro-10,19-dimethy 1-20-oxa-3,4,10,11,23-pentaazapentacyclo[19.3.1.0^{8,12}.0^{13,18}.0^{2,6}]pentacosa-1(25),2(6),4,8(9),11, 13(18),14,16,21(22),23-decaen-22-amine

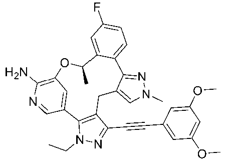

The synthetic method of Example 125 is the same as that of Example 47. LC-MS (ESI) [M+H]⁺: 579.4. ¹H NMR (400 MHz, CDCl₃), 7.49-7.58 (brs, 1H), 7.46 (s, 1H), 7.29 (dd, J = 10.0 Hz, 2.8 Hz, 1H), 7.16 (dd, J = 8.4 Hz, 5.6 Hz, 1H), 7.01 (td, J = 8.4 Hz, 2.8 Hz, 1H), 6.76 (d, J = 2.0 Hz, 2H), 6.54 (s, 1H), 6.48 (t, J = 2.0 Hz, 1H), 5.38-5.45 (m, 1H), 4.92 (s, 2H), 4.11-4.16 (m, 2H), 3.90 (s, 3H), 3.86 (d, J = 15.6 Hz, 1H), 3.81 (s, 6H), 2.96 (d, J = 15.6 Hz, 1H), 1.79 (d, J = 6.4 Hz, 3H), 1.40 (t, J = 7.2 Hz, 3H).

### Example 126: 2-(4-(3-((19R)-22-amino-3-ethyl-16-fluoro-10,19-dimethyl-20-oxa-3,4,10, 11,23-pentaazapentacyclo[19.3.1.0^{8,12}.0^{2,6}.0^{13,18}]pentacosa-1(24),2(6),4,8(9),11,13(14),15,17,21 (25),22-decaen-5-yl)prop-2-yn-1-yl)piperazin-1-yl)ethan-1-ol

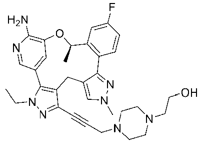

The synthetic method of Example 126 is the same as that of Example 47. LC-MS (ESI) [M+H]⁺: 585.5. ¹H NMR (400 MHz, CDCl₃), 7.48 (d, J = 1.6 Hz, 1H), 7.42 (s, 1H), 7.27 (dd, J = 10.0 Hz, 2.8 Hz, 1H), 7.12 (dd, J = 8.4 Hz, 5.6 Hz, 1H), 6.98 (td, J = 8.4 Hz, 2.8 Hz, 1H), 6.48 (d, J = 1.6 Hz, 1H), 5.35-5.42 (m, 1H), 4.85 (s, 2H), 4.05-4.14 (m, 2H), 3.91 (s, 3H), 3.74 (d, J = 15.6 Hz, 1H), 3.69 (t, J = 5.2 Hz, 2H), 3.65 (s, 2H), 2.89 (d, J = 15.6 Hz, 1H), 2.61-2.88 (m, 10H), 1.76 (d, J = 6.4 Hz, 3H), 1.37 (t, J = 7.2 Hz, 3H).

### Example 127: (19R)-5-((4-chlorophenyl)ethynyl)-3-ethyl-16-fluoro-10,19-dimethyl-20-o xa-3,4,10,11,23-pentaazapentacyclo[19.3.1.0^{8,12}.0^{13,18}.0^{2,6}]pentacosa-1(25),2(6),4,8(9),11,13(1 8),14,16,21(22),23-decaen-22-amine

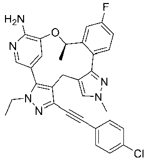

The synthetic method of Example 127 is the same as that of Example 47. LC-MS (ESI) [M+H]⁺: 553.3. ¹H NMR (400 MHz, CDCl₃), 7.58 (s, 1H), 7.52 (d, J = 8.4 Hz, 2H), 7.44 (s, 1H), 7.34 (d, J = 8.4 Hz, 2H), 7.26-7.31 (m, 1H), 7.17 (dd, J = 8.4 Hz, 5.6 Hz, 1H), 7.02 (td, J = 8.4 Hz, 2.8 Hz, 1H), 6.55 (s, 1H), 5.37-5.44 (m, 1H), 4.80-5.30 (brs, 2H), 4.04-4.18 (m, 2H), 3.91 (s, 3H), 3.85 (d, J = 15.6 Hz, 1H), 2.96 (d, J = 15.6 Hz, 1H), 1.80 (d, J = 6.4 Hz, 3H), 1.40 (t, J = 7.2 Hz, 3H).

### Example 128: (19R)-3-ethyl-16-fluoro-5-(piperidin-3-ylethynyl)-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,13(18),1 4,16,21(22),23-decaen-22-amine

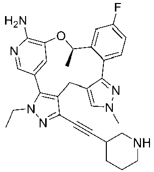

The synthetic method of Example 128 is the same as that of Example 47. LC-MS (ESI) [M+H]⁺: 526.4. ¹H NMR (400 MHz, CD₃OD), 7.53-7.56 (m, 2H), 7.42 (d, J = 1.2 Hz, 1H), 7.19 (dd, J = 8.4 Hz, 5.6 Hz, 1H), 7.08 (td, J = 8.4 Hz, 2.8 Hz, 1H), 6.46 (d, J = 1.2 Hz, 1H), 5.37-5.44 (m, 1H), 4.02-4.17 (m, 2H), 3.92 (s, 3H), 3.74 (d, J = 15.6 Hz, 1H), 3.30-3.38 (m, 1H), 2.82-3.11 (m, 5H), 2.12-2.20 (m, 1H), 1.87-1.96 (m, 1H), 1.74-1.85 (m, 4H), 1.61-1.72 (m, 1H), 1.31 (t, J = 7.2 Hz, 3H).

### Example 129: (19R)-3-ethyl-16-fluoro-5-((5-fluoropyridin-2-yl)ethynyl)-10,19-dimethyl-20-oxa-3,4,10,11,23-pentaazapentacyclo[19.3.1.0^{2,6}.0^{8,12}.0^{13,18} ]pentacosa-1(25),2(6),4,8(9),11,1 3(14),15,17,21(22),23-decaen-22-amine

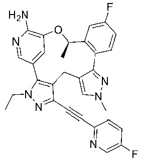

The synthetic method of Example 129 is the same as that of Example 47. LC-MS (ESI) [M+H]⁺: 538.4. ¹H NMR (400 MHz, CDCl₃), 8.50 (d, J = 2.8 Hz, 1H), 7.63 (dd, J = 8.8 Hz, 4.4 Hz, 1H), 7.52 (s, 1H), 7.47 (s, 1H), 7.43 (td, J = 8.4 Hz, 2.8 Hz, 1H), 7.29 (dd, J = 10.0 Hz, 2.8 Hz, 1H), 7.14 (dd, J = 8.4 Hz, 5.6 Hz, 1H), 7.01 (td, J = 8.4 Hz, 2.8 Hz, 1H), 6.56 (s, 1H), 5.39-5.45 (m, 1H), 4.97 (s, 2H), 4.15-4.21 (m, 2H), 3.89-3.94 (m, 4H), 2.97 (d, J = 15.6 Hz, 1H), 1.79 (d, J = 6.4 Hz, 3H), 1.43 (t, J = 7.2 Hz, 3H).

### Example 130: 4-((19R)-22-amino-3-ethyl-16-fluoro-10,19-dimethyl-20-oxa-3,4,10,11,23 -pentaazapentacyclo[19.3.1.0^{2,6}.0^{8,12}.0^{13,18}]pentacosa-1(24),2(6),4,8(9),11,13(18),14,16,21(25), 22-decaen-5-yl)-1,1,1-trifluorobut-3-yn-2-ol

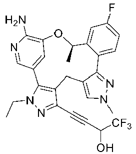

The synthetic method of Example 130 is the same as that of Example 47. LC-MS (ESI) [M+H]⁺: 541.4. ¹H NMR (400 MHz, CD₃OD), 7.36-7.60 (m, 3H), 7.19 (dd, J = 8.4 Hz, 6.0 Hz, 1H), 7.08 (td, J = 8.4 Hz, 2.8 Hz, 1H), 6.48 (s, 1H), 5.38-5.46 (m, 1H), 5.15 (q, J = 6.0 Hz, 1H), 4.04-4.19 (m, 2H), 3.92 (s, 3H), 3.75-3.80 (m, 1H), 2.92 (d, J = 15.6 Hz, 1H), 1.81 (d, J = 6.4 Hz, 3H), 1.32 (t, J = 7.2 Hz, 3H).

### Example 131: 3-((19R)-22-amino-3-ethyl-16-fluoro-10,19-dimethyl-20-oxa-3,4,10,11,23 -pentaazapentacyclo[19.3.1.0^{2,6}.0^{8,12}.0^{13,18}]pentacosa-1(24),2(6),4,8(9),11,13(18),14,16,21(25), 22-decaen-5-yl)-N,N-dimethylprop-2-ynamide

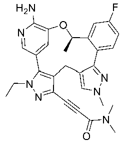

The synthetic method of Example 131 is the same as that of Example 47. LC-MS (ESI) [M+H]⁺: 514.3. ¹H NMR (400 MHz, CD₃OD), 7.59 (s, 1H), 7.55 (dd, J = 10.0 Hz, 2.8 Hz, 1H), 7.39-7.51 (brs, 1H), 7.21 (dd, J = 8.4 Hz, 5.6 Hz, 1H), 7.09 (td, J = 8.4 Hz, 2.8 Hz, 1H), 6.48 (s, 1H), 5.39-5.46 (m, 1H), 4.08-4.23 (m, 2H), 3.92 (s, 3H), 3.83 (d, J = 15.6 Hz, 1H), 3.39 (s, 3H), 3.05 (s, 3H), 2.96 (d, J = 15.6 Hz, 1H), 1.81 (d, J = 6.4 Hz. 3H), 1.34 (t, J = 7.2 Hz, 3H).

### Example 132: 3-((19R)-22-amino-3-ethyl-16-fluoro-10,19-dimethyl-20-oxa-3,4,10,11,23 -pentaazapentacyclo[19.3.1.0^{2,6}.0^{8,12}.0^{13,18}]pentacosa-1(24),2(6),4,8(9),11,13(18),14,16,21(25), 22-decaen-5-yl)-N-methylprop-2-ynamide

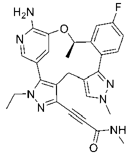

The synthetic method of Example 132 is the same as that of Example 47. LC-MS (ESI) [M+H]⁺: 500.3. ¹H NMR (400 MHz, CD₃OD), 7.56 (s, 1H), 7.54 (dd, J = 10.0 Hz, 2.8 Hz, 1H), 7.39-7.52 (brs, 1H), 7.20 (dd, J = 8.4 Hz, 6.0 Hz, 1H), 7.08 (td, J = 8.4 Hz, 2.8 Hz, 1H), 6.47 (s, 1H), 5.38-5.44 (m, 1H), 4.06-4.20 (m, 2H), 3.92 (s, 3H), 3.84 (d, J = 15.6 Hz, 1H), 2.92 (d, J = 15.6 Hz, 1H), 2.84 (s, 3H), 1.81 (d, J = 6.4 Hz, 3H), 1.32 (t, J = 7.2 Hz, 3H).

### Example 133: (19R)-5-((3,5-difluorophenyl)ethynyl)-3-ethyl-16-fluoro-10,19-dimethyl-2 0-oxa-3,4,10,11,23-pentaazapentacyclo[19.3.1.0^{1,12}.0^{13,18}.0^{2,6} ]pentacosa-1(25),2(6),4,8(9),11,13 (18),14,16,21(22),23-decaen-22-amine

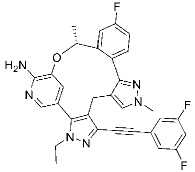

The synthetic method of Example 133 is the same as that of Example 47. LC-MS (ESI) [M+H]⁺: 555.3. ¹H NMR (400 MHz, CDCl₃), 7.54 (s, 1H), 7.41 (s, 1H), 7.30 (dd, J = 10.0 Hz, 2.8 Hz, 1H), 7.17 (dd, J = 8.4 Hz, 5.6 Hz, 1H), 7.06-7.13 (m, 2H), 7.02 (td, J = 8.4 Hz, 2.8 Hz, 1H), 6.80-6.86 (m, 1H), 6.55 (s, 1H), 5.40-5.45 (m, 1H), 4.88-5.10 (brs, 2H), 4.13-4.18 (m, 2H), 3.92 (s, 3H), 3.83 (d, J = 15.6 Hz, 1H), 2.98 (d, J = 15.6 Hz, 1H), 1.81 (d, J = 6.4 Hz, 3H), 1.43 (t, J = 7.2 Hz, 3H).

### Example 134: 1-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)ethynyl)cycloprop-1-ol

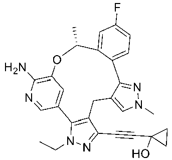

The synthetic method of Example 134 is the same as that of Example 47. LC-MS (ESI) [M+H]⁺: 499.4. ¹H NMR (400 MHz, CD₃OD), 7.57 (s, 1H), 7.52 (dd, J = 10.4 Hz, 2.8 Hz, 1H), 7.34-7.51 (brs, 1H), 7.18 (dd, J = 8.4 Hz, 5.6 Hz, 1H), 7.06 (td, J = 8.4 Hz, 2.8 Hz, 1H), 6.47 (s, 1H), 5.37-5.44 (m, 1H), 3.99-4.16 (m, 2H), 3.92 (s, 3H), 3.75 (d, J = 15.6 Hz, 1H), 2.87 (d, J = 15.6 Hz, 1H), 1.80 (d, J = 6.4 Hz, 3H), 1.30 (t, J = 7.2 Hz, 3H), 1.08-1.14 (m, 4H).

### Example 135: 6-((19R)-22-amino-3-ethyl-16-fluoro-10,19-dimethyl-20-oxa-3,4,10,11,23 -pentaazapentacyclo[19.3.1.0^{8,11}.0^{13,18}.0^{2,6}]pentacosa-1(25),2(6),4,8(9),11,13(18),14,16,21(22), 23-decaen-5-yl)-2-methylhexy-5-yn-2-ol

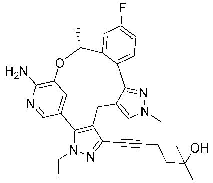

The synthetic method of Example 135 is the same as that of Example 47. LC-MS (ESI) [M+H]⁺:529.4. ¹H NMR (400 MHz, CDCl₃), 7.50 (d, J = 1.2 Hz, 1H), 7.42 (s, 1H), 7.27 (dd, J = 10.4 Hz, 2.8 Hz, 1H), 7.14 (dd, J = 8.4 Hz, 5.6 Hz, 1H), 6.99 (td, J = 8.4 Hz, 2.8 Hz, 1H), 6.51 (d, J = 1.2 Hz, 1H), 5.36-5.43 (m, 1H), 5.00 (s, 2H), 4.03-4.12 (m, 2H), 3.91 (s, 3H), 3.73 (d, J = 15.6 Hz, 1H), 2.87 (d, J = 15.6 Hz, 1H), 2.62 (t, J = 8.0 Hz, 2H), 1.89 (t, J = 8.0 Hz, 2H), 1.78 (d, J = 6.0 Hz, 3H), 1.36 (t, J = 7.2 Hz, 3H), 1.28 (s, 6H).

### Example 136: 3-((19R)-22-amino-3-ethyl-16-fluoro-10,19-dimethyl-20-oxa-3,4,10,11,23 -pentaazapentacyclo[19.3.1.0^{2,6}.0^{8,12}.0^{13,18}]pentacosa-1(25),2(6),4,8(9),11,13(14),15,17,21(22), 23-decaen-5-yl)-1-(3,3-difluoroazetidin-1-yl)prop-2-yn-1-one

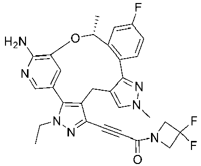

The synthetic method of Example 136 is the same as that of Example 47. LC-MS (ESI) [M+H]⁺: 562.4. ¹H NMR (400 MHz, CDCl₃), 7.41 (s, 1H), 7.36 (s, 1H), 7.29 (dd, J = 10.0 Hz, 2.8 Hz, 1H), 7.15 (dd, J = 8.4 Hz, 5.6 Hz, 1H), 7.05 (td, J = 8.4 Hz, 2.8 Hz, 1H), 6.61 (s, 1H), 5.64-5.96 (brs, 2H), 5.41-5.48 (m, 1H), 4.64-4.71 (m, 2H), 4.44 (t, J = 12.0 Hz, 2H), 4.11-4.17 (m, 2H), 3.92 (s, 3H), 3.82 (d, J = 16.0 Hz, 1H), 2.96 (d, J = 15.6 Hz, 1H), 1.83 (d, J = 6.0 Hz, 3H), 1.43 (t, J = 7.2 Hz, 3H).

### Example 137: (19R)-3-ethyl-16-fluoro-10,19-dimethyl-5-((4-methyl-1,3-thiazol-5-yl)eth ynyl)-20-oxa-3,4,10,11,23-pentaazapentacyclo[19.3. 1.0^{8,11}.0^{13,18}.0^{2,6}]pentacosa-1(25),2(6),4,8 (9),11,13(18),14,16,21(22),23-decaen-22-amine

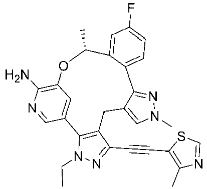

The synthetic method of Example 137 is the same as that of Example 47. LC-MS (ESI) [M+H]⁺: 540.4. ¹H NMR (400 MHz, CDCl₃), 8.66 (s, 1H), 7.47-7.67 (brs, 1H), 7.43 (s, 1H), 7.29 (dd, J = 10.0 Hz, 2.4 Hz, 1H), 7.15 (dd, J = 8.4 Hz, 5.6 Hz, 1H), 7.01 (td, J = 8.4 Hz, 2.4 Hz, 1H), 6.55 (s, 1H), 5.38-5.46 (m, 1H), 5.23 (s, 2H), 4.08-4.17 (m, 2H), 3.91 (s, 3H), 3.82 (d, J = 15.6 Hz, 1H), 2.98 (d, J = 15.6 Hz, 1H), 2.64 (s, 3H), 1.80 (d, J = 6.4 Hz, 3H), 1.41 (t, J = 7.2 Hz, 3H).

### Example 138: (19R)-3-ethyl-16-fluoro-5-((2-methoxypyridin-3-yl)ethynyl)-10,19-dimeth yl-20-oxa-3,4,10,11,23-pentaazapentacyclo[19.3.1.0^{8,12}.0^{13,18}.0^{2,6} ]pentacosa-1(25),2(6),4,8(9),1 1,13(18),14,16,21(22),23-decaen-22-amine

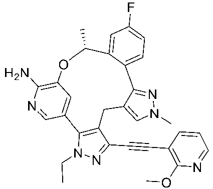

The synthetic method of Example 138 is the same as that of Example 47. LC-MS (ESI) [M+H]⁺: 550.4. ¹H NMR (400 MHz, CDCl₃), 8.14 (dd, J = 5.2 Hz, 1.6 Hz, 1H), 7.79 (dd, J = 7.6 Hz, 2.0 Hz, 1H), 7.51-7.60 (m, 2H), 7.28 (dd, J = 10.0 Hz, 2.8 Hz, 1H), 7.14 (dd, J = 8.4 Hz. 5.6 Hz, 1H), 7.00 (td, J = 8.4 Hz, 2.8 Hz, 1H), 6.90 (dd, J = 7.2 Hz, 5.2 Hz, 1H), 6.58 (s, 1H), 5.40-5.48 (m, 1H), 5.17-5.36 (brs, 2H), 4.08-4.18 (m, 2H), 3.98 (s, 3H), 3.91 (s, 3H), 3.90 (d, J = 15.2 Hz, 1H), 2.96 (d, J = 15.2 Hz, 1H), 1.80 (d, J = 6.4 Hz, 3H), 1.39 (d, J = 7.2 Hz, 3H).

### Example 139: (19R)-3-ethyl-16-fluoro-5-((6-methoxypyridin-3-yl)ethynyl)-10,19-dimeth yl-20-oxa-3,4,10,11,23-pentaazapentacyclo[19.3.1.0^{8,12}.0^{13,18}.0^{2,6}]pentacosa-1(25),2(6),4,8(9),1 1,13(18),14,16,21(22),23-decaen-22-amine

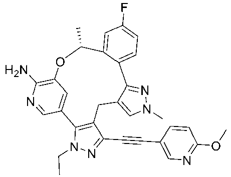

The synthetic method of Example 139 is the same as that of Example 47. LC-MS (ESI) [M+H]⁺: 550.4. ¹H NMR (400 MHz, CDCl₃), 8.42 (d, J = 2.0 Hz, 1H), 7.74 (dd, J = 8.4 Hz, 2.0 Hz, 1H), 7.49-7.59 (brs, 1H), 7.45 (s, 1H), 7.28 (dd, J = 10.0 Hz, 2.8 Hz, 1H), 7.16 (dd, J = 8.4 Hz, 5.6 Hz, 1H), 7.02 (td, J = 8.4 Hz, 2.8 Hz, 1H), 6.75 (d, J = 8.4 Hz, 1H), 6.58 (s, 1H), 5.22-5.48 (m, 3H), 4.08-4.18 (m, 2H), 3.96 (s, 3H), 3.90 (s, 3H), 3.85 (d, J = 15.6 Hz, 1H), 2.96 (d, J = 15.6 Hz, 1H), 1.80 (d, J = 6.8 Hz, 3H), 1.41 (t, J = 7.2 Hz, 3H).

### Example 140: (19R)-3-ethyl-16-fluoro-10,19-dimethyl-5-((3-methylisooxazol-5-yl)ethyn yl)-20-oxa-3,4,10,11,23-pentaazapentacyclo[19.3.1.0^{8,12}.0^{13,18}.0^{2,6}]pentacosa-1(25),2(6),4,8(9),1 1,13(18),14,16,21(22),23-decaen-22-amine

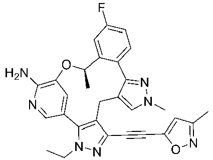

The synthetic method of Example 140 is the same as that of Example 47. LC-MS (ESI) [M+H]⁺: 523.4. ¹H NMR (400 MHz, CD₃OD), 7.40-7.65 (m, 3H), 7.22 (dd, J = 8.4 Hz, 5.6 Hz, 1H), 7.08 (td, J = 8.4 Hz, 2.8 Hz, 1H), 6.67 (s, 1H), 6.51 (s, 1H), 5.40-5.47 (m, 1H), 4.09-4.22 (m, 2H), 3.92 (s, 3H), 3.82 (d, J = 15.6 Hz, 1H), 2.98 (d, J = 15.6 Hz, 1H), 2.34 (s, 3H), 1.82 (d, J = 6.4 Hz, 3H), 1.36 (t, J = 7.2 Hz, 3H).

### Example 141: 3-((19R)-22-amino-3-ethyl-16-fluoro-10,19-dimethyl-20-oxa-3,4,10,11,23 -pentaazapentacyclo[19.3.1.0^{8,12}.0^{2,6}.0^{13,18}]pentacosa-1(24),2(6),4,8(9),11,13(14),15,17,21(25), 22-decaen-5-yl)-1-(4-(2-hydroxyethyl)piperazin-1-yl)prop-2-yn-1-one

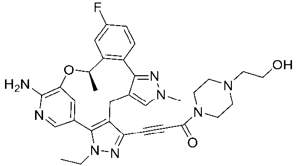

The synthetic method of Example 141 is the same as that of Example 47. LC-MS (ESI) [M+H]⁺: 598.4. ¹H NMR (400 MHz, CDCl₃), 7.50 (s, 1H), 7.41 (s, 1H), 7.28 (dd, J = 10.0 Hz, 1H), 7.13 (dd, J = 8.4 Hz, 5.6 Hz, 1H), 7.00 (td, J = 8.4 Hz, 2.8 Hz, 1H), 6.53 (d, J = 1.6 Hz, 1H), 5.39-5.45 (m, 1H), 4.99 (s, 2H), 4.11-4.20 (m, 2H), 3.97-4.00 (m, 2H), 3.91 (s, 3H), 3.76-3.84 (m, 3H), 3.70-3.72 (m, 2H), 2.96 (d, J = 15.6 Hz, 1H), 2.58-2.74 (m, 6H), 1.79 (d, J = 6.4 Hz, 3H), 1.40 (t, J = 7.2 Hz, 3H).

### Example 142: (19R)-3-ethyl-16-fluoro-5-(((3S)-tetrahydrofuran-3-yl)ethynyl)-10,19-dim ethyl-20-oxa-3,4,10,11,23-pentaazapentacyclo[19.3.1.0^{8,12}.0^{13,18}.0^{2,6}] pentacosa- 1(24),2(6),4,8 (9),11,13(14),15,17,21(25),22-decaen-22-amine

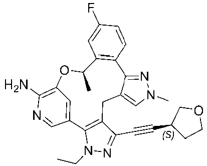

The synthetic method of Example 142 is the same as that of Example 47. LC-MS (ESI) [M+H]⁺: 513.3. ¹H NMR (400 MHz, CDCl₃), 7.51 (s, 1H), 7.39 (s, 1H), 7.28 (dd, J = 10.0 Hz, 2.8 Hz, 1H), 7.14 (dd, J = 8.4 Hz, 5.6 Hz, 1H), 6.99 (td, J = 8.4 Hz, 2.8 Hz, 1H), 6.50 (d, J = 1.6 Hz, 1H), 5.37-5.43 (m, 1H), 4.95 (s, 2H), 4.06-4.15 (m, 3H), 3.95-4.01 (m, 1H), 3.87-3.93 (m, 4H), 3.79-3.83 (m, 1H), 3.72 (d, J = 15.6 Hz, 1H), 3.28-3.32 (m, 1H), 2.89 (d, J = 15.6 Hz, 1H), 2.29-2.38 (m, 1H), 2.11-2.19 (m, 1H), 1.79 (d, J = 6.0 Hz, 3H), 1.37 (t, J = 7.2 Hz, 3H).

### Example 143: (19R)-3-ethyl-16-fluoro-5-(((3R)-1,4-oxaazacyclohexan-3-yl)ethynyl)-10, 19-dimethyl-20-oxa-3,4,10,11,23-pentaazapentacyclo[19.3.1.0^{8,12}.0^{13,18}.0^{2,6}]pentacosa-1(25),2 (6),4,8(9),11,13(18),14,16,21(22),23-decaen-22-amine

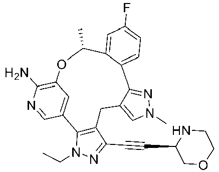

The synthetic method of Example 143 is the same as that of Example 47. LC-MS (ESI) [M+H]⁺: 528.4. ¹H NMR (400 MHz, CDCl₃), 7.48 (s, 1H), 7.44 (s, 1H), 7.28 (dd, J = 10.0 Hz, 2.8 Hz, 1H), 7.12 (dd, J = 8.4 Hz, 5.6 Hz, 1H), 6.99 (td, J = 8.4 Hz, 2.8 Hz, 1H), 6.51 (s, 1H), 5.37 (m, 1H), 5.08-5.24 (brs, 2H), 3.98-4.12 (m, 4H), 3.90 (s, 1H), 3.64-3.82 (m, 4H), 3.10-3.20 (m, 1H), 2.86-2.98 (m, 2H), 1.78 (d, J = 6.0 Hz, 3H), 1.38 (t, J = 7.2 Hz, 3H).

### Example 144: (19R)-3-ethyl-16-fluoro-5-(((2S)-1,4-oxaazacyclohexan-2-yl)ethynyl)-10,1 9-dimethyl-20-oxa-3,4,10,11,23-pentaazapentacyclo[19.3.1.0^{2,6}.0^{8,12}.0^{13,18}]pentacosa-1(25),2(6), 4,8(9),11,13(14),15,17,21(22),23-decaen-22-amine

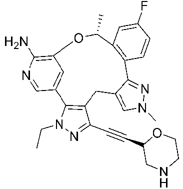

The synthetic method of Example 144 is the same as that of Example 47. LC-MS (ESI) [M+H]⁺: 528.4. ¹H NMR (400 MHz, CDCl₃), 7.51 (d, J = 1.2 Hz, 1H), 7.43 (s, 1H), 7.29 (dd, J = 10.0 Hz, 2.8 Hz, 1H), 7.13 (dd, J = 8.4 Hz, 5.6 Hz, 1H), 6.99 (td, J = 8.4 Hz, 2.8 Hz, 1H), 6.51 (d, J = 1.2 Hz, 1H), 5.38-5.44 (m, 1H), 4.81 (s, 2H), 4.66 (dd, J = 6.8 Hz, 2.8 Hz, 1H), 4.02-4.16 (m, 3H), 3.91 (s, 3H), 3.77 (d, J = 15.6 Hz, 1H), 3.67-3.74 (m, 1H), 3.27 (dd, J = 12.8 Hz, 3.2 Hz, 1H), 3.09 (dd, J = 12.8 Hz, 6.8 Hz, 1H), 2.90-2.98 (m, 3H), 1.79 (d, J = 6.0 Hz, 3H), 1.39 (t, J = 7.2 Hz, 3H).

### Example 145: (19R)-3-ethyl-16-fluoro-10,19-dimethyl-5-((1-methyl piperidin-2-yl)ethy nyl)-20-oxa-3,4,10,11,23-pentaazapentacyclo[19.3.1.0^{8,12}.0^{13,18}.0^{2,6}]pentacosa-1(24),2(6),4,8(9), 11,13(14),15,17,21(25),22-decaen-22-amine

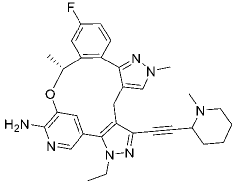

The synthetic method of Example 145 is the same as that of Example 47. LC-MS (ESI) [M+H]⁺: 540.4. ¹H NMR (400 MHz, CDCl₃), 7.38-7.54 (m, 2H), 7.29 (dd, J = 10.0 Hz, 2.8 Hz, 1H), 7.14 (dd, J = 8.4 Hz, 5.6 Hz, 1H), 6.97-7.02 (m, 1H), 6.49-6.50 (m, 1H), 5.38-5.45 (m, 1H), 4.82 (s, 2H), 4.06-4.20 (m, 2H), 3.85-4.03 (m, 4H), 3.72-3.77 (m, 1H), 2.80-2.98 (m, 2H), 2.51-2.74 (m, 4H), 1.97-2.24 (m, 2H), 1.70-1.90 (m, 5H), 1.56-1.68 (m, 2H), 1.39 (t, J = 7.2 Hz, 3H).

### Example 146: 3-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)azetidin-3-ol

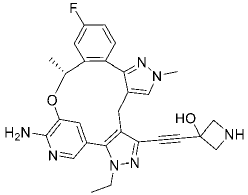

The synthetic method of Example 146 is the same as that of Example 47. LC-MS (ESI) [M+H]⁺: 514.4. ¹H NMR (400 MHz, CD₃OD+CDCl₃), 7.36-7.48 (m, 2H), 7.30 (dd, J = 9.6 Hz, 2.4 Hz, 1H), 7.09 (dd, J = 8.4 Hz, 5.6 Hz, 1H), 6.99 (td, J = 8.4 Hz, 2.8 Hz, 1H), 6.59 (s, 1H), 5.32-5.40 (m, 1H), 4.33-4.37 (m, 2H), 4.17-4.20 (m, 2H), 3.97-4.07 (m, 2H), 3.84 (s, 3H), 3.74 (d, J = 15.6 Hz, 1H), 2.83 (d, J = 15.6 Hz, 1H), 1.78 (d, J = 6.0 Hz, 3H), 1.31 (t, J = 7.2 Hz, 3H).

### Example 147: (19R)-3-ethyl-16-fluoro-5-(4-methoxybut-1-yn-1-yl)-10,19-dimethyl-20-o xa-3,4,10,11,23-pentaazapentacyclo[19.3.1.0^{8,12}.0^{13,18}.0^{2,6}]pentacosa-1(25),2(6),4,8(9),11,13(1 8),14,16,21(22),23-decaen-22-amine

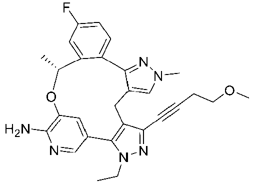

The synthetic method of Example 147 is the same as that of Example 47. LC-MS (ESI) [M+H]⁺: 501.4. ¹H NMR (400 MHz, CDCl₃), 7.47 (s, 1H), 7.23-7.30 (m, 2H), 7.19 (dd, J = 8.4 Hz, 5.6 Hz, 1H), 7.10 (td, J = 8.4 Hz, 2.8 Hz, 1H), 6.76 (s, 1H), 5.46-5.54 (m, 1H), 3.98-4.10 (m, 2H), 3.92 (s, 3H), 3.85 (d, J = 15.6 Hz, 1H), 3.65 (t, J = 7.2 Hz, 2H), 3.41 (s, 3H), 3.22-3.40 (brs, 2H), 2.88 (d, J = 15.6 Hz, 1H), 2.79 (t, J = 7.2 Hz, 2H), 1.87 (d, J = 6.4 Hz, 3H), 1.40 (t, J = 7.2 Hz, 3H).

### Example 148: (19R)-3-ethyl-16-fluoro-10,19-dimethyl-5-(thiophen-2-ylethynyl)-20-oxa-3,4,10,11,23-pentaazapentacyclo[19.3.1.0^{8,11}.0^{13,18}.0^{2,6}]pentacosa-1(25),2(6),4,8(9),11,13(18),1 4,16,21(22),23-decaen-22-amine

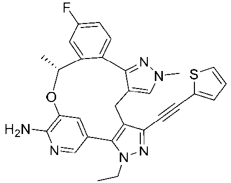

The synthetic method of Example 148 is the same as that of Example 47. LC-MS (ESI) [M+H]⁺: 525.3. ¹H NMR (400 MHz, CDCl₃), 7.49 (s, 1H), 7.46 (s, 1H), 7.35 (dd, J = 7.6 Hz, 1.2 Hz, 1H), 7.32 (dd, J = 9.2 Hz, 1.2 Hz, 1H), 7.29 (dd, J = 10.0 Hz, 2.8 Hz, 1H), 7.16 (dd, J = 8.4 Hz, 5.6 Hz, 1H), 6.99-7.04 (m, 2H), 6.57 (s, 1H), 5.40-5.46 (m, 1H), 5.23 (s, 2H), 4.11-4.17 (m, 2H), 3.91 (s, 3H), 3.85 (d, J = 15.6 Hz, 1H), 2.95 (d, J = 15.6 Hz, 1H), 1.81 (d, J = 6.4 Hz, 3H), 1.42 (t, J = 7.2 Hz, 3H).

### Example 149: (19R)-3-ethyl-16-fluoro-10,19-dimethyl-5-(thiophen-3-ylethynyl)-20-oxa-3,4,10,11,23-pentaazapentacyclo[19.3.1.0^{8,12}.0^{13,18}.0^{2,6}]pentacosa-1(25),2(6),4,8(9),11,13(18),1 4,16,21(22),23-decaen-22-amine

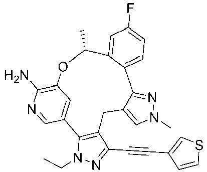

The synthetic method of Example 149 is the same as that of Example 47. LC-MS (ESI) [M+H]⁺: 525.3. ¹H NMR (400 MHz, CDCl₃), 7.59 (dd, J = 2.4 Hz, 1.2 Hz, 1H), 7.50 (s, 1H), 7.46 (s, 1H), 7.28-7.33 (m, 2H), 7.24-7.26 (m, 1H), 7.16 (dd, J = 8.4 Hz, 5.6 Hz, 1H), 7.01 (td, J = 8.4 Hz, 2.8 Hz, 1H), 6.56 (d, J = 1.2 Hz, 1H), 5.40-5.46 (m, 1H), 5.15 (s, 2H), 4.10-4.16 (m, 2H), 3.90 (s, 3H), 3.85 (d, J = 15.6 Hz, 1H), 2.95 (d, J = 15.6 Hz, 1H), 1.80 (d, J = 6.4 Hz, 3H), 1.42 (t, J = 7.2 Hz, 3H).

### Example 150: (19R)-3-ethyl-16-fluoro-10,19-dimethyl-5-(pyrimidin-4-ylethynyl)-20-oxa -3,4,10,11,23-pentaazapentacyclo[19.3.1.0^{2,6}.0^{8,12}.0^{13,18}]pentacosa-1(24),2(6),4,8(9),11,13(18),1 4,16,21(25),22-decaen-22-amine

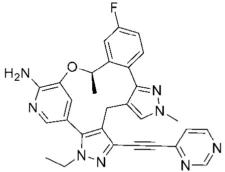

The synthetic method of Example 150 is the same as that of Example 47. LC-MS (ESI) [M+H]⁺: 521.3. ¹H NMR (400 MHz, CDCl₃), 9.10-9.42 (brs, 1H), 8.58-8.97 (brs, 1H), 7.36-7.70 (m, 3H), 7.23-7.30 (m, 1H), 7.19 (dd, J = 8.4 Hz, 5.6 Hz, 1H), 7.08 (td, J = 8.4 Hz, 2.0 Hz, 1H), 6.99 (s, 1H), 5.43-5.52 (m, 1H), 4.10-4.24 (m, 2H), 3.97 (d, J = 15.6 Hz, 1H), 3.91 (s, 3H), 3.00 (d, J = 15.6 Hz, 1H), 1.85 (d, J = 6.0 Hz, 3H), 1.43-1.50 (m, 3H).

### Example 151: (19R)-5-((2-aminopyrimidin-5-yl)ethynyl)-3-ethyl-16-fluoro-10,19-dimeth yl-20-oxa-3,4,10,11,23-pentaazapentacyclo[19.3.1.0^{8,12}.0^{13,18}.0^{2,6}]pentacosa-1(24),2(6),4,8(9),1 1,13(14),15,17,21\(25),22-decaen-22-amine

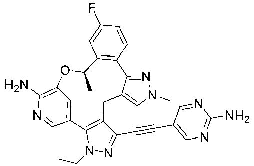

The synthetic method of Example 151 is the same as that of Example 47. LC-MS (ESI) [M+H]⁺: 536.3. ¹H NMR (400 MHz, CDCl₃), 8.51 (s, 2H), 7.53 (d, J = 2.0 Hz, 1H), 7.42 (s, 1H), 7.29 (dd, J = 9.6 Hz, 2.8 Hz, 1H), 7.15 (dd, J = 8.4 Hz, 5.6 Hz, 1H), 7.00 (td, J = 8.4 Hz, 2.8 Hz, 1H), 6.53 (d, J = 2.0 Hz, 1H), 5.39-5.46 (m, 1H), 5.33 (s, 2H), 4.95 (s, 2H), 4.11-4.18 (m, 2H), 3.91 (s, 3H), 3.82 (d, J = 15.6 Hz, 1H), 2.96 (d, J = 15.6 Hz, 1H), 1.79 (d, J = 6.4 Hz, 3H), 1.42 (t, J = 7.2 Hz, 3H).

### Example 152: (19R)-3-ethyl-16-fluoro-10,19-dimethyl-5-(3-methyl-3-(methyldioxo-λ⁶-s ulfanyl)but-1-yn-1-yl)-20-oxa-3,4,10,11,23-pentaazapentacyclo[19.3.1.0^{8,12},0^{13,18}.0^{2,6} ]pentacosa -1(24),2(6),4,8(9),11,13(14),15,17,21(25),22-decaen-22-amine

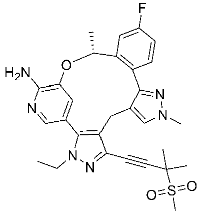

The synthetic method of Example 152 is the same as that of Example 47. LC-MS (ESI) [M+H]⁺: 563.4. ¹H NMR (400 MHz, CDCl₃), 7.56-7.87 (brs, 1H), 7.54 (s, 1H), 7.24-7.27 (m, 1H), 7.13 (dd, J = 8.4 Hz, 5.6 Hz, 1H), 7.00 (td, J = 8.4 Hz, 2.8 Hz,1H), 6.54 (s, 1H), 5.54 (s, 2H), 5.36-5.43 (m, 1H), 4.01-4.14 (m, 2H), 3.91 (s, 3H), 3.71 (d, J = 15.6 Hz, 1H), 3.12 (s, 3H), 2.93 (d, J = 15.6 Hz, 1H), 1.79-1.81 (m, 9H), 1.33 (t, J = 7.2 Hz, 3H).

### Example 153: 1-(3-((19R)-22-amino-3-ethyl-16-fluoro-10,19-dimethyl-20-oxa-3,4,10,11, 23-pentaazapentacyclo[19.3.1.0^{1,12}.0^{13,18}.0^{2,6}]pentacosa-1(25),2(6),4,8(9),11,13(18),14,16,21(2 2),23-decaen-5-yl)prop-2-yn-1-yl)cyclobut-1-ol

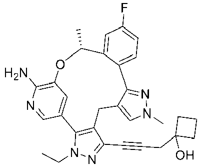

The synthetic method of Example 153 is the same as that of Example 47. LC-MS (ESI) [M+H]⁺: 527.4. ¹H NMR (400 MHz, CDCl₃), 7.51 (s, 1H), 7.42 (s, 1H), 7.28 (d, J = 10.4 Hz, 1H), 7.11-7.14 (m, 1H), 6.96-7.01 (m, 1H), 6.49 (s, 1H), 5.34-5.44 (m, 1H), 4.89 (s, 2H), 4.04-4.14 (m, 2H), 3.89 (s, 3H), 3.73 (d, J = 15.6 Hz, 1H), 2.89 (d, J = 15.6 Hz, 1H), 2.84 (s, 2H), 1.95-2.26 (m, 5H), 1.78 (d, J = 5.2 Hz, 3H), 1.56-1.68 (m, 1H), 1.37 (t, J = 6.8 Hz, 3H).

### Example 154: (19R)-3-ethyl-16-fluoro-10,19-dimethyl-5-((4-methyl-1,4-oxaazacyclohex an-3-yl)ethynyl)-20-oxa-3,4,10,11,23-pentaazapentacyclo[19.3.1.0^{2,6}.0^{8,12}.0^{13,18}]pentacosa-1(2 5),2(6),4,8(9),11,13(14),15,17,21(22),23-decaen-22-amine

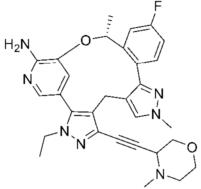

The synthetic method of Example 154 is the same as that of Example 47. LC-MS (ESI) [M+H]⁺: 542.3. ¹H NMR (400 MHz, CDCl₃), 7.44-7.53 (m, 2H), 7.28 (dd, J = 10.0 Hz, 2.8 Hz, 1H), 7.12-7.16 (m, 1H), 7.00 (td, J = 8.4 Hz, 2.8 Hz, 1H), 6.53 (s, 1H), 5.40-5.46 (m, 1H), 4.90-5.12 (brs, 2H), 3.98-4.18 (m, 3H), 3.70-3.96 (m, 7H), 3.48-3.68 (brs, 1H), 2.82-2.97 (m, 2H), 2.38-2.68 (m, 4H), 1.80 (d, J = 6.4 Hz, 3H), 1.39 (t, J = 7.2 Hz, 3H).

### Example 155: (19R)-3-ethyl-16-fluoro-10,19-dimethyl-5-((4-methyl-1,4-oxaazacyclohex an-2-yl)ethynyl)-20-oxa-3,4,10,11,23-pentaazapentacyclo[19.3.1.0^{2,6}.0^{8,12}.0^{13,18}]pentacosa-1(2 5),2(6),4,8(9),11,13(14),15,17,21(22),23-decaen-22-amine

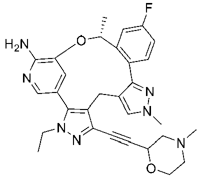

The synthetic method of Example 155 is the same as that of Example 47. LC-MS (ESI) [M+H]⁺: 542.4. ¹H NMR (400 MHz, CDCl₃), 7.50 (s, 1H), 7.38-7.44 (m, 1H), 7.28 (dd, J = 10.0 Hz, 2.8 Hz, 1H), 7.13 (dd, J = 8.4 Hz, 5.6 Hz, 1H), 6.97-7.03 (m, 1H), 6.52 (s, 1H), 5.38-5.45 (m, 1H), 4.67-5.04 (m, 3H), 4.08-4.14 (m, 2H), 4.00-4.07 (m, 1H), 3.84-3.97 (m, 4H), 3.77 (d, J = 15.2 Hz, 1H), 2.98-3.21 (m, 1H), 2.91 (d, J = 15.2 Hz, 1H), 2.30-2.84 (m, 6H), 1.79 (d, J = 6.4 Hz, 3H), 1.39 (t, J = 7.6 Hz, 3H).

### Example 156: (19R)-3-ethyl-16-fluoro-10,19-dimethyl-5-(3-methyl-3-(1,4-oxaazacycloh exan-4-yl)but-1-yn-1-yl)-20-oxa-3,4,10,11,23-pentaazapentacyclo[19.3.1.0^{8,12}.0^{13,18}.0^{2,6}]pentac osa-1(25),2(6),4,8(9),11,13(18),14,16,21(22),23-decaen-22-amine

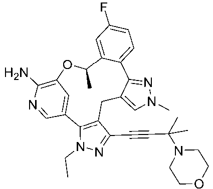

The synthetic method of Example 156 is the same as that of Example 47. LC-MS (ESI) [M+H]⁺: 570.4. ¹H NMR (400 MHz, CD₃OD), 7.59 (s, 1H), 7.53 (dd, J = 10.0 Hz, 2.8 Hz, 1H), 7.39-7.50 (brs, 1H), 7.20 (dd, J = 8.4 Hz, 5.6 Hz, 1H), 7.08 (td, J = 8.4 Hz, 2.8 Hz, 1H), 6.48 (s, 1H), 5.38-5.44 (m, 1H), 4.02-4.18 (m, 2H), 3.92 (s, 3H), 3.74-3.79 (m, 5H), 2.91 (d, J = 15.6 Hz, 1H), 2.82-2.84 (m, 4H), 1.81 (d, J = 6.4 Hz, 3H), 1.55 (s, 3H), 1.54 (s, 3H), 1.31 (t, J = 7.2 Hz, 3H).

### Example 157: 2-(4-(((19R)-22-amino-3-ethyl-16-fluoro-10,19-dimethyl-20-oxa-3,4,10,1 1,23-pentaazapentacyclo[19.3.1.0^{8,12}.0^{13,18}.0^{2,6}]pentacosa-1(25),2(6),4,8(9),11,13(18),14,16,21 (22),23-decaen-5-yl)ethynyl)-1,3-thiazol-2-yl)prop-2-ol

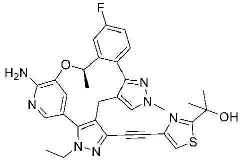

The synthetic method of Example 157 is the same as that of Example 47. LC-MS (ESI) [M+H]⁺: 583.4. ¹H NMR (400 MHz, CD₃OD), 7.55-7.94 (m, 3H), 7.54 (dd, J = 10.0 Hz, 2.4 Hz, 1H), 7.24 (dd, J = 8.4 Hz, 5.6 Hz, 1H), 7.10 (td, J = 8.4 Hz, 2.4 Hz, 1H), 6.62 (s, 1H), 5.44-5.52 (m, 1H), 4.07-4.24 (m, 2H), 3.86-3.94 (m, 4H), 2.95 (d, J = 15.6 Hz, 1H), 1.85 (d, J = 6.4 Hz, 3H), 1.64 (s, 6H), 1.34 (t, J = 7.2 Hz, 3H).

### Example 158: (19R)-5-(2,3-dihydro-1H-indol-7-ylethynyl)-3-ethyl-16-fluoro-10,19-dime thyl-20-oxa-3,4,10,11,23-pentaazapentacyclo[19.3.1.0^{8,12}.0^{13,18}.0^{2,6}]pentacosa-1(25),2(6),4,8(9), 11,13(18),14,16,21(22),23-decaen-22-amine

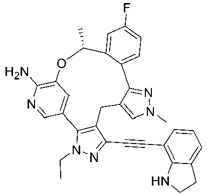

The synthetic method of Example 158 is the same as that of Example 47. LC-MS (ESI) [M+H]⁺: 560.4. ¹H NMR (400 MHz, CD₃OD), 7.61 (s, 1H), 7.48 (dd, J = 8.4 Hz, 2.8 Hz, 1H), 7.45 (d, J = 1.6 Hz, 1H), 7.20 (dd, J = 8.4 Hz, 5.6 Hz, 1H), 7.13 (d, J = 7.2 Hz, 1H), 7.02-7.09 (m, 2H), 6.63 (t, J = 7.6 Hz, 1H), 6.52 (d, J = 1.6 Hz, 1H), 5.37-5.44 (m, 1H), 4.05-4.20 (m, 2H), 3.90 (s, 3H), 3.86 (d, J = 15.6 Hz, 1H), 3.61 (t, J = 8.4 Hz, 2H), 3.07 (t, J = 8.4 Hz, 2H), 2.94 (d, J = 15.6 Hz, 1H), 1.81 (d, J = 6.4 Hz, 3H), 1.35 (t, J = 7.2 Hz, 3H).

### Example 159: 2-(5-(((19R)-22-amino-3-ethyl-16-fluoro-10,19-dimethyl-20-oxa-3,4,10,1 1,23-pentaazapentacyclo[19.3.1.0^{8,12}.0^{2,6}.0^{13,18}]pentacosa-1(24),2(6),4,8(9),11,13(14),15,17,21 (25),22-decaen-5-yl)ethynyl)pyrimidin-2-yl)prop-2-ol

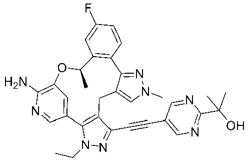

The synthetic method of Example 159 is the same as that of Example 47. LC-MS (ESI) [M+H]⁺: 579.4. ¹H NMR (400 MHz, CDCl₃), 8.90 (s, 2H), 7.54 (d, J = 1.6 Hz, 1H), 7.40 (s, 1H), 7.30 (dd, J = 9.6 Hz, 2.8 Hz, 1H), 7.16 (dd, J = 8.4 Hz, 5.6 Hz, 1H), 7.01 (td, J = 8.4 Hz, 2.8 Hz, 1H), 6.53 (d, J = 1.6 Hz, 1H), 5.39-5.46 (m, 1H), 4.86 (s, 2H), 4.50 (s, 1H), 4.18 (q, J = 7.2 Hz, 2H), 3.92 (s, 3H), 3.84 (d, J = 15.6 Hz, 1H), 3.01 (d, J = 15.6 Hz, 1H), 1.80 (d, J = 6.4 Hz, 3H), 1.62 (s, 6H), 1.44 (t, J = 7.2 Hz, 3H).

### Example 160: (5-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)pyrimidin-2-yl)methanol

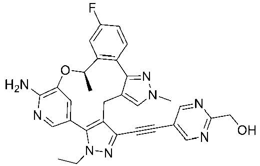

The synthetic method of Example 160 is the same as that of Example 47. LC-MS (ESI) [M+H]⁺: 551.4. ¹H NMR (400 MHz, CDCl₃), 8.91 (s, 2H), 7.53 (d, J = 1.6 Hz, 1H), 7.40 (s, 1H), 7.30 (dd, J = 9.6 Hz, 2.8 Hz, 1H), 7.17 (dd, J = 8.8 Hz, 6.0 Hz, 1H), 7.01 (td, J = 8.4 Hz, 2.8 Hz, 1H), 7.54 (d, J = 1.6 Hz, 1H), 5.40-5.46 (m, 1H), 4.94 (s, 2H), 4.88 (s, 2H), 4.17 (q, J = 7.2 Hz, 2H), 3.92 (s, 3H), 3.84 (d, J = 15.6 Hz, 1H), 3.46-3.62 (brs, 1H), 3.01 (d, J = 15.6 Hz, 1H), 1.80 (d, J = 6.4 Hz, 3H), 1.44 (t, J = 7.2 Hz, 3H).

### Example 161: (19R)-3-ethyl-16-fluoro-10,19-dimethyl-5-(1,3-oxazol-2-ylethynyl)-20-ox a-3,4,10,11,23-pentaazapentacyclo[19.3.1.0^{8,12}.0^{13,18}.0^{2,6}]pentacosa-1(24),2(6),4,8(9),11,13 (14), 15,17,21(25),22-decaen-22-amine

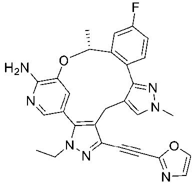

The synthetic method of Example 161 is the same as that of Example 47. LC-MS (ESI) [M+H]⁺: 510.4. ¹H NMR (400 MHz, CD₃OD), 8.04 (s, 1H), 7.59 (s, 1H), 7.53 (dd, J = 10.4 Hz, 2.8 Hz, 1H), 7.40-7.52 (brs, 1H), 7.34 (s, 1H), 7.20 (dd, J = 8.4 Hz, 5.6 Hz, 1H), 7.07 (td, J = 8.4 Hz, 2.8 Hz, 1H), 6.48 (s, 1H), 5.38-5.45 (m, 1H), 4.08-4.23 (m, 2H), 3.91 (s, 3H), 3.83 (d, J = 15.6 Hz, 1H), 2.97 (d, J = 15.6 Hz, 1H), 1.79 (d, J = 6.0 Hz, 3H), 1.35 (t, J = 7.2 Hz, 3H).

### Example 162: 3-(3-((19R)-22-amino-3-ethyl-16-fluoro-10,19-dimethyl-20-oxa-3,4,10,11, 23-pentaazapentacyclo[19.3.1.0^{8,12}.0^{13,18}.0^{2,6}]pentacosa-1(25),2(6),4,8(9),11,13(18),14,16,21 (2 2),23-decaen-5-yl)prop-2-yn-1-yl)oxetan-3-ol

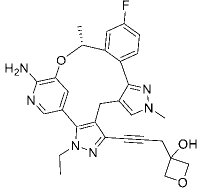

The synthetic method of Example 162 is the same as that of Example 47. LC-MS (ESI) [M+H]⁺: 529.4. ¹H NMR (400 MHz, CDCl₃), 7.45-7.57 (brs, 1H), 7.42 (s, 1H), 7.28 (dd, J = 10.0 Hz, 2.8 Hz, 1H), 7.12 (dd, J = 8.4 Hz, 5.6 Hz, 1H), 6.99 (td, J = 8.4 Hz, 2.8 Hz, 1H), 6.49 (s, 1H), 5.36-542 (m, 1H), 4.93 (s, 2H), 4.64-4.71 (m, 4H), 4.05-4.14 (m, 2H), 3.89 (s, 3H), 3.71 (d, J = 15.6 Hz, 1H), 3.08 (s, 2H), 2.89 (d, J = 15.6 Hz, 1H), 1.78 (d, J = 6.0 Hz, 3H), 1.37 (t, J = 7.2 Hz, 3H).

### Example 163: (19R)-3-ethyl-16-fluoro-10,19-dimethyl-5-((2-methylpyrazol-3-yl)ethynyl) -20-oxa-3,4,10,11,23-pentaazapentacyclo[19.3.1.0^{2,6}.0^{8,12}.0^{13,18}]pentacosa-1(25),2(6),4,8(9),11, 13(14),15,17,21(22),23-decaen-22-amine

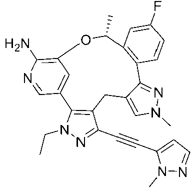

The synthetic method of Example 163 is the same as that of Example 47. LC-MS (ESI) [M+H]⁺: 523.4. ¹H NMR (400 MHz, CDCl₃), 7.52 (d, J = 1.6 Hz, 1H), 7.49 (d, J = 2.0 Hz, 1H), 7.42 (s, 1H), 7.30 (dd, J = 9.6 Hz, 2.8 Hz, 1H), 7.15 (dd, J = 8.4 Hz, 6.0 Hz, 1H), 7.01 (td, J = 8.4 Hz, 2.8 Hz, 1H), 6.55 (d, J = 2.0 Hz, 1H), 6.53 (d, J = 1.6 Hz, 1H), 5.40-5.45 (m, 1H), 4.98 (s, 2H), 4.13-4.20 (m, 2H), 4.05 (s, 3H), 3.92 (s, 3H), 3.81 (d, J = 15.6 Hz, 1H), 2.99 (d, J = 15.6 Hz, 1H), 1.80 (d, J = 6.8 Hz, 3H), 1.43 (t, J = 7.2 Hz, 3H).

### Example 164: (19R)-5-((1-(difluoromethyl)pyrazol-4-yl)ethynyl)-3-ethyl-16-fluoro-10,1 9-dimethyl-20-oxa-3,4,10,11,23-pentaazapentacyclo[19.3.1.0^{8,12}.0^{13,18}.0^{2,6}]pentacosa-1(25),2(6), 4,8(9),11,13(18),14,16,21(22),23-decaen-22-amine

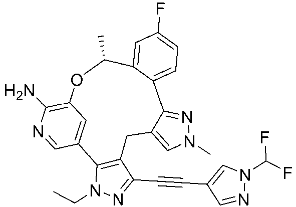

The synthetic method of Example 164 is the same as that of Example 47. LC-MS (ESI) [M+H]⁺: 559.4. ¹H NMR (400 MHz, CD₃OD), 8.41 (s, 1H), 7.93 (s, 1H), 7.58 (s, 1H), 7.53 (dd, J = 10.0 Hz, 2.4 Hz, 1H), 7.52 (t, J = 59.6 Hz, 1H), 7.46 (s, 1H), 7.19 (dd, J = 8.4 Hz, 5.6 Hz, 1H), 7.07 (td, J = 8.4 Hz, 2.4 Hz, 1H), 6.50 (s, 1H), 5.38-5.46 (m, 1H), 4.04-4.20 (m, 2H), 3.91 (s, 3H), 3.82 (d, J = 15.6 Hz, 1H), 2.93 (d, J = 15.6 Hz, 1H), 1.80 (d, J = 6.4 Hz, 3H), 1.33 (t, J = 7.2 Hz, 3H).

### Example 165: (19R)-3-ethyl-16-fluoro-10,19-dimethyl-9-vinyl-20-oxa-3,4,10,11,23-pent aazapentacyclo[19.3.1.0^{8,12} 0^{13,18}.0^{2,6}]pentacosa-1(25),2(6),4,8(9),11,13(18),14,16,21(22),23-de caen-22-amine

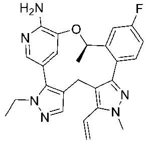

The synthetic method of Example 165 is the same as that of Example 47. LC-MS (ESI) [M+H]⁺: 445.3. ¹H NMR (400 MHz, CD₃OD), 7.57 (s, 1H), 7.52 (dd, J = 10.0 Hz, 2.8 Hz, 1H), 7.30-7.43 (brs, 1H), 7.15 (dd, J = 8.4 Hz, 5.6 Hz, 1H), 7.04 (td, J = 8.4 Hz, 2.8 Hz, 1H), 6.75 (dd, J = 18.4 Hz, 11.6 Hz, 1H), 6.56 (s, 1H), 5.69-5.73 (m, 2H), 5.56-5.61 (m, 1H), 3.99-4.14 (m, 2H), 3.90 (s, 3H), 3.82 (d, J = 15.6 Hz, 1H), 3.16 (d, J = 15.6 Hz, 1H), 1.84 (d, J = 6.4 Hz, 3H), 1.28 (t, J = 7.2 Hz, 3H).

### Example 166: 1-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)ethynyl)cyclobut-1-ol

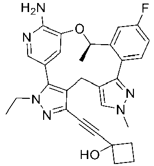

The synthetic method of Example 166 is the same as that of Example 47. LC-MS (ESI) [M+H]⁺: 513.4. ¹H NMR (400 MHz, CD₃OD), 7.60 (s, 1H), 7.43-7.56 (m, 2H), 7.20 (dd, J = 8.4 Hz, 6.0 Hz, 1H), 7.08 (td, J = 8.4 Hz, 2.8 Hz, 1H), 6.48 (s, 1H), 5.38-5.44 (m, 1H), 4.02-4.17 (m, 2H), 3.92 (s, 3H), 3.79 (d, J = 15.6 Hz, 1H), 2.90 (d, J = 15.6 Hz, 1H), 2.52-2.58 (m, 2H), 2.32-2.41 (m, 2H), 1.87-1.98 (m, 2H), 1.81 (d, J = 6.4 Hz, 3H), 1.30 (t, J = 7.2 Hz, 3H).

### Example 167: 2-(4-(((19R)-22-amino-3-ethyl-16-fluoro-10,19-dimethyl-20-oxa-3,4,10,1 1,23-pentaazapentacyclo[19.3.1.0^{8,12}.0^{13,18}.0^{2,6}]pentacosa-1(25),2(6),4,8(9),11,13(18),14,16,21 (22),23-decaen-5-yl)ethynyl)phenyl)prop-2-ol

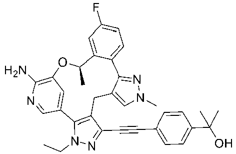

The synthetic method of Example 167 is the same as that of Example 47. LC-MS (ESI) [M+H]⁺: 577.3. ¹H NMR (400 MHz, CD₃OD+CDCl₃), 7.40-7.55 (m, 7H), 7.19 (dd, J = 8.4 Hz, 5.6 Hz, 1H), 7.04 (td, J = 8.4 Hz, 2.8 Hz, 1H), 6.52 (s, 1H), 5.36-5.43 (m, 1H), 4.06-4.20 (m, 2H), 3.91 (s, 3H), 3.85 (d, J = 15.6 Hz, 1H), 2.95 (d, J = 15.6 Hz, 1H), 1.81 (d, J = 6.4 Hz, 3H), 1.54 (s, 6H), 1.36 (t, J = 7.2 Hz, 3H).

### Example 168: 5-(((19R)-22-amino-3-ethyl-16-fluoro-10,19-dimethyl-20-oxa-3,4,10,11,2 3-pentaazapentacyclo[19.3.1.0^{2,6}.0^{8,12}.0^{13,18}]pentacosa-1(24),2(6),4,8(9),11,13(18),14,16,21(25), 22-decaen-5-yl)ethynyl)pyridin-2-carbonitrile

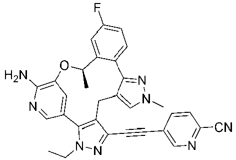

The synthetic method of Example 168 is the same as that of Example 47. LC-MS (ESI) [M+H]⁺: 545.3. ¹H NMR (400 MHz, CD₃OD), 8.90 (d, J = 2.0 Hz, 1H), 8.17 (dd, J = 8.0 Hz, 2.4 Hz, 1H), 7.91 (d, J = 8.0 Hz, 1H), 7.60 (s, 1H), 7.54 (dd, J = 10.0 Hz, 2.8 Hz, 1H), 7.46 (d, J = 2.0 Hz, 1H), 7.22 (dd, J = 8.4 Hz, 5.6 Hz, 1H), 7.08 (td, J = 8.4 Hz, 2.8 Hz, 1H), 6.50 (d, J = 2.0 Hz, 1H), 5.40-5.46 (m, 1H), 4.10-4.22 (m, 2H), 3.91 (s, 3H), 3.88 (d, J = 15.6 Hz, 1H), 2.98 (d, J = 15.6 Hz, 1H), 1.82 (d, J = 6.4 Hz, 3H), 1.36 (t, J = 7.2 Hz, 3H).

### Example 169: (19R)-5-((dimethyl(oxo)-λ⁶-sulfanylidene)amino)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(25),2(6),4, 8(9),11,13(18),14,16,21(22),23-decaen-22-amine

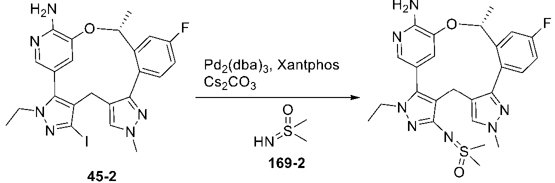

Under nitrogen protection, a solution of compound **45-2** (30 mg), compound **169-2** (10 mg), Pd₂(dba)₃ (5 mg), Xantphos (6 mg) and cesium carbonate (54 mg) in toluene (2 mL) was heated to 100 °C and reacted overnight. After the reaction was complete, the reaction solution was concentrated under reduced pressure, and the resulting residue was purified through flash silica gel chromatography (dichloromethane:methanol = 30:1) to obtain Example 169. LC-MS (ESI) [M+H]⁺: 510.5. ¹H NMR (400 MHz, CDCl₃), 7.58 (s, 1H), 7.48 (s, 1H), 7.27 (dd, J = 10.0 Hz, 2.8 Hz, 1H), 7.11 (dd, J = 8.4 Hz, 6.0 Hz, 1H), 6.97 (td, J = 8.4 Hz, 2.8 Hz, 1H), 6.56 (s, 1H), 5.38-5.44 (m, 1H), 4.85 (s, 2H), 4.00 (q, J = 7.2 Hz, 2H), 3.90 (s, 3H), 3.67 (d, J = 15.6 Hz, 1H), 3.43 (s, 3H), 3.28 (s, 3H), 2.73 (d, J = 15.6 Hz, 1H), 1.78 (d, J = 6.0 Hz, 3H), 1.34 (t, J = 7.2 Hz, 3H).

### Example 170: (19R)-3-ethyl-16-fluoro-10,19-dimethyl-5-(1,2,5-thiadiazol-3-ylethynyl)-2 0-oxa-3,4,10,11,23-pentaazapentacyclo[19.3.1.0^{8,12}.0^{13,18}.0^{2,6}]pentacosa-1(25),2(6),4,8(9),11,13 (18),14,16,21(22),23-decaen-22-amine

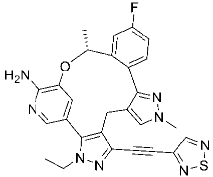

The synthetic method of Example 170 is the same as that of Example 47. LC-MS (ESI) [M+H]⁺: 527.3. ¹H NMR (400 MHz, CDCl₃), 8.75 (s, 1H), 7.46 (s, 1H), 7.19-7.29 (m, 3H), 7.09 (td, J = 8.0 Hz, 2.8 Hz, 1H), 6.73 (s, 1H), 5.46-5.54 (m, 1H), 5.24-5.42 (brs, 2H), 4.09-4.16 (m, 2H), 3.97 (d, J = 15.6 Hz, 1H), 3.92 (s, 3H), 3.00 (d, J = 15.6 Hz, 1H), 1.87 (d, J = 6.4 Hz, 3H), 1.46 (t, J = 6.8 Hz, 3H).

### Example 171: 5-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)ethynyl)-1,2-dihydropyridin-2-one

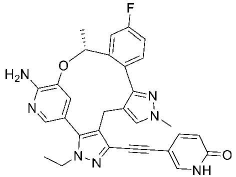

The synthetic method of Example 171 is the same as that of Example 47. LC-MS (ESI) [M+H]⁺: 536.4. ¹H NMR (400 MHz, CDCl₃), 12.50-13.42 (brs, 1H), 7.71 (d, J = 2.4 Hz, 1H), 7.61 (dd, J = 9.2 Hz, 2.4 Hz, 1H), 7.53 (d, J = 1.6 Hz, 1H), 7.40 (s, 1H), 7.30 (dd, J = 9.6 Hz, 2.8 Hz, 1H), 7.15 (dd, J = 8.4 Hz, 5.6 Hz, 1H), 7.00 (td, J = 8.4 Hz, 2.8 Hz, 1H), 6.59 (d, J = 9.2 Hz, 1H), 6.52 (d, J = 1.6 Hz, 1H), 5.38-5.45 (m, 1H), 4.91 (s, 2H), 4.13-4.19 (m, 2H), 3.92 (s, 3H), 3.78 (d, J = 15.6 Hz, 1H), 2.96 (d, J = 15.6 Hz, 1H), 1.79 (d, J = 6.4 Hz, 3H), 1.42 (t, J = 7.2 Hz, 3H).

### Example 172: (19R)-5-((3,3-difluorocyclobutyl)ethynyl)-3-ethyl-16-fluoro-10,19-dimeth yl-20-oxa-3,4,10,11,23-pentaazapentacyclo[19.3.1.0^{8,12}.0^{13,18}.0^{2,6}]pentacosa-1(25),2(6),4,8(9),1 1,13(18),14,16,21(22),23-decaen-22-amine

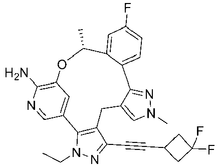

The synthetic method of Example 172 is the same as that of Example 47. LC-MS (ESI) [M+H]⁺: 533.4. ¹H NMR (400 MHz, CDCl₃), 7.51 (d, J = 1.6 Hz, 1H), 7.39 (s, 1H), 7.29 (dd, J = 10.0 Hz, 2.8 Hz, 1H), 7.14 (dd, J = 8.4 Hz, 5.6 Hz, 1H), 6.99 (td, J = 8.4 Hz, 2.8 Hz, 1H), 6.49 (d, J = 1.6 Hz, 1H), 5.37-5.44 (m, 1H), 4.85 (s, 2H), 4.08-4.13 (m, 2H), 3.91 (s, 3H), 3.72 (d, J = 15.6 Hz, 1H), 3.16-3.26 (m, 1H), 2.94-3.06 (m, 2H), 2.76-2.92 (m, 3H), 1.78 (d, J = 6.0 Hz, 3H), 1.39 (t, J = 7.2 Hz, 3H).

### Example 173: 5-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)ethynyl)-1-methyl-1,2-dihydropyridin-2-one

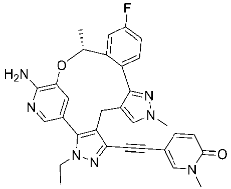

The synthetic method of Example 173 is the same as that of Example 47. LC-MS (ESI) [M+H]⁺: 550.4. ¹H NMR (400 MHz, CDCl₃), 7.63 (d, J = 2.4 Hz, 1H), 7.53 (s, 1H), 7.48 (dd, J = 9.6 Hz, 2.4 Hz, 1H), 7.40 (s, 1H), 7.30 (dd, J = 9.6 Hz, 2.8 Hz, 1H), 7.13 (dd, J = 8.4 Hz, 5.6 Hz, 1H), 7.00 (td, J = 8.4 Hz, 2.8 Hz, 1H), 6.57 (d, J = 9.6 Hz, 1H), 6.51 (s, 1H), 5.38-5.44 (m, 1H), 4.83 (s, 2H), 4.14 (q, J = 7.2 Hz, 2H), 3.92 (s, 3H), 3.78 (d, J = 5.6 Hz, 1H), 3.57 (s, 3H), 2.95 (d, J = 15.6 Hz, 1H), 1.79 (d, J = 6.4 Hz, 3H), 1.42 (t, J = 7.2 Hz, 3H).

### Example 174: (19R)-3-ethyl-16-fluoro-10,19-dimethyl-5-((1-methyl pyrazol-3-yl)ethyny 1)-20-oxa-3,4,10,11,23-pentaazapentacyclo[19.3.1.0^{2,6}.0^{8,12}.0^{13,18}]pentacosa-1(25),2(6),4,8(9),1 1,13(14),15,17,21(22),23-decaen-22-amine

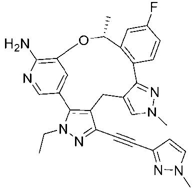

The synthetic method of Example 174 is the same as that of Example 47. LC-MS (ESI) [M+H]⁺: 523.4. ¹H NMR (400 MHz, CDCl₃), 7.51 (d, J = 1.6 Hz, 1H), 7.49 (s, 1H), 7.35 (d, J = 2.4 Hz, 1H), 7.28 (dd, J = 10.0 Hz, 2.8 Hz, 1H), 7.12 (dd, J = 8.4 Hz, 5.6 Hz, 1H), 7.00 (td, J = 8.4 Hz, 2.8 Hz, 1H), 6.57 (d, J = 1.6 Hz, 1H), 6.52 (d, J = 2.0 Hz, 1H), 5.39-5.46 (m, 1H), 4.97 (s, 2H), 4.11-4.17 (m, 2H), 3.94 (s, 3H), 3.90 (d, J = 15.6 Hz, 1H), 3.89 (s, 3H), 2.93 (d, J = 15.6 Hz, 1H), 1.79 (d, J = 6.4 Hz, 3H), 1.42 (t, J = 7.2 Hz, 3H).

### Example 175: (19R)-5-((4-(difluoromethyl)phenyl)ethynyl)-3-ethyl-16-fluoro-10,19-dim ethyl-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine

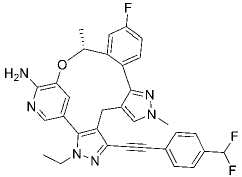

The synthetic method of Example 175 is the same as that of Example 47. LC-MS (ESI) [M+H]⁺: 569.4. ¹H NMR (400 MHz, CD₃OD), 7.69 (d, J = 8.0 Hz, 2H), 7.50-7.59 (m, 5H), 7.20 (dd, J = 8.4 Hz, 5.6 Hz, 1H), 7.05 (t, J = 7.6 Hz, 1H), 6.80 (t, J = 56.0 Hz, 1H), 6.48 (s, 1H), 5.35-5.43 (m, 1H), 4.03-4.23 (m, 2H), 3.90 (s, 3H), 3.86 (d, J = 15.6 Hz, 1H), 2.92 (d, J = 15.6 Hz, 1H), 1.83 (d, J = 5.6 Hz, 3H), 1.30-1.39 (m, 3H).

### Example 176: 5-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)ethynyl)pyridin-3-carbonitrile

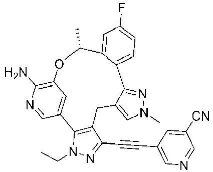

The synthetic method of Example 176 is the same as that of Example 47. LC-MS (ESI) [M+H]⁺: 545.4. ¹H NMR (400 MHz, CD₃OD), 8.99 (d, J = 1.6 Hz, 1H), 8.88 (d, J = 2.0 Hz, 1H), 8.40-8.42 (m, 1H), 7.60 (s, 1H), 7.53 (dd, J = 10.0 Hz, 2.8 Hz, 1H), 7.46 (s, 1H), 7.21 (dd, J = 8.4 Hz, 5.6 Hz, 1H), 7.07 (td, J = 8.4 Hz, 2.8 Hz, 1H), 6.49 (d, J = 1.6 Hz, 1H), 5.39-5.46 (m, 1H), 4.08-4.24 (m, 2H), 3.91 (s, 3H), 3.87 (d, J = 15.6 Hz, 1H), 2.97 (d, J = 15.6 Hz, 1H), 1.81 (d, J = 6.4 Hz, 3H), 1.35 (t, J = 7.2 Hz, 3H).

### Example 177: 1-(3-((19R)-22-amino-3-ethyl-16-fluoro-10,19-dimethyl-20-oxa-3,4,10,11, 23-pentaazapentacyclo[19.3.1.0^{8,12}.0^{13,18}.0^{2,6}]pentacosa-1(24),2(6),4,8(9),11,13(14),15,17,21(2 5),22-decaen-5-yl)prop-2-yn-1-yl)tetrahydropyrrol-2-one

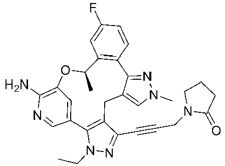

The synthetic method of Example 177 is the same as that of Example 47. LC-MS (ESI) [M+H]⁺: 540.4. ¹H NMR (400 MHz, CDCl₃), 7.50 (d, J = 1.6 Hz, 1H), 7.38 (s, 1H), 7.28 (dd, J = 10.0 Hz, 2.8 Hz, 1H), 7.13 (dd, J = 8.4 Hz, 5.6 Hz, 1H), 6.99 (td, J = 8.4 Hz, 2.8 Hz, 1H), 6.51 (d, J = 1.6 Hz, 1H), 5.37-5.44 (m, 1H), 4.83 (s, 2H), 4.43 (s, 2H), 4.08-4.14 (m, 2H), 3.92 (s, 3H), 3.73 (d, J = 15.6 Hz, 1H), 3.61 (t, J = 7.2 Hz, 2H), 2.90 (d, J = 15.6 Hz, 1H), 2.40 (t, J = 8.0 Hz, 2H), 2.04-2.12 (m, 2H), 1.79 (d, J = 6.0 Hz, 3H), 1.39 (t, J = 7.2 Hz, 3H).

### Example 178: (19R)-3-ethyl-16-fluoro-10,19-dimethyl-5-((5-methyl-1,3,4-thiadiazol-2-y l)ethynyl)-20-oxa-3,4,10,11,23-pentaazapentacyclo[19.3. 1.0^{8,12}.0^{13,18}.0^{2,6}]pentacosa-1(24),2(6), 4,8(9),11,13(14),15,17,21(25),22-decaen-22-amine

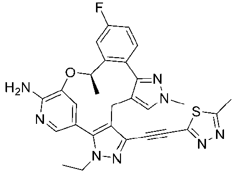

The synthetic method of Example 178 is the same as that of Example 47. LC-MS (ESI) [M+H]⁺: 541.3. ¹H NMR (400 MHz, CDCl₃), 7.53 (s, 1H), 7.43 (s, 1H), 7.29 (dd, J = 10.0 Hz, 2.8 Hz, 1H), 7.14 (dd, J = 8.4 Hz, 5.6 Hz, 1H), 7.01 (td, J = 8.4 Hz, 2.8 Hz, 1H), 6.55 (d, J = 1.6 Hz, 1H), 5.39-5.46 (m, 1H), 4.93 (s, 2H), 4.18 (q, J = 7.2 Hz, 2H), 3.91 (s, 3H), 3.88 (d, J = 15.6 Hz, 1H), 2.99 (d, J = 15.6 Hz, 1H), 2.83 (s, 3H), 1.80 (d, J = 6.8 Hz, 3H), 1.44 (t, J = 7.2 Hz, 3H).

### Example 179: 1-(4-(((19R)-22-amino-3-ethyl-16-fluoro-10,19-dimethyl-20-oxa-3,4,10,1 1,23-pentaazapentacyclo[19.3.1.0^{8,12}.0^{13,18}.0^{2,6}]pentacosa-1(24),2(6),4,8(9),11,13(14),15,17,21 (25),22-decaen-5-yl)ethynyl)piperidin-1-yl)ethan-1-one

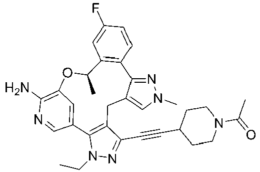

The synthetic method of Example 179 is the same as that of Example 47. LC-MS (ESI) [M+H]⁺: 568.3. ¹H NMR (400 MHz, CDCl₃), 7.51 (s, 1H), 7.37 (s, 1H), 7.29 (dd, J = 10.0 Hz, 2.8 Hz, 1H), 7.12-7.17 (m, 1H), 7.00 (td, J = 8.4 Hz, 2.8 Hz, 1H), 6.49 (s, 1H), 5.37-5.44 (m, 1H), 4.87 (s, 2H), 3.96-4.14 (m, 3H), 3.91 (s, 3H), 3.70-3.80 (m, 2H), 3.32-3.46 (m, 2H), 2.96-3.03 (m, 1H), 2.90 (d, J = 15.6 Hz, 1H), 2.11 (s, 1.5H), 2.10 (s, 1.5H), 1.91-2.03 (m, 2H), 1.73-1.84 (m, 5H), 1.39 (t, J = 7.2 Hz, 3H).

### Example 180: (19R)-3-ethyl-16-fluoro-10,19-dimethyl-5-((1-(methyldioxo-λ⁶-sulfanyl)pi peridin-4-yl)ethynyl)-20-oxa-3,4,10,11,23-pentaazapentacyclo[ 19.3.1.0^{8,12}.0^{2,6}.0^{13,18}]pentacosa-1(24),2(6),4,8(9),11,13(14),15,17,21(25),22-decaen-22-amine

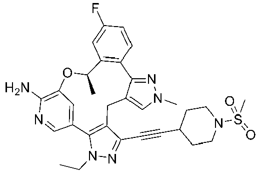

The synthetic method of Example 180 is the same as that of Example 47. LC-MS (ESI) [M+H]⁺: 604.3. ¹H NMR (400 MHz, CDCl₃), 7.50 (d, J = 1.6 Hz, 1H), 7.37 (s, 1H), 7.29 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.49 (d, J = 1.6 Hz, 1H), 5.37-5.43 (m, 1H), 4.88 (s, 2H), 4.08-4.14 (m, 2H), 3.92 (s, 3H), 3.71 (d, J = 15.6 Hz, 1H), 3.52-3.59 (m, 2H), 3.14-3.21 (m, 2H), 2.89-2.98 (m, 2H), 2.78 (s, 3H), 2.04-2.12 (m, 2H), 1.88-1.98 (m, 2H), 1.79 (d, J = 6.4 Hz, 3H), 1.39 (t, J = 7.2 Hz, 3H).

### Example 181: 4-(((19R)-22-amino-3-ethyl-16-fluoro-10,19-dimethyl-20-oxa-3,4,10,11, 23-pentaazapentacyclo[19.3.1.0^{8,12}.0^{13,18}.0^{2,6}]pentacosa-1(24),2(6),4,8(9),11,13(14),15,17,21(2 5),22-decaen-5-yl)ethynyl)cyclohexan-1-one

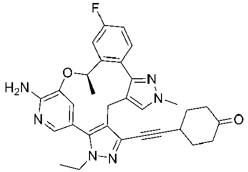

The synthetic method of Example 181 is the same as that of Example 47. LC-MS (ESI) [M+H]⁺: 539.4. ¹H NMR (400 MHz, CDCl₃), 7.50 (d, J = 1.6 Hz, 1H), 7.37 (s, 1H), 7.29 (dd, J = 10.0 Hz, 2.4 Hz, 1H), 7.14 (dd, J = 8.4 Hz, 5.¹H NMR (400 MHz, CD₃OD),6 Hz, 1H), 7.00 (td, J = 8.4 Hz, 2.4 Hz, 1H), 6.49 (d, J = 1.6 Hz, 1H), 5.37-5.44 (m, 1H), 4.85 (s, 2H), 4.08-4.14 (m, 2H), 3.91 (s, 3H), 3.72 (d, J = 15.6 Hz, 1H), 3.16-3.22 (m, 1H), 2.91 (d, J = 15.6 Hz, 1H), 2.67-2.75 (m, 2H), 2.37-2.44 (m, 2H), 2.12-2.23 (m, 4H), 1.79 (d, J = 6.4 Hz, 3H), 1.39 (t, J = 7.2 Hz, 3H).

### Example 182: (19R)-3-ethyl-9-ethynyl-16-fluoro-10,19-dimethyl-20-oxa-3,4,10,11,23-pe ntaazapentacyclo[19.3.1.0^{2,6}.0^{8,12}.0^{13,18}]pentacosa-1(24),2(6),4,8(9),11,13(14),15,17,21(25),22-decaen-22-amine

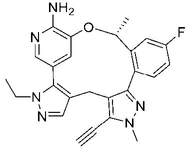

The synthetic method of Example 182 is the same as that of Example 47. LC-MS (ESI) [M+H]⁺: 443.4. ¹H NMR (400 MHz, CD₃OD), 7.68 (s, 1H), 7.24-7.61 (m, 2H), 7.13 (dd, J = 8.4 Hz, 5.6 Hz, 1H), 7.05 (td, J = 8.4 Hz, 5.6 Hz, 1H), 6.50 (s, 1H), 5.48-5.54 (m, 1H), 4.42 (s, 1H), 4.00-4.15 (m, 2H), 3.95 (s, 3H), 3.77 (d, J = 15.6 Hz, 1H), 3.09 (d, J = 15.6 Hz, 1H), 1.82 (d, J = 6.0 Hz, 3H), 1.30 (t, J = 7.2 Hz, 3H).

### Example 183: (19R)-3-ethyl-16-fluoro-10,19-dimethyl-5-((1-methyl-1,2,4-triazol-,3-yl)et hynyl)-20-oxa-3,4,10,11,23-pentaazapentacyclo[19.3. 1.0^{2,6}.0^{8,12}.0^{13,18}]pentacosa-1(24),2(6),4,8 (9),11,13(18),14,16,21(25),22-decaen-22-amine

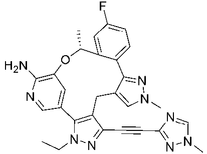

The synthetic method of Example 183 is the same as that of Example 47. LC-MS (ESI) [M+H]⁺: 443.4. ¹H NMR (400 MHz, CD₃OD), 8.48 (s, 1H), 7.62 (s, 1H), 7.53 (dd, J = 10.0 Hz, 2.8 Hz, 1H), 7.46 (d, J = 2.0 Hz, 1H), 7.20 (dd, J = 8.4 Hz, 5.6 Hz, 1H), 7.08 (td, J = 8.4 Hz, 2.8 Hz, 1H), 6.50 (d, J = 2.0 Hz, 1H), 5.40-5.46 (m, 1H), 4.08-4.23 (m, 2H), 3.98 (s, 3H), 3.92 (s, 3H), 3.86 (d, J = 15.6 Hz, 1H), 2.96 (d, J = 15.6 Hz, 1H), 1.81 (d, J = 6.4 Hz, 3H), 1.35 (t, J = 7.2 Hz, 3H).

### Example 184: (19R)-5-((1,2-dimethylimidazol-4-yl)ethynyl)-3-ethyl-16-fluoro-10,19-di methyl-20-oxa-3,4,10,11,23-pentaazapentacyclo[19.3.1.0^{2,6}.0^{8,12}.0^{13,18}]pentacosa-1(24),2(6),4,8 (9),11,13(18),14,16,21(25),22-decaen-22-amine

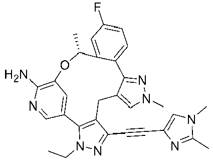

The synthetic method of Example 184 is the same as that of Example 47. LC-MS (ESI) [M+H]⁺: 537.4. ¹H NMR (400 MHz, CD₃OD), 7.62 (s, 1H), 7.53 (dd, J = 10.0 Hz, 2.4 Hz, 1H), 7.44 (d, J = 1.6 Hz, 1H), 7.36 (s 1H), 7.18 (dd, J = 8.4 Hz, 5.6 Hz, 1H), 7.08 (td, J = 8.4 Hz, 2.4 Hz, 1H), 6.50 (d, J = 1.6 Hz, 1H), 5.39-5.46 (m, 1H), 4.04-4.20 (m, 2H), 3.92 (s, 3H), 3.83 (d, J = 15.6 Hz, 1H), 3.65 (s, 3H), 2.91 (d, J = 15.6 Hz, 1H), 2.38 (s, 3H), 1.81 (d, J = 6.4 Hz, 3H), 1.33 (t, J = 7.2 Hz, 3H).

### Example 185: (19R)-3-ethyl-16-fluoro-10,19-dimethyl-5-((3-methylimidazol-4-yl)ethyny l)-20-oxa-3,4,10,11,23-pentaazapentacyclo[19.3.1 .0^{2,6}.0^{8,12}.0^{13,18}]pentacosa-1(24),2(6),4,8(9), 1 1,13(18),14,16,21(25),22-decaen-22-amine

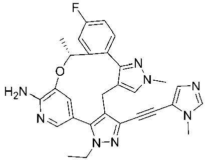

The synthetic method of Example 185 is the same as that of Example 47. LC-MS (ESI) [M+H]⁺: 523.4. ¹H NMR (400 MHz, CD₃OD), 7.82 (s, 1H), 7.60 (s, 1H), 7.54 (dd, J = 10.0 Hz, 2.8 Hz, 1H), 7.46 (s, 1H), 7.34-7.44 (brs, 1H), 7.22 (dd, J = 8.4 Hz, 5.6 Hz, 1H), 7.08 (td, J = 8.4 Hz, 2.8 Hz, 1H), 6.50 (d, J = 1.6 Hz, 1H), 5.40-5.47 (m, 1H), 4.10-4.21 (m, 2H), 3.92 (s, 3H), 3.85 (s, 3H), 3.83 (d, J = 15.6 Hz, 1H), 2.97 (d, J = 15.6 Hz, 1H), 1.82 (d, J = 6.4 Hz, 3H), 1.35 (t, J = 7.2 Hz, 3H).

### Example 186: (19R)-3-ethyl-16-fluoro-10,19-dimethyl-5-((1-methyl-1,2,3-triazol-,4-yl)et hynyl)-20-oxa-3,4,10,11,23-pentaazapentacyclo[19.3.1.0^{2,6}.0^{8,12}.0^{13,18}]pentacosa-1(24),2(6),4,8 (9),11,13(18),14,16,21(25),22-decaen-22-amine

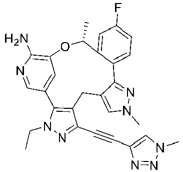

The synthetic method of Example 186 is the same as that of Example 47. LC-MS (ESI) [M+H]⁺: 524.3. ¹H NMR (400 MHz, CD₃OD), 8.26 (s, 1H), 7.61 (s, 1H), 7.53 (dd, J = 10.0 Hz, 2.8 Hz, 1H), 7.45 (d, J = 1.6 Hz, 1H), 7.19 (dd, J = 8.4 Hz, 5.6 Hz, 1H), 7.07 (td, J = 8.4 Hz, 2.8 Hz, 1H), 6.50 (d, J = 1.6 Hz, 1H), 5.39-5.45 (m, 1H), 4.06-4.22 (m, 5H), 3.92 (s, 3H), 3.83 (d, J = 15.6 Hz, 1H), 2.94 (d, J = 15.6 Hz, 1H), 1.80 (d, J = 6.4 Hz, 3H), 1.34 (t, J = 7.2 Hz, 3H).

### Example 187: 3-(((19R)-22-amino-3-ethyl-16-fluoro-10,19-dimethyl-20-oxa-3,4,10,11,2 3-pentaazapentacyclo[19.3.1.0^{2,6}.0^{8,12}.0^{13,18}]pentacosa-1(25),2(6),4,8(9),11,13(14),15,17,21(22), 23-decaen-5-yl)ethynyl)-1-methyl cyclobut-1-ol

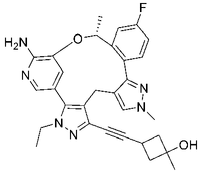

The synthetic method of Example 187 is the same as that of Example 47. LC-MS (ESI) [M+H]⁺: 527.3. ¹H NMR (400 MHz, CDCl₃), 7.49 (s, 1H), 7.41 (s, 1H), 7.29 (dd, J = 10.0 Hz, 2.8 Hz, 1H), 7.15 (dd, J = 8.4 Hz, 5.6 Hz, 1H), 7.00 (td, J = 8.4 Hz, 2.8 Hz, 1H), 6.52 (s, 1H), 5.38-5.45 (m, 1H), 4.97 (s, 2H), 4.06-4.16 (m, 2H), 3.91 (s, 3H), 3.75 (d, J = 15.6 Hz, 1H), 2.81-2.91 (m, 2H), 2.51-2.56 (m, 2H), 2.17-2.23 (m, 2H), 1.79 (d, J = 6.4 Hz, 3H), 1.39 (t, J = 7.2 Hz, 3H), 3.40 (s, 3H).

### Example 188: (19R)-3-ethyl-16-fluoro-10,19-dimethyl-5-((1-(oxetan-3-yl)pyrazol-4-yl)et hynyl)-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine

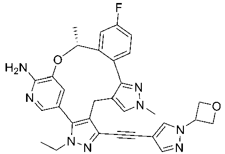

The synthetic method of Example 188 is the same as that of Example 47. LC-MS (ESI) [M+H]⁺: 527.3. ¹H NMR (400 MHz, CDCl₃), 7.81 (s, 1H), 7.78 (s, 1H), 7.53 (d, J = 1.2 Hz, 1H), 7.43 (s, 1H), 7.29 (dd, J = 10.0 Hz, 2.8 Hz, 1H), 7.14 (dd, J = 8.4 Hz, 5.6 Hz, 1H), 6.99 (td, J = 8.4 Hz, 2.8 Hz, 1H), 6.52 (d, J = 1.2 Hz, 1H), 5.38-5.49 (m, 2H), 5.06 (d, J = 6.4 Hz, 4H), 6.85 (s, 2H), 4.11-4.16 (m, 2H), 3.90 (s, 3H), 3.81 (d, J = 15.6 Hz, 1H), 2.94 (d, J = 15.6 Hz, 1H), 1.78 (d, J = 6.0 Hz, 3H), 1.41 (t, J = 7.2 Hz, 3H).

### Example 189: (19R)-5-((3-(difluoromethyl)imidazol-4-yl)ethynyl)-3-ethyl-16-fluoro-10, 19-dimethyl-20-oxa-3,4,10,11,23-pentaazapentacyclo[19.3.1.0^{1,12}.0^{13,18}.0^{2,6} ]pentacosa-1(25),2 (6),4,8(9),11,13(18),14,16,21(22),23-decaen-22-amine

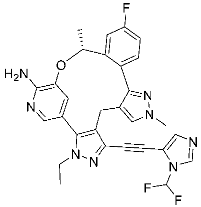

The synthetic method of Example 189 is the same as that of Example 47. LC-MS (ESI) [M+H]⁺: 559.3. ¹H NMR (400 MHz, CD₃OD), 8.20 (s, 1H), 7.71 (t, J = 58.8 Hz, 1H), 7.57 (s, 1H), 7.54 (dd, J = 10.0 Hz, 2.8 Hz, 1H), 7.50 (s, 1H), 7.46 (d, J = 1.6 Hz, 1H), 7.19 (dd, J = 8.4 Hz, 5.6 Hz, 1H), 7.08 (td, J = 8.4 Hz, 2.8 Hz, 1H), 6.50 (d, J = 1.6 Hz, 1H), 5.40-5.46 (m, 1H), 4.08-4.23 (m, 2H), 3.91 (s, 3H), 3.82 (d, J = 15.6 Hz, 1H), 2.96 (d, J = 15.6 Hz, 1H), 1.81 (d, J = 6.4 Hz, 3H), 1.35 (t, J = 7.2 Hz, 3H).

### Example 190: (19R)-5-((1-(difluoromethyl)imidazol-4-yl)ethynyl)-3-ethyl-16-fluoro-10, 19-dimethyl-20-oxa-3,4,10,11,23-pentaazapentacyclo[19.3.1.0^{8,12}.0^{13,18}.0^{2,6}]pentacosa-1(25),2 (6),4,8(9),11,13(18),14,16,21(22),23-decaen-22-amine

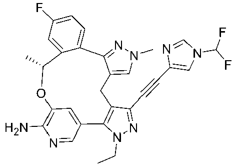

The synthetic method of Example 190 is the same as that of Example 47. LC-MS (ESI) [M+H]⁺: 559.3. ¹H NMR (400 MHz, CD₃OD), 8.20 (s, 1H), 7.90 (d, J = 1.6 Hz, 1H), 7.62 (s, 1H), 7.60 (t, J = 60.0 Hz, 1H), 7.53 (dd, J = 10.4 Hz, 2.8 Hz, 1H), 7.49 (s, 1H), 7.20 (dd, J = 8.4 Hz, 5.6 Hz, 1H), 7.08 (td, J = 8.4 Hz, 2.8 Hz, 1H), 6.52 (d, J = 1.6 Hz, 1H), 5.40-5.46 (m, 1H), 4.05-4.20 (m, 2H), 3.92 (s, 3H), 3.84 (d, J = 15.6 Hz, 1H), 2.93 (d, J = 15.6 Hz, 1H), 1.81 (d, J = 6.4 Hz, 3H), 1.33 (t, J = 7.2 Hz, 3H).

### Example 191: (19R)-5-((1-cyclopropylpyrazol-4-yl)ethynyl)-3-ethyl-16-fluoro-10,19-di methyl-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine

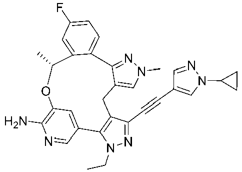

The synthetic method of Example 191 is the same as that of Example 47. LC-MS (ESI) [M+H]⁺: 549.3. ¹H NMR (400 MHz, CDCl₃ +CD₃OD), 7.88 (s, 1H), 7.52-7.82 (brs, 2H), 7.51 (s, 1H), 7.44 (dd, J = 10.0 Hz, 2.8 Hz, 1H), 7.17 (dd, J = 8.4 Hz, 5.6 Hz, 1H), 7.04 (td, J = 8.4 Hz, 2.8 Hz, 1H), 6.53 (s, 1H), 5.36-5.42 (m, 1H), 4.05-4.16 (m, 2H), 3.91 (s, 3H), 3.79 (d, J = 15.6 Hz, 1H), 3.62-3.67 (m, 1H), 2.91 (d, J = 15.6 Hz, 1H), 1.81 (d, J = 6.4 Hz, 3H), 1.35 (t, J = 7.2 Hz, 3H), 1.03-1.14 (m, 4H).

### Example 192: (5-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)ethynyl)pyridin-2-yl)methanol

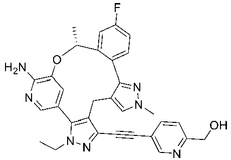

The synthetic method of Example 192 is the same as that of Example 47. LC-MS (ESI) [M+H]⁺: 550.4. ¹H NMR (400 MHz, CD₃OD), 8.69 (d, J = 2.0 Hz, 1H), 8.04 (dd, J = 8.0 Hz, 2.0 Hz, 1H), 7.61-7.63 (m, 2H), 7.54 (dd, J = 10.0 Hz, 2.8 Hz, 1H), 7.46 (d, J = 2.0 Hz, 1H), 7.23 (dd, J = 8.4 Hz, 6.0 Hz, 1H), 7.08 (td, J = 8.4 Hz, 2.8 Hz, 1H), 6.50 (d, J = 2.0 Hz, 1H), 5.40-5.46 (m, 1H), 4.73 (s, 2H), 4.08-4.23 (m, 2H), 3.92 (s, 3H), 3.87 (d, J = 15.6 Hz, 1H), 2.97 (d, J = 15.6 Hz, 1H), 1.82 (d, J = 6.4 Hz, 3H), 1.35 (t, J = 7.2 Hz, 3H).

### Example 193: N-(4-((19R)-22-amino-3-ethyl-16-fluoro-10,19-dimethyl-20-oxa-3,4,10,11, 23-pentaazapentacyclo[19.3.1.0^{8,12}.0^{13,18}.0^{2,6}]pentacosa-1(25),2(6),4,8(9),11,13(18),14,16,21 (2 2),23-decaen-5-yl)-2-methylbut-3-yn-2-yl)methanesulfonamide

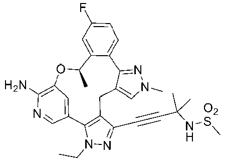

The synthetic method of Example 193 is the same as that of Example 47. LC-MS (ESI) [M+H]⁺: 578.4. ¹H NMR (400 MHz, CD₃OD), 7.72 (s, 1H), 7.52 (dd, J = 10.4 Hz, 2.8 Hz, 1H), 7.44 (s, 1H), 7.19 (dd, J = 8.4 Hz, 5.6 Hz, 1H), 7.07 (td, J = 8.4 Hz, 2.8 Hz, 1H), 6.49 (s, 1H), 5.39-5.45 (m, 1H), 4.02-4.18 (m, 2H), 3.92 (s, 3H), 3.77 (d, J = 15.6 Hz, 1H), 3.17 (s, 3H), 2.89 (d, J = 15.6 Hz, 1H), 1.81 (d, J = 6.4 Hz, 3H), 1.75 (s, 3H), 1.72 (s, 3H), 1.31 (t, J = 7.2 Hz, 3H).

### Example 194: (19R)-3-ethyl-16-fluoro-10,19-dimethyl-5-((3-(oxetan-3-yl)imidazol-4-yl) ethynyl)-20-oxa-3,4,10,11,23-pentaazapentacyclo[19.3.1.0^{8,12}.0^{13,18}.0^{2,6}]pentacosa-1(25),2(6),4, 8(9),11,13(18),14,16,21(22),23-decaen-22-amine

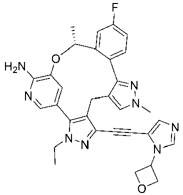

The synthetic method of Example 194 is the same as that of Example 47. LC-MS (ESI) [M+H]⁺: 565.4. ¹H NMR (400 MHz, CDCl₃), 7.64 (s, 1H), 7.56 (d, J = 1.2 Hz, 1H), 7.53 (d, J = 1.2 Hz, 1H), 7.50 (s, 1H), 7.28 (dd, J = 10.0 Hz, 2.8 Hz, 1H), 7.12 (dd, J = 8.4 Hz, 5.6 Hz, 1H), 6.99 (td, J = 8.4 Hz, 2.8 Hz, 1H), 6.56 (d, J = 1.6 Hz, 1H), 5.40-5.45 (m, 1H), 5.25-5.33 (m, 1H), 5.11-5.15 (m, 2H), 4.78-4.87 (m, 4H), 4.12-4.17 (m, 2H), 3.90 (d, J = 15.6 Hz, 1H), 3.89 (s, 3H), 2.94 (d, J = 15.6 Hz, 1H), 1.79 (d, J = 6.0 Hz, 3H), 1.42 (t, J = 7.2 Hz, 3H).

### Example 195: (19R)-5-(dimethyl(oxo)-λ⁵-phosphanyl)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,1 3(18),14,16,21(22),23-decaen-22-amine

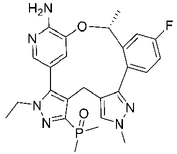

The synthetic method of Example 195 is the same as that of Example 169. LC-MS (ESI) [M+H]⁺: 495.3. ¹H NMR (400 MHz, CDCl₃), 7.86 (s, 1H), 7.48 (s, 1H), 7.26 (dd, J = 10.0 Hz, 2.8 Hz, 1H), 7.10 (dd, J = 8.4 Hz, 5.6 Hz, 1H), 6.96 (td, J = 8.4 Hz, 2.8 Hz, 1H), 6.60 (d, J = 1.6 Hz, 1H), 5.44-5.50 (m, 1H), 4.84 (s, 2H), 4.06-4.17 (m, 3H), 3.88 (s, 3H), 2.98 (d, J = 15.6 Hz, 1H), 1.97 (d, J = 13.6 Hz, 3H), 1.79 (d, J = 6.4 Hz, 3H), 1.76 (d, J = 13.6 Hz, 3H), 1.37 (t, J = 7.2 Hz, 3H).

### Example 196: (19R)-3-ethyl-16-fluoro-10,19-dimethyl-5-((4-methyl-3,4,5,6-tetrahydro-2 H-pyran-4-yl)ethynyl)-20-oxa-3,4,10,11,23-pentaazapentacyclo[19.3.1.0^{8,12}.0^{13,18}.0^{2,6}]pentacosa -1(24),2(6),4,8(9),11,13(14),15,17,21(25),22-decaen-22-amine

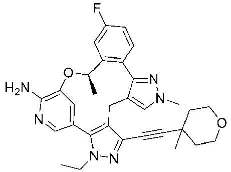

The synthetic method of Example 196 is the same as that of Example 47. LC-MS (ESI) [M+H]⁺: 541.4. ¹H NMR (400 MHz, CDCl₃), 7.50 (d, J = 1.6 Hz, 1H), 7.40 (s, 1H), 7.29 (dd, J = 10.0 Hz, 2.8 Hz, 1H), 7.16 (dd, J = 8.4 Hz, 5.6 Hz, 1H), 7.00 (td, J = 8.4 Hz, 2.8 Hz, 1H), 6.50 (d, J = 1.6 Hz, 1H), 5.39-5.44 (m, 1H), 4.85 (s, 2H), 4.07-4.16 (m, 2H), 3.84-3.93 (m, 7H), 3.73 (d, J = 15.6 Hz, 1H), 2.92 (d, J = 15.6 Hz, 1H), 1.80 (d, J = 6.4 Hz, 3H), 1.58-1.70 (m, 4H), 1.43 (s, 3H), 1.39 (t, J = 7.2 Hz, 3H).

### Example 197: 4-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)tetrahydropyran-4-carbonitrile

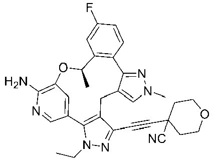

The synthetic method of Example 197 is the same as that of Example 47. LC-MS (ESI) [M+H]⁺: 552.4. ¹H NMR (400 MHz, CDCl₃), 7.52 (s, 1H), 7.38 (s, 1H), 7.29 (dd, J = 10.0 Hz, 2.8 Hz, 1H), 7.15 (dd, J = 8.4 Hz, 6.0 Hz, 1H), 7.00 (td, J = 8.4 Hz, 2.8 Hz, 1H), 6.50 (d, J = 1.2 Hz, 1H), 5.38-5.45 (m, 1H), 4.76-4.96 (brs, 2H), 4.11-4.16 (m, 2H), 3.81-4.00 (m, 7H), 3.71 (d, J = 15.6 Hz, 1H), 2.95 (d, J = 15.6 Hz, 1H), 2.15-2.31 (m, 4H), 1.79 (d, J = 6.4 Hz, 3H), 1.40 (t, J = 7.2 Hz, 3H).

### Example 198: (19R)-3-ethyl-16-fluoro-5-((1-(2-methoxyethyl)imidazol-4-yl)ethynyl)-10, 19-dimethyl-20-oxa-3,4,10,11,23-pentaazapentacyclo[19.3.1.0^{2,6}.0^{8,12}.0^{13,18}]pentacosa-1(25),2 (6),4,8(9),11,13(14),15,17,21(22),23-decaen-22-amine

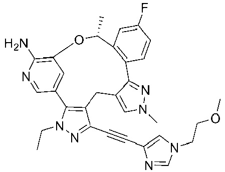

The synthetic method of Example 198 is the same as that of Example 47. LC-MS (ESI) [M+H]⁺: 567.4. ¹H NMR (400 MH¹H NMR (400 MHz, CD₃OD), 7.50-7.55 (m, 2H), 7.46 (s, 1H), 7.30 (s, 1H), 7.27 (dd, J = 10.0 Hz, 2.8 Hz, 1H), 7.13 (dd, J = 8.4 Hz, 6.0 Hz, 1H), 7.01 (td, J = 8.4 Hz, 2.8 Hz, 1H), 6.61 (s, 1H), 5.30-5.47 (m, 3H), 4.04-4.15 (m, 4H), 3.92 (d, J = 15.6 Hz, 1H), 3.89 (s, 3H), 3.64 (t, J = 4.8 Hz, 2H), 3.36 (s, 3H), 2.92 (d, J = 15.6 Hz, 1H), 1.80 (d, J = 6.4 Hz, 3H), 1.42 (t, J = 7.2 Hz, 3H).

### Example 199: (19R)-3-ethyl-16-fluoro-5-((1-(2-methoxyethyl)pyrazol-4-yl)ethynyl)-10,1 9-dimethyl-20-oxa-3,4,10,11,23-pentaazapentacyclo[19.3.1.0^{2,6}.0^{8,12}.0^{13,18}]pentacosa-1(25),2(6), 4,8(9),11,13(14),15,17,21(22),23-decaen-22-amine

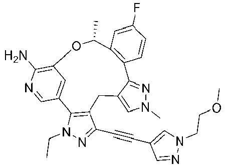

The synthetic method of Example 199 is the same as that of Example 47. LC-MS (ESI) [M+H]⁺: 567.4. ¹H NMR (400 MHz, CDCl₃), 7.33-7.80 (m, 4H), 7.28 (dd, J = 10.0 Hz, 2.8 Hz, 1H), 7.15 (dd, J = 8.4 Hz, 5.6 Hz, 1H), 7.01 (td, J = 8.4 Hz, 2.8 Hz, 1H), 6.56 (s, 1H), 5.38-5.45 ((m, 1H), 4.76-5.32 (brs, 2H), 4.30 (t, J = 5.2 Hz, 2H), 4.09-4.14 (m, 2H), 3.91 (s, 3H), 3.83 (d, J = 15.6 Hz, 1H), 3.75 (t, J = 5.2 Hz, 2H), 3.35 (s, 3H), 2.93 (d, J = 15.6 Hz, 1H), 1.80 (d, J = 6.4 Hz, 3H), 1.40 (t, J = 7.2 Hz, 3H).

### Example 200: (6-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)ethynyl)pyridin-2-yl)methanol

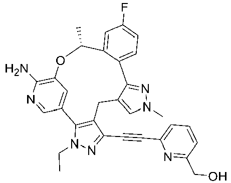

The synthetic method of Example 200 is the same as that of Example 47. LC-MS (ESI) [M+H]⁺: 550.4. ¹H NMR (400 MHz, CD₃OD), 7.89 (t, J = 8.0 Hz, 1H), 7.65 (s, 1H), 7.58 (d, J = 8.0 Hz, 2H), 7.54 (dd, J = 10.0 Hz, 2.8 Hz, 1H), 7.34-7.53 (brs, 1H), 7.21 (dd, J = 8.8 Hz, 6.0 Hz, 1H), 7.08 (td, J = 8.4 Hz, 2.8 Hz, 1H), 6.51 (s, 1H), 5.40-5.46 (m, 1H), 4.71 (s, 2H), 4.08-4.23 (m, 2H), 3.93 (d, J = 15.6 Hz, 1H), 3.91 (s, 3H), 2.95 (d, J = 15.6 Hz, 1H), 1.81 (d, = 6.4 Hz, 3H), 1.35 (t, J = 7.2 Hz, 3H).

### Example 201: 2-(6-(((19R)-22-amino-3-ethyl-16-fluoro-10,19-dimethyl-20-oxa-3,4,10,1 1 ,23-pentaazapentacyclo[19.3.1.0^{2,6}.0^{8,12}.0^{13,18}]pentacosa-1\(24),2(6),4,8(9),11,13(18),14,16,21 (25),22-decaen-5-yl)ethynyl)pyridin-3-yl)prop-2-ol

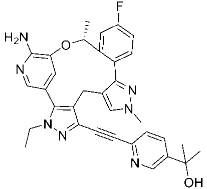

The synthetic method of Example 201 is the same as that of Example 47. LC-MS (ESI) [M+H]⁺: 578.4. ¹H NMR (400 MHz, CDCl₃), 8.76 (d, J = 2.0 Hz, 1H), 7.84 (dd, J = 8.0 Hz, 2.0 Hz, 1H), 7.58 (d, J = 8.0 Hz, 1H), 7.48-7.52 (m, 2H), 7.28 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.57 (d, J = 1.6 Hz, 1H), 5.39-5.45 (m, 1H), 4.11-4.20 (m, 2H), 3.94 (d, J = 15.6 Hz, 1H), 3.88 (s, 3H), 2.96 (d, J = 15.6 Hz, 1H), 1.79 (d, J = 6.4 Hz, 3H), 1.62 (s, 6H), 1.43 (t, J = 7.2 Hz, 3H).

### Example 202: (6-(((19R)-22-amino-3-ethyl-16-fluoro-10,19-dimethyl-20-oxa-3,4,10,11,2 3-pentaazapentacyclo[19.3.1.0^{2,6}.0^{8,12}.0^{13,18}]pentacosa-1(24),2(6),4,8(9),11,13(18),14,16,21(25), 22-decaen-5-yl)ethynyl)pyridin-3-yl)methanol

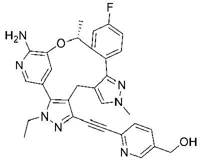

The synthetic method of Example 202 is the same as that of Example 47. LC-MS (ESI) [M+H]⁺: 550.4. ¹H NMR (400 MHz, CD₃OD), 8.62 (s, 1H), 7.96-7.99 (m, 1H), 7.78 (dd, J = 7.6 Hz, 3.2 Hz, 1H), 7.67 (s, 1H), 7.56 (dd, J = 10.0 Hz, 2.8 Hz, 1H), 7.50 (s, 1H), 7.27-7.31 (m, 1H), 7.16 (td, J = 8.4 Hz, 2.8 Hz, 1H), 6.80 (s, 1H), 5.56-5.63 (m, 1H), 4.72 (s, 2H), 4.13-4.23 (m, 2H), 4.01 (d, J = 15.6 Hz, 1H), 3.91 (s, 3H), 3.01 (d, J = 15.6 Hz, 1H), 1.88 (d, J = 6.4 Hz, 3H), 1.37 (t, J = 7.2 Hz, 3H).

### Example 203: 2-(6-(((19R)-22-amino-3-ethyl-16-fluoro-10,19-dimethyl-20-oxa-3,4,10,1 1,23-pentaazapentacyclo[19.3.1.0^{8,12}.0^{13,18}.0^{2,6}]pentacosa-1(25),2(6),4,8(9),11,13(18),14,16,21 (22),23-decaen-5-yl)ethynyl)pyridin-2-yl)prop-2-ol

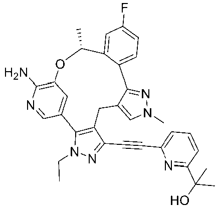

The synthetic method of Example 203 is the same as that of Example 47. LC-MS (ESI) [M+H]⁺: 578.4. ¹H NMR (400 MHz, CD₃OD), 7.33-7.91 (m, 6H), 7.20-7.24 (m, 1H), 7.06-7.10 (m, 1H), 6.53 (s, 1H), 5.38-5.50 (m, 1H), 4.05-4.26 (m, 2H), 3.78-3.99 (m, 4H), 2.96 (d, J = 14.8 Hz, 1H), 1.82 (d, J = 6.0 Hz, 3H), 1.56 (s, 6H), 1.30-1.39 (m, 3H).

### Example 204: (19R)-5-((1-cyclopropylimidazol-4-yl)ethynyl)-3-ethyl-16-fluoro-10,19-di methyl-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine

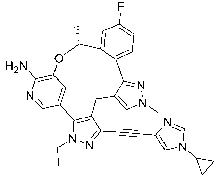

The synthetic method of Example 204 is the same as that of Example 47. LC-MS (ESI) [M+H]⁺: 549.4. ¹H NMR (400 MHz, CD₃OD), 7.78 (s,1 H), 7.61 (s, 1H), 7.55 (s, 1H), 7.53 (dd, J = 10.0 Hz, 2.8 Hz, 1H), 7.44 (d, J = 1.6 Hz, 1H), 7.19 (dd¹H NMR (400 MHz, CDCl₃), J = 8.4 Hz, 5.6 Hz, 1H), 7.07 (td, J = 8.4 Hz, 2.8 Hz, 1H), 6.50 (d, J = 1.6 Hz, 1H), 5.40-5.46 (m, 1H), 4.05-4.20 (m, 2H), 3.92 (s, 3H), 3.83 (d, J = 15.6 Hz, 1H), 3.50-3.56 (m, 1H), 2.92 (d, J = 15.6 Hz, 1H), 1.81 (d, J = 6.4 Hz, 3H), 1.33 (t, J = 7.2 Hz, 3H), 04-1.08 (m, 4H).

### Example 205: (19R)-5-((1-cyclopropylimidazol-5-yl)ethynyl)-3-ethyl-16-fluoro-10,19-di methyl-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine

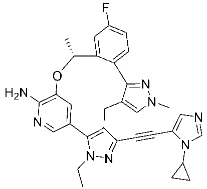

The synthetic method of Example 205 is the same as that of Example 47. LC-MS (ESI) [M+H]⁺: 549.4. ¹H NMR (400 MHz, CD₃OD), 7.77 (s, 1H), 7.60 (s, 1H), 7.54 (dd, J = 10.0 Hz, 2.8 Hz, 1H), 7.46 (d, J = 1.6 Hz, 1H), 7.32 (s, 1H), 7.20 (dd, J = 8.4 Hz, 5.6 Hz, 1H), 7.07 (td, J = 8.4 Hz, 2.8 Hz, 1H), 6.49 (d, J = 1.6 Hz, 1H), 5.40-5.46 (m, 1H), 4.08-4.23 (m, 2H), 3.91 (s, 3H), 3.86 (d, J = 15.6 Hz, 1H), 3.48-3.54 (m, 1H), 2.97 (d, J = 15.6 Hz, 1H), 1.81 (d, J = 6.4 Hz, 3H), 1.35 (t, J = 7.2 Hz, 3H), 1.12-1.13 (m, 4H).

### Example 206: (19R)-9-((dimethyl(oxo)-λ⁶-sulfanylidene)amino)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(24),2(6),4, 8(9),11,13(14),15,17,21(25),22-decaen-22-amine

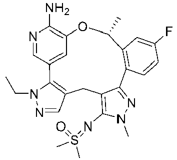

The synthetic method of Example 206 is the same as that of Example 169. LC-MS (ESI) [M+H]⁺: 510.3. ¹H NMR (400 MHz, CDCl₃), 7.69 (s, 1H), 7.39 (s, 1H), 7.25 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.84 (s, 1H), 5.66-5.98 (brs, 2H), 5.56-5.63 (m, 1H), 4.07-4.20 (m, 2H), 3.80 (s, 3H), 3.71 (d, J = 15.6 Hz, 1H), 3.27 (s, 3H), 3.03 (d, J = 15.6 Hz, 1H), 2.58 (s, 3H), 1.84 (d, J = 6.4 Hz, 3H), 1.37 (t, J = 7.2 Hz, 3H).

### Example 207: (19R)-3-ethyl-16-fluoro-10,19-dimethyl-5-((1-(2,2,2-trifluoroethyl)imidaz ol-4-yl)ethynyl)-20-oxa-3,4,10,11,23-pentaazapentacyclo[19.3.1.0^{2,6}.0^{8,12}.0^{13,18}]pentacosa-1(2 4),2(6),4,8(9),11,13(18),14,16,21(25),22-decaen-22-amine

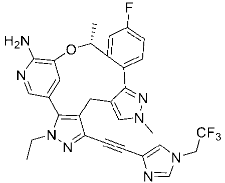

The synthetic method of Example 207 is the same as that of Example 47. LC-MS (ESI) [M+H]⁺: 591.4. ¹H NMR (400 MHz, CD₃OD), 7.85 (s, 1H), 7.63 (s, 2H), 7.53 (dd, J = 10.0 Hz, 2.8 Hz, 1H), 7.45 (s, 1H), 7.20 (dd, J = 8.4 Hz, 5.6 Hz, 1H), 7.07 (td, J = 8.4 Hz, 2.8 Hz, 1H), 6.50 (s, 1H), 5.40-5.46 (m, 1H), 4.95 (q, J = 8.8 Hz, 2H), 4.05-4.21 (m, 2H), 3.92 (s, 3H), 3.85 (d, J = 15.6 Hz, 1H), 2.93 (d, J = 15.6 Hz, 1H), 1.81 (d, J = 6.4 Hz, 3H), 1.34 (t, J = 7.2 Hz, 3H).

### Example 208: (19R)-3-ethyl-16-fluoro-10,19-dimethyl-5-((1-(2,2,2-trifluoroethyl)pyrazol -4-yl)ethynyl)-20-oxa-3,4,10,11,23-pentaazapentacyclo[19.3.1.0^{2,6}.0^{8,12}.0^{13,18}]pentacosa-1(24),2 (6),4,8(9),11,13(18),14,16,21(25),22-decaen-22-amine

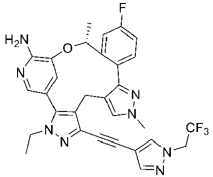

The synthetic method of Example 208 is the same as that of Example 47. LC-MS (ESI) [M+H]⁺: 591.4. ¹H NMR (400 MHz, CD₃OD), 8.11 (s, 1H), 7.81 (s, 1H), 7.58 (s, 1H), 7.53 (dd, J = 10.0 Hz, 2.8 Hz, 1H), 7.45 (s, 1H), 7.21 (dd, J = 8.4 Hz, 5.6 Hz, 1H), 7.07 (td, J = 8.4 Hz, 2.8 Hz, 1H), 6.49 (s, 1H), 5.39-5.46 (m, 1H), 4.99 (q, J = 8.8 Hz, 2H), 4.05-4.20 (m, 2H), 3.92 (s, 3H), 3.82 (d, J = 15.6 Hz, 1H), 2.93 (d, J = 15.6 Hz, 1H), 1.81 (d, J = 6.4 Hz, 3H), 1.33 (t, J = 7.2 Hz, 3H).

### Example 209: (19R)-3-ethyl-16-fluoro-10,19-dimethyl-5-((3-(oxetan-3-yl)imidazol-5-yl) ethynyl)-20-oxa-3,4,10,11,23-pentaazapentacyclo[19.3. 1.0^{1,12}.0^{13,18}.0^{2,6}]pentacosa-1(25),2(6),4, 8(9),11,13(18),14,16,21(22),23-decaen-22-amine

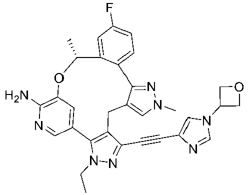

The synthetic method of Example 209 is the same as that of Example 47. LC-MS (ESI) [M+H]⁺: 591.4. ¹H NMR (400 MHz, CDCl₃), 7.88 (s, 1H), 7.53 (d, J = 1.6 Hz, 1H), 7.45 (s, 1H), 7.42 (s, 1H), 7.29 (dd, J = 9.6 Hz, 2.8 Hz, 1H), 7.13 (dd, J = 8.4 Hz, 5.6 Hz, 1H), 7.00 (td, J = 8.4 Hz, 2.8 Hz, 1H), 6.51 (s, 1H), 5.56-5.63 (m, 1H), 5.38-5.45 (m, 1H), 5.04-5.16 (m, 4H), 4.84 (s, 2H), 4.16 (q, J = 7.2 Hz, 2H), 3.91 (s, 3H), 3.79 (d, J = 15.6 Hz, 1H), 2.97 (d, J = 15.6 Hz, 1H), 1.79 (d, J = 6.4 Hz, 3H), 1.43 (t, J = 7.2 Hz, 3H).

### Example 210: 4-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)nitrilo)-4λ⁶-1,4-oxathiancyclohexan-4-one

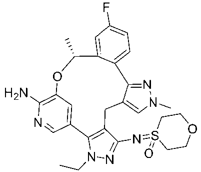

The synthetic method of Example 210 is the same as that of Example 169. LC-MS (ESI) [M+H]⁺: 552.3. ¹H NMR (400 MHz, CDCl₃), 7.59 (s, 1H), 7.49 (d, J = 1.6 Hz, 1H), 7.28 (dd, J = 10.0 Hz, 2.8 Hz, 1H), 7.11 (dd, J = 8.4 Hz, 6.0 Hz, 1H), 6.97 (td, J = 8.4 Hz, 2.8 Hz, 1H), 6.54 (d, J = 1.6 Hz, 1H), 5.36-5.43 (m, 1H), 4.80 (s, 2H), 4.18-4.31 (m, 3H), 3.95-4.12 (m, 4H), 3.89 (s, 3H), 3.68 (d, J = 15.6 Hz, 1H), 3.44-3.57 (m, 2H), 3.26-3.33 (m, 1H), 2.74 (d, J = 15.6 Hz, 1H), 1.78 (d, J = 6.4 Hz, 3H), 1.31 (t, J = 7.2 Hz, 3H).

### Example 211: 1-(((19R)-22-amino-3-ethyl-16-fluoro-10,19-dimethyl-20-oxa-3,4,10,11,23 -pentaazapentacyclo[19.3.1.0^{8,12}.0^{13,18}.0^{2,6}]pentacosa-1(25),2(6),4,8(9),11,13(18),14,16,21(22), 23-decaen-5-yl)nitrilo)tetrahydro-1λ⁶-thiophen-1-one

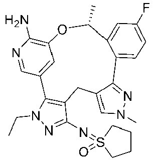

The synthetic method of Example 211 is the same as that of Example 169. LC-MS (ESI) [M+H]⁺: 536.4. ¹H NMR (400 MHz, CDCl₃), 7.57 (s, 1H), 7.49 (s, 1H), 7.27 (dd, J = 9.6 Hz, 2.4 Hz, 1H), 7.10 (dd, J = 8.4 Hz, 5.6 Hz, 1H), 6.96 (td, J = 8.4 Hz, 2.8 Hz, 1H), 6.55 (d, J = 1.2 Hz, 1H), 5.37-5.42 (m, 1H), 4.79 (s, 2H), 3.99 (q, J = 7.2 Hz, 2H), 3.89 (s, 3H), 3.79-3.86 (m, 1H), 3.59-3.68 (m, 2H), 3.16-3.30 (m, 2H), 2.72 (d, J = 15.2 Hz, 1H), 2.22-2.42 (m, 4H), 1.77 (d, J = 6.4 Hz, 3H), 1.33 (t, J = 7.2 Hz, 3H).

### Example 212: (19R)-3-ethyl-16-fluoro-5-((4-fluoro-3,4,5,6-tetrahydro-2H-pyran-4-yl)eth ynyl)-10,19-dimethyl-20-oxa-3,4,10,11,23-pentaazapentacyclo[19.3.1.0^{8,12}.0^{13,18}.0^{2,6}]pentacosa-1(24),2(6),4,8(9),11,13(14),15,17,21(25),22-decaen-22-amine

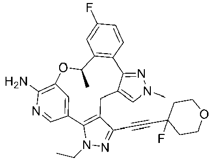

The synthetic method of Example 212 is the same as that of Example 47. LC-MS (ESI) [M+H]⁺: 545.4. ¹H NMR (400 MHz, CDCl₃), 7.51 (s, 1H), 7.38 (s, 1H), 7.29 (dd, J = 10.0 Hz, 2.8 Hz, 1H), 7.15 (dd, J = 8.4 Hz, 5.6 Hz, 1H), 7.01 (td, J = 8.4 Hz, 2.8 Hz, 1H), 6.51 (s, 1H), 5.37-5.45 (m, 1H), 4.80-4.98 (brs, 2H), 4.10-4.16 (m, 2H), 3.81-4.01 (m, 7H), 3.71 (d, J = 15.2 Hz, 1H), 2.95 (d, J = 15.2 Hz, 1H), 2.13-2.33 (m, 4H), 1.80 (d, J = 6.4 Hz, 3H), 1.40 (t, J = 7.2 Hz, 3H).

### Example 213: 1-(4-(((19R)-22-amino-3-ethyl-16-fluoro-10,19-dimethyl-20-oxa-3,4,10,1 1 ,23-pentaazapentacyclo[ 19.3.1.0^{2,6}.0^{8,12}.0^{13,18}]pentacosa-1(24),2(6),4,8(9),11,13(18),14,16,21 (25),22-decaen-5-yl)ethynyl)pyrazol-1-yl)-2-methylprop-2-ol

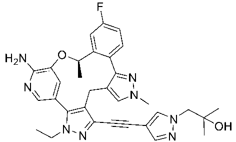

The synthetic method of Example 213 is the same as that of Example 47. LC-MS (ESI) [M+H]⁺: 581.4. ¹H NMR (400 MHz, CD₃OD), 7.96 (s, 1H), 7.72 (s, 1H), 7.58 (s, 1H), 7.54 (dd, J = 10.0 Hz, 2.8 Hz, 1H), 7.44 (d, J = 1.6 Hz, 1H), 7.21 (dd, J = 8.4 Hz, 6.0 Hz, 1H), 7.08 (td, J = 8.4 Hz, 2.8 Hz, 1H), 6.50 (d, J = 1.6 Hz, 1H), 5.39-5.45 (m, 1H), 4.05-4.20 (m, 4H), 3.92 (s, 3H), 3.81 (d, J = 16.0 Hz, 1H), 2.92 (d, J = 16.0 Hz, 1H), 1.81 (d, J = 6.4 Hz, 3H), 1.34 (t, J = 7.2 Hz, 3H), 1.18 (s, 6H).

### Example 214: 1-(4-(((19R)-22-amino-3-ethyl-16-fluoro-10,19-dimethyl-20-oxa-3,4,10,1 1 ,23-pentaazapentacyclo[ 19.3.1.0^{2,6}.0^{8,12}.0^{13,18}]pentacosa-1(24),2(6),4,8(9),11,13(18),14,16,21 (25),22-decaen-5-yl)ethynyl)imidazol-1-yl)-2-methylprop-2-ol

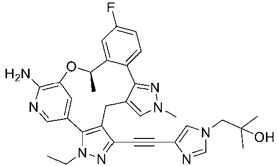

The synthetic method of Example 214 is the same as that of Example 47. LC-MS (ESI) [M+H]⁺: 581.4. ¹H NMR (400 MHz, CD₃OD), 7.69 (d, J = 1.2 Hz, 1H), 7.63 (s, 1H), 7.54 (dd, J = 10.0 Hz, 2.8 Hz, 1H), 7.51 (d, J = 1.2 Hz, 1H), 7.45 (d, J = 1.6 Hz, 1H), 7.20 (dd, J = 8.4 Hz, 6.0 Hz, 1H), 7.08 (td, J = 8.4 Hz, 2.8 Hz, 1H), 6.51 (d, J = 1.6 Hz, 1H), 5.40-5.46 (m, 1H), 4.05-4.20 (m, 2H), 3.99 (s, 2H), 3.92 (s, 3H), 3.85 (d, J = 15.6 Hz, 1H), 2.92 (d, J = 15.6 Hz, 1H), 1.81 (d, J = 6.4 Hz, 3H), 1.33 (t, J = 7.2 Hz, 3H), 1.19 (s, 6H).

### Example 215: (19R)-3-ethyl-16-fluoro-10,19-dimethyl-5-((1-(2,2,2-trifluoroethyl)imidaz ol-5-yl)ethynyl)-20-oxa-3,4,10,11,23-pentaazapentacyclo[19.3.1 .0^{2,6}.0^{8,12}.0^{13,18}]pentacosa-1(2 4),2(6),4,8(9),11,13(18),14, 16,21(25),22-decaen-22-amine

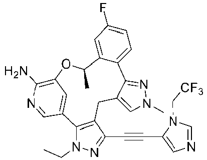

The synthetic method of Example 215 is the same as that of Example 47. LC-MS (ESI) [M+H]⁺: 591.4. ¹H NMR (400 MHz, CD₃OD), 7.96 (s, 1H), 7.57 (s, 1H), 7.54 (dd, J = 10.0 Hz, 2.8 Hz, 1H), 7.46 (s, 2H), 7.19 (dd, J = 8.4 Hz, 6.0 Hz, 1H), 7.09 (td, J = 8.4 Hz, 2.8 Hz, 1H), 6.49 (d, J = 1.2 Hz, 1H), 5.39-5.45 (m, 1H), 5.01-5.09 (m, 2H), 4.09-4.24 (m, 2H), 3.91 (s, 3H), 3.81 (d, J = 16.0 Hz, 1H), 2.96 (d, J = 16.0 Hz, 1H), 1.81 (d, J = 6.4 Hz, 3H), 1.36 (t, J = 7.2 Hz, 3H).

### Example 216: 1-((((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)nitrilo)-1λ⁶-thiacyclohexan-1-one

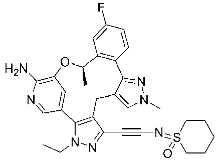

The synthetic method of Example 216 is the same as that of Example 169. LC-MS (ESI) [M+H]⁺: 574.4. ¹H NMR (400 MHz, CDCl₃), 7.51 (s, 1H), 7.38 (s, 1H), 7.29 (dd, J = 10.0 Hz, 2.8 Hz, 1H), 7.15 (dd, J = 8.4 Hz, 5.6 Hz, 1H), 7.01 (td, J = 8.4 Hz, 2.8 Hz, 1H), 6.51 (s, 1H), 5.37-5.44 (m, 1H), 4.78-5.01 (brs, 2H), 4.10-4.17 (m, 2H), 3.81-4.01 (m, 7H), 3.71 (d, J = 15.6 Hz, 1H), 2.95 (d, J = 15.6 Hz, 1H), 2.14-2.32 (m, 4H), 1.80 (d, J = 6.0 Hz, 3H), 1.50-1.67 (m, 2H), 1.40 (t, J = 7.2 Hz, 3H).

### Example 217: (19R)-5-((6-aminopyridin-3-yl)ethynyl)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(24),2(6),4,8(9),11,1 \3(14),15,17,21(25),22-decaen-22-amine

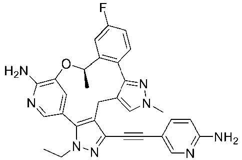

The synthetic method of Example 217 is the same as that of Example 47. LC-MS (ESI) [M+H]⁺: 534.4. ¹H NMR (400 MHz, CDCl₃), 8.33 (d, J = 1.6 Hz, 1H), 7.62 (dd, J = 8.4 Hz, 2.0 Hz, 1H), 7.52 (s, 1H), 7.46 (s, 1H), 7.29 (dd, J = 10.0 Hz, 2.4 Hz, 1H), 7.16 (dd, J = 8.4 Hz, 6.0 Hz, 1H), 7.00 (td, J = 8.4 Hz, 2.4 Hz, 1H), 6.55 (s, 1H), 6.50 (d, J = 8.4 Hz, 1H), 5.39-5.46 (m, 1H), 4.96 (s, 2H), 4.56-4.84 (brs, 2H), 4.10-4.17 (m, 2H), 3.90 (s, 3H), 3.84 (d, J = 15.6 Hz, 1H), 2.95 (d, J = 15.6 Hz, 1H), 1.80 (d, J = 6.4 Hz, 3H), 1.42 (t, J = 7.2 Hz, 3H).

### Example 218: (19R)-3-ethyl-16-fluoro-10,19-dimethyl-5-((5-methyl-1,3,4-oxadiazol-2-y 1)ethynyl)-20-oxa-3,4,10,11,23-pentaazapentacyclo[19.3.1.0^{8,12}.0¹³¹⁸.0^{2,6}]pentacosa-1(25),2(6), 4,8(9),11,13(18),14,16,21(22),23-decaen-22-amine

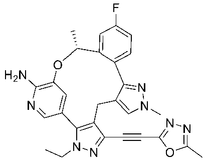

The synthetic method of Example 218 is the same as that of Example 47. LC-MS (ESI) [M+H]⁺: 525.4. ¹H NMR (400 MHz, CDCl₃), 7.51 (s, 1H), 7.40 (s, 1H), 7.29 (dd, J = 10.0 Hz, 2.8 Hz, 1H), 7.14 (dd, J = 8.4 Hz, 5.6 Hz, 1H), 7.02 (td, J = 8.4 Hz, 2.8 Hz, 1H), 6.56 (d, J = 1.2 Hz, 1H), 5.40-5.46 (m, 1H), 5.09-5.27 (brs, 2H), 4.17 (q, J = 6.8 Hz, 2H), 3.91 (s, 3H), 3.85 (d, J = 15.6 Hz, 1H), 2.99 (d, J = 15.6 Hz, 1H), 2.61 (s, 3H), 1.80 (d, J = 6.4 Hz, 3H), 1.44 (t, J = 7.2 Hz, 3H).

### Example 219: (19R)-3-ethyl-16-fluoro-10,19-dimethyl-5-(3-methylbut-1-yn-1-yl)-20-oxa -3,4,10,11,23-pentaazapentacyclo[19.3.1.0^{2,6}.0^{8,12}.0^{13,18}]pentacosa-1(24),2(6),4,8(9),11,13(18),1 4,16,21(25),22-decaen-22-amine

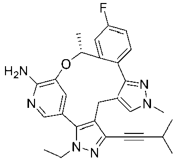

The synthetic method of Example 219 is the same as that of Example 47. LC-MS (ESI) [M+H]⁺: 485.4. ¹H NMR (400 MHz, CD₃OD), 7.51-7.54 (m, 2H), 7.43 (s, 1H), 7.20 (dd, J = 8.4 Hz, 5.6 Hz, 1H), 7.08 (td, J = 8.4 Hz, 2.8 Hz, 1H), 6.48 (d, J = 1.6 Hz, 1H), 5.38-5.45 (m, 1H), 4.01-4.13 (m, 2H), 3.92 (s, 3H), 3.74 (d, J = 15.6 Hz, 1H), 2.85-2.93 (m, 2H), 1.81 (d, J = 6.4 Hz, 3H), 1.34 (s, 3H), 1.33 (s, 3H), 1.30 (t, J = 7.2 Hz, 3H).

### Example 220: (19R)-5-((2-aminopyridin-4-yl)ethynyl)-3-ethyl-16-fluoro-10,19-dimethyl-20-oxa-3,4,10,11,23-pentaazapentacyclo[19.3.1.0^{8,12}.0^{13,18}.0^{2,6}]pentacosa-1(24),2(6),4,8(9),11,1 3(14),15,17,21(25),22-decaen-22-amine

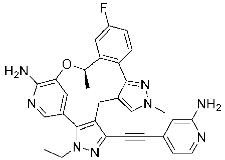

The synthetic method of Example 220 is the same as that of Example 47. LC-MS (ESI) [M+H]⁺: 535.4. ¹H NMR (400 MHz, CDCl₃), 7.93 (d, J = 5.6 Hz, 1H), 7.52 (d, J = 1.6 Hz, 1H), 7.38 (s, 1H), 7.30 (dd, J = 10.0 Hz, 2.4 Hz, 1H), 7.16 (dd, J = 8.4 Hz, 5.6 Hz, 1H), 7.02 (td, J = 8.4 Hz, 2.8 Hz, 1H), 6.82-6.84 (m, 2H), 6.53 (d, J = 1.6 Hz, 1H), 5.51-5.96 (brs, 2H), 5.39-5.45 (m, 1H), 5.05 (s, 2H), 4.12-4.20 (m, 2H), 3.92 (s, 3H), 3.82 (d, J = ¹H NMR (400 MHz, CD₃OD),15.9 Hz, 1H), 2.99 (d, J = 15.6 Hz, 1H), 1.80 (d, J = 6.4 Hz, 3H), 1.43 (t, J = 7.2 Hz, 3H).

### Example 221: (19R)-5-((2-amino-1,3-thiazol-5-yl)ethynyl)-3-ethyl-16-fluoro-10,19-dime thyl-20-oxa-3,4,10,11,23-pentaazapentacyclo[19.3.1.0^{8,12}.0^{13,18}.0^{2,6}]pentacosa-1(24),2(6),4,8(9), 11,13(14),15,17,21(25),22-decaen-22-amine

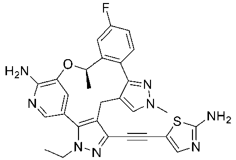

The synthetic method of Example 221 is the same as that of Example 47. LC-MS (ESI) [M+H]⁺: 541.4. ¹H NMR (400 MHz, CDCl₃), 7.50 (d, J = 1.6 Hz, 1H), 7.42 (s, 1H), 7.35 (s, 1H), 7.29 (dd, J = 10.0 Hz, 2.8 Hz, 1H), 7.15 (dd, J = 8.4 Hz, 6.0 Hz, 1H), 7.00 (td, J = 8.4 Hz, 2.8 Hz, 1H), 6.54 (d, J = 1.6 Hz, 1H), 5.39-5.46 (m, 1H), 5.15- (s, 2H), 5.04 (s, 2H), 4.10-4.16 (m, 2H), 3.92 (s, 3H), 3.80 (d, J = 15.6 Hz, 1H), 2.94 (d, J = 15.6 Hz, 1H), 1.80 (d, J = 6.4 Hz, 3H), 1.41 (t, J = 7.2 Hz, 3H).

### Example 222: (19R)-5-((1E)-3-ethoxyprop-1-en-1-yl)-3-ethyl-16-fluoro-10,19-dimethyl-20-oxa-3,4,10,11,23-pentaazapentacyclo[19.3.1.0^{8,12}.0^{13,182,6}]pentacosa-1(24),2(6),4,8(9),11,1 3(14),15,17,21(25),22-decaen-22-amine

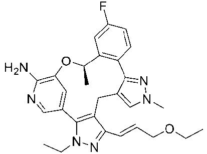

The synthetic method of Example 222 is the same as that of Example 47. LC-MS (ESI) [M+H]⁺: 503.4. ¹H NMR (400 MHz, CDCl₃), 7.47 (s, 1H), 7.27-7.30 (m, 2H), 7.14 (dd, J = 8.4 Hz, 6.0 Hz, 1H), 7.01 (td, J = 8.4 Hz, 2.8 Hz, 1H), 6.76 (d, J = 16.4 Hz, 1H), 6.60 (dt, J = 16.4 Hz, 6.0 Hz, 1H), 6.54 (s, 1H), 5.37-5.42 (m, 1H), 5.15 (s, 2H), 4.21 (dd, J = 6.0 Hz, 1.2 Hz, 2H), 4.05-4.15 (m, 2H), 3.90 (s, 3H), 3.69 (d, J = 16.0 Hz, 1H), 3.60 (q, J = 7.2 Hz, 2H), 2.93 (d, J = 16.0 Hz, 1H), 1.79 (d, J = 6.4 Hz, 3H), 1.38 (t, J = 7.2 Hz, 3H), 1.27 (t, J = 7.2 Hz, 3H).

### Example 223: (19R)-5-((6-aminopyridin-2-yl)ethynyl)-3-ethyl-16-fluoro-10,19-dimethyl-20-oxa-3,4,10,11,23-pentaazapentacyclo[19.3.1.0^{2,6}.0^{8,12}.0^{13,18}]pentacosa-1(24),2(6),4,8(9),11,1 3(18),14,16,21(25),22-decaen-22-amine

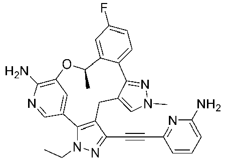

The synthetic method of Example 223 is the same as that of Example 47. LC-MS (ESI) [M+H]⁺: 535.4. ¹H NMR (400 MHz, CD₃OD), 7.64 (s, 1H), 7.54 (dd, J = 10.0 Hz, 2.8 Hz, 1H), 7.48 (dd, J = 8.8 Hz, 6.8 Hz, 1H), 7.45 (d, J = 1.6 Hz, 1H), 7.22 (dd, J = 8.4 Hz, 6.0 Hz, 1H), 7.08 (td, J = 8.4 Hz, 2.8 Hz, 1H), 6.91 (d, J = 7.2 Hz, 1H), 6.60 (d, J = 8.4 Hz, 1H), 6.51 (d, J = 1.6 Hz, 1H), 5.40-5.46 (m, 1H), 4.06-4.22 (m, 2H), 3.92 (s, 3H), 3.91 (d, J = 15.6 Hz, 1H), 2.94 (d, J = 15.6 Hz, 1H), 1.82 (d, J = 6.4 Hz, 3H), 1.34 (t, J = 7.2 Hz, 3H).

### Example 224: (19R)-5-((2-aminopyridin-3-yl)ethynyl)-3-ethyl-16-fluoro-10,19-dimethyl-20-oxa-3,4,10,11,23-pentaazapentacyclo[19.3. 1.0^{2,6}.0^{8,12}.0^{13,18}]pentacosa-1(24),2(6),4,8(9),11,1 3(18),14,16,21(25),22-decaen-22-amine

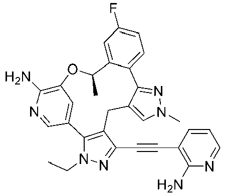

The synthetic method of Example 224 is the same as that of Example 47. LC-MS (ESI) [M+H]⁺: 535.4. ¹H NMR (400 MHz, CD₃OD), 7.97 (dd, J = 5.2 Hz, 2.0 Hz, 1H), 7.72 (dd, J = 7.6 Hz, 2.0 Hz, 1H), 7.63 (s, 1H), 7.54 (dd, J = 10.0 Hz, 2.8 Hz, 1H), 7.46 (d, J = 2.0 Hz, 1H), 7.23 (dd, = 8.4 Hz, 6.0 Hz, 1H), 7.08 (td, J = 8.4 Hz, 2.8 Hz, 1H), 6.68 (dd, J = 7.6 Hz, 5.2 Hz, 1H), 6.50 (d, J = 2.0 Hz, 1H), 5.39-5.46 (m, 1H), 4.10-4.23 (m, 2H), 3.91 (s, 3H), 3.87 (d, J = 15.6 Hz, 1H), 2.96 (d, J = 15.6 Hz, 1H), 1.82 (d, J = 6.4 Hz, 3H), 1.37 (t, J = 7.2 Hz, 3H).

### Example 225: (19R)-5-((6-amino-1,2-pyridazin-3-yl)ethynyl)-3-ethyl-16-fluoro-10,19-di methyl-20-oxa-3,4,10,11,23-pentaazapentacyclo[19.3.1.0^{2,6}.0^{8,12}.0^{13,18}]pentacosa-1(25),2(6),4,8 (9),11,13(14),15,17,21(22),23-decaen-22-amine

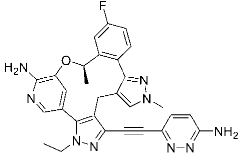

The synthetic method of Example 225 is the same as that of Example 47. LC-MS (ESI) [M+H]⁺: 536.4. ¹H NMR (400 MHz, CDCl₃), 7.54 (s, 1H), 7.51 (s, 1H), 7.46 (d, J = 8.8 Hz, 1H), 7.28 (dd, J = 10.4 Hz, 2.8 Hz, 1H), 7.14 (dd, J = 8.4 Hz, 6.0 Hz, 1H), 7.00 (td, J = 8.4 Hz, 2.8 Hz, 1H), 6.75 (d, J = 8.8 Hz, 1H), 6.57 (s, 1H), 5.40-5.46 (m, 1H), 4.96 (s, 2H), 4.91 (s, 2H), 4.13-4.19 (m, 2H), 3.93 (d, J = 15.6 Hz, 1H), 3.89 (s, 3H), 2.96 (d, J = 15.6 Hz, 1H), 1.79 (d, J = 6.4 Hz, 3H), 1.43 (t, J = 7.2 Hz, 3H).

### Example 226: (19R)-5-((5-aminopyrazin-2-yl)ethynyl)-3-ethyl-16-fluoro-10,19-dimethyl -20-oxa-3,4,10,11,23-pentaazapentacyclo[19.3.1.0^{2,6}.0^{8,12}.0^{13,18}]pentacosa-1(25),2(6),4,8(9),11, 13(14),15,17,21(22),23-decaen-22-amine

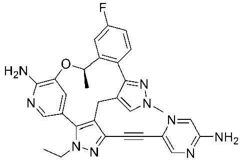

The synthetic method of Example 226 is the same as that of Example 47. LC-MS (ESI) [M+H]⁺: 536.4. ¹H NMR (400 MHz, CDCl₃), 8.31 (s, 1H), 8.01 (s, 1H), 7.54 (s, 1H), 7.48 (s, 1H), 7.29 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.56 (s, 1H), 5.39-5.46 (m, 1H), 4.87 (s, 2H), 4.77 (s, 2H), 4.16 (q, J = 7.2 Hz, 2H), 3.90 (d, J = 15.6 Hz, 1H), 3.89 (s, 3H), 2.96 (d, J = 15.6 Hz, 1H), 1.79 (d, J = 6.4 Hz, 3H), 1.43 (t, J = 7.2 Hz, 3H).

### Example 227: (19R)-5-((6-aminopyrazin-2-yl)ethynyl)-3-ethyl-16-fluoro-10,19-dimethyl -20-oxa-3,4,10,11,23-pentaazapentacyclo[19.3.1.0^{2,6}.0^{8,12}.0^{13,18}]pentacosa-1(25),2(6),4,8(9),11, 13(14),15,17,21(22),23-decaen-22-amine

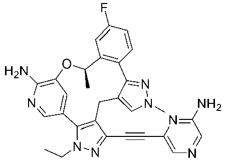

The synthetic method of Example 227 is the same as that of Example 47. LC-MS (ESI) [M+H]⁺: 536.4. ¹H NMR (400 MHz, CDCl₃), 8.20 (s, 1H), 7.93 (s, 1H), 7.54 (d, J = 1.6 Hz, 1H), 7.45 (s, 1H), 7.29 (dd, J = 10.0 Hz, 2.8 Hz, 1H), 7.14 (dd, J = 8.4 Hz, 6.0 Hz, 1H), 7.00 (td, J = 8.4 Hz, 2.8 Hz, 1H), 6.54 (d, J = 1.6 Hz, 1H), 5.39-5.45 (m, 1H), 4.84 (s, 2H), 4.71 (s, 2H), 4.13-4.20 (m, 2H), 3.90 (s, 3H), 3.89 (d, J = 15.6 Hz, 1H), 2.98 (d, J = 15.6 Hz, 1H), 1.79 (d, J = 6.4 Hz, 3H), 1.43 (t, J = 7.2 Hz, 3H).

### Example 228: (19R)-5-((diethyl(oxo)-λ⁶-sulfanylidene)amino)-3-ethyl-16-fluoro-10,19-di methyl-20-oxa-3,4,10,11,23-pentaazapentacyclo[19.3.1.0^{8,12}.0^{13,18}.0^{2,6}]pentacosa-1(25),2(6),4,8 (9),11,13(18),14,16,21(22),23-decaen-22-amine

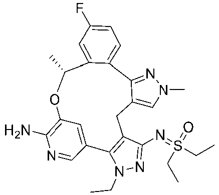

The synthetic method of Example 228 is the same as that of Example 169. LC-MS (ESI) [M+H]⁺: 538.4. ¹H NMR (400 MHz, CDCl₃), 7.59 (s, 1H), 7.38-7.58 (brs, 1H), 7.26 (dd, J = 10.0 Hz, 2.8 Hz, 1H), 7.12 (dd, J = 8.4 Hz, 6.0 Hz, 1H), 6.97 (td, J = 8.4 Hz, 2.8 Hz, 1H), 6.56 (s, 1H), 5.37-5.42 (m, 1H), 4.74-5.26 (brs, 2H), 3.96 (q, J = 7.2 Hz, 2H), 3.89 (s, 3H), 3.69 (d, J = 15.6 Hz, 1H), 3.48-3.63 (m, 2H), 3.21-3.41 (m, 2H), 2.71 (d, J = 15.6 Hz, 1H), 1.78 (d, J = 6.0 Hz, 3H), 1.51 (t, J = 7.2 Hz, 3H), 1.46 (t, J = 7.2 Hz, 3H), 1.30 (t, J = 7.2 Hz, 3H).

### Example 229: 1-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(25),2(6),4,8(9),11,13(18),14,16,21(22), 23-decaen-5-yl)nitrilo)-1λ⁶-thiacyclobut-1-one

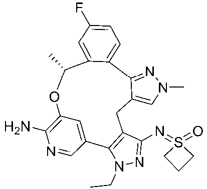

The synthetic method of Example 229 is the same as that of Example 169. LC-MS (ESI) [M+H]⁺: 522.4. ¹H NMR (400 MHz, CDCl₃), 7.53 (s, 1H), 7.50 (d, J = 1.2 Hz, 1H), 7.27 (dd, J = 10.0 Hz, 2.8 Hz, 1H), 7.09 (dd, J = 8.4 Hz, 6.0 Hz, 1H), 6.97 (td, J = 8.4 Hz, 2.8 Hz, 1H), 6.56 (d, J = 1.2 Hz, 1H), 5.36-5.43 (m, 1H), 4.68-4.82 (m, 3H), 4.53-4.62 (m, 1H), 4.19-4.27 (m, 1H), 4.08-4.15 (m, 1H), 4.00 (q, J = 7.2 Hz, 2H), 3.88 (s, 3H), 3.66 (d, J = 15.6 Hz, 1H), 2.74 (d, J = 15.6 Hz, 1H), 2.28-2.46 (m, 2H), 1.78 (d, J = 6.8 Hz, 3H), 1.34 (t, J = 7.2 Hz, 3H).

### Example 230: 2-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6} ]pentacosa-1(25),2(6),4,8(9),11,13(18),14,16,21(22), 23-decaen-5-yl)nitrilo)-2λ⁶-2-thiaspiro[3.3]heptan-2-one

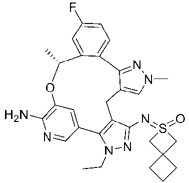

The synthetic method of Example 230 is the same as that of Example 169. LC-MS (ESI) [M+H]⁺: 562.4. ¹H NMR (400 MHz, CDCl₃), 7.48 (d, *J* = 7.1 Hz, 2H), 7.28 (d, *J =* 2.3 Hz, 1H), 7.09 (m, 1H), 7.02 - 6.84 (m, 1H), 6.56 (s, 1H), 5.39 (d, *J =* 6.1 Hz, 1H), 5.05 (d, *J =* 12.9 Hz, 1H), 4.99 - 4.85 (m, 6H), 4.44 (d, *J =* 13.3 Hz, 1H), 4.32 (d, *J* = 13.3 Hz, 1H), 3.96 (m, 2H), 3.88 (s, 3H), 3.63 (d, *J =* 15.5 Hz, 1H), 2.72 (d, *J =* 15.4 Hz, 1H), 1.78 (d, *J =* 6.2 Hz, 3H), 1.30 (m, 4H).

### Example 231: (19R)-3-ethyl-16-fluoro-l 0, 19-dimethyl-5-((1-(trifluoromethyl)pyrazol-4-yl)ethynyl)-20-oxa-3,4,10,11,23-pentaazapentacyclo[ 19.3.1.0^{1,12}.0^{13,18}.0^{2,6}]pentacosa-1(25),2(6), 4,8(9),11,13(18),14,16,21(22),23-decaen-22-amine

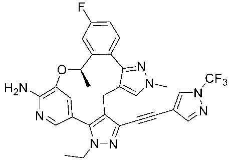

The synthetic method of Example 231 is the same as that of Example 47. LC-MS (ESI) [M+H]⁺: 577.4. ¹H NMR (400 MHz, CD₃OD), 8.61 (s, 1H), 8.06 (s, 1H), 7.58 (s, 1H), 7.54 (dd, J = 10.0 Hz, 2.8 Hz, 1H), 7.45 (d, J = 1.6 Hz, 1H), 7.20 (dd, J = 8.4 Hz, 5.6 Hz, 1H), 7.08 (td, J = 8.4 Hz, 2.8 Hz, 1H), 6.50 (d, J = 1.6 Hz, 1H), 5.40-5.46 (m, 1H), 4.08-4.20 (m, 2H), 3.92 (s, 3H), 3.83 (d, J = 16.0 Hz, 1H), 2.94 (d, J = 16.0 Hz, 1H), 1.82 (d, J = 6.4 Hz, 3H), 1.34 (t, J = 7.2 Hz, 3H).

### Example 232: (19R)-10-(difluoromethyl)-3-ethyl-16-fluoro-5-((1E)-3-methoxyprop-1-en -1-yl)-19-methyl-20-oxa-3,4,10,11,23-pentaazapentacyclo[19.3.1.0^{8,12}.0^{13,18}.0^{2,6}]pentacosa-1(2 5),2(6),4,8(9),11,13(18),14,16,21(22),23-decaen-22-amine

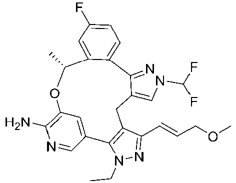

The synthetic method of Example 232 is the same as that of Example 45. LC-MS (ESI) [M+H]⁺: 525.4. ¹H NMR (400 MHz, CD₃OD), 7.81 (s, 1H), 7.58 (dd, J = 10.0 Hz, 2.8 Hz, 1H), 7.483 (t, J = 59.6 Hz, 1H), 7.43 (s, 1H), 7.27 (dd, J = 8.4 Hz, 6.0 Hz, 1H), 7.12 (td, J = 8.4 Hz, 2.8 Hz, 1H), 6.82 (d, J = 16.4 Hz, 1H), 6.57 (dt, J = 16.4 Hz, 6.0 Hz, 1H), 6.34 (d, J = 1.6 Hz, 1H), 5.34-5.40 (m, 1H), 4.02-4.19 (m, 4H), 3.80 (d, J = 16.0 Hz, 1H), 3.44 (s, 3H), 2.93 (d, J = 16.0 Hz, 1H), 1.80 (d, J = 6.4 Hz, 3H), 1.33 (t, J = 7.2 Hz, 3H).

### Example 233: (19R)-10-(difluoromethyl)-3-ethyl-5-ethynyl-16-fluoro-19-methyl-20-oxa-3,4,10,11,23-pentaazapentacyclo[19.3.1.0^{8,12}.0^{13,18}.0^{2,6}]pentacosa-1(25),2(6),4,8(9),11,13(18),1 4,16,21(22),23-decaen-22-amine

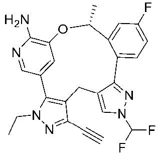

The synthetic method of Example 233 is the same as that of Example 47. LC-MS (ESI) [M+H]⁺: 479.2. ¹H NMR (400 MHz, CD₃OD), 7.93 (s, 1H), 7.57 (dd, J = 10.0 Hz, 2.8 Hz, 1H), 7.50 (t, J = 60.0 Hz, 1H), 7.45 (s, 1H), 7.24 (dd, J = 8.4 Hz, 5.6 Hz, 1H), 7.11 (td, J = 8.4 Hz, 2.4 Hz, 1H), 6.32 (s, 1H), 5.35-5.41 (m, 1H), 4.04-4.19 (m, 2H), 3.89 (s, 1H), 3.83 (d, J = 15.6 Hz, 1H), 2.94 (d, J = 15.6 Hz, 1H), 1.80 (d, J = 6.4 Hz, 3H), 1.33 (t, J = 7.2 Hz, 3H).

### Example 234: (19R)-5-((2-amino-1,3-thiazol-4-yl)ethynyl)-3-ethyl-16-fluoro-10,19-dime thyl-20-oxa-3,4,10,11,23-pentaazapentacyclo[19.3.1.0^{8,12}.0^{13,18}.0^{2,6}]pentacosa-1(24),2(6),4,8(9), 11,13(14),15,17,21(25),22-decaen-22-amine

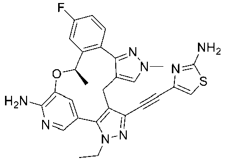

The synthetic method of Example 234 is the same as that of Example 47. LC-MS (ESI) [M+H]⁺: 541.4. ¹H NMR (400 MHz, CDCl₃), 7.49 - 7.43 (m, 2H), 7.28 (dd, *J* = 9.9, 2.7 Hz, 1H), 7.12 (dd, *J* = 8.5, 5.8 Hz, 1H), 7.01 (td, *J* = 8.2, 2.5 Hz, 1H), 6.85 (s, 1H), 6.57 (d, *J* = 1.4 Hz, 1H), 5.48 - 5.26 (m, 5H), 4.20 - 4.06 (m, 2H), 3.89 (s, 4H), 2.92 (d, *J* = 15.5 Hz, 1H), 1.79 (d, *J* = 6.3 Hz, 3H), 1.41 (t, *J =* 7.2 Hz, 3H).

### Example 235: (19R)-3-ethyl-16-fluoro-10,19-dimethyl-5-(pyrazolo[1,5-a]pyridin-5-yleth ynyl)-20-oxa-3,4,10,11,23-pentaazapentacyclo[19.3.1.0^{2,6}.0^{1,12}.0^{13,18}]pentacosa-1(24),2(6),4,8 (9),11,13(18),14,16,21(25),22-decaen-22-amine

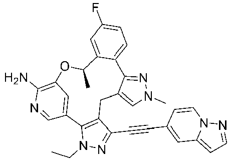

The synthetic method of Example 235 is the same as that of Example 47. LC-MS (ESI) [M+H]⁺: 559.4. ¹H NMR (400 MHz, CD₃OD), 8.55 (d, J = 7.2 Hz, 1H), 8.00 (d, J = 2.4 Hz, 1H), 7.94 (s, 1H), 7.62 (s, 1H), 7.54 (dd, J = 10.0 Hz, 2.8 Hz, 1H), 7.46 (d, J = 2.4 Hz, 1H), 7.24 (dd, J = 8.4 Hz, 6.0 Hz, 1H), 7.09 (td, J = 8.4 Hz, 2.8 Hz, 1H), 7.00 (dd, J = 7.2 Hz, 2.0 Hz, 1H), 6.70 (d, J = 2.4 Hz, 1H), 6.51 (d, J = 2.0 Hz, 1H), 5.41-5.47 (m, 1H), 4.08-4.24 (m, 2H), 3.92 (s, 3H), 3.89 (d, J = 15.6 Hz, 1H), 2.98 (d, J = 15.6 Hz, 1H), 1.82 (d, J = 6.4 Hz, 3H), 1.36 (t, J = 7.2 Hz, 3H).

### Example 236: (19R)-3-ethyl-16-fluoro-5-(imidazo[1,2-a]pyridin-6-ylethynyl)-10,19-dim ethyl-20-oxa-3,4,10,11,23-pentaazapentacyclo[19.3.1.0^{2,6}.0^{8,12}.0^{13,18}]pentacosa-1(24),2(6),4,8 (9),11,13(18),14,16,21(25),22-decaen-22-amine

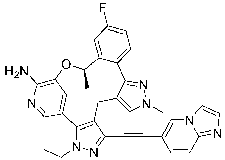

The synthetic method of Example 236 is the same as that of Example 47. LC-MS (ESI) [M+H]⁺: 559.4. ¹H NMR (400 MHz, CD₃OD), 8.83 (s, 1H), 7.90 (s, 1H), 7.59-7.64 (m, 3H), 7.54 (dd, J = 10.0 Hz, 2.8 Hz, 1H), 7.44-7.47 (m, 2H), 7.22 (dd, J = 8.4 Hz, 5.6 Hz, 1H), 7.08 (td, J = 8.4 Hz, 2.8 Hz, 1H), 6.51 (d, J = 2.0 Hz, 1H), 5.40-5.48 (m, 1H), 4.08-4.24 (m, 2H), 3.92 (s, 3H), 3.88 (d, J = 15.6 Hz, 1H), 2.97 (d, J = 15.6 Hz, 1H), 1.82 (d, J = 6.4 Hz, 3H), 1.36 (t, J = 7.2 Hz, 3H).

### Example 237: (19R)-10-(difluoromethyl)-5-((dimethyl(oxo)-λ⁶-sulfanylidene)amino)-3-e thyl-16-fluoro-19-methyl-20-oxa-3,4,10,11,23-pentaazapentacyclo[19.3.1.0^{8,12}.0^{13,18}.0^{2,6}]pentac osa-1(25),2(6),4,8(9),11,13(18),14,16,21(22),23-decaen-22-amine

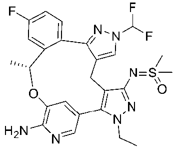

The synthetic method of Example 237 is the same as that of Example 169. LC-MS (ESI) [M+H]⁺: 546.4. ¹H NMR (400 MHz, CD₃OD), 8.12 (s, 1H), 7.55 (dd, J = 10.0 Hz, 2.8 Hz, 1H), 7.47 (t, J = 60.4 Hz, 1H), 7.40 (s, 1H), 7.20 (dd, J = 8.4 Hz, 5.6 Hz, 1H), 7.10 (td, J = 8.4 Hz, 2.8 Hz, 1H), 6.35 (d, J = 1.6 Hz, 1H), 5.34-5.40 (m, 1H), 3.91-4.06 (m, 2H), 3.72 (d, J = 15.6 Hz, 1H), 3.40 (s, 3H), 3.35 (s, 3H), 2.72 (d, J = 15.6 Hz, 1H), 1.80 (d, J = 6.4 Hz, 3H), 1.28 (t, J = 7.2 Hz, 3H).

### Example 238: ((((19R)-22-amino-3-ethyl-16-fluoro-10,19-dimethyl-20-oxa-3,4,10,11,23-pentaazapentacyclo[19.3. 1.0^{8,12}.0^{13,18}.0^{2,6}]pentacosa-1(25),2(6),4,8(9),11,13(18),14,16,21(22),2 3-decaen-5-yl)ethynyl)cyclopropyl)methanol

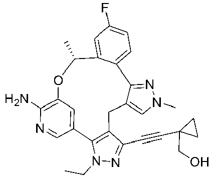

The synthetic method of Example 238 is the same as that of Example 47. LC-MS (ESI) [M+H]⁺: 513.4. ¹H NMR (400 MHz, CD₃OD), 7.63 (s, 1H), 7.52 (dd, J = 10.4 Hz, 2.8 Hz, 1H), 7.36-7.50 (brs, 1H), 7.19 (dd, J = 8.4 Hz, 6.0 Hz, 1H), 7.07 (td, J = 8.4 Hz, 2.8 Hz, 1H), 6.47 (s, 1H), 5.38-5.44 (m, 1H), 3.99-4.15 (m, 2H), 3.92 (s, 3H), 3.76 (d, J = 15.6 Hz, 1H), 3.57-3.63 (m, 2H), 2.86 (d, J = 15.6 Hz, 1H), 1.81 (d, J = 6.4 Hz, 3H), 1.29 (d, J = 7.2 Hz, 3H), 1.04-1.08 (m, 2H), 0.94-0.97 (m, 2H).

### Example 239: (19R)-3-ethyl-16-fluoro-10,19-dimethyl-5-((1E)-3-(oxetan-3-yloxy)prop-1 -en-1-yl)-20-oxa-3,4,10,11,23-pentaazapentacyclo[19.3.1.0^{8,12}.0^{13,18}.0².⁶]pentacosa-1(24),2(6),4, 8(9),11,13(14),15,17,21(25),22-decaen-22-amine

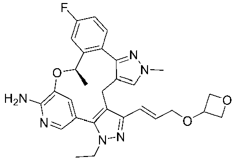

The synthetic method of Example 239 is the same as that of Example 45. LC-MS (ESI) [M+H]⁺: 531.4. ¹H NMR (400 MHz, CDCl₃), 7.47 (s, 1H), 7.33-7.43 (brs, 1H), 7.29 (dd, J = 10.0 Hz, 2.8 Hz, 1H), 7.14 (dd, J = 8.4 Hz, 6.0 Hz, 1H), 7.00 (td, J = 8.4 Hz, 2.8 Hz, 1H), 6.76 (d, J = 16.4 Hz, 1H), 6.55 (dt, J = 16.4 Hz, 6.0 Hz, 1H), 6.51 (s, 1H), 5.35-5.42 (m, 1H), 5.02 (s, 2H), 4.82-4.90 (m, 1H), 4.77-4.81 (m, 2H), 4.54-4.58 (m, 2H), 4.05-4.16 (m, 4H), 3.90 (s, 3H), 3.66 (d, J = 16.0 Hz, 1H), 2.93 (d, J = 16.0 Hz, 1H), 1.78 (d, J = 6.0 Hz, 3H), 1.38 (t, J = 7.2 Hz, 3H).

### Example 240: 6-(((19R)-22-amino-3-ethyl-16-fluoro-10,19-dimethyl-20-oxa-3,4,10,11,2 3-pentaazapentacyclo[19.3.1_{.}0^{8,12}.0^{13,18}.0^{2,6}]pentacosa-1(24),2(6),4,8(9),11,13(14),15,17,21(25), 22-decaen-5-yl)ethynyl)-1,2-dihydropyridin-2-one

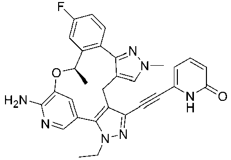

The synthetic method of Example 240 is the same as that of Example 47. LC-MS (ESI) [M+H]⁺: 536.4. ¹H NMR (400 MHz, CDCl₃), 7.36-7.51 (m, 3H), 7.29 (dd, J = 10.0 Hz, 2.8 Hz, 1H), 7.15 (dd, J = 8.4 Hz, 6.0 Hz, 1H), 7.05 (td, J = 8.4 Hz, 2.8 Hz, 1H), 6.64 (s, 1H), 6.55 (d, J = 7.2 Hz, 1H), 6.45 (d, J = 8.8 Hz, 1H), 5.44-5.51 (m, 1H), 4.02-4.20 (m, 2H), 3.85-3.90 (m, 4H), 2.86 (d, J = 15.6 Hz, 1H), 1.84 (d, J = 6.4 Hz, 3H), 1.40 (t, J = 7.2 Hz, 3H).

### Example 241: 4-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)-1,2-dihydropyridin-2-one

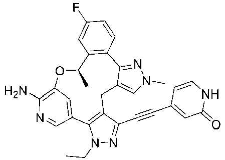

The synthetic method of Example 241 is the same as that of Example 47. LC-MS (ESI) [M+H]⁺: 536.4. ¹H NMR (400 MHz, CDCl₃), 12.05-12.88 (brs, 1H), 7.42-7.58 (brs, 1H), 7.40 (s, 1H), 7.35 (d, J = 6.8 Hz, 1H), 7.29 (dd, J = 10.0 Hz, 2.4 Hz, 1H), 7.19 (dd, J = 8.4 Hz, 6.0 Hz, 1H), 7.06 (td, J = 8.4 Hz, 2.4 Hz, 1H), 6.79 (s, 1H), 6.63 (s, 1H), 6.41 (d, J = 6.4 Hz, 1H), 5.87-6.29 (brs, 2H), 5.42-5.50 (m, 1H), 4.08-4.23 (m, 2H), 3.92 (s, 3H), 3.86 (d, J = 16.0 Hz, 1H), 2.98 (d, J = 16.0 Hz, 1H), 1.83 (d, J = 6.4 Hz, 3H), 1.44 (t, J = 7.2 Hz, 3H).

### Example 242: 6-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)-1-methyl-1,2-dihydropyridin-2-one

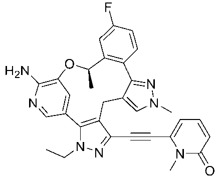

The synthetic method of Example 242 is the same as that of Example 47. LC-MS (ESI) [M+H]⁺: 550.4. ¹H NMR (400 MHz, CD₃OD), 7.61 (s, 1H), 7.54 (dd, J = 10.0 Hz, 2.8 Hz, 1H), 7.50 (dd, J = 9.2 Hz, 7.2 Hz, 1H), 7.46 (s, 1H), 7.22 (dd, J = 8.4 Hz, 6.0 Hz, 1H), 7.07 (td, J = 8.4 Hz, 2.8 Hz, 1H), 6.80 (dd, J = 6.8 Hz, 1.2 Hz, 1H), 6.62 (dd, J = 9.2 Hz, 1.2 Hz, 1H), 6.48 (d, J = 1.6 Hz, 1H), 5.39-5.46 (m, 1H), 4.09-4.24 (m, 2H), 3.91 (s, 3H), 3.82-3.85 (m, 4H), 2.99 (d, J = 15.6 Hz, 1H), 1.81 (d, J = 6.4 Hz, 3H), 1.35 (t, J = 7.2 Hz, 3H).

### Example 243: 4-(((19R)-22-amino-3-ethyl-16-fluoro-10,19-dimethyl-20-oxa-3,4,10,11,2 3-pentaazapentacyclo[19.3.1.0^{8,12}.0^{13,18}.0^{2,6}]pentacosa-1(24),2(6),4,8(9),11,13(14),15,17,21(25), 22-decaen-5-yl)ethynyl)-1-methyl-1,2-dihydropyridin-2-one

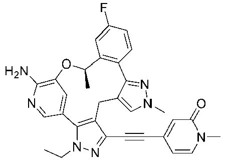

The synthetic method of Example 243 is the same as that of Example 47. LC-MS (ESI) [M+H]⁺: 550.4. ¹H NMR (400 MHz, CD₃OD), 7.68 (d, J = 7.2 Hz, 1H), 7.56 (s, 1H), 7.54 (dd, J = 10.0 Hz, 2.8 Hz, 1H), 7.45 (d, J = 1.6 Hz, 1H), 7.23 (dd, J = 8.4 Hz, 6.0 Hz, 1H), 7.08 (td, J = 8.4 Hz, 2.8 Hz, 1H), 6.72 (d, J = 1.6 Hz, 1H), 6.47-6.50 (m, 2H), 5.39-5.45 (m, 1H), 4.08-4.23 (m, 2H), 3.91 (s, 3H), 3.83 (d, J = 15.6 Hz, 1H), 3.58 (s, 3H), 2.96 (d, J = 15.6 Hz, 1H), 1.81 (d, J = 6.4 Hz, 3H), 1.35 (t, J = 7.2 Hz, 3H).

### Example 244: (19R)-3-ethyl-16-fluoro-10,19-dimethyl-5-(prop-2-en-1-yl)-20-oxa-3,4,10, 11,23-pentaazapentacyclo[19.3.1.0^{8,12}.0^{13,18}.0^{2,6}]pentacosa-1(25),2(6),4,8(9),11,13(18),14,16,21 (22),23-decaen-22-amine

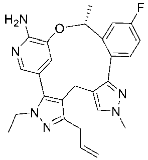

The synthetic method of Example 244 is the same as that of Example 45. LC-MS (ESI) [M+H]⁺: 459.4. ¹H NMR (400 MHz, CDCl₃), 7.42-7.60 (brs, 1H), 7.24-7.29 (m, 2H), 7.13 (dd, J = 8.4 Hz, 6.0 Hz, 1H), 6.99 (td, J = 8.4 Hz, 2.8 Hz, 1H), 6.55 (s, 1H), 6.07-6.17 (m, 1H), 5.24-5.56 (m, 3H), 5.12-5.23 (m, 2H), 3.98-4.10 (m, 2H), 3.90 (s, 3H), 3.54-3.58 (m, 3H), 2.88 (d, J = 16.0 Hz, 1H), 1.79 (d, J = 6.0 Hz, 3H), 1.31 (t, J = 7.2 Hz, 3H).

### Example 245: (19R)-5-((cyclopropyl(methyl)(oxo)-λ⁶-sulfanylidene)amino)-3-ethyl-16-f1 uoro-10,19-dimethyl-20-oxa-3,4,10,11,23-pentaazapentacyclo[19.3.1.0^{8,12}.0^{13,18}.0^{2,6}]pentacosa-1(25),2(6),4,8(9),11,13(18),14,16,21(22),23-decaen-22-amine

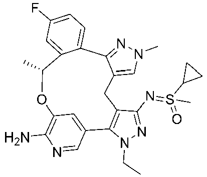

The synthetic method of Example 245 is the same as that of Example 169. LC-MS (ESI) [M+H]⁺: 536.5. ¹H NMR (400 MHz, CDCl₃), 7.58 (s, 1H), 7.50 (s, 1H), 7.24-7.29 (m, 1H), 7.11 (dd, J = 8.4 Hz, 5.6 Hz, 1H), 6.97 (td, J = 8.4 Hz, 2.4 Hz, 1H), 6.55 (s, 1H), 5.36-5.42 (m, 1H), 4.62-4.89 (brs, 2H), 3.93-4.04 (m, 2H), 3.89 (s, 3H), 3.62 (d, J = 15.6 Hz, 1H), 3.41 (s, 3H), 2.68-2.75 (m, 2H), 1.78 (d, J = 6.0 Hz, 3H), 1.36-1.44 (m, 2H), 1.32 (t, J = 6.4 Hz, 3H), 1.09-1.14 (m, 2H).

### Example 246: (19R)-10-(difluoromethyl)-3-ethyl-16-fluoro-19-methyl-5-(prop-2-en-1-yl) -20-oxa-3,4,10,11,23-pentaazapentacyclo[19.3.1.0^{8,12}.0^{13,18}.0^{2,6}]pentacosa-1(25),2(6),4,8(9),11, 13(18),14,16,21(22),23-decaen-22-amine

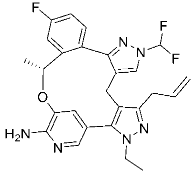

The synthetic method of Example 246 is the same as that of Example 45. LC-MS (ESI) [M+H]⁺: 495.4. ¹H NMR (400 MHz, CD₃OD), 7.91 (s, 1H), 7.56 (dd, J = 10.0 Hz, 2.4 Hz, 1H), 7.47 (t, J = 60.0 Hz, 1H), 7.39 (d, J = 1.6 Hz, 1H), 7.24 (dd, J = 8.4 Hz, 5.6 Hz, 1H), 7.10 (td, J = 8.4 Hz, 2.8 Hz, 1H), 6.33 (d, J = 1.6 Hz, 1H), 6.07-6.17 (m, 1H), 5.34-5.40 (m, 1H), 5.13-5.21 (m, 2H), 4.00-4.16 (m, 2H), 3.67 (d, J = 16.0 Hz, 1H), 3.60 (d, J = 6.4 Hz, 2H), 2.88 (d, J = 16.0 Hz, 1H), 1.80 (d, J = 6.0 Hz, 3H), 1.30 (t, J = 7.2 Hz, 3H).

### Example 247: N-(4-((19R)-22-amino-3-ethyl-16-fluoro-10,19-dimethyl-20-oxa-3,4,10,11, 23-pentaazapentacyclo[19.3.1.0^{8,12}.0^{13,18}.0^{2,6}]pentacosa-1(24),2(6),4,8(9),11,13(14),15,17,21 (2 5),22-decaen-5-yl)-2-methylbut-3-yn-2-yl)acetamide

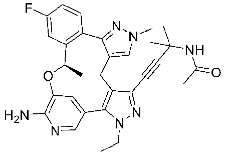

The synthetic method of Example 247 is the same as that of Example 47. LC-MS (ESI) [M+H]⁺: 542.4. ¹H NMR (400 MHz, CDCl₃), 7.67 (s, 1H), 7.46 (s, 1H), 7.27 (dd, J = 10.0 Hz, 2.8 Hz, 1H), 7.15 (dd, J = 8.4 Hz, 5.6 Hz, 1H), 6.99 (td, J = 8.4 Hz, 2.8 Hz, 1H), 6.57 (d, J = 1.6 Hz, 1H), 5.77 (s, 1H), 5.26-5.52 (m, 3H), 4.00-4.14 (m, 2H), 3.93 (s, 3H), 3.78 (d, J = 15.6 Hz, 1H), 2.89 (d, J = 15.6 Hz, 1H), 1.96 (s, 3H), 1.80 (d, J = 6.4 Hz, 3H), 1.78 (s, 3H), 1.76 (s, 3H), 1.36 (t, J = 7.2 Hz, 3H).

### Example 248: N-(4-((19R)-22-amino-3-ethyl-16-fluoro-10,19-dimethyl-20-oxa-3,4,10,11, 23-pentaazapentacyclo[19.3.1.0^{8,12}.0^{2,6}.0^{13,18}]pentacosa-1(24),2(6),4,8(9),11,13(14),15,17,21(2 5),22-decaen-5-yl)-2-methylbut-3-yn-2-yl)propane-2-sulfonamide

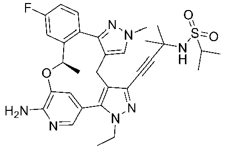

The synthetic method of Example 248 is the same as that of Example 47. LC-MS (ESI) [M+H]⁺: 606.5. ¹H NMR (400 MHz, CDCl₃), 7.70 (s, 1H), 7.39 (s, 1H), 7.27 (dd, J = 10.0 Hz, 2.8 Hz, 1H), 7.16 (dd, J = 8.4 Hz, 6.0 Hz, 1H), 7.03 (td, J = 8.4 Hz, 2.8 Hz, 1H), 6.64 (s, 1H), 5.93-6.13 (brs, 2H), 5.44-5.48 (m, 1H), 4.00-4.14 (m, 3H), 3.91 (s, 3H), 3.80 (d, J = 15.6 Hz, 1H), 2.90 (d, J = 15.6 Hz, 1H), 1.82 (d, J = 6.0 Hz, 3H), 1.73-1.78 (m, 12H), 1.38 (t, J = 7.2 Hz, 3H).

### Example 249: 3-(((19R)-22-amino-3-ethyl-16-fluoro-10,19-dimethyl-20-oxa-3,4,10,11,2 3-pentaazapentacyclo[19.3.1.0^{2,6}.0^{8,12}.0^{13,18}]pentacosa-1(25),2(6),4,8(9),11,13(14),15,17,21(22), 23-decaen-5-yl)ethynyl)-1,2-dihydropyridin-2-one

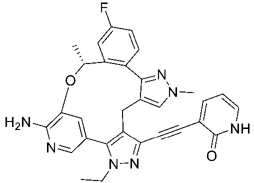

The synthetic method of Example 249 is the same as that of Example 47. LC-MS (ESI) [M+H]⁺: 536.4. ¹H NMR (400 MHz, CDCl₃), 8.31 (dd, J = 4.8 Hz, 1.6 Hz, 1H), 7.97 (dd, J = 7.2 Hz, 1.6 Hz, 1H), 7.56 (s, 1H), 7.46 (s, 1H), 7.26-7.31 (m, 2H), 7.17-7.21 (m, 2H), 7.00 (td, J = 8.4 Hz, 2.8 Hz, 1H), 6.64 (s, 1H), 5.46-5.50 (m, 1H), 4.83 (s, 2H), 4.13-4.26 (m, 3H), 3.88 (s, 3H), 3.04 (d, J = 15.6 Hz, 1H), 1.81 (d, J = 6.4 Hz, 3H), 1.45 (t, J = 7.2 Hz, 3H).

### Example 250: 2-(4-(((19R)-22-amino-3-ethyl-16-fluoro-10,19-dimethyl-20-oxa-3,4,10,1 1,23-pentaazapentacyclo[19.3.1. 0^{2,6}.0^{8,12}.0^{13,18}]pentacosa-1(25),2(6),4,8(9),11,13(14),15,17,21 (22),23-decaen-5-yl)ethynyl)pyrazol-1-yl)ethan-1-ol

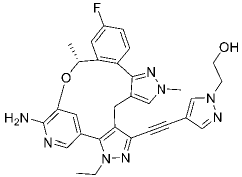

The synthetic method of Example 250 is the same as that of Example 47. LC-MS (ESI) [M+H]⁺: 553.4. ¹H NMR (400 MHz, CDCl₃), 7.73 (s, 1H), 7.70 (s, 1H), 7.54 (s, 1H), 7.44 (s, 1H), 7.28 (dd, J = 10.0 Hz, 2.4 Hz, 1H), 7.15 (dd, J = 8.4 Hz, 6.0 Hz, 1H), 7.01 (td, J = 8.4 Hz, 2.4 Hz, 1H), 6.56 (s, 1H), 5.37-5.45 (m, 1H), 4.84-5.36 (brs, 2H), 4.27 (t, J = 4.8 Hz, 2H), 4.11 (q, J = 7.2 Hz, 2H), 4.03 (t, J = 4.8 Hz, 2H), 3.91 (s, 3H), 3.82 (d, J = 15.6 Hz, 1H), 2.93 (d, J = 15.6 Hz, 1H), 1.80 (d, J = 6.4 Hz, 3H), 1.40 (t, J = 7.2 Hz, 3H).

### Example 251: 3-(((19R)-22-amino-3-ethyl-16-fluoro-10,19-dimethyl-20-oxa-3,4,10,11,2 3-pentaazapentacyclo[19.3.1.0^{2,6}.0^{8,12.}0^{13,18}]pentacosa-1(25),2(6),4,8(9),11,13(14),15,17,21(22), 23-decaen-5-yl)ethynyl)-1-methyl-1,2-dihydropyridin-2-one

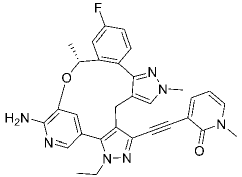

The synthetic method of Example 251 is the same as that of Example 47. LC-MS (ESI) [M+H]⁺: 550.4. ¹H NMR (400 MHz, CDCl₃), 7.76 (s, 1H), 7.69 (dd, J = 6.8 Hz, 2.0 Hz, 1H), 7.53 (s, 1H), 7.32 (dd, J = 6.8 Hz, 2.0 Hz, 1H), 7.27 (dd, J = 10.0 Hz, 2.8 Hz, 1H), 7.14 (dd, J = 8.4 Hz, 5.6 Hz, 1H), 6.98 (td, J = 8.4 Hz, 2.8 Hz, 1H), 6.60 (s, 1H), 6.19 (t, J = 7.2 Hz, 1H), 5.44-5.49 (m, 1H), 4.90 (s, 2H), 4.10-4.16 (m, 2H), 3.95 (d, J = 15.6 Hz, 1H), 3.92 (s, 3H), 3.61 (s, 3H), 2.96 (d, J = 15.6 Hz, 1H), 1.80 (d, J = 6.4 Hz, 3H), 1.41 (t, J = 7.2 Hz, 3H).

### Example 252: (5-(((19R)-22-amino-3-ethyl-16-fluoro-10,19-dimethyl-20-oxa-3,4,10,11,2 3-pentaazapentacyclo[19.3.1.0^{2,6}.0^{8,12}.0^{13,18}]pentacosa-1(24),2(6),4,8(9),11,13(18),14,16,21(25), 22-decaen-5-yl)ethynyl)pyrazin-2-yl)methanol

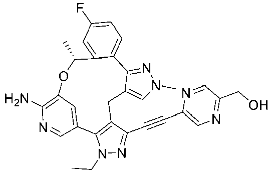

The synthetic method of Example 252 is the same as that of Example 47. LC-MS (ESI) [M+H]⁺: 551.4. ¹H NMR (400 MHz, CD₃OD), 8.80 (s, 1H), 8.76 (s, 1H), 7.64 (s, 1H), 7.54 (dd, J = 10.0 Hz, 2.8 Hz, 1H), 7.47 (d, J = 1.2 Hz, 1H), 7.23 (dd, J = 8.4 Hz, 5.6 Hz, 1H), 7.08 (td, J = 8.4 Hz, 2.8 Hz, 1H), 6.51 (d, J = 1.6 Hz, 1H), 5.41-5.46 (m, 1H), 4.80 (s, 2H), 4.11-4.23 (m, 2H), 3.92 (d, J = 15.6 Hz, 1H), 3.91 (s, 3H), 2.98 (d, J = 15.6 Hz, 1H), 1.82 (d, J = 6.4 Hz, 3H), 1.36 (t, J = 7.2 Hz, 3H).

### Example 253: N-(4-((19R)-22-amino-3-ethyl-16-fluoro-10,19-dimethyl-20-oxa-3,4,10,11, 23-pentaazapentacyclo[19.3.1.0^{2,6}.0^{8,12}.0^{13,18}]pentacosa-1(25),2(6),4,8(9),11,13(14),15,17,21(2 2),23-decaen-5-yl)-2-methylbut-3-yn-2-yl)-1-(methylamino)methanamide

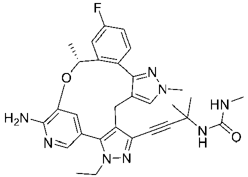

The synthetic method of Example 253 is the same as that of Example 47. LC-MS (ESI) [M+H]⁺: 557.4. ¹H NMR (400 MHz, CDCl₃), 7.53 (s, 1H), 7.50 (s, 1H), 7.28 (dd, J = 10.4 Hz, 2.8 Hz, 1H), 7.14 (dd, J = 8.4 Hz, 6.0 Hz, 1H), 7.00 (td, J = 8.4 Hz, 2.8 Hz, 1H), 6.54 (d, J = 1.6 Hz, 1H), 5.03 (s, 2H), 4.91-4.98 (m, 1H), 4.61 (s, 1H), 4.07-4.13 (m, 2H), 3.93 (s, 3H), 3.73 (d, J = 15.6 Hz, 1H), 2.92 (d, J = 15.6 Hz, 1H), 2.78 (d, J = 4.8 Hz, 3H), 1.80 (d, J = 6.4 Hz, 3H), 1.74 (s, 3H), 1.72 (s, 3H), 1.39 (t, J = 7.2 Hz, 3H).

### Example 254: 1-((4-((19R)-22-amino-3-ethyl-16-fluoro-10,19-dimethyl-20-oxa-3,4,10,1 1,23-pentaazapentacyclo[19.3.1.0^{2,6}.0^{8,12}.0^{13,18}]pentacosa-1(25),2(6),4,8(9),11,13(14),15,17,21 (22),23-decaen-5-yl)-2-methylbut-3-yn-2-yl)amino)-N,N-dimethylmethanamide

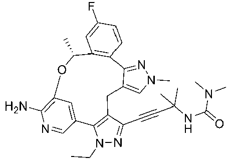

The synthetic method of Example 254 is the same as that of Example 47. LC-MS (ESI) [M+H]⁺: 571.5. ¹H NMR (400 MHz, CDCl₃), 7.76 (s, 1H), 7.50 (d, J = 1.6 Hz,1H), 7.28 (dd, J = 10.0 Hz, 2.8 Hz, 1H), 7.14 (dd, J = 8.4 Hz, 6.0 Hz, 1H), 6.98 (dd, J = 8.4 Hz, 2.8 Hz, 1H), 6.55 (d, J = 2.0 Hz, 1H), 5.41-5.48 (m, 1H), 4.86 (s, 2H), 4.60 (s, 1H), 4.04-4.14 (m, 2H), 3.93 (s, 3H), 3.80 (d, J = 15.2 Hz, 1H), 2.87-2.91 (m, 7H), 1.78-1.81 (m, 6H), 1.76 (s, 3H), 1.37 (t, J = 7.2 Hz, 3H).

### Example 255: methyl ((4-((19R)-22-amino-3-ethyl-16-fluoro-10,19-dimethyl-20-oxa-3, 4,10,11,23-pentaazapentacyclo[19.3.1.0^{2,6}.0^{8,12}.0^{13,18}]pentacosa-1(25),2(6),4,8(9),11,13(14),15, 17,21(22),23-decaen-5-yl)-2-methylbut-3-yn-2-yl)carbamate

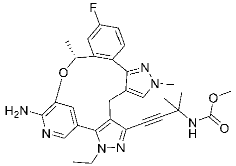

The synthetic method of Example 255 is the same as that of Example 47. LC-MS (ESI) [M+H]⁺: 558.4. ¹H NMR (400 MHz, CDCl₃), 7.64 (s, 1H), 7.56 (s, 1H), 7.24-7.27 (m, 1H), 7.15 (dd, J = 8.4 Hz, 5.6 Hz, 1H), 7.00 (td, J = 8.4 Hz, 2.8 Hz, 1H), 6.57 (s, 1H), 5.38-5.44 (m, 1H), 4.99 (s, 1H), 3.97-4.11 (m, 2H), 3.93 (s, 3H), 3.79 (d, J = 15.6 Hz, 1H), 3.61 (s, 3H), 2.88 (d, J = 15.6 Hz, 1H), 1.80 (d, J = 6.4 Hz, 3H), 1.76 (s, 3H), 1.73 (s, 3H), 1.32 (t, J = 7.2 Hz, 3H).

### Example 256: N-(4-((19R)-22-amino-3-ethyl-16-fluoro-10,19-dimethyl-20-oxa-3,4,10,11, 23-pentaazapentacyclo[19.3.1.0^{8,12}.0^{2,6}.0^{13,18}]pentacosa-1(24),2(6),4,8(9),11,13(14),15,17,21(2 5),22-decaen-5-yl)-2-methylbut-3-yn-2-yl)cyclopropanesulfonamide

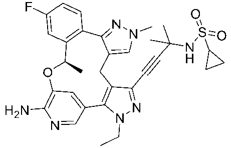

The synthetic method of Example 256 is the same as that of Example 47. LC-MS (ESI) [M+H]⁺: 604.4. ¹H NMR (400 MHz, CDCl₃), 7.60 (s, 1H), 7.48 (s, 1H), 7.27 (dd, J = 10.0 Hz, 2.8 Hz, 1H), 7.14 (dd, J = 8.4 Hz, 6.0 Hz, 1H), 7.00 (td, J = 8.4 Hz, 2.8 Hz, 1H), 6.56 (s, 1H), 5.40-5.44 (m, 1H), 5.18-5.38 (brs, 2H), 4.79 (s, 1H), 4.04-4.14 (m, 2H), 3.91 (s, 3H), 3.75 (d, J = 15.6 Hz, 1H), 2.90 (d, J = 15.6 Hz, 1H), 2.79-2.86 (m, 1H), 1.81 (s, 3H), 1.80 (d, J = 6.8 Hz, 3H), 1.78 (s, 3H), 1.37 (t, J = 7.2 Hz, 3H), 1.21-1.28 (m, 2H), 1.00-1.07 (m, 2H).

### Example 257: 1,1,1-trifluoro-N-(4-((19R)-22-amino-3-ethyl-16-fluoro-10,19-dimethyl-20 -oxa-3,4,10,11,23-pentaazapentacyclo[19.3.1.0^{8,12}.0^{2,6}.0^{13,18}]pentacosa-1(24),2(6),4,8(9),11,13 (14),15,17,21(25),22-decaen-5-yl)-2-methylbut-3-yn-2-yl)methanesulfonamide

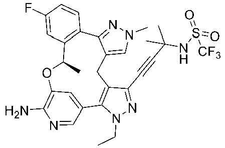

The synthetic method of Example 257 is the same as that of Example 47. LC-MS (ESI) [M+H]⁺: 632.6. ¹H NMR (400 MHz, CDCl₃), 7.52 (s, 1H), 7.44 (s, 1H), 7.28 (dd, J = 10.0 Hz, 2.8 Hz, 1H), 7.15 (dd, J = 8.4 Hz, 5.6 Hz, 1H), 7.02 (td, J = 8.4 Hz, 2.8 Hz, 1H), 6.59 (d, J = 1.2 Hz, 1H), 5.60-5.78 (brs, 2H), 5.42-5.46 (m, 1H), 4.01-4.16 (m, 2H), 3.90 (s, 3H), 3.72 (d, J = 15.6 Hz, 1H), 2.88 (d, J = 15.6 Hz, 1H), 1.87 (s, 3H), 1.86 (s, 3H), 1.82 (d, J = 6.0 Hz, 3H), 1.38 (t, J = 7.2 Hz, 3H).

### Example 258: 5-(((19R)-22-amino-3-ethyl-16-fluoro-10,19-dimethyl-20-oxa-3,4,10,11,2 3-pentaazapentacyclo[19.3.1.0^{8,12}.0^{2,6}.0^{13,18}]pentacosa-1(24),2(6),4,8(9),11,13(14),15,17,21(25), 22-decaen-5-yl)ethynyl)-1-(2,2,2-trifluoroethyl)-1,2-dihydropyridin-2-one

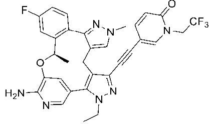

The synthetic method of Example 258 is the same as that of Example 47. LC-MS (ESI) [M+H]⁺: 618.4. ¹H NMR (400 MHz, CDCl₃), 7.61 (d, J = 1.6 Hz, 1H), 7.50 (dd, J = 9.6 Hz, 1.6 Hz, 1H), 7.46 (s, 1H), 7.40 (s, 1H), 7.30 (dd, J = 9.6 Hz, 2.4 Hz, 1H), 7.16 (dd, J = 8.4 Hz, 5.6 Hz, 1H), 7.03 (td, J = 8.4 Hz, 2.4 Hz, 1H), 6.64 (d, J = 9.6 Hz, 1H), 6.58 (d, J = 1.6 Hz, 1H), 5.50-5.76 (brs, 2H), 5.41-5.46 (m, 1H), 4.35-4.71 (m, 2H), 4.04.08-4.17 (m, 2H), 3.92 (s, 3H), 3.80 (d, J = 15.6 Hz, 1H), 2.96 (d, = 15.6 Hz, 1H), 1.81 (d, J = 6.0 Hz, 3H), 1.43 (d, J = 7.2 Hz, 3H).

### Example 259: 3-ethyl-16-fluoro-10-methyl-3,4,9,10,11,23,26-heptaazahexacyclo[22.3.1. 0^{8,12}.0^{13,18}.0^{19,23}.0^{2,6}]octacosa-1(28),2(6),4,8(9),11,13(18),14,16,24(25),26-decaen-25-amine

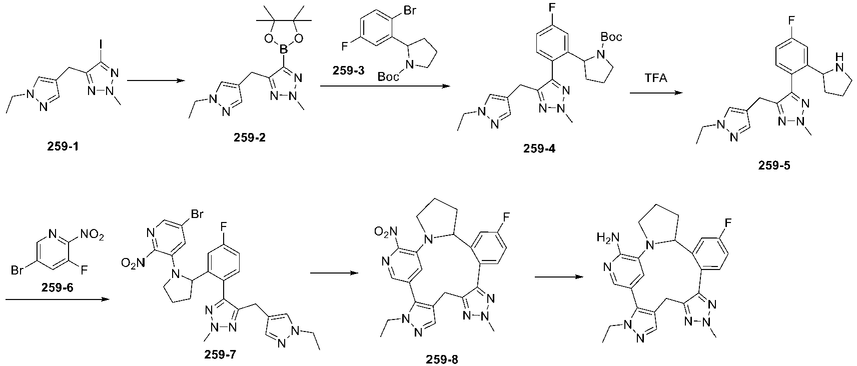

Step 1: Under nitrogen protection, a solution of compound 259-1 (317 mg), bis(pinacolato)diboron (500 mg), potassium acetate (400 mg) and Pd(dppf)Cl₂ (73 mg) in dioxane (5 mL) was heated to 90 °C and stirred overnight, the resulting solution of compound **259-2** was directly used in the next step.

Step 2: To the solution of compound **259-2** obtained last step were added water (1 mL), compound **259-3** (300 mg), anhydrous sodium carbonate (200 mg) and Ruphos-Pd-G3 (100 mg). The reaction solution was heated to 100 °C and stirred overnight. The reaction solution was cooled to room temperature and concentrated under reduced pressure, and the resulting residue was purified through flash silica gel chromatography (dichloromethane:methanol = 50:1) to obtain compound **259-4** (110 mg).

Step 3: To compound **259-4** (110 mg) was added a solution of HCl in dioxane (4 N, 5 mL), and the mixture was stirred at room temperature for 3 hours. The reaction solution was concentrated under reduced pressure to obtain compound **259-5** (70 mg).

Step 4: To a solution of compound **259-5** (70 mg) in DMF (2 mL) were added compound **259-6** (44 mg) and DIEA (103 mg). The reaction solution was heated to 60 °C and stirred for 1 hour. The mixture was cooled to room temperature and concentrated under reduced pressure, and the resulting residue was purified through flash silica gel chromatography (dichloromethane:methanol = 30:1) to obtain compound **259-7** (55 mg).

Step 5: Compound **259-7** (55 mg) was used as the starting material to obtain compound **259-8** (52 mg) through General Synthesis Method F.

Step 6: To a solution of compound **259-8** (55 mg) in ethanol (5 mL) /water (1 mL) were added ammonium chloride (40 mg) and iron powder for reduction (50 mg). The reaction solution was heated to 80 °C and stirred for 1.5 hours. The reaction solution was cooled to room temperature and filtered, the filtrate was concentrated under reduced pressure, and the resulting residue was purified through flash silica gel chromatography (dichloromethane:methanol = 30:1) to obtain Example 259 (12 mg). LC-MS (ESI) [M+H]⁺: 445.5. ¹H NMR (400 MHz, CDCl₃), 7.73 (s, 1H), 7.62 (s, 1H), 7.32 (dd, J = 10.4 Hz, 2.8 Hz, 1H), 7.02 (dd, J = 8.4 Hz, 6.0 Hz, 1H), 6.94 (td, J = 8.4 Hz, 2.8 Hz, 1H), 6.44 (s, 1H), 5.20 (s, 2H), 4.16-4.24 (m, 4H), 4.03-4.13 (m, 2H), 3.80-3.86 (m, 1H), 3.75 (d, J = 15.6 Hz, 1H), 3.16 (d, J = 15.6 Hz, 1H), 2.42-2.51 (m, 2H), 2.14-2.25 (m, 1H), 1.93-2.11 (m, 2H), 1.42 (t, J = 7.2 Hz, 3H).

### Example 260: 5-chloro-3-ethyl-16-fluoro-10-methyl-3,4,10,11,23,26-hexaazahexacyclo [22.3.1.0^{8,12}.0^{13,18}.0^{19,23}.0^{2,6}]octacosa-1(28),2(6),4,8(9),11,13(18),14,16,24(25),26-decaen-25-a mine

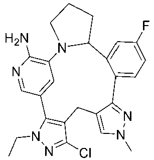

The synthetic method of Example 260 is the same as that ofExample 259. LC-MS (ESI) [M+H]⁺: 478.4. ¹H NMR (400 MHz, CDCl₃), 7.58 (d, J = 1.6 Hz, 1H), 7.41 (s, 1H), 7.29 (dd, J = 10.4 Hz, 2.8 Hz, 1H), 7.06 (dd, J = 8.4 Hz, 6.0 Hz, 1H), 6.92 (td, J = 8.4 Hz, 2.8 Hz, 1H), 6.67 (d, J = 2.0 Hz, 1H), 5.36 (s, 2H), 4.32-4.36 (m, 1H), 4.02-4.08 (m, 2H), 3.92 (s, 3H), 3.83-3.88 (m, 1H), 3.60 (d, J = 15.6 Hz, 1H), 2.82 (d, J = 15.6 Hz, 1H), 2.46-2.55 (m, 2H), 2.15-2.24 (m, 1H), 1.93-2.10 (m, 2H), 1.41 (t, J = 7.2 Hz, 3H).

### Example 261: (3R)-19-ethyl-17-ethynyl-6,8-difluoro-3,12-dimethyl-2-oxa-11,12,18,19,2 3-pentaazapentacyclo[19.3.1.0^{4,9}.0^{10,14}.0^{16,20}]pentacosa-1(25),4(5),6,8,10(11),13,16(20),17,21(2 2),23-decaen-24-amine

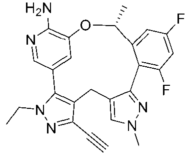

The synthetic method of Example 261 is the same as that of Example 47. LC-MS (ESI) [M+H]⁺: 461.4. ¹H NMR (400 MHz, CDCl₃), 7.55 (d, J = 1.6 Hz, 1H), 7.45 (s, 1H), 7.11-7.14 (m, 1H), 6.77-6.83 (m, 1H), 6.53 (d, J = 1.6 Hz, 1H), 5.34-5.40 (m, 1H), 4.81 (s, 2H), 4.10-4.16 (m, 2H), 3.93 (s, 3H), 3.82 (d, J = 15.6 Hz, 1H), 3.29 (s, 1H), 2.84 (dd, J = 15.6 Hz, 1.6 Hz, 1H), 1.77 (d, J = 6.4 Hz, 3H), 1.41 (t, J = 7.2 Hz, 3H).

### Example 262: (3R)-8-chloro-19-ethyl-17-ethynyl-6-fluoro-3,12-dimethyl-2-oxa-11,12,18, 19,23-pentaazapentacyclo[19.3.1.0^{4,9}.0^{10,14}.0^{16,20}]pentacosa-1(25),4(5),6,8,10(11),13,16(20),17, 21(22),23-decaen-24-amine

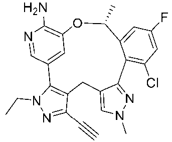

The synthetic method of Example 262 is the same as that of Example 47. LC-MS (ESI) [M+H]⁺: 477.4.

### Example 263: (3R)-8,17-dichloro-19-ethyl-6-fluoro-3,12-dimethyl-2-oxa-11,12,18,19,23-pentaazapentacyclo[19.3.1.0^{4,9}.0^{10,14}.0^{16,20}]pentacosa-1(25),4(5),6,8,10(11),13,16(20),17,21(22), 23-decaen-24-amine

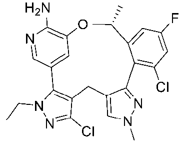

The synthetic method of Example 263 is the same as that of Example 47. LC-MS (ESI) [M+H]⁺: 487.4. ¹H NMR (400 MHz, CDCl₃), 7.45 (s, 1H), 7.41 (s, 1H), 7.23 (dd, J = 8.8 Hz, 2.8 Hz, 1H), 7.15 (dd, J = 7.6 Hz, 2.8 Hz, 1H), 6.59 (s, 1H), 5.25-5.31 (m, 1H), 4.90 (s, 2H), 4.10 (q, J = 7.2 Hz, 2H), 3.94 (s, 3H), 3.69 (d, J = 16.0 Hz, 1H), 2.76 (d, J = 16.0 Hz, 1H), 1.74 (d, J = 6.0 Hz, 3H), 1.40 (t, J = 6.8 Hz, 3H).

### Example 264: (3R)-17-chloro-19-ethyl-8-ethynyl-6-fluoro-3,12-dimethyl-2-oxa-11,12,18, 19,23-pentaazapentacyclo[19.3.1.0^{4,9}.0^{10,14}.0^{16,20}]pentacosa-1(25),4(5),6,8,10(11),13,16(20),17, 21(22),23-decaen-24-amine

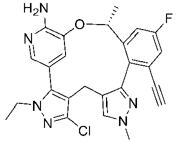

The synthetic method of Example 264 is the same as that of Example 47. LC-MS (ESI) [M+H]⁺: 477.4.

### Example 265: (3R)-19-ethyl-17-ethynyl-6-fluoro-3,12-dimethyl-8,11,12,18,19,23-hexaaz a-2-oxapentacyclo[19.3.1.0^{4,9}.0^{10,14}.0^{16,20}]pentacosa-1(25),4(5),6,8,10(11),13,16(20),17,21(22), 23-decaen-24-amine

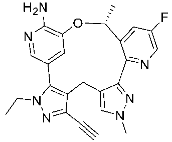

The synthetic method of Example 265 is the same as that of Example 47. LC-MS (ESI) [M+H]⁺: 444.4. ¹H NMR (400 MHz, CDCl₃), 8.46 (d, J = 2.8 Hz, 1H), 7.63 (dd, J = 9.2 Hz, 2.8 Hz, 1H), 7.54 (s, 1H), 7.46 (s, 1H), 6.53 (d, J = 1.6 Hz, 1H), 5.45-5.51 (m, 1H), 4.88 (s, 2H), 4.07-4.13 (m, 2H), 3.94 (d, J = 15.6 Hz, 1H), 3.91 (s, 3H), 3.29 (s, 1H), 2.93 (d, J = 15.6 Hz, 1H), 1.83 (d, J = 6.4 Hz, 3H), 1.39 (t, J = 7.2 Hz, 3H).

### Example 266: (3R)-24-amino-17-chloro-19-ethyl-6-fluoro-3,12-dimethyl-2-oxa-11,12,18, 19,23-pentaazapentacyclo[19.3.1.0^{4,9}.0^{10,14}.0^{16,20}]pentacosa-1(25),4(5),6,8,10(11),13,16(20),17, 21(22),23-decaen-8-carbonitrile

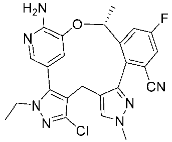

The synthetic method of Example 266 is the same as that of Example 47. LC-MS (ESI) [M+H]⁺: 477.3.

### Example 267: (19R)-3-ethyl-5-ethynyl-16-fluoro-19-methyl-20-oxa-3,4,9,11,23-pentaaza -10-thiapentacyclo[19.3.1.0^{2,6}.0^{8,12} 0^{13,18}]pentacosa-1(24),2(6),4,8(9),11,13(14),15,17,21(25),22 -decaen-22-amine

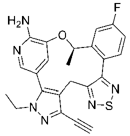

The synthetic method of Example 267 is the same as that of Example 1. LC-MS (ESI) [M+H]⁺: 447.4. ¹H NMR (400 MHz, CD₃OD), 7.61 (dd, J = 10.0 Hz, 2.4 Hz, 1H), 7.44 (d, J = 1.6 Hz, 1H), 7.32 (dd, J = 8.4 Hz, 6.0 Hz, 1H), 7.15 (td, J = 8.4 Hz, 2.4 Hz, 1H), 5.83 (d, J = 1.6 Hz, 1H), 5.16-5.21 (m, 1H), 4.05-4.16 (m, 3H), 3.74 (s, 1H), 3.53 (d, J = 15.6 Hz, 1H), 1.81 (d, J = 6.8 Hz, 3H), 1.32 (t, J = 7.2 Hz, 3H).

### Example 268: (19R)-3-ethyl-5-ethynyl-16-fluoro-19-methyl-20-oxa-3,4,8,11,23-pentaaza pentacyclo[19.3.1.0^{2,6}.0^{8,12}.0^{13,18}]pentacosa-1(24),2(6),4,9,11,13(14),15,17,21(25),22-decaen-22 -amine

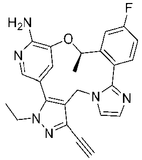

The synthetic method of Example 268 is the same as that of Example 1. LC-MS (ESI) [M+H]⁺: 429.3. ¹H NMR (400 MHz, CDCl₃), 7.16-7.68 (m, 5H), 7.04-7.09 (m, 1H), 5.93 (s, 1H), 5.56-5.70 (brs, 1H), 5.17-5.38 (brs, 2H), 5.04 (d, J = 13.2 Hz, 1H), 4.42 (d, J = 14.8 Hz, 1H), 4.14-4.19 (m, 2H), 3.39 (s, 1H), 1.82-1.96 (m, 3H), 1.44 (t, J = 7.6 Hz, 3H).

### Example 269: (19R)-3-ethyl-5-ethynyl-16-fluoro-19-methyl-20-oxa-3,4,11,12,23-pentaaz apentacyclo[19.3.1.0^{2,6}.0^{8,12}.0^{13,18}]pentacosa-1(24),2(6),4,8(9),10,13(14),15,17,21(25),22-decae n-22-amine

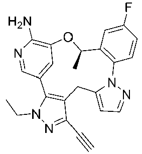

The synthetic method of Example 269 is the same as that of Example 1. LC-MS (ESI) [M+H]⁺: 429.3. ¹H NMR (400 MHz, CDCl₃), 7.66 (d, J = 1.6 Hz, 1H), 7.58 (s, 1H), 7.35 (dd, J = 9.2 Hz, 2.8 Hz, 1H), 7.15 (dd, J = 8.4 Hz, 5.6 Hz, 1H), 7.05-7.10 (m, 1H), 6.41 (s, 1H), 6.26 (s, 1H), 5.28-5.33 (m,1H), 4.72-5.18 (brs, 2H), 4.12-4.19 (m, 2H), 3.99 (d, J = 16.0 Hz, 1H), 3.30 (s, 1H), 3.14 (d, J = 16.0 Hz, 1H), 1.81 (d, J = 6.4 Hz, 3H), 1.42 (t, J = 7.2 Hz, 3H).

### Example 270: (19R)-3-ethyl-5-ethynyl-16-fluoro-19-methyl-20-oxa-3,4,11,23-tetraaza-1 0-thiapentacyclo[19.3.1.0^{2,6}.0^{8,12}.0^{13,18}]pentacosa-1(24),2(6),4,8(9),11,13(14),15,17,21(25),22-d ecaen-22-amine

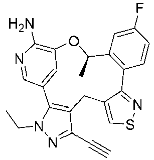

The synthetic method of Example 270 is the same as that of Example 1. LC-MS (ESI) [M+H]⁺: 446.3. ¹H NMR (400 MHz, CDCl₃), 8.74 (d, J = 1.6 Hz, 1H), 7.53 (s, 1H), 7.32 (dd, J = 10.0 Hz, 2.4 Hz, 1H), 7.11 (dd, J = 8.4 Hz, 5.6 Hz, 1H), 7.03 (td, J = 8.4 Hz, 2.4 Hz, 1H), 6.08 (d, J = 1.6 Hz, 1H), 5.19-5.24 (m, 1H), 4.87 (s, 2H), 4.11-4.16 (m, 2H), 3.91 (dd, J = 15.6 Hz, 1.6 Hz, 1H), 3.33 (s, 1H), 3.17 (d, J = 15.6 Hz, 1H), 1.81 (d, J = 6.4 Hz, 3H), 1.41 (t, J = 7.2 Hz, 3H).

### Example 271: (19R)-3-ethyl-5-ethynyl-16-fluoro-19-methyl-10,20-dioxa-3,4,11,23-tetraa zapentacyclo[19.3.1.0^{2,6}.0^{8,12}.0^{13,18}]pentacosa-1(24),2(6),4,8(9),11,13(14),15,17,21(25),22-deca en-22-amine

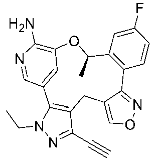

The synthetic method of Example 271 is the same as that of Example 1. LC-MS (ESI) [M+H]⁺: 430.3. ¹H NMR (400 MHz, CDCl₃), 8.52 (d, J = 1.2 Hz, 1H), 7.56 (s, 1H), 7.35 (dd, J = 9.6 Hz, 2.4 Hz, 1H), 7.16 (dd, J = 8.4 Hz, 5.6 Hz, 1H), 7.10 (td, J = 8.4 Hz, 2.4 Hz, 1H), 6.32 (d, J = 1.2 Hz, 1H), 5.36-5.42 (m, 1H), 5.06-5.24 (brs, 2H), 4.04-4.16 (m, 2H), 3.81 (dd, J = 16.0 Hz, 1.6 Hz, 1H), 3.33 (s, 1H), 2.80 (d, J = 16.0 Hz, 1H), 1.81 (d, J = 6.4 Hz, 3H), 1.38 (t, J = 7.2 Hz, 3H).

### Example 272: (20R)-3-ethyl-5-ethynyl-17-fluoro-11,20-dimethyl-21-oxa-3,4,12,24-tetraa zapentacyclo[20.3.1.0^{2,6}.0^{8,13}.0^{14,19}]hexacosa-1(25),2(6),4,8(9),10,12,14(15),16,18,22(26),23-un decaen-23-amine

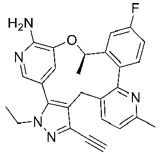

The synthetic method of Example 272 is the same as that of Example 1. LC-MS (ESI) [M+H]⁺: 454.5. ¹H NMR (400 MHz, CD₃OD), 7.96 (d, J = 8.0 Hz, 1H), 7.54 (dd, J = 10.0 Hz, 2.8 Hz, 1H), 7.45 (s, 1H), 7.33 (d, J = 8.4 Hz, 1H), 7.25 (dd, J = 8.4 Hz, 5.6 Hz, 1H), 7.08 (td, J = 8.4 Hz, 2.8 Hz, 1H), 6.25 (d, J = 1.6 Hz, 1H), 5.14-5.20 (m, 1H), 4.12-4.22 (m, 2H), 3.92 (d, J = 15.6 Hz, 1H), 3.88 (s, 1H), 3.25 (d, J = 15.6 Hz, 1H), 2.54 (s, 3H), 1.85 (d, J = 6.8 Hz, 3H), 1.36 (t, J = 7.2 Hz, 3H).

### Example 273: (20R)-3-ethyl-5-ethynyl-17-fluoro-20-methyl-21-oxa-3,4,11,24-tetraazape ntacyclo[20.3.1.0^{2,6}.0^{8,13}.0^{14,19}]hexacosa-1(25),2(6),4,8(9),10,12,14(15),16,18,22(26),23-undeca en-23-amine

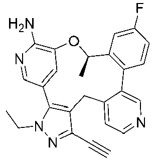

The synthetic method of Example 273 is the same as that of Example 1. LC-MS (ESI) [M+H]⁺: 440.3. ¹H NMR (400 MHz, CDCl₃), 8.52-8.66 (brs, 1H), 8.30-8.40 (brs, 1H), 7.62-7.70 (brs, 1H), 7.54 (s,1 H), 7.32 (dd, J = 10.0 Hz, 2.8 Hz, 1H), 7.10 (dd, J = 8.4 Hz, 5.6 Hz, 1H), 7.04 (td, J = 8.4 Hz, 2.8 Hz, 1H), 6.18 (d, J = 1.6 Hz, 1H), 5.17-5.24 (m, 1H), 4.87 (s, 2H), 4.18 (q, J = 7.2 Hz, 2H), 3.88 (d, J = 15.6 Hz, 1H), 3.33 (s, 1H), 3.28 (d, J = 15.6 Hz, 1H), 1.83 (d, J = 6.0 Hz, 3H), 1.43 (t, J = 7.2 Hz, 3H).

### Example 274: (20R)-3-ethyl-5-ethynyl-17-fluoro-20-methyl-21-oxa-3,4,10,24-tetraazape ntacyclo[20.3.1.0^{2,6}.0^{8,13}.0^{14,19}]hexacosa-1(25),2(6),4,8(9),10,12,14(15),16,18,22(26),23-undeca en-23-amine

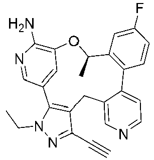

The synthetic method of Example 274 is the same as that of Example 1. LC-MS (ESI) [M+H]⁺: 440.3. ¹H NMR (400 MHz, CD₃OD), 8.78-8.96 (brs, 1H), 8.38-8.48 (brs, 1H), 7.56 (dd, J = 10.0 Hz, 2.8 Hz, 1H), 7.47 (s, 1H), 7.27 (s, 1H), 7.20 (dd, J = 8.4 Hz, 5.6 Hz, 1H), 7.11 (td, J = 8.4 Hz, 2.8 Hz, 1H), 6.26 (s, 1H), 5.26-5.33 (m, 1H), 4.11-4.25 (m, 2H), 3.98 (d, J = 15.6 Hz, 1H), 3.94 (s, 1H), 3.29 (d, J = 15.6 Hz, 1H), 1.85 (d, J = 6.4 Hz, 3H), 1.36 (t, J = 7.2 Hz, 3H).

### Example 275: (20R)-3-ethyl-5-ethynyl-17-fluoro-20-methyl-21-oxa-3,4,12,24-tetraazape ntacyclo[20.3.1.0^{2,6}.0^{8,13}.0^{14,19}]hexacosa-1(25),2(6),4,8(9),10,12,14(15),16,18,22(26),23-undeca en-23-amine

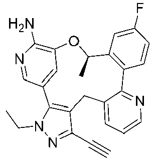

The synthetic method of Example 275 is the same as that of Example 1. LC-MS (ESI) [M+H]⁺: 440.3. ¹H NMR (400 MHz, CD₃OD), 8.51 (dd, J = 4.8 Hz, 0.8 Hz, 1H), 8.11 (d, J = 8.0 Hz, 1H), 7.54 (dd, J = 10.0 Hz, 2.4 Hz, 1H), 7.45-7.48 (m, 2H), 7.25 (dd, J = 8.4 Hz, 5.6 Hz, 1H), 7.09 (td, J = 8.4 Hz, 2.4 Hz, 1H), 6.21 (d, J = 1.6 Hz, 1H), 5.14-5.20 (m, 1H), 4.10-4.24 (m, 2H), 3.97 (d, J = 15.6 Hz, 1H), 3.89 (s, 1H), 3.27 (d, J = 15.6 Hz, 1H), 1.84 (d, J = 6.0 Hz, 3H), 1.36 (t, J = 7.2 Hz, 3H).

### Example 276: (20R)-3-ethyl-5-ethynyl-17-fluoro-10,20-dimethyl-21-oxa-3,4,12,24-tetraa zapentacyclo[20.3.1.0^{2,6}.0^{8,13}.0^{14,19}]hexacosa-1(25),2(6),4,8(9),10,12,14(15),16,18,22 (26),23-u ndecaen-23-amine

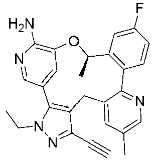

The synthetic method of Example 276 is the same as that of Example 1. LC-MS (ESI) [M+H]⁺: 454.4. ¹H NMR (400 MHz, CD₃OD), 8.35 (s, 1H), 7.91 (s, 1H), 7.52 (dd, J = 10.4 Hz, 2.8 Hz, 1H), 7.46 (s, 1H), 7.23 (dd, J = 8.4 Hz, 5.6 Hz, 1H), 7.07 (td, J = 8.4 Hz, 2.8 Hz, 1H), 6.23 (d, J = 1.6 Hz, 1H), 5.14-5.20 (m, 1H), 4.12-4.22 (m, 2H), 3.94 (d, J = 15.6 Hz, 1H), 3.91 (s, 1H), 3.24 (d, J = 15.6 Hz, 1H), 2.39 (s, 3H), 1.83 (d, J = 6.0 Hz, 3H), 1.37 (t, J = 7.2 Hz, 3H).

### Example 277: (20R)-3-ethyl-5-ethynyl-17-fluoro-20-methyl-21-+-3,4,10,12,24-pentaazap entacyclo[20.3.1.0^{2,6}.0^{8,13}.0^{14,19}]hexacosa-1(25),2(6),4,8(9),10,12,14(15),16,18,22(26),23-undec aen-23-amine

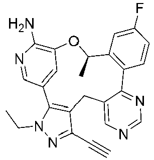

The synthetic method of Example 277 is the same as that of Example 1. LC-MS (ESI) [M+H]⁺: 441.4. ¹H NMR (400 MHz, CDCl₃), 9.15 (s, 1H), 9.11 (s, 1H), 7.50 (s, 1H), 7.35 (dd, J = 9.6 Hz, 2.4 Hz, 1H), 7.10 (dd, J = 8.4 Hz, 5.6 Hz, 1H), 7.05 (td, J = 8.4 Hz, 2.4 Hz, 1H), 6.14 (s, 1H), 5.12-5.29 (m, 3H), 4.15 (q, J = 6.8 Hz, 2H), 4.02 (d, J = 15.6 Hz, 1H), 3.36 (s, 1H), 3.29 (d, J = 15.6 Hz, 1H), 1.86 (d, J = 6.0 Hz, 3H), 1.41 (t, J = 7.2 Hz, 3H).

### Example 278: (20R)-3-ethyl-5-ethynyl-17-fluoro-11,20-dimethyl-21-oxa-3,4,10,12,24-p entaazapentacyclo[20.3.1.0^{2,1}.0^{1,13}.0^{14,19}]hexacosa-1(25),2(6),4,8(9),10,12,14(15), 16,18,22(2 6),23-undecaen-23-amine

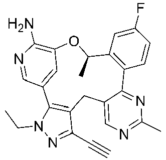

The synthetic method of Example 278 is the same as that of Example 1. LC-MS (ESI) [M+H]⁺: 455.4.

### Example 279: (20R)-3-ethyl-5-ethynyl-17-fluoro-20-methyl-21-oxa-3,4,11,12,24-pentaaz apentacyclo[20.3.1.0^{2,6}.0^{8,13}.0^{14,19}]hexacosa-1(25),2(6),4,8(9),10,12,14(15),16,18,22(26),23-und ecaen-23-amine

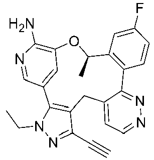

The synthetic method of Example 279 is the same as that of Example 1. LC-MS (ESI) [M+H]⁺: 441.3.

### Example 280: (19R)-3-ethyl-5-ethynyl-16-fluoro-19-methyl-9,20-dioxa-3,4,11,23-tetraaz apentacyclo[19.3.1.0^{2,6}.0^{8,12}.0^{13,18}]pentacosa-1(24),2(6),4,8(12),10,13(14),15,17,21(25),22-deca en-22-amine

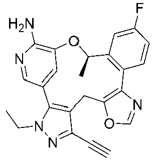

The synthetic method of Example 280 is the same as that of Example 1. LC-MS (ESI) [M+H]⁺: 430.4.

### Example 281: (19R)-3-ethyl-5-ethynyl-16-fluoro-19-methyl-20-oxa-3,4,11,23-tetraaza-9-thiapentacyclo[19.3.1.0^{2,6}.0^{8,12}.0^{13,18}]pentacosa-1(24),2(6),4,8(12),10,13(14),15,17,21(25),22-de caen-22-amine

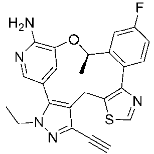

The synthetic method of Example 281 is the same as that of Example 1. LC-MS (ESI) [M+H]⁺: 446.3. ¹H NMR (400 MHz, CD₃OD+CDCl₃), 8.75 (s, 1H), 7.34-7.60 (brs, 1H), 7.26-7.29 (m, 1H), 7.09 (dd, J = 8.4 Hz, 5.6 Hz, 1H), 6.98 (td, J = 8.4 Hz, 2.8 Hz, 1H), 6.32 (s, 1H), 5.06-5.13 (m, 1H), 4.01-4.11 (m, 3H), 3.40 (d, J = 16.0 Hz, 1H), 3.34 (s, 1H), 1.75 (d, J = 6.4 Hz, 3H), 1.33 (t, J = 7.2 Hz, 3H).

### Example 282: (19R)-3-ethyl-5-ethynyl-16-fluoro-19-methyl-10-(trideuteromethyl)-20-ox a-3,4,10,11,23-pentaazapentacyclo[19.3.1.0^{8,12}.0^{13,18}.0^{2,6}]pentacosa-1(24),2(6),4,8(9),11,13(14), 15,17,21(25),22-decaen-22-amine

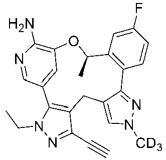

The synthetic method of Example 282 is the same as that of Example 1. LC-MS (ESI) [M+H]⁺: 446.3. ¹H NMR (400 MHz, CDCl₃), 7.52 (s, 1H), 7.43 (s, 1H), 7.28 (dd, J = 10.0 Hz, 2.8 Hz, 1H), 7.13 (dd, J = 8.4 Hz, 5.6 Hz, 1H), 7.00 (td, J = 8.4 Hz, 2.8 Hz, 1H), 6.52 (d, J = 1.2 Hz, 1H), 5.38-5.44 (m, 1H), 4.87 (s, 2H), 4.09-4.15 (m, 2H), 3.79 (d, J = 15.6 Hz, 1H), 3.30 (s, 1H), 2.92 (d, J = 15.6 Hz, 1H), 1.79 (d, J = 6.4 Hz, 3H), 1.40 (t, J = 7.2 Hz, 3H).

### Example 283: (20R)-3-ethyl-5-ethynyl-10,17-difluoro-20-methyl-21-oxa-3,4,12,24-tetraa zapentacyclo[20.3.1.0^{8,13}.0^{14,19}.0^{2,6}]hexacosa-1(25),2(6),4,8(9),10,12,14(19),15,17, 22(26),23-u ndecaen-23-amine

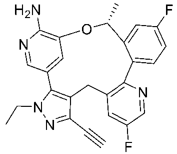

The synthetic method of Example 283 is the same as that of Example 1. LC-MS (ESI) [M+H]⁺: 458.3. ¹H NMR (400 MHz, CDCl₃), 8.43 (d, J = 2.0 Hz, 1H), 7.78 (dd, J = 9.6 Hz, 2.4 Hz, 1H), 7.42-7.52 (brs, 1H), 7.30 (dd, J =9.6 Hz, 2.4 Hz, 1H), 7.12 (dd, J = 8.4 Hz, 5.6 Hz, 1H), 7.03 (td, J = 8.4 Hz, 2.4 Hz, 1H), 6.23 (s, 1H), 5.54-5.74 (brs, 2H), 5.15-5.20 (m, 1H), 4.16 (q, J = 7.2 Hz, 2H), 3.99 (d, J = 15.6 Hz, 1H), 3.36 (s, 1H), 3.30 (dd, J = 15.6 Hz, 1.6 Hz, 1H), 1.84 (d, J = 6.4 Hz, 3H), 1.44 (t, J = 7.2 Hz, 3H).

### Example 284: (20R)-3-ethyl-5-ethynyl-17-fluoro-11-methoxy-20-methyl-21-oxa-3,4,12,2 4-tetraazapentacyclo[20.3.1.0^{8,13}.0^{14,19}.0^{2,6}]hexacosa-1(26),2(6),4,8(9),10,12, 14(19),15,17,22 (23),24-undecaen-23-amine

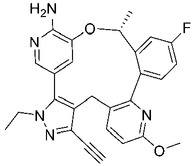

The synthetic method of Example 284 is the same as that of Example 1. LC-MS (ESI) [M+H]⁺: 470.4. ¹H NMR (400 MHz, CD₃OD), 7.92 (d, J = 8.8 Hz, 1H), 7.50 (dd, J = 10.0 Hz, 2.8 Hz, 1H), 7.44 (d, J = 2.0 Hz, 1H), 7.20 (dd, J = 8.4 Hz, 6.0 Hz, 1H), 7.05 (td, J = 8.4 Hz, 2.8 Hz, 1H), 6.83 (d, J = 8.8 Hz, 1H), 6.37 (d, J = 1.6 Hz, 1H), 5.33-5.39 (m, 1H), 4.10-4.20 (m, 2H), 3.89 (s, 4H), 3.88 (d, J = 15.6 Hz, 1H), 3.23 (d, J = 15.6 Hz, 1H), 1.86 (d, J = 6.4 Hz, 3H), 1.35 (t, J = 7.2 Hz, 3H).

### Example 285: (19R)-3-(cyclopropylmethyl)-5-ethynyl-16-fluoro-10,19-dimethyl-20-oxa-3,4,10,11,23-pentaazapentacyclo[19.3.1.0^{8.12}.0^{13,18}.0^{2,6}]pentacosa-1(24),2(6),4,8(9),11,13(18),1 4,16,21(25),22-decaen-22-amine

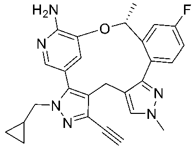

The synthetic method of Example 285 is the same as that of Example 1. LC-MS (ESI) [M+H]⁺: 469.4. ¹H NMR (400 MHz, CDCl₃), 7.50-7.74 (brs, 1H), 7.45 (s, 1H), 7.25-7.28 (m, 1H), 7.15 (dd, J = 8.4 Hz, 5.6 Hz, 1H), 7.03 (td, J = 8.4 Hz, 2.8 Hz, 1H), 6.60 (s, 1H), 5.58-6.14 (brs, 2H), 5.40-5.46 (m, 1H), 4.08 (dd, J = 14.0 Hz, 6.0 Hz, 1H), 3.92 (s, 3H), 3.80-3.87 (m, 2H), 3.32 (s, 1H), 2.92 (d, J = 15.2 Hz, 1H), 1.82 (d, J = 6.4 Hz, 3H), 1.13-1.24 (m, 1H), 0.42-0.56 (m, 2H), 0.30-0.38 (m, 1H), 0.14-0.22 (m, 1H).

### Example 286: (19R)-5-ethynyl-16-fluoro-3-(2-methoxyethyl)-10,19-dimethyl-20-oxa-3,4, 10,11,23-pentaazapentacyclo[19.3.1.0^{8,12}.0^{13,18}.0^{2,6}]pentacosa-1(25),2(6),4,8(9),11,13(18),14,16, 21 (22),23-decaen-22-amine

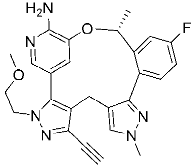

The synthetic method of Example 286 is the same as that of Example 1. LC-MS (ESI) [M+H]⁺: 473.5. ¹H NMR (400 MHz, CDCl₃), 7.80 (d, J = 1.6 Hz, 1H), 7.43 (s, 1H), 7.28 (dd, J = 10.0 Hz, 2.4 Hz, 1H), 7.11 (dd, J = 8.4 Hz, 6.0 Hz, 1H), 6.98 (td, J = 8.4 Hz, 2.4 Hz, 1H), 6.50 (d, J = 1.6 Hz, 1H), 5.36-5.41 (m, 1H), 4.83 (s, 2H), 4.24-4.31 (m, 1H), 4.08-4.14 (m, 1H), 3.90 (s, 3H), 3.84-3.89 (m, 1H), 3.77 (d, J = 15.6 Hz, 1H), 3.70-3.75 (m, 1H), 3.31 (s, 1H), 3.23 (s, 3H), 2.92 (d, J = 15.6 Hz, 1H), 1.77 (d, J = 6.4 Hz, 3H).

### Example 287: 2-((19R)-22-amino-5-ethynyl-16-fluoro-10,19-dimethyl-20-oxa-3,4,10,11, 23-pentaazapentacyclo[19.3.1.0^{8,12}.0^{13,18}.0^{2,6}]pentacosa-1(25),2(6),4,8(9),11,13(18),14,16,21(2 2),23-decaen-3-yl)ethan-1-ol

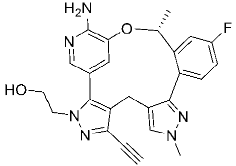

The synthetic method of Example 287 is the same as that of Example 1. LC-MS (ESI) [M+H]⁺: 459.4. ¹H NMR (400 MHz, CDCl₃), 7.52-7.64 (brs, 1H), 7.41 (s, 1H), 7.28 (dd, J = 10.0 Hz, 2.8 Hz, 1H), 7.11 (dd, J = 8.4 Hz, 5.6 Hz, 1H), 6.98 (td, J = 8.4 Hz, 2.8 Hz, 1H), 6.49 (s, 1H), 5.35-5.42 (m, 1H), 4.93 (s, 2H), 4.22-4.28 (m, 1H), 4.08-4.13 (m, 1H), 3.94-4.01 (m, 2H), 3.91 (s, 3H), 3.78 (d, J = 15.6 Hz, 1H), 3.31 (s, 1H), 2.92 (d, J = 15.6 Hz, 1H), 1.77 (d, J = 6.4 Hz, 3H).

### Example 288: (19R)-5-ethynyl-16-fluoro-10,19-dimethyl-3-(1,1,1-trifluoroethan-2-yl)-20 -oxa-3,4,10,11,23-pentaazapentacyclo[19.3.1.0^{8,12}.0^{13,18}.0^{2,6}]pentacosa-1(25),2(6),4,8(9),11,13 (18),14,16,21 (22),23-decaen-22-amine

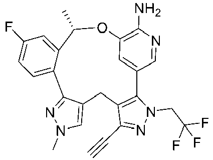

The synthetic method of Example 288 is the same as that of Example 1. LC-MS (ESI) [M+H]⁺: 497.5. ¹H NMR (400 MHz, CDCl₃), 7.49-7.58 (brs, 1H), 7.45 (s, 1H), 7.29 (dd, J = 10.0 Hz, 2.4 Hz, 1H), 7.12 (dd, J = 8.4 Hz, 6.0 Hz, 1H), 7.00 (td, J = 8.4 Hz, 2.4 Hz, 1H), 6.48 (s, 1H), 5.38-5.47 (m, 1H), 4.87 (s, 2H), 4.56-4.78 (m, 2H), 3.93 (s, 3H), 3.79 (d, J = 15.6 Hz, 1H), 3.35 (s, 1H), 2.95 (d, J = 15.6 Hz, 1H), 1.80 (d, J = 6.4 Hz, 3H).

### Example 289: (19R)-3-(difluoromethyl)-5-ethynyl-16-fluoro-10,19-dimethyl-20-oxa-3,4, 10,11,23-pentaazapentacyclo[19.3.1.0^{8,12}.0^{13,18}.0^{2,6}]pentacosa-1(25),2(6),4,8(9),11,13(18),14,16, 21(22),23-decaen-22-amine

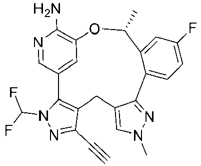

The synthetic method of Example 289 is the same as that of Example 1. LC-MS (ESI) [M+H]⁺: 465.4. ¹H NMR (400 MHz, CD₃OD), 7.58 (s, 1H), 7.55 (dd, J = 10.0 Hz, 2.8 Hz, 1H), 7.49 (s, 1H), 7.38 (t, J = 58.0 Hz, 1H), 7.21 (dd, J = 8.4 Hz, 5.6 Hz, 1H), 7.09 (td, J = 8.4 Hz, 2.8 Hz, 1H), 6.53 (d, J = 2.0 Hz, 1H), 5.41-5.47 (m, 1H), 4.03 (s, 1H), 3.92 (s, 3H), 3.81 (d, J = 15.6 HZ, 1H), 2.96 (d, J = 15.6 Hz, 1H), 1.82 (d, J = 6.4 Hz, 3H).

### Example 290: (19R)-3-(cyclopropylmethyl)-5-ethynyl-16-fluoro-19-methyl-10-(trideuter omethyl)-20-oxa-3,4,10,11,23-pentaazapentacyclo[19.3.1.0^{8,12}.0^{13,18}.0^{2,6}]pentacosa-1(25),2(6),4, 8(9),11,13(18),14,16,21(22),23-decaen-22-amine

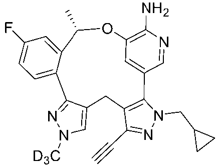

The synthetic method of Example 290 is the same as that of Example 1. LC-MS (ESI) [M+H]⁺: 472.5. ¹H NMR (400 MHz, CDCl₃), 7.61 (s, 1H), 7.45 (s, 1H), 7.26 (dd, J = 9.6 Hz, 2.8 Hz, 1H), 7.13 (dd, J = 8.4 Hz, 5.6 Hz, 1H), 7.00 (t, J = 8.4 Hz, 2.8 Hz, 1H), 6.54 (d, J = 1.2 Hz, 1H), 5.38-5.44 (m, 1H), 5.06-5.52 (brs, 2H), 4.07 (dd, J = 14.4 Hz, 6.4 Hz, 1H), 3.83 (dd, J = 14.4 Hz, 7.2 Hz, 1H), 3.79 (d, J = 15.6 Hz, 1H), 3.31 (s, 1H), 2.91 (d, J = 15.6 Hz, 1H), 1.79 (d, J = 6.4 Hz, 3H), 1.12-1.22 (m, 1H), 0.40-0.52 (m, 2H), 0.26-0.32 (m, 1H), 0.12-0.18 (m, 1H).

### Biological Assay

### 1. ALK Compound Mutant Kinase Inhibitory Activity Assay of the Compound (IC₅₀ Determination)

In this patent, homogeneous time-resolved fluorescence (HTRF) assay systems were used to establish ALK G1202R/L1196M and ALK I1171N/D1203N kinase activity detection platforms, respectively, for the determination of compound activity. ALK G1202R/L1196M reaction buffer: 50 mM HEPES, pH 7.5; 0.1 mM Na₃VO₄; 0.001% Tween-20; 5 mM MnCl₂; 1 mM DTT; 50 nM SEB (purchased from Cisbio); and 0.01% BSA. The final concentration of ATP was 4 µM, and the final concentration of the TK peptide substrate was 2 µM (purchased from Cisbio). ALK^{I1171N/D1203N} reaction buffer: 50 mM HEPES, pH 7.5; 0.1 mM Na₃VO₄; 0.001% Tween-20; 5 mM MnCl₂; 1 mM DTT; 10 nM SEB (purchased from Cisbio) and 0.01% BSA. The final concentration of ATP was 1 µM, and the final concentration of the TK peptide substrate was 1 µM (purchased from Cisbio).

First, the 10 mM compound was diluted 50-fold with DMSO, followed by 5-fold serial dilutions (8 concentrations in total, including 0 mM). For each diluted concentration, 2 µL of the compound solution was added to 48 µL of reaction buffer and mixed well. Then, 2.5 µL of the diluted compound was added to a 384-well plate (OptiPlate-384, purchased from PerkinElmer), followed by the addition of 2.5 µL of a mixture of ATP and TK peptide substrate. Subsequently, 5 µL of His-ALK^{G1202R/L1196M} (final concentration: 0.5 nM)/ His-ALK^{I1171N/D1203N} (final concentration: 0.3 nM) was added. The plate was centrifuged to initiate the reaction, with a total reaction volume of 10 µL. The 384-well plate was placed in an incubator at 23 °C for 1 hour. Then, 5 µL of TK Antibody (purchased from Cisbio) and 5 µL of Streptavidin-XL665 (purchased from Cisbio) were added to stop the reaction. After another 1-hour incubation in the incubator, fluorescence values were read on an Envision instrument (purchased from PerkinElmer) (excitation at 320 nm; emission detected at 665 nm and 620 nm; the ratio of the two represents the activity signal value of ALK^{G1202R/L1196M} and ALK^{I1171N/D1203N} kinases). The signal values at each concentration of the compound were measured, and the IC₅₀ value of the compound against His-ALK^{G1202R/L1196M} / His-ALK^{I1171N/D1203N} enzyme activity was calculated using GraphPad Prism software (Table 1). Data marked as A in the table indicate high activity: IC₅₀ ≤ 5 nM. N.D. means not determined.

**Table 1. Inhibitory Activity of Example Compounds Against ALK^{G1202R/L1196M}**

| Compound | ALK^{G1202R/L1196M} Enzyme, IC₅₀ (nM) | ALK^{I1171N/D1203N} Enzyme, IC₅₀ (nM) |
|---|---|---|
| Example 1 | A | N.D. |
| Example 2 | A | N.D. |
| Example 3 | A | N.D. |
| Example 5 | A | N.D. |
| Example 6 | A | N.D. |
| Example 7 | A | A |
| Example 8 | A | N.D. |
| Example 9 | A | N.D. |
| Example 10 | A | N.D. |
| Example 11 | A | N.D. |
| Example 13 | A | N.D. |
| Example 16 | A | N.D. |
| Example 17 | A | N.D. |
| Example 20 | A | N.D. |
| Example 22 | A | N.D. |
| Example 23 | A | N.D. |
| Example 24 | A | N.D. |
| Example 25 | A | N.D. |
| Example 27 | A | A |
| Example 28 | A | A |
| Example 29 | A | A |
| Example 36 | A | N.D. |
| Example 39 | A | N.D. |
| Example 40 | A | N.D. |
| Example 45 | A | N.D. |
| Example 46 | A | A |
| Example 47 | A | A |
| Example 48 | A | A |
| Example 49 | A | A |
| Example 50 | A | N.D. |
| Example 51 | A | A |
| Example 52 | A | A |
| Example 53 | A | N.D. |
| Example 54 | A | A |
| Example 56 | A | A |
| Example 57 | A | N.D. |
| Example 58 | A | A |
| Example 59 | A | N.D. |
| Example 60 | A | N.D. |
| Example 61 | A | N.D. |
| Example 62 | A | N.D. |
| Example 63 | A | A |
| Example 64 | A | N.D. |
| Example 65 | A | N.D. |
| Example 66 | A | N.D. |
| Example 68 | A | N.D. |
| Example 69 | A | A |
| Example 70 | A | A |
| Example 71 | A | A |
| Example 72 | A | A |
| Example 73 | A | N.D. |
| Example 74 | A | N.D. |
| Example 75 | A | N.D. |
| Example 76 | A | N.D. |
| Example 77 | A | N.D. |
| Example 78 | A | N.D. |
| Example 79 | A | A |
| Example 80 | A | A |
| Example 81 | A | N.D. |
| Example 82 | A | N.D. |
| Example 83 | A | N.D. |
| Example 84 | A | N.D. |
| Example 85 | A | N.D. |
| Example 86 | A | A |
| Example 88 | A | N.D. |
| Example 89 | A | A |
| Example 90 | A | N.D. |
| Example 90 | A | N.D. |
| Example 91 | A | N.D. |
| Example 92 | A | A |
| Example 93 | A | A |
| Example 94 | A | A |
| Example 95 | A | N.D. |
| Example 96 | A | N.D. |
| Example 41 | A | N.D. |
| Example 97 | A | A |
| Example 98 | A | A |
| Example 99 | A | A |
| Example 100 | A | A |
| Example 101 | A | N.D. |
| Example 102 | A | A |
| Example 103 | A | N.D. |
| Example 104 | A | A |
| Example 105 | A | A |
| Example 106 | A | N.D. |
| Example 107 | A | A |
| Example 108 | A | A |
| Example 109 | A | N.D. |
| Example 110 | A | A |
| Example 111 | A | A |
| Example 112 | A | N.D. |
| Example 113 | A | N.D. |
| Example 114 | A | A |
| Example 115 | A | N.D. |
| Example 116 | A | N.D. |
| Example 117 | A | A |
| Example 118 | A | A |
| Example 120 | A | N.D. |
| Example 122 | A | N.D. |
| Example 123 | A | N.D. |
| Example 124 | A | A |
| Example 125 | A | N.D. |
| Example 126 | A | A |
| Example 127 | A | N.D. |
| Example 128 | A | A |
| Example 129 | A | N.D. |
| Example 130 | A | A |
| Example 131 | A | N.D. |
| Example 132 | A | N.D. |
| Example 134 | A | A |
| Example 135 | A | A |
| Example 136 | A | N.D. |
| Example 137 | A | N.D. |
| Example 138 | A | N.D. |
| Example 139 | A | N.D. |
| Example 140 | A | N.D. |
| Example 141 | A | N.D. |
| Example 142 | A | A |
| Example 143 | A | A |
| Example 144 | A | A |
| Example 145 | A | N.D. |
| Example 146 | A | N.D. |
| Example 147 | A | A |
| Example 150 | A | N.D. |
| Example 151 | A | A |
| Example 152 | A | N.D. |
| Example 153 | A | A |
| Example 154 | A | N.D. |
| Example 155 | A | N.D. |
| Example 156 | A | N.D. |
| Example 157 | A | N.D. |
| Example 159 | A | N.D. |
| Example 160 | A | A |
| Example 161 | A | A |
| Example 162 | A | N.D. |
| Example 165 | A | N.D. |
| Example 166 | A | N.D. |
| Example 167 | A | N.D. |
| Example 168 | A | N.D. |
| Example 169 | A | A |
| Example 171 | A | N.D. |
| Example 172 | A | N.D. |
| Example 173 | A | A |
| Example 174 | A | A |
| Example 176 | A | N.D. |
| Example 177 | A | A |
| Example 178 | A | N.D. |
| Example 179 | A | A |
| Example 180 | A | A |
| Example 181 | A | A |
| Example 182 | A | N.D. |
| Example 183 | A | N.D. |
| Example 184 | A | N.D. |
| Example 185 | A | A |
| Example 186 | A | A |
| Example 187 | A | A |
| Example 188 | A | N.D. |
| Example 189 | A | A |
| Example 190 | A | N.D. |
| Example 191 | A | N.D. |
| Example 192 | A | A |
| Example 193 | A | A |
| Example 194 | A | N.D. |
| Example 196 | A | N.D. |
| Example 197 | A | N.D. |
| Example 198 | A | N.D. |
| Example 199 | A | A |
| Example 200 | A | A |
| Example 201 | A | N.D. |
| Example 202 | A | A |
| Example 203 | A | N.D. |
| Example 204 | A | N.D. |
| Example 205 | A | N.D. |
| Example 206 | A | N.D. |
| Example 207 | A | N.D. |
| Example 208 | A | N.D. |
| Example 209 | A | N.D. |
| Example 210 | A | N.D. |
| Example 211 | A | N.D. |
| Example 213 | A | N.D. |
| Example 214 | A | N.D. |
| Example 215 | A | N.D. |
| Example 216 | A | N.D. |
| Example 217 | A | N.D. |
| Example 218 | A | N.D. |
| Example 219 | A | N.D. |
| Example 220 | A | N.D. |
| Example 221 | A | N.D. |
| Example 222 | A | N.D. |
| Example 223 | A | N.D. |
| Example 224 | A | N.D. |
| Example 225 | A | N.D. |
| Example 226 | A | N.D. |
| Example 227 | A | N.D. |
| Example 228 | A | A |
| Example 229 | A | A |
| Example 230 | A | N.D. |
| Example 231 | A | N.D. |
| Example 232 | A | N.D. |
| Example 233 | A | N.D. |
| Example 234 | A | N.D. |
| Example 235 | A | N.D. |
| Example 236 | A | N.D. |
| Example 237 | A | N.D. |
| Example 238 | A | N.D. |
| Example 239 | A | N.D. |
| Example 240 | A | N.D. |
| Example 241 | A | N.D. |
| Example 242 | A | N.D. |
| Example 243 | A | N.D. |
| Example 244 | A | N.D. |
| Example 245 | A | N.D. |
| Example 246 | A | N.D. |
| Example 247 | A | N.D. |
| Example 248 | A | N.D. |
| Example 249 | A | N.D. |
| Example 250 | A | N.D. |
| Example 251 | A | N.D. |
| Example 252 | A | N.D. |
| Example 253 | A | N.D. |
| Example 254 | A | N.D. |
| Example 255 | A | N.D. |
| Example 256 | A | N.D. |
| Example 257 | A | N.D. |
| Example 258 | A | N.D. |
| Example 260 | A | N.D. |
| Example 267 | A | N.D. |
| Example 268 | A | N.D. |
| Example 272 | A | N.D. |
| Example 275 | A | A |
| Example 276 | A | N.D. |
| Example 277 | A | N.D. |
| Example 281 | A | N.D. |
| Example 282 | A | N.D. |
| Example 283 | A | N.D. |
| Example 284 | A | N.D. |
| Example 285 | A | N.D. |
| Example 286 | A | N.D. |
| Example 289 | A | N.D. |
| Example 290 | A | N.D. |

### 2. Cell Proliferation Inhibition Assay of Compounds (IC₅₀ Determination)

Baf3 EML4-ALK V1^{G1202R/L1196M} cells and Baf3 EML4-ALK V1^{I1171N/D1203N} cells were cultured in suspension using RPMI-1640 medium supplemented with 10% fetal bovine serum (FBS, purchased from Gibco, Brazil), 1% penicillin/streptomycin (P/S, purchased from Life Technology), and 2 µg/mL puromycin (purchased from Solarbio). The cells were cultured in a cell culture incubator at 37 °C with 5% CO₂. One day prior to compound testing, Baf3 EML4-ALK V1^{G1202R/L1196M} cells were seeded into 96-well plates (purchased from Corning) at a density of 1000 cells/195 µL/well, and Baf3 EML4-ALK V1^{I1171N/D1203N} cells were seeded into 96-well plates at a density of 2000 cells/195 µL/well. After 24 hours, the compound was serially diluted 3-fold in DMSO starting from 10 mM (11 concentrations in total). Then, 4 µL of each concentration was taken and further diluted into 96 µL of serum-free medium. Then, 5 µL of the diluted compound from each concentration was added to the cell culture plate, and the plate was incubated in the cell culture incubator for an additional 3 days. After 3 days, 35 µL of Cell-Titer Glo reagent (purchased from Promega) was added directly to the medium. The plate was incubated at room temperature for 5-10 minutes. Fluorescence signals were read on an Envision instrument, and the IC₅₀ value for inhibition of cell proliferation by the compound was calculated using GraphPad Prism software (Table 2). Data marked as A in the table indicate high activity: IC₅₀ ≤ 10 nM.

**Table 2. Inhibitory Activity of Example Compounds on BaF3 EML4-ALK^{G1202R/L1196M} Cell Proliferation**

| Compound | BaF3 EML4-ALK^{G1102R/L1196M}, IC₅₀ (nM) | BaF3 EML4-ALK^{I1171N/D1203N} IC₅₀ (nM) |
|---|---|---|
| Example 3 | A | N.D. |
| Example 5 | A | N.D. |
| Example 6 | A | N.D. |
| Example 7 | A | A |
| Example 27 | A | A |
| Example 28 | A | N.D. |
| Example 29 | A | N.D. |
| Example 47 | A | A |
| Example 48 | A | A |
| Example 49 | A | A |
| Example 50 | A | N.D. |
| Example 51 | A | N.D. |
| Example 52 | A | A |
| Example 54 | A | A |
| Example 56 | A | N.D. |
| Example 59 | A | A |
| Example 60 | A | N.D. |
| Example 61 | A | A |
| Example 62 | A | N.D. |
| Example 63 | A | N.D. A |
| Example 69 | A | A |
| Example 70 | A | A |
| Example 71 | A | N.D. |
| Example 72 | A | A |
| Example 75 | A | N.D. |
| Example 79 | A | A |
| Example 80 | A | A |
| Example 81 | A | N.D. |
| Example 86 | A | A |
| Example 89 | A | N.D. |
| Example 92 | A | A |
| Example 93 | A | A |
| Example 94 | A | A |
| Example 97 | A | A |
| Example 98 | A | A |
| Example 99 | A | A |
| Example 100 | A | A |
| Example 102 | A | A |
| Example 104 | A | A |
| Example 105 | A | A |
| Example 107 | A | A |
| Example 108 | A | A |
| Example 109 | A | N.D. |
| Example 110 | A | A |
| Example 111 | A | A |
| Example 114 | A | A |
| Example 117 | A | N.D. |
| Example 123 | A | N.D. |
| Example 124 | A | N.D. |
| Example 126 | A | A |
| Example 128 | A | N.D. |
| Example 130 | A | N.D. |
| Example 134 | A | A |
| Example 135 | A | A |
| Example 136 | A | N.D. |
| Example 142 | A | A |
| Example 143 | A | A |
| Example 144 | A | A |
| Example 147 | A | A |
| Example 151 | A | A |
| Example 153 | A | A |
| Example 154 | A | A |
| Example 156 | A | A |
| Example 160 | A | A |
| Example 166 | A | N.D. |
| Example 169 | A | N.D. |
| Example 173 | A | A |
| Example 177 | A | A |
| Example 179 | A | A |
| Example 180 | A | A |
| Example 181 | A | A |
| Example 183 | A | N.D. |
| Example 185 | A | A |
| Example 186 | A | A |
| Example 187 | A | A |
| Example 189 | A | A |
| Example 192 | A | A |
| Example 193 | A | A |
| Example 196 | A | N.D. |
| Example 199 | A | A |
| Example 200 | A | N.D. |
| Example 202 | A | N.D. |
| Example 205 | A | N.D. |
| Example 209 | A | N.D. |
| Example 210 | A | N.D. |
| Example 211 | A | N.D. |
| Example 220 | A | N.D. |
| Example 222 | A | N.D. |
| Example 226 | A | A |
| Example 227 | A | N.D. |
| Example 228 | A | A |
| Example 229 | A | A |
| Example 234 | A | N.D. |
| Example 243 | A | N.D. |
| Example 250 | A | N.D. |
| Example 251 | A | N.D. |
| Example 252 | A | N.D. |
| Example 254 | A | N.D. |
| Example 255 | A | N.D. |
| Example 39 | A | N.D. |
| Example 272 | A | N.D. |
| Example 275 | A | A |
| Example 276 | A | N.D. |
| Example 282 | A | N.D. |
| Example 283 | A | N.D. |
| Example 285 | A | N.D. |
| Example 290 | A | N.D. |

### 3. Pharmacokinetic Data

Male SD rats were obtained from Beijing Vital River Laboratory Animal Technology Co., Ltd. The rats were divided into groups of three, and each group received a single oral gavage dose of the test sample suspension (5 mg/kg). The animals were fasted overnight before the experiment, from 10 hours prior to dosing to 4 hours post-dose. Blood samples were collected at 0.25, 0.5, 1, 2, 4, 6, 8, and 24 hours after administration. After anesthesia with isoflurane using a small animal anesthesia machine, 0.3 mL of whole blood was collected from the ophthalmic venous plexus into heparinized anticoagulant tubes. The samples were centrifuged at 4 °C and 4000 rpm for 5 minutes, and the plasma was transferred to centrifuge tubes and stored at -80 °C until analysis. Plasma samples were extracted using protein precipitation, and the extract was analyzed by LC/MS/MS (Table 3).

**Table 3. Pharmacokinetic Data of Example Compounds**

| **Example** | **Dosage (mg/kg)** | **T_{1/2} (hr)** | **Tmax (hr)** | **Cₘₐₓ (ng/mL)** | **AUC_{0-inf} (hr*ng/mL)** |
|---|---|---|---|---|---|
| 27 | 5 | 3.00 | 0.50 | 1596 | 10721 |
| 59 | 5 | 2.98 | 0.33 | 3801 | 10063 |
| 63 | 5 | 2.89 | 3.00 | 2753 | 31917 |
| 186 | 5 | 3.81 | 1.00 | 2012 | 13245 |
| 226 | 5 | 10.0 | 2.83 | 1140 | 9130 |
| 228 | 5 | 2.75 | 0.33 | 3146 | 11143 |
| 229 | 5 | 4.16 | 3.67 | 1859 | 15504 |
| 275 | 5 | 3.81 | 0.833 | 1425 | 8619 |

### 4. In Vivo Efficacy of Example Compounds in an ALK Mutant Cell Line Xenograft Model

The compound was formulated as a solution for administration at 10 mL/kg, with a vehicle of 20% HP-β-CD. The experimental animals were obtained from Beijing Vital River Laboratory Animal Technology Co., Ltd. Female BALB/c-nude mice, 6 - 8 weeks old, were used. Ba/F3 EML4-ALK^{G1202R/L1196M} mutant cells were cultured in vitro in RPMI1640 medium supplemented with 10% fetal bovine serum, 100 U/mL penicillin, 100 µg/mL streptomycin, and 2 µg/mL puromycin, in an incubator at 37 °C with 5% CO₂. Cells were passaged twice a week. When the cells reached 80% - 90% confluence and the required number was achieved, the cells were harvested, counted, and inoculated.

Ba/F3 EML4-ALK^{G1202R/L1196M} mutant cells (1 × 10⁶ cells/0.1 mL) were subcutaneously inoculated into the back of each mouse. When the average tumor volume reached approximately 150 mm³, the mice were grouped and administered with the compound. Mice were weighed three times per week, and tumor diameters were measured with a vernier caliper three times per week (Table 4). Tumor volume was calculated using the formula: V = 0.5 × a × b², where a and b represent the long diameter and short diameter of the tumor, respectively. The antitumor efficacy of the compound was evaluated by the tumor growth inhibition rate (TGI%) or the relative tumor proliferation rate T/C (%). Tumor Growth Inhibition Rate TGI (%): TGI (%) = [1 - (mean tumor volume of the treatment group at the end of administration - mean tumor volume of the treatment group at the start of administration) / (mean tumor volume of the vehicle control group at the end of treatment - mean tumor volume of the vehicle control group at the start of treatment)] × 100%. Relative Tumor Proliferation Rate T/C (%) = T_{RTV}/C_{RTV} × 100% (T_{RTV}: mean RTV of the treatment group; C_{RTV}: mean RTV of the negative control group). The relative tumor volume (RTV) was calculated based on the tumor measurement results according to the formula: RTV = Vt/Vo, where Vo is the tumor volume measured at the time of grouping and dosing (i.e., day 0), and Vt is the tumor volume at a given measurement. T_{RTV} and C_{RTV} were taken from the same day. From the results of the Ba/F3 EML4-ALK^{G1202R/L1196M} mutant cell mouse xenograft model experiment, it can be seen that the compound of the present invention exhibits excellent antitumor efficacy and safety in the Ba/F3 EML4-ALK^{G1202R/L1196M} mutant cell tumor model.

**Table 4. In Vivo Antitumor Efficacy Evaluation of Example Compounds in the Ba/F3 EML4-ALK^{G1202R/L1196M} Model**

| Compound | TGI (%) | | | | Body Weight Change (% of vehicle) | | | |
|---|---|---|---|---|---|---|---|---|
| | Day 2 | Day 4 | Day 7 | Day 11 | Day 2 | Day 4 | Day 7 | Day 11 |
| Example 27 (PO, 1 mg/kg, BID) | 71.1 | 78.4 | 84.0 | 92.8 | 100.2 | 96.9 | 93.6 | 94.8 |
| Example 63 (PO, 1 mg/kg, BID) | 84.9 | 93.0 | 95.1 | 93.4 | 99.3 | 99.1 | 92.6 | 91.8 |
| Example 229 (PO, 1 mg/kg, BID) | 71.6 | 88.7 | 92.6 | 93.1 | 99.0 | 98.9 | 97.1 | 97.5 |

## Claims

1. A compound of formula (II) or a pharmaceutically acceptable salt, solvate, polymorph or isomer thereof: 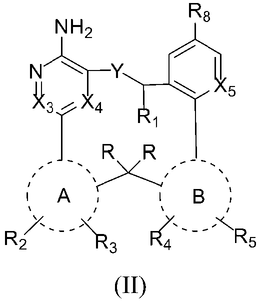 wherein,
each R is independently H, halogen, -CN, or C₁₋₄ alkyl, or two R are taken together to form a 3-4 membered carbocycle,
Y is -O- or -NR₁₀-,
R₁₀ is H or C₁₋₆ alkyl,
R₁ is H, C₁₋₆ alkyl, halo-C₁₋₆ alkyl, C₂₋₆ alkenyl, or 3-6 membered cycloalkyl, the alkyl, alkenyl and cycloalkyl are optionally substituted with halogen, -OH or -NH₂, or
R₁ and R₁₀ are optionally joined together to form a 4-8 membered heterocycle, the heterocycle is optionally substituted with halogen, -CN, -OH or -NH₂,
X₅ is N or CR₉,
R₈ and R₉ are each independently H, F, Cl, or ethynyl,
X₄ is N and CH,
X₃ is N and CH,
Ring A and Ring B are 5-6 membered aromatic rings,
R₂ and R₃ are each independently H, halogen, -CN, C₁₋₄ alkyl, -O-C₁₋₄ alkyl, halo-C₁₋₄ alkyl, 3-4 membered cycloalkylmethyl, 3-6 membered cycloalkyl, 3-6 membered heterocyclyl, 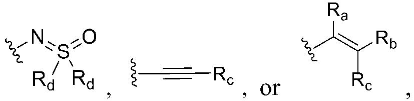 the alkyl, methyl, cycloalkyl and heterocyclyl are optionally substituted with halogen, -CN, -OH, -NH₂, C₂₋₆ alkenyl, or C₂₋₆ alkynyl,
R₄ and R₅ are each independently H, halogen, -CN, C₁₋₄ alkyl, -O-C₁₋₄ alkyl, halo-C₁₋₄ alkyl, 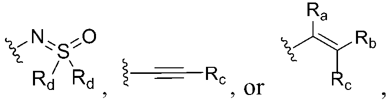 the alkyl is optionally substituted with halogen, -CN, - OH, -NH₂, C₂₋₆ alkenyl, or C₂₋₆ alkynyl,
X₃, R₂, R₃, R₄ and R₅ satisfy the following condition:
when X₃ is CH,
at least one of R₂, R₃, R₄ and R₅ is 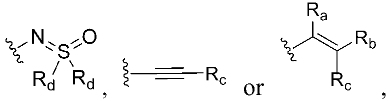 Rₐ is H, halogen, C₁₋₆ alkyl, or halo-C₁₋₆ alkyl,
R_{b} is H, halogen, C₁₋₆ alkyl, or halo-C₁₋₆ alkyl,
R_{c} is H, halogen, C₁₋₆ alkyl, 3-8 membered cycloalkyl, 3-8 membered heterocyclyl, 6-10 membered aryl, 5-12 membered heteroaryl, -(CO)-NH₂, -(CO)-NH-C₁₋₆ alkyl, -(CO)-O-C₁₋₆ alkyl, -(CO)-C₁₋₆ alkyl, the alkyl, cycloalkyl, heterocyclyl, aryl and heteroaryl are optionally substituted with halogen, -OH, -NH₂, -CN, -O-C₁₋₆ alkyl, -NH-C₁₋₆ alkyl, or -N(C₁₋₆ alkyl)(C₁₋₆ alkyl), or
R_{b} and R_{c} are optionally joined together to form a 4-8 membered carbocycle or 4-8 membered heterocycle, the carbocycle and heterocycle are optionally substituted with halogen, - OH, -NH₂, -CN, C₁₋₆ alkyl, C₂₋₆ alkenyl, C₂₋₆ alkynyl, -O-C₁₋₆ alkyl, -NH-C₁₋₆ alkyl, or -N(C₁₋₆ alkyl)(C₁₋₆ alkyl), the alkyl, alkenyl and alkynyl are optionally substituted with halogen, -OH, - NH₂, or -CN,
each R_{d} is independently C₁₋₆ alkyl.

2. The compound according to claim 1, or a pharmaceutically acceptable salt, solvate, polymorph, or isomer thereof, wherein Y is -O-,
R₁ is H, C₁₋₆ alkyl, halo-C₁₋₆ alkyl, C₂₋₆ alkenyl, or 3-6 membered cycloalkyl, the alkyl, alkenyl and cycloalkyl are optionally substituted with halogen, -OH or -NH₂,
R₈ and R₉ are each independently H or F.

3. The compound according to claim 2, or a pharmaceutically acceptable salt, solvate, polymorph, or isomer thereof, wherein R₂ and R₃ are each independently H, halogen, -CN, C₁₋₄ alkyl, -O-C₁₋₄ alkyl, halo-C₁₋₄ alkyl, 3-4 membered cycloalkylmethyl, 3-6 membered cycloalkyl, 3-6 membered heterocyclyl, 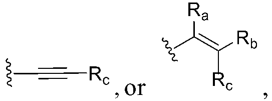 the alkyl, methyl, cycloalkyl and heterocyclyl are optionally substituted with halogen, -CN, -OH, -NH₂, C₂₋₆ alkenyl, or C₂₋₆ alkynyl,
R₄ and R₅ are each independently H, halogen, -CN, C₁₋₄ alkyl, -O-C₁₋₄ alkyl, halo-C₁₋₄ alkyl, 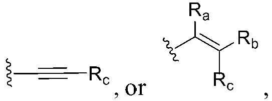 the alkyl is optionally substituted with halogen, -CN, -OH, -NH₂, C₂₋₆ alkenyl, or C₂₋₆ alkynyl,
X₃, R₂, R₃, R₄ and R₅ satisfy the following condition:
when X₃ is CH,
at least one of R₂, R₃, R₄ and R₅ is 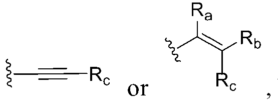 Rₐ, R_{b} and R_{c} are as defined in claim 1.

4. The compound according to claim 2, or a pharmaceutically acceptable salt, solvate, polymorph, or isomer thereof, wherein R is H.

5. The compound according to claim 2, or a pharmaceutically acceptable salt, solvate, polymorph, or isomer thereof, wherein R₈ is F.

6. The compound according to claim 2, or a pharmaceutically acceptable salt, solvate, polymorph, or isomer thereof, wherein R₁ is methyl or ethyl.

7. The following compound, or a pharmaceutically acceptable salt, solvate, polymorph, or isomer thereof:

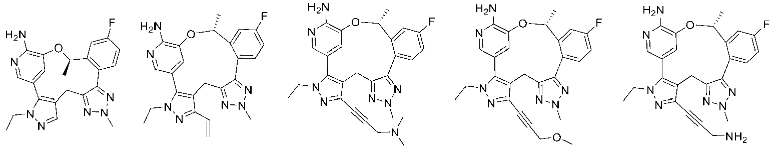

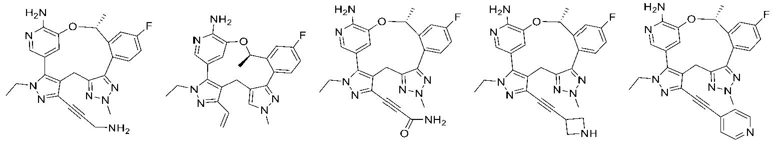

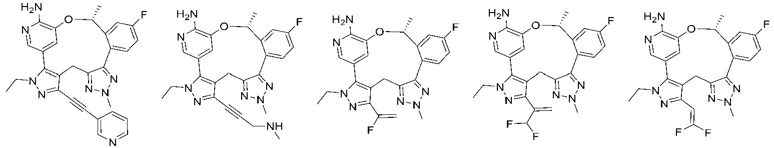

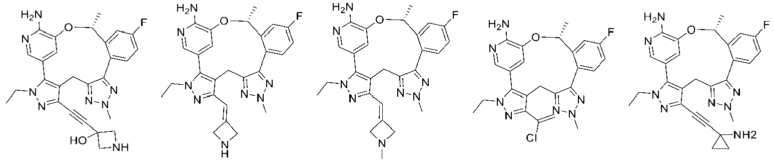

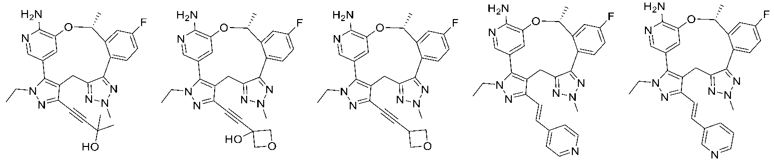

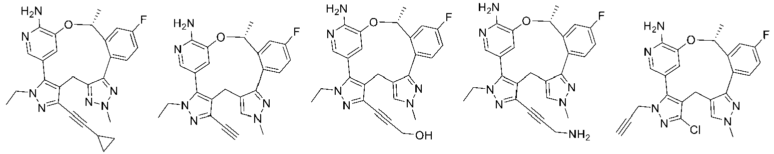

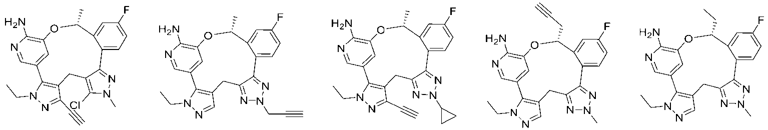

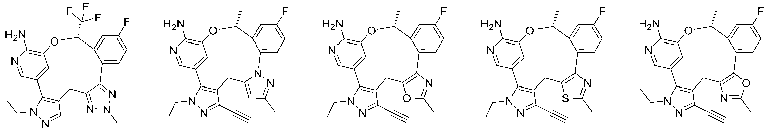

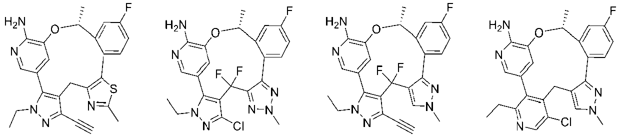

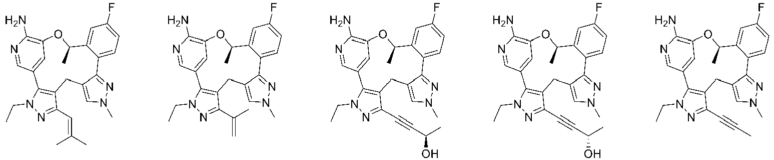

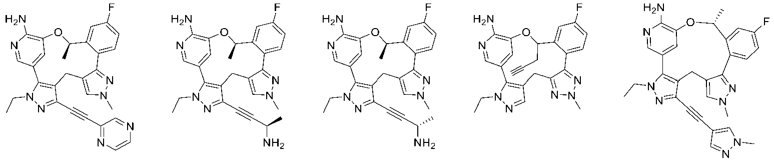

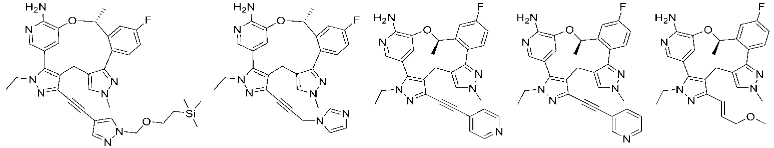

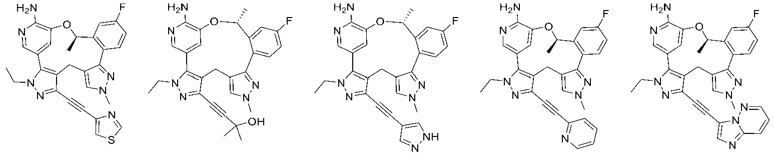

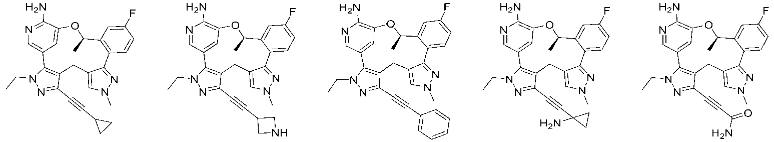

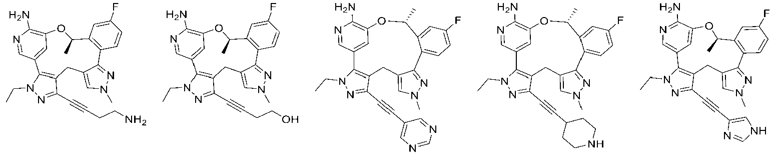

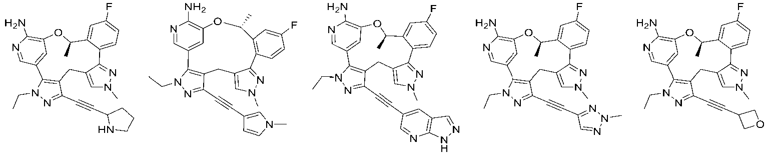

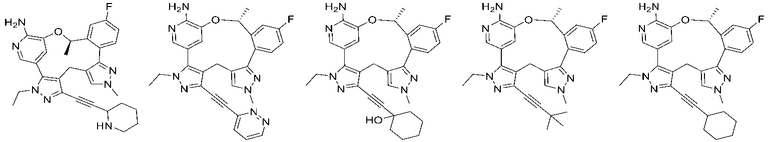

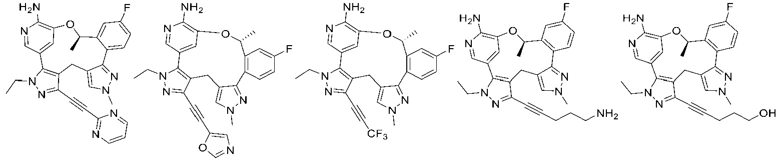

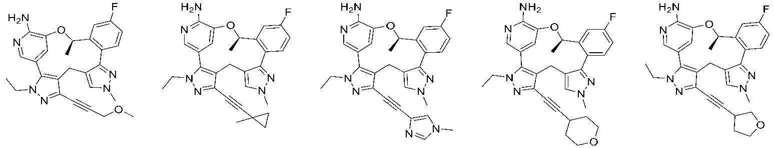

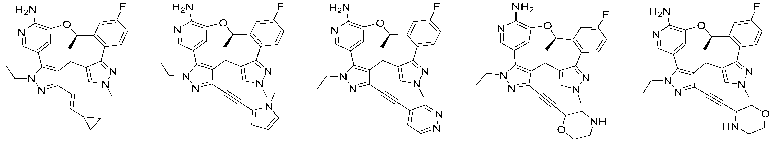

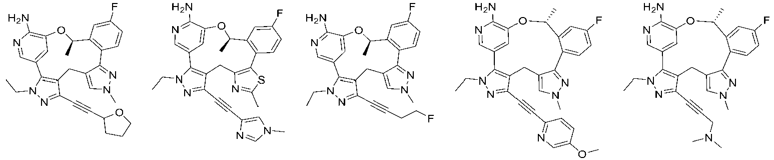

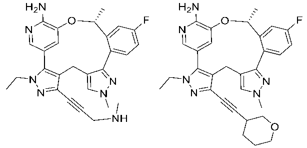

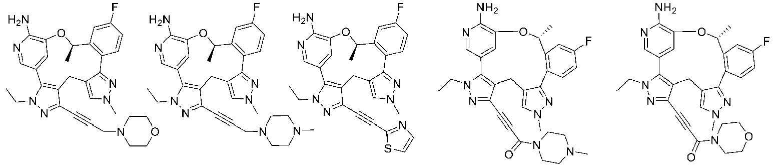

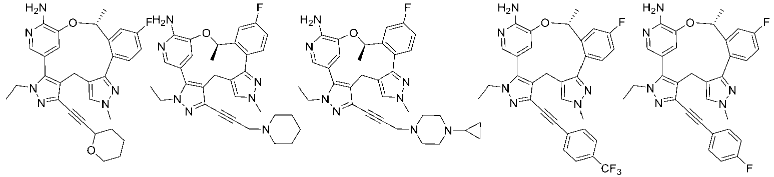

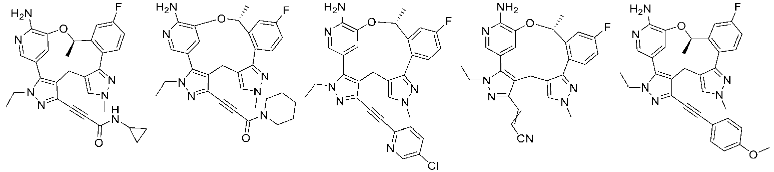

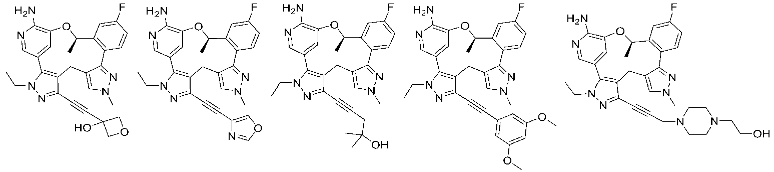

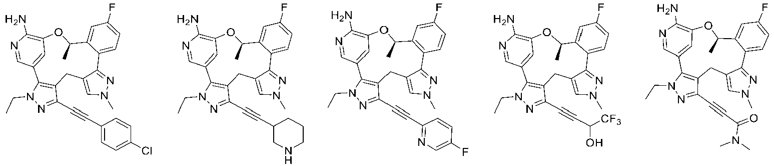

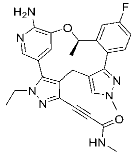

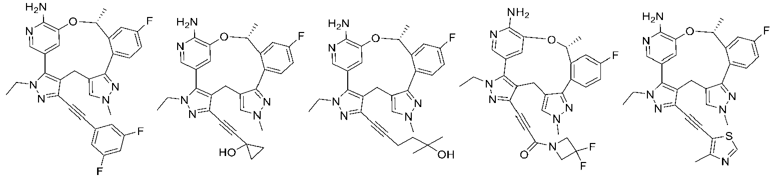

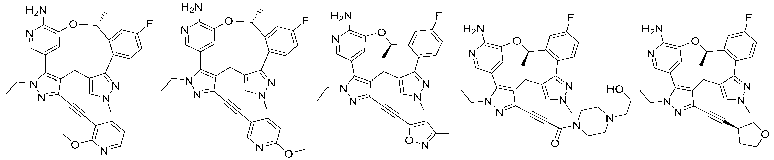

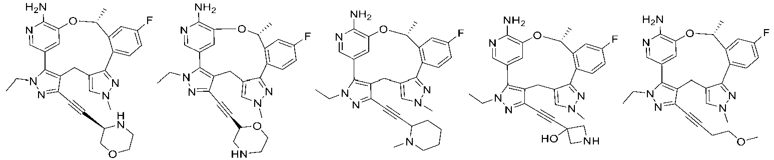

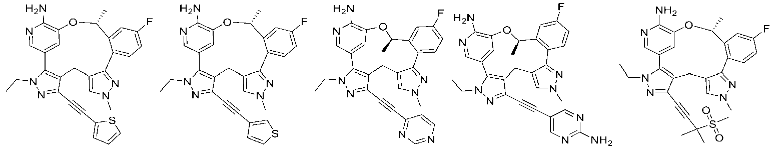

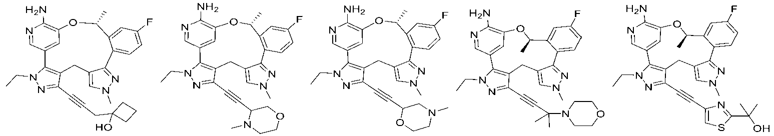

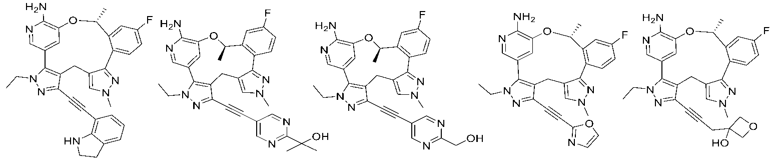

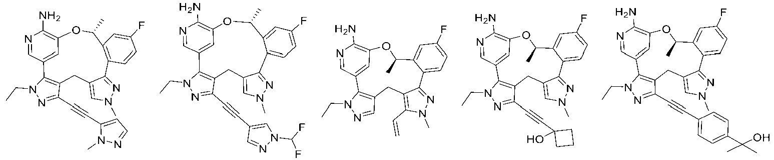

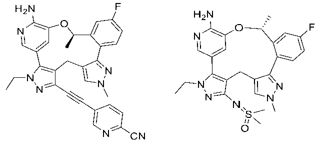

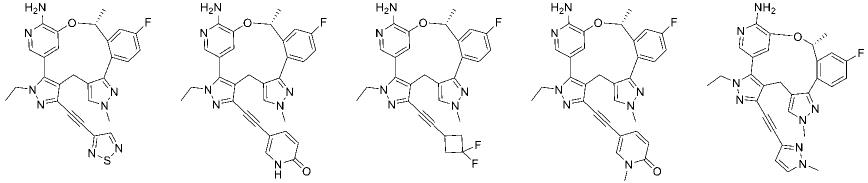

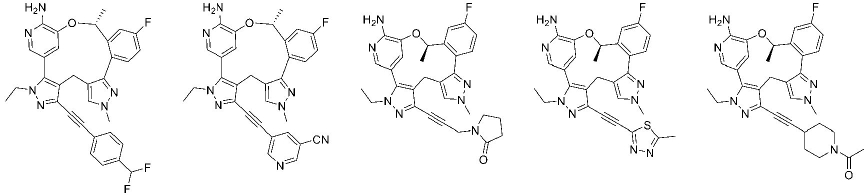

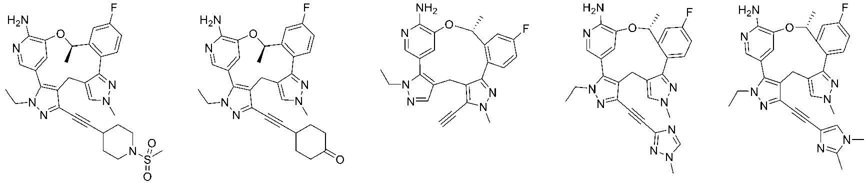

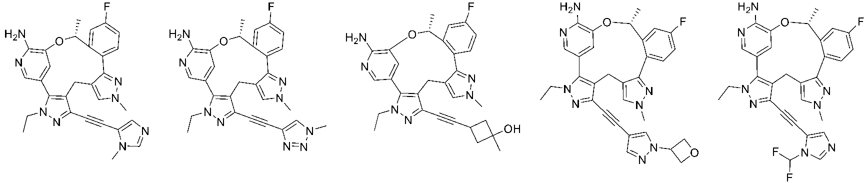

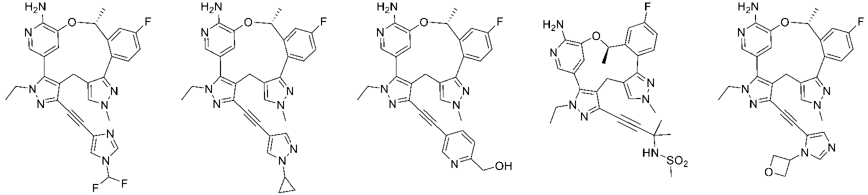

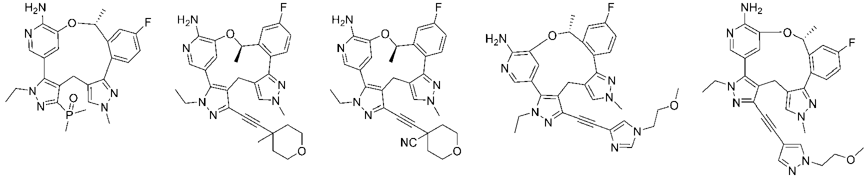

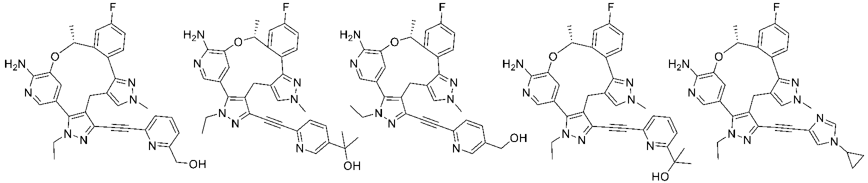

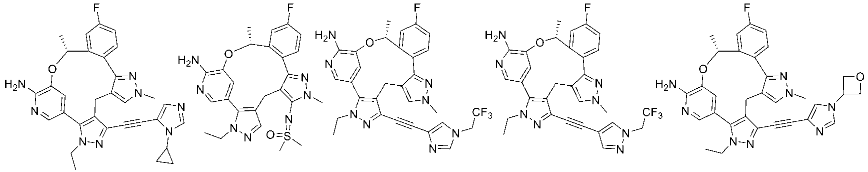

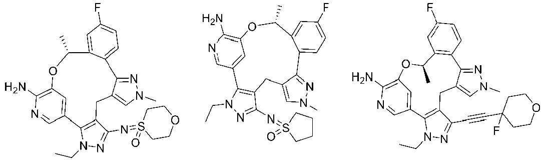

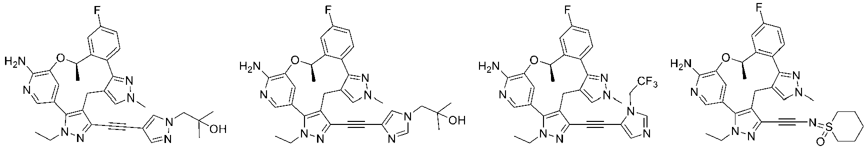

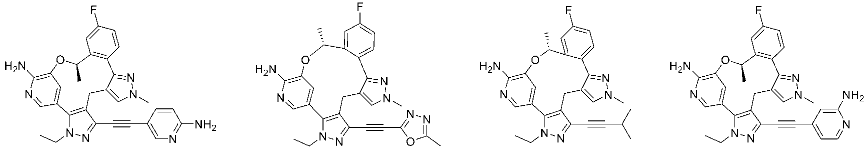

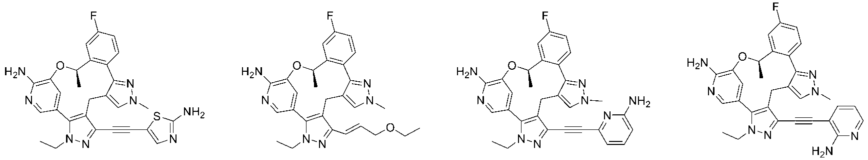

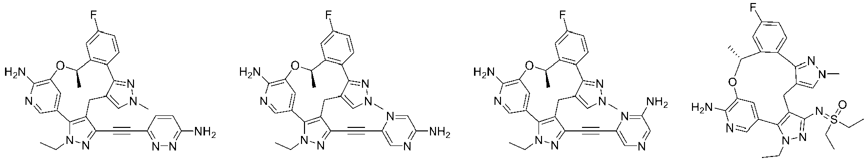

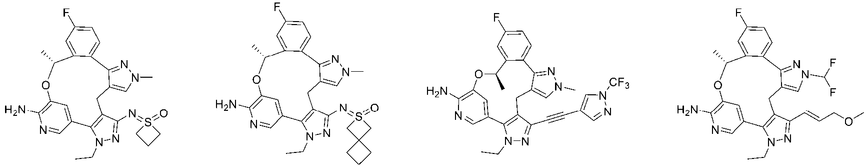

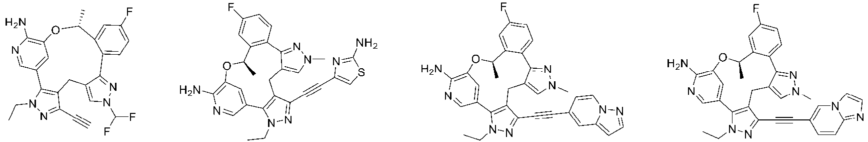

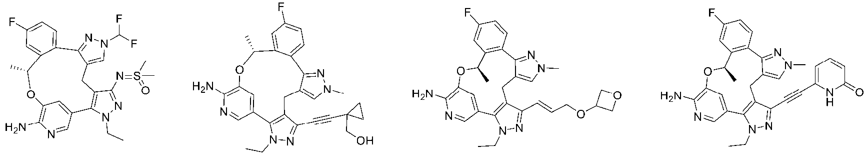

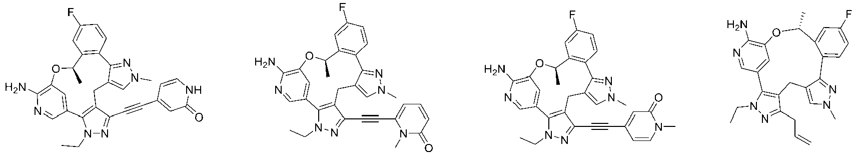

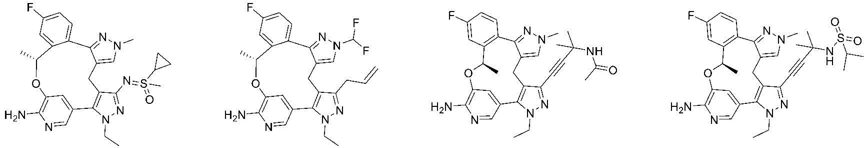

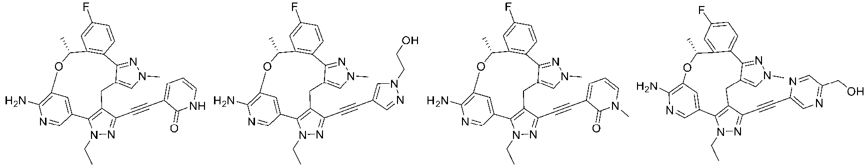

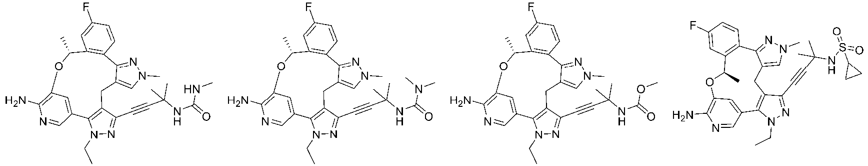

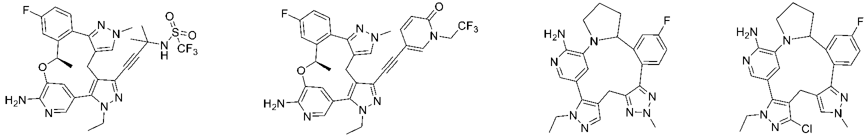

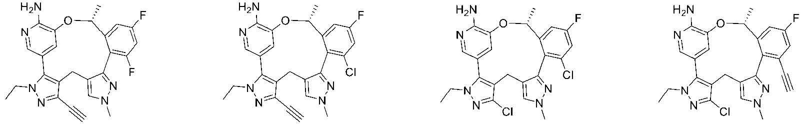

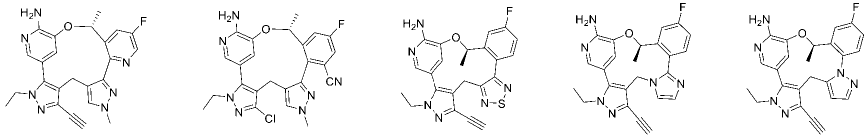

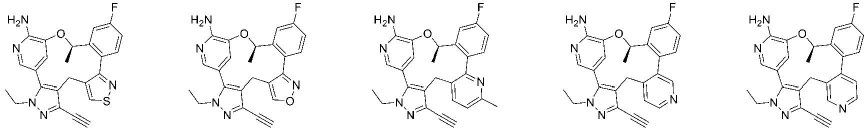

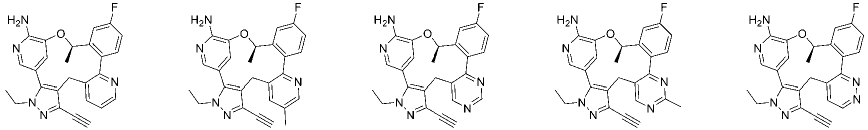

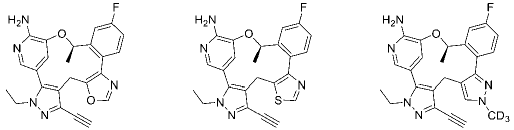

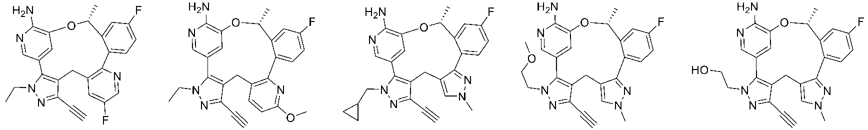

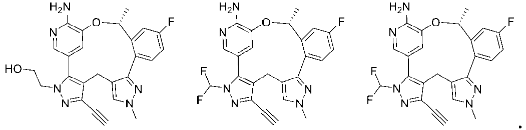

8. A pharmaceutical composition comprising the compound according to any one of claims 1 to 7, or a pharmaceutically acceptable salt, solvate, polymorph, or isomer thereof, and optionally a pharmaceutically acceptable carrier.

9. Use of the compound according to any one of claims 1 to 7, or a pharmaceutically acceptable salt, solvate, polymorph, or isomer thereof, or the composition according to claim 8 in the manufacture of a medicament for treating an ALK-mediated disease.

10. The use according to claim 9, wherein the ALK-mediated disease is ALK-positive non-small cell lung cancer, anaplastic large cell lymphoma, inflammatory myofibroblastic tumor, nasopharyngeal carcinoma, breast cancer, colorectal cancer, diffuse large B-cell lymphoma, systemic histiocytosis, or neuroblastoma.
